# EUROPEAN PATENT SPECIFICATION

(11) **EP 2 199 393 B1**
(45) Date of publication and mention of the grant of the patent: **31.10.2012**
(21) Application number: 10156326.0
(22) Date of filing: 17.04.2001
(51) Int. Cl.: C12N 15/64, C12N 15/86, C12N 15/10, C12N 15/66, C40B 40/02

(54) **Methods of constructing display libraries of genetic packages for members of a diverse family of peptides**
Verfahren zur Einrichtung von Bibliotheken für genetische Verpackungen unterschiedlicher Familien von Peptiden
Nouveaux procédés de construction de bibliothèques de paquets génétiques qui affichent collectivement une famille diverse de peptides, polypeptides ou protéines

(30) Priority: 17.04.2000 US 198069 P
(43) Date of publication of application: 23.06.2010
(62) Divisional of application: 01927108.9
(73) Proprietor: Dyax Corp., Cambridge, MA 02139 (US)
(72) Inventor: Ladner, Robert, C., Ijamsville, MD 21854 (US); Cohen, Edward, Hirsch, Pasadena, CA 91109-2855 (US); Nastri, Horacio, Gabriel, Newton, MA 02464 (US); Rookey, Kristin, L., Revere, MA 02151 (US); Hoet, Rene, NL-6226 CZ, Maastricht (NL)
(74) Representative: Vossius & Partner

(56) References cited:
- WO-A-97/20923
- WO-A-97/49809
- WO-A1-00/18905
- WO-A1-99/55367
- HOOGENBOOM H R ET AL: "MULTI-SUBUNIT PROTEINS ON THE SURFACE OF FILAMENTOUS PHAGE: METHODOLOGIES FOR DISPLAYING ANTIBODY (FAB) HEAVY AND LIGHT CHAINS" NUCLEIC ACIDS RESEARCH, OXFORD UNIVERSITY PRESS, SURREY, GB, vol. 19, no. 15, 1 January 1991 (1991-01-01), pages 4133-4137, XP001019229 ISSN: 0305-1048
- CLACKSON T ET AL: "IN VITRO SELECTION FROM PROTEIN AND PEPTIDE LIBRARIES" TRENDS IN BIOTECHNOLOGY, ELSEVIER PUBLICATIONS, CAMBRIDGE, GB LNKD- DOI:10.1016/0167-7799(94)90079-5, vol. 12, 1 May 1994 (1994-05-01), pages 173-184, XP000619299 ISSN: 0167-7799
- SMITH G P ET AL: "PHAGE DISPLAY" CHEMICAL REVIEWS, ACS,WASHINGTON, DC, US LNKD- DOI:10.1021/CR960065D, vol. 97, no. 2, 1 March 1997 (1997-03-01), pages 391-410, XP000683275 ISSN: 0009-2665
- FAN Z-C ET AL: "Three-dimensional structure of an Fv from a human IgM immunoglobulin" JOURNAL OF MOLECULAR BIOLOGY, LONDON, GB LNKD- DOI:10.1016/0022-2836(92)90500-J, vol. 228, no. 1, 5 November 1992 (1992-11-05), pages 188-207, XP024020018 ISSN: 0022-2836 [retrieved on 1992-11-05]
- BARBAS C F ET AL: "SEMISYNTHETIC COMBINATORIAL ANTIBODY LIBRARIES: A CHEMICAL SOLUTION TO THE DIVERSITY PROBLEM" PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.89.10.4457, vol. 89, 1 May 1992 (1992-05-01), pages 4457-4461, XP002024223 ISSN: 0027-8424
- BARBAS C F ET AL: "ASSEMBLY OF COMBINATORIAL ANTIBODY LIBRARIES ON PHAGE SURFACES: THE GENE III SITE" PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.88.18.7978, vol. 88, 1 September 1991 (1991-09-01), pages 7978-7982, XP000652426 ISSN: 0027-8424
- COURTNEY B C ET AL: "A phage display vector with improved stability, applicability and ease of manipulation" GENE, ELSEVIER, AMSTERDAM, NL LNKD- DOI:10.1016/0378-1119(95)00526-C, vol. 165, no. 1, 7 November 1995 (1995-11-07), pages 139-140, XP004043198 ISSN: 0378-1119
- HOET RENÉ MICHAEL ET AL: "Generation of high-affinity human antibodies by combining donor-derived and synthetic complementarity-determining-region diversity." NATURE BIOTECHNOLOGY MAR 2005 LNKD- PUBMED:15723048, vol. 23, no. 3, March 2005 (2005-03), pages 344-348, XP002581478 ISSN: 1087-0156
- SCHOONBROODT SONIA ET AL: "Oligonucleotide-assisted cleavage and ligation: a novel directional DNA cloning technology to capture cDNAs. Application in the construction of a human immune antibody phage-display library." NUCLEIC ACIDS RESEARCH 2005 LNKD- PUBMED:15905471, vol. 33, no. 9, 2005, page E81, XP002581479 ISSN: 1362-4962

### SEQUENCE LISTING

<110> DYAX CORPORATION
<120> NOVEL METHODS OF CONSTRUCTING LIBRARIES OF GENETIC PACKAGES THAT COLLECTIVELY DISPLAY THE MEMBERS OF A DIVERSE FAMILY OF PEPTIDES, POLYPEPTIDES OR PROTEINS
<130> F3042 EP/1 S3
<150> EP 01 92 7108.9
   <151> 2001-04-17
<150> 60/198,069 <151> 2000-04-17
<160> 428
<170> PatentIn Ver. 2.1
<210> 1
   <211> 17
   <212> DNA
   <213> Artificial Sequence
<220>
   <223> Description of Artificial Sequence: Synthetic oligonucleotide
<400> 1
   catgtgtatt actgtgc 17
<210> 2
   <211> 44
   <212> DNA
   <213> Artificial Sequence
<220>
   <223> Description of Artificial Sequence: Synthetic oligonucleotide
<400> 2
   cacatccgtg cttcttgcac ggatgtggca cagtaataca catg 44
<210> 3
   <211> 18
   <212> DNA
   <213> Artificial Sequence
<220>
   <223> Description of Artificial Sequence: Synthetic oligonucleotide
<400> 3
   gtgtattaga ctgctgcc 18
<210> 4
   <211> 43
   <212> DNA
   <213> Artificial Sequence
<220>
   <223> Description of Artificial Sequence: Synthetic oligonucleotide
<400> 4
   ggcagcagtc taatacacca catccgtgtt cttcacggat gtg 43
<210> 5
   <211> 47
   <212> DNA
   <213> Artificial Sequence
<220>
   <223> Description of Artificial Sequence: Synthetic oligonucleotide
<400> 5
   cacatccgtg tttgttacac ggatgtggtg tcttacagtc cattctg 47
<210> 6
   <211> 20
   <212> DNA
   <213> Artificial Sequence
<220>
   <223> Description of Artificial Sequence: Synthetic oligonucleotide
<400> 6
   cagaatggac tgtaagacac 20
<210> 7
   <211> 43
   <212> DNA
   <213> Artificial Sequence
<220>
   <223> Description of Artificial Sequence: Synthetic oligonucleotide
<400> 7
   atcgagtctc actgagccac atccgtggtt ttccacggat gtg 43
<210> 8
   <211> 17
   <212> DNA
   <213> Artificial Sequence
<220>
   <223> Description of Artificial Sequence: Synthetic oligonucleotide
<400> 8
   gctcagtgag actcgat 17
<210> 9
   <211> 19
   <212> DNA
   <213> Artificial Sequence
<220>
   <223> Description of Artificial Sequence: Synthetic oligonucleotide
<400> 9
   atgaccgaat tgctacaag 19
<210> 10
   <211> 46
   <212> DNA
   <213> Artificial Sequence
<220>
   <223> Description of Artificial Sequence: Synthetic oligonucleotide
<400> 10
   gactcctcag cttcttgctg aggagtcctt gtagcaattc ggtcat 46
<210> 11
   <211> 30
   <212> DNA
   <213> Artificial Sequence
<220>
   <223> Description of Artificial Sequence: Synthetic oligonucleotide
<400> 11
   acctcactgg cttccggatt cactttctct 30
<210> 12
   <211> 42
   <212> DNA
   <213> Artificial Sequence
<220>
   <223> Description of Artificial Sequence: Synthetic oligonucleotide
<400> 12
   agaaacccac tccaaacctt taccaggagc ttggcgaacc ca 42
<210> 13
   <211> 51
   <212> DNA
   <213> Artificial Sequence
<220>
   <223> Description of Artificial Sequence: Synthetic oligonucleotide
<400> 13
   ggaaggcagt gatctagaga tagtgaagcg acctttaacg gagtcagcat a 51
<210> 14
   <211> 23
   <212> DNA
   <213> Artificial Sequence
<220>
   <223> Description of Artificial Sequence: Synthetic oligonucleotide
<400> 14
   ggaaggcagt gatctagaga tag 23
<210> 15
   <211> 24
   <212> DNA
   <213> Artificial Sequence
<220>
   <223> Description of Artificial Sequence: Synthetic oligonucleotide
<220>
   <221> modified_base
   <222> (11) .. (24)
   <223> a, t, c, g, other or unknown
<400> 15
   cacggatgtg nnnnnnnnnn nnnn 24
<210> 16
   <211> 24
   <212> DNA
   <213> Artificial Sequence
<220>
   <223> Description of Artificial Sequence: Synthetic oligonucleotide
<220>
   <221> modified_base
   <222> (1) .. (14)
   <223> a, t, c, g, other or unknown
<400> 16
   nnnnnnnnnn nnnncacatc cgtg 24
<210> 17
   <211> 14
   <212> DNA
   <213> Artificial Sequence
<220>
   <223> Description of Artificial Sequence: Synthetic oligonucleotide
<400> 17
   gtgtattact gtgc 14
<210> 18
   <211> 34
   <212> DNA
   <213> Artificial Sequence
<220>
   <223> Description of Artificial Sequence: Synthetic oligonucleotide
<400> 18
   cacatccgtg cacggatgtg gcacagtaat acac 34
<210> 19
   <211> 14
   <212> DNA
   <213> Artificial Sequence
<220>
   <223> Description of Artificial Sequence: Synthetic oligonucleotide
<400> 19
   gtgtattaga ctgc 14
<210> 20
   <211> 34
   <212> DNA
   <213> Artificial Sequence
<220>
   <223> Description of Artificial Sequence: Synthetic oligonucleotide
<400> 20
   gcagtctaat acaccacatc cgtgcacgga tgtg 34
<210> 21
   <211> 34
   <212> DNA
   <213> Artificial Sequence
<220>
   <223> Description of Artificial Sequence: Synthetic oligonucleotide
<400> 21
   cacatccgtg cacggatgtg gtgtcttaca gtcc 34
<210> 22
   <211> 14
   <212> DNA
   <213> Artificial Sequence
<220>
   <223> Description of Artificial Sequence: Synthetic oligonucleotide
<400> 22
   ggactgtaag acac 14
<210> 23
   <211> 34
   <212> DNA
   <213> Artificial Sequence
<220>
   <223> Description of Artificial Sequence: Synthetic oligonucleotide
<400> 23
   gagtctcact gagccacatc cgtgcacgga tgtg 34
<210> 24
   <211> 14
   <212> DNA
   <213> Artificial Sequence
<220>
   <223> Description of Artificial Sequence: Synthetic oligonucleotide
<400> 24
   gctcagtgag actc 14
<210> 25
   <211> 14
   <212> DNA
   <213> Artificial Sequence
<220>
   <223> Description of Artificial Sequence: Synthetic oligonucleotide
<400> 25
   gtgtattact gtgc 14
<210> 26
   <211> 14
   <212> DNA
   <213> Artificial Sequence
<220>
   <223> Description of Artificial Sequence: Synthetic oligonucleotide
<400> 26
   gtatattact gtgc 14
<210> 27
   <211> 14
   <212> DNA
   <213> Artificial Sequence
<220>
   <223> Description of Artificial Sequence: Synthetic oligonucleotide
<400> 27
   gtgtattact gtaa 14
<210> 28
   <211> 14
   <212> DNA
   <213> Artificial Sequence
<220>
   <223> Description of Artificial Sequence: Synthetic oligonucleotide
<400> 28
   gtgtattact gtac 14
<210> 29
   <211> 14
   <212> DNA
   <213> Artificial Sequence
<220>
   <223> Description of Artificial Sequence: Synthetic oligonucleotide
<400> 29
   ttgtattact gtgc 14
<210> 30
   <211> 14
   <212> DNA
   <213> Artificial Sequence
<220>
   <223> Description of Artificial Sequence: Synthetic oligonucleotide
<400> 30
   ttgtatcact gtgc 14
<210> 31
   <211> 14
   <212> DNA
   <213> Artificial Sequence
<220>
   <223> Description of Artificial Sequence: Synthetic oligonucleotide
<400> 31
   acatattact gtgc 14
<210> 32
   <211> 14
   <212> DNA
   <213> Artificial Sequence
<220>
   <223> Description of Artificial Sequence: Synthetic oligonucleotide
<400> 32
   acgtattact gtgc 14
<210> 33
   <211> 14
   <212> DNA
   <213> Artificial Sequence
<220>
   <223> Description of Artificial Sequence: Synthetic oligonucleotide
<400> 33
   atgtattact gtgc 14
<210> 34
   <211> 24
   <212> DNA
   <213> Artificial Sequence
<220>
   <223> Description of Artificial Sequence: Primer
<400> 34
   tggaagaggc acgttctttt cttt 24
<210> 35
   <211> 24
   <212> DNA
   <213> Artificial Sequence
<220>
   <223> Description of Artificial Sequence: Primer
<400> 35
   aaagaaaaga acgtgcctct tcca 24
<210> 36
   <211> 24
   <212> DNA
   <213> Artificial Sequence
<220>
   <223> Description of Artificial Sequence: Primer
<400> 36
   acactctccc ctgttgaagc tctt 24
<210> 37
   <211> 23
   <212> DNA
   <213> Artificial Sequence
<220>
   <223> Description of Artificial Sequence: Primer
<400> 37
   tgaacattct gtaggggcca ctg 23
<210> 38
   <211> 23
   <212> DNA
   <213> Artificial Sequence
<220>
   <223> Description of Artificial Sequence: Primer
<400> 38
   agagcattct gcaggggcca ctg 23
<210> 39
   <211> 51
   <212> DNA
   <213> Artificial Sequence
<220>
   <223> Description of Artificial Sequence: Primer
<400> 39
   accgcctcca ccgggcgcgc cttattaaca ctctcccctg ttgaagctct t 51
<210> 40
   <211> 50
   <212> DNA
   <213> Artificial Sequence
<220>
   <223> Description of Artificial Sequence: Primer
<400> 40
   accgcctcca ccgggcgcgc cttattatga acattctgta ggggccactg 50
<210> 41
   <211> 50
   <212> DNA
   <213> Artificial Sequence
<220>
   <223> Description of Artificial Sequence: Primer
<400> 41
   accgcctcca ccgggcgcgc cttattaaga gcattctgca ggggccactg 50
<210> 42
   <211> 98
   <212> DNA
   <213> Homo sapiens
<400> 42
   agggtcacca tgaccaggga cacgtccatc agcacagcct acatggagct gagcaggctg 60
   agatctgacg acacggccgt gtattactgt gcgagaga                         98
<210> 43
   <211> 98
   <212> DNA
   <213> Homo sapiens
<400> 43
   agagtcacca ttaccaggga cacatccgcg agcacagcct acatggagct gagcagcctg 60
   agatctgaag acacggctgt gtattactgt gcgagaga                         98
<210> 44
   <211> 98
   <212> DNA
   <213> Homo sapiens
<400> 44
   agagtcacca tgaccaggaa cacctccata agcacagcct acatggagct gagcagcctg 60
   agatctgagg acacggccgt gtattactgt gcgagagg                         98
<210> 45
   <211> 98
   <212> DNA
   <213> Homo sapiens
<400> 45
   agagtcacca tgaccacaga cacatccacg agcacagcct acatggagct gaggagcctg 60
   agatctgacg acacggccgt gtattactgt gcgagaga                         98
<210> 46
   <211> 98
   <212> DNA
   <213> Homo sapiens
<400> 46
   agagtcacca tgaccgagga cacatctaca gacacagcct acatggagct gagcagcctg 60
   agatctgagg acacggccgt gtattactgt gcaacaga                         98
<210> 47
   <211> 98
   <212> DNA
   <213> Homo sapiens
<400> 47
   agagtcacca ttaccaggga caggtctatg agcacagcct acatggagct gagcagcctg 60
   agatctgagg acacagccat gtattactgt gcaagata 98
<210> 48
   <211> 98
   <212> DNA
   <213> Homo sapiens
<400> 48
   agagtcacca tgaccaggga cacgtccacg agcacagtct acatggagct gagcagcctg 60
   agatctgagg acacggccgt gtattactgt gcgagaga 98
<210> 49
   <211> 98
   <212> DNA
   <213> Homo sapiens
<400> 49
   agagtcacca ttaccaggga catgtccaca agcacagcct acatggagct gagcagcctg 60
   agatccgagg acacggccgt gtattactgt gcggcaga 98
<210> 50
   <211> 98
   <212> DNA
   <213> Homo sapiens
<400> 50
   agagtcacga ttaccgcgga cgaatccacg agcacagcct acatggagct gagcagcctg 60
   agatctgagg acacggccgt gtattactgt gcgagaga 98
<210> 51
   <211> 98
   <212> DNA
   <213> Homo sapiens
<400> 51
   agagtcacga ttaccgcgga caaatccacg agcacagcct acatggagct gagcagcctg 60
   agatctgagg acacggccgt gtattactgt gcgagaga 98
<210> 52
   <211> 98
   <212> DNA
   <213> Homo sapiens
<400> 52
   agagtcacca taaccgcgga cacgtctaca gacacagcct acatggagct gagcagcctg 60
   agatctgagg acacggccgt gtattactgt gcaacaga 98
<210> 53
   <211> 100
   <212> DNA
   <213> Homo sapiens
<400> 53
   aggctcacca tcaccaagga cacctccaaa aaccaggtgg tccttacaat gaccaacatg 60
   gaccctgtgg acacagccac atattactgt gcacacagac 100
<210> 54
   <211> 100
   <212> DNA
   <213> Homo sapiens
<400> 54
   aggctcacca tctccaagga cacctccaaa agccaggtgg tccttaccat gaccaacatg 60
   gaccctgtgg acacagccac atattactgt gcacggatac 100
<210> 55
   <211> 100
   <212> DNA
   <213> Homo sapiens
<400> 55
   aggctcacca tctccaagga cacctccaaa aaccaggtgg tccttacaat gaccaacatg 60
   gaccctgtgg acacagccac gtattactgt gcacggatac 100
<210> 56
   <211> 98
   <212> DNA
   <213> Homo sapiens
<400> 56
   cgattcacca tctccagaga caacgccaag aactcactgt atctgcaaat gaacagcctg 60
   agagccgagg acacggctgt gtattactgt gcgagaga 98
<210> 57
   <211> 100
   <212> DNA
   <213> Homo sapiens
<400> 57
   cgattcacca tctccagaga caacgccaag aactccctgt atctgcaaat gaacagtctg 60
   agagctgagg acacggcctt gtattactgt gcaaaagata 100
<210> 58
   <211> 98
   <212> DNA
   <213> Homo sapiens
<400> 58
   cgattcacca tctccaggga caacgccaag aactcactgt atctgcaaat gaacagcctg 60
   agagccgagg acacggccgt gtattactgt gcgagaga                         98
<210> 59
   <211> 98
   <212> DNA
   <213> Homo sapiens

<400> 59
   cgattcacca tctccagaga aaatgccaag aactccttgt atcttcaaat gaacagcctg 60
   agagccgggg acacggctgt gtattactgt gcaagaga                         98
<210> 60
   <211> 98
   <212> DNA
   <213> Homo sapiens
<400> 60
   agattcacca tctcaagaga tgattcaaaa aacacgctgt atctgcaaat gaacagcctg 60
   aaaaccgagg acacagccgt gtattactgt accacaga                         98
<210> 61
   <211> 98
   <212> DNA
   <213> Homo sapiens
<400> 61
   cgattcacca tctccagaga caacgccaag aactccctgt atctgcaaat gaacagtctg 60
   agagccgagg acacggcctt gtatcactgt gcgagaga                         98
<210> 62
   <211> 98
   <212> DNA
   <213> Homo sapiens
<400> 62
   cgattcacca tctccagaga caacgccaag aactcactgt atctgcaaat gaacagcctg 60
   agagccgagg acacggctgt gtattactgt gcgagaga                         98
<210> 63
   <211> 98
   <212> DNA
   <213> Homo sapiens
<400> 63
   cggttcacca tctccagaga caattccaag aacacgctgt atctgcaaat gaacagcctg 60
   agagccgagg acacggccgt atattactgt gcgaaaga                         98
<210> 64
   <211> 98
   <212> DNA
   <213> Homo sapiens
<400> 64
   cgattcacca tctccagaga caattccaag aacacgctgt atctgcaaat gaacagcctg 60
   agagctgagg acacggctgt gtattactgt gcgaaaga                         98
<210> 65
   <211> 98
   <212> DNA
   <213> Homo sapiens
<400> 65
   cgattcacca tctccagaga caattccaag aacacgctgt atctgcaaat gaacagcctg 60
   agagctgagg acacggctgt gtattactgt gcgagaga                         98
<210> 66
   <211> 98
   <212> DNA
   <213> Homo sapiens
<400> 66
   cgattcacca tctccagaga caattccaag aacacgctgt atctgcaaat gaacagcctg 60
   agagctgagg acacggctgt gtattactgt gcgaaaga                         98
<210> 67
   <211> 98
   <212> DNA
   <213> Homo sapiens
<400> 67
   cgattcacca tctccagaga caattccaag aacacgctgt atctgcaaat gaacagcctg 60
   agagccgagg acacggctgt gtattactgt gcgagaga                         98
<210> 68
   <211> 100
   <212> DNA
   <213> Homo sapiens
<400> 68
   cgattcacca tctccagaga caacagcaaa aactccctgt atctgcaaat gaacagtctg 60
   agaactgagg acaccgcctt gtattactgt gcaaaagata                       100
<210> 69
   <211> 98
   <212> DNA
   <213> Homo sapiens
<400> 69
   cgattcacca tctccagaga caatgccaag aactcactgt atctgcaaat gaacagcctg 60
   agagacgagg acacggctgt gtattactgt gcgagaga                         98
<210> 70
   <211> 98
   <212> DNA
   <213> Homo sapiens
<400> 70
   agattcacca tctcaagaga tggttccaaa agcatcgcct atctgcaaat gaacagcctg 60
   aaaaccgagg acacagccgt gtattactgt actagaga                         98
<210> 71
   <211> 98
   <212> DNA
   <213> Homo sapiens
<400> 71
   cgattcacca tctccagaga caattccaag aacacgctgt atcttcaaat gaacagcctg 60
   agagccgagg acacggccgt gtattactgt gcgagaga                         98
<210> 72
   <211> 98
   <212> DNA
   <213> Homo sapiens
<400> 72
   agattcacca tctccagaga caattccaag aacacgctgt atcttcaaat gggcagcctg 60
   agagctgagg acatggctgt gtattactgt gcgagaga                         98
<210> 73
   <211> 98
   <212> DNA
   <213> Homo sapiens
<400> 73
   agattcacca tctccagaga caattccaag aacacgctgt atcttcaaat gaacagcctg 60
   agagctgagg acacggctgt gtattactgt gcgagaga 98
<210> 74
   <211> 98
   <212> DNA
   <213> Homo sapiens
<400> 74
   agattcacca tctcaagaga tgattcaaag aactcactgt atctgcaaat gaacagcctg 60
   aaaaccgagg acacggccgt gtattactgt gctagaga 98
<210> 75
   <211> 98
   <212> DNA
   <213> Homo sapiens
<400> 75
   aggttcacca tctccagaga tgattcaaag aacacggcgt atctgcaaat gaacagcctg 60
   aaaaccgagg acacggccgt gtattactgt actagaca 98
<210> 76
   <211> 98
   <212> DNA
   <213> Homo sapiens
<400> 76
   cgattcacca tctccagaga caacgccaag aacacgctgt atctgcaaat gaacagtctg 60
   agagccgagg acacggctgt gtattactgt gcaagaga 98
<210> 77
   <211> 98
   <212> DNA
   <213> Homo sapiens
<400> 77
   agattcacca tctccagaga caattccaag aacacgctgc atcttcaaat gaacagcctg 60
   agagctgagg acacggctgt gtattactgt aagaaaga 98
<210> 78
   <211> 98
   <212> DNA
   <213> Homo sapiens
<400> 78
   cgagtcacca tatcagtaga caagtccaag aaccagttct ccctgaagct gagctctgtg 60
   accgccgcgg acacggccgt gtattactgt gcgagaga 98
<210> 79
   <211> 98
   <212> DNA
   <213> Homo sapiens
<400> 79
   cgagtcacca tgtcagtaga cacgtccaag aaccagttct ccctgaagct gagctctgtg 60
   accgccgtgg acacggccgt gtattactgt gcgagaaa                        98
<210> 80
   <211> 98
   <212> DNA
   <213> Homo sapiens
<400> 80
   cgagttacca tatcagtaga cacgtctaag aaccagttct ccctgaagct gagctctgtg 60
   actgccgcgg acacggccgt gtattactgt gcgagaga                        98
<210> 81
   <211> 98
   <212> DNA
   <213> Homo sapiens
<400> 81
   cgagtcacca tatcagtaga caggtccaag aaccagttct ccctgaagct gagctctgtg 60
   accgccgcgg acacggccgt gtattactgt gccagaga                        98
<210> 82
   <211> 98
   <212> DNA
   <213> Homo sapiens
<400> 82
   cgagttacca tatcagtaga cacgtccaag aaccagttct ccctgaagct gagctctgtg 60
   actgccgcag acacggccgt gtattactgt gccagaga                        98
<210> 83
   <211> 98
   <212> DNA
   <213> Homo sapiens
<400> 83
   cgagttacca tatcagtaga cacgtctaag aaccagttct ccctgaagct gagctctgtg 60
   actgccgcgg acacggccgt gtattactgt gcgagaga                        98
<210> 84
   <211> 98
   <212> DNA
   <213> Homo sapiens
<400> 84
   cgagtcacca tatcagtaga cacgtccaag aaccagttct ccctgaagct gagctctgtg 60
   accgccgcgg acacggctgt gtattactgt gcgagaga 98
<210> 85
   <211> 98
   <212> DNA
   <213> Homo sapiens
<400> 85
   cgagtcacca tatccgtaga cacgtccaag aaccagttct ccctgaagct gagctctgtg 60
   accgccgcag acacggctgt gtattactgt gcgagaca 98
<210> 86
   <211> 98
   <212> DNA
   <213> Homo sapiens
<400> 86
   cgagtcacca tatcagtaga cacgtccaag aaccagttct ccctgaagct gagctctgtg 60
   accgctgcgg acacggccgt gtattactgt gcgagaga 98
<210> 87
   <211> 98
   <212> DNA
   <213> Homo sapiens
<400> 87
   cgagtcacca tatcagtaga cacgtccaag aaccagttct ccctgaagct gagctctgtg 60
   accgctgcgg acacggccgt gtattactgt gcgagaga 98
<210> 88
   <211> 98
   <212> DNA
   <213> Homo sapiens
<400> 88
   cgagtcacca tatcagtaga cacgtccaag aaccagttct ccctgaagct gagctctgtg 60
   accgccgcag acacggccgt gtattactgt gcgagaga 98
<210> 89
   <211> 98
   <212> DNA
   <213> Homo sapiens
<400> 89
   caggtcacca tctcagccga caagtccatc agcaccgcct acctgcagtg gagcagcctg 60
   aaggcctcgg acaccgccat gtattactgt gcgagaca 98
<210> 90
   <211> 96
   <212> DNA
   <213> Homo sapiens
<400> 90
   cacgtcacca tctcagctga caagtccatc agcactgcct acctgcagtg gagcagcctg 60
   aaggcctcgg acaccgccat gtattactgt gcgaga 96
<210> 91
   <211> 98
   <212> DNA
   <213> Homo sapiens
<400> 91
   cgaataacca tcaacccaga cacatccaag aaccagttct ccctgcagct gaactctgtg 60
   actcccgagg acacggctgt gtattactgt gcaagaga 98
<210> 92
   <211> 98
   <212> DNA
   <213> Homo sapiens
<400> 92
   cggtttgtct tctccttgga cacctctgtc agcacggcat atctgcagat ctgcagccta 60
   aaggctgagg acactgccgt gtattactgt gcgagaga 98
<210> 93
   <211> 22
   <212> DNA
   <213> Artificial Sequence
<220>
   <223> Description of Artificial Sequence: Probe
<400> 93
   agttctccct gcagctgaac tc 22
<210> 94
   <211> 22
   <212> DNA
   <213> Artificial Sequence
<220>
   <223> Description of Artificial Sequence: Probe
<400> 94
   cactgtatct gcaaatgaac ag 22
<210> 95
   <211> 22
   <212> DNA
   <213> Artificial Sequence
<220>
   <223> Description of Artificial Sequence: Probe
<400> 95
   ccctgtatct gcaaatgaac ag 22
<210> 96
   <211> 22
   <212> DNA
   <213> Artificial Sequence
<220>
   <223> Description of Artificial Sequence: Probe
<400> 96
   ccgcctacct gcagtggagc ag 22
<210> 97
   <211> 22
   <212> DNA
   <213> Artificial Sequence
<220>
   <223> Description of Artificial Sequence: Probe
<400> 97
   cgctgtatct gcaaatgaac ag 22
<210> 98
   <211> 22
   <212> DNA
   <213> Artificial Sequence
<220>
   <223> Description of Artificial Sequence: Probe
<400> 98
   cggcatatct gcagatctgc ag 22
<210> 99
   <211> 22
   <212> DNA
   <213> Artificial Sequence
<220>
   <223> Description of Artificial Sequence: Probe
<400> 99
   cggcgtatct gcaaatgaac ag 22
<210> 100
   <211> 22
   <212> DNA
   <213> Artificial Sequence
<220>
   <223> Description of Artificial Sequence: Probe
<400> 100
   ctgcctacct gcagtggagc ag 22
<210> 101
   <211> 22
   <212> DNA
   <213> Artificial Sequence
<220>
   <223> Description of Artificial Sequence: Probe
<400> 101
   tcgcctatct gcaaatgaac ag 22
<210> 102
   <211> 22
   <212> DNA
   <213> Artificial Sequence
<220>
   <223> Description of Artificial Sequence: Synthetic oligonucleotide
<400> 102
   agttctccct gcagctgaac tc 22
<210> 103
   <211> 20
   <212> DNA
   <213> Artificial Sequence
<220>
   <223> Description of Artificial Sequence: Synthetic oligonucleotide
<400> 103
   ttctccctgc agctgaactc 20
<210> 104
   <211> 18
   <212> DNA
   <213> Artificial Sequence
<220>
   <223> Description of Artificial Sequence: Synthetic oligonucleotide
<400> 104
   ttctccctgc agctgaac 18
<210> 105
   <211> 22
   <212> DNA
   <213> Artificial Sequence
<220>
   <223> Description of Artificial Sequence: Synthetic oligonucleotide
<400> 105
   cgctgtatct gcaaatgaac ag 22
<210> 106
   <211> 20
   <212> DNA
   <213> Artificial Sequence
<220>
   <223> Description of Artificial Sequence: Synthetic oligonucleotide
<400> 106
   ctgtatctgc aaatgaacag 20
<210> 107
   <211> 18
   <212> DNA
   <213> Artificial Sequence
<220>
   <223> Description of Artificial Sequence: Synthetic oligonucleotide
<400> 107
   ctgtatctgc aaatgaac 18
<210> 108
   <211> 22
   <212> DNA
   <213> Artificial Sequence
<220>
   <223> Description of Artificial Sequence: Synthetic oligonucleotide
<400> 108
   cactgtatct gcaaatgaac ag 22
<210> 109
   <211> 22
   <212> DNA
   <213> Artificial Sequence
<220>
   <223> Description of Artificial Sequence: Synthetic oligonucleotide
<400> 109
   ccgcctacct gcagtggagc ag 22
<210> 110
   <211> 42
   <212> DNA
   <213> Artificial Sequence
<220>
   <223> Description of Artificial Sequence: Synthetic oligonucleotide
<400> 110
   cgcttcacta agtctagaga caactctaag aatactctct ac 42
<210> 111
   <211> 21
   <212> DNA
   <213> Artificial Sequence
<220>
   <223> Description of Artificial Sequence: Synthetic oligonucleotide
<400> 111
   ttgcagatga acagcttaag g 21
<210> 112
   <211> 45
   <212> DNA
   <213> Artificial Sequence
<220>
   <223> Description of Artificial Sequence: Synthetic oligonucleotide
<400> 112
   caagtagaga gtattcttag agttgtctct agacttagtg aagcg 45
<210> 113
   <211> 54
   <212> DNA
   <213> Artificial Sequence
<220>
   <223> Description of Artificial Sequence: Synthetic oligonucleotide
<400> 113
   cgcttcacta agtctagaga caactctaag aatactctct acttgcagct gaac 54
<210> 114
   <211> 54
   <212> DNA
   <213> Artificial Sequence
<220>
   <223> Description of Artificial Sequence: Synthetic oligonucleotide
<400> 114
   cgcttcacta agtctagaga caactctaag aatactctct acttgcaaat gaac 54

<210> 115
   <211> 54
   <212> DNA
   <213> Artificial Sequence
<220>
   <223> Description of Artificial Sequence: Synthetic oligonucleotide
<400> 115
   cgcttcacta agtctagaga caactctaag aatactctct acttgcagtg gagc 54
<210> 116
   <211> 21
   <212> DNA
   <213> Artificial Sequence
<220>
   <223> Description of Artificial Sequence: Synthetic oligonucleotide
<400> 116
   cgcttcacta agtctagaga c 21
<210> 117
   <211> 22
   <212> DNA
   <213> Artificial Sequence
<220>
   <223> Description of Artificial Sequence: Probe
<400> 117
   acatggagct gagcagcctg ag 22
<210> 118
   <211> 22
   <212> DNA
   <213> Artificial Sequence
<220>
   <223> Description of Artificial Sequence: Probe
<400> 118
   acatggagct gagcaggctg ag 22
<210> 119
   <211> 22
   <212> DNA
   <213> Artificial Sequence
<220>
   <223> Description of Artificial Sequence: Probe
<400> 119
   acatggagct gaggagcctg ag 22
<210> 120
   <211> 22
   <212> DNA
   <213> Artificial Sequence
<220>
   <223> Description of Artificial Sequence: Probe
<400> 120
   acctgcagtg gagcagcctg aa 22
<210> 121
   <211> 22
   <212> DNA
   <213> Artificial Sequence
<220>
   <223> Description of Artificial Sequence: Probe
<400> 121
   atctgcaaat gaacagcctg aa 22
<210> 122
   <211> 22
   <212> DNA
   <213> Artificial Sequence
<220>
   <223> Description of Artificial Sequence: Probe
<400> 122
   atctgcaaat gaacagcctg ag 22
<210> 123
   <211> 22
   <212> DNA
   <213> Artificial Sequence
<220>
   <223> Description of Artificial Sequence: Probe
<400> 123
   atctgcaaat gaacagtctg ag 22
<210> 124
   <211> 22
   <212> DNA
   <213> Artificial Sequence
<220>
   <223> Description of Artificial Sequence: Probe
<400> 124
   atctgcagat ctgcagccta aa 22
<210> 125
   <211> 22
   <212> DNA
   <213> Artificial Sequence
<220>
   <223> Description of Artificial Sequence: Probe
<400> 125
   atcttcaaat gaacagcctg ag 22
<210> 126
   <211> 22
   <212> DNA
   <213> Artificial Sequence
<220>
   <223> Description of Artificial Sequence: Probe
<400> 126
   atcttcaaat gggcagcctg ag 22
<210> 127
   <211> 22
   <212> DNA
   <213> Artificial Sequence
<220>
   <223> Description of Artificial Sequence: Probe
<400> 127
   ccctgaagct gagctctgtg ac 22
<210> 128
   <211> 22
   <212> DNA
   <213> Artificial Sequence
<220>
   <223> Description of Artificial Sequence: Probe
<400> 128
   ccctgcagct gaactctgtg ac 22
<210> 129
   <211> 22
   <212> DNA
   <213> Artificial Sequence
<220>
   <223> Description of Artificial Sequence: Probe
<400> 129
   tccttacaat gaccaacatg ga 22
<210> 130
   <211> 22
   <212> DNA
   <213> Artificial Sequence
<220>
   <223> Description of Artificial Sequence: Probe
<400> 130
   tccttaccat gaccaacatg ga 22
<210> 131
   <211> 22
   <212> DNA
   <213> Artificial Sequence
<220>
   <223> Description of Artificial Sequence: Synthetic oligonucleotide
<400> 131
   acatggagct gagcagcctg ag 22
<210> 132
   <211> 22
   <212> DNA
   <213> Artificial Sequence
<220>
   <223> Description of Artificial Sequence: Synthetic oligonucleotide
<400> 132
   ccctgaagct gagctctgtg ac 22
<210> 133
   <211> 54
   <212> DNA
   <213> Artificial Sequence
<220>
   <223> Description of Artificial Sequence: Synthetic oligonucleotide
<400> 133
   cgcttcacta agtctagaga caactctaag aatactctct acttgcagat gaac 54
<210> 134
   <211> 60
   <212> DNA
   <213> Artificial Sequence
<220>
   <223> Description of Artificial Sequence: Synthetic oligonucleotide
<400> 134
   cgcttcactc agtctagaga taacagtaaa aatactttgt acttgcagct gagcagcctg 60
<210> 135
   <211> 60
   <212> DNA
   <213> Artificial Sequence
<220>
   <223> Description of Artificial Sequence: Synthetic oligonucleotide
<400> 135
   cgcttcactc agtctagaga taacagtaaa aatactttgt acttgcagct gagctctgtg 60
<210> 136
   <211> 52
   <212> DNA
   <213> Artificial Sequence
<220>
   <223> Description of Artificial Sequence: Synthetic oligonucleotide
<400> 136
   tcagctgcaa gtacaaagta tttttactgt tatctctaga ctgagtgaag cg 52
<210> 137
   <211> 24
   <212> DNA
   <213> Artificial Sequence
<220>
   <223> Description of Artificial Sequence: Synthetic oligonucleotide
<400> 137
   cgcttcactc agtctagaga taac 24
<210> 138
   <211> 22
   <212> DNA
   <213> Artificial Sequence
<220>
   <223> Description of Artificial Sequence: Synthetic oligonucleotide
<400> 138
   ccgtgtatta ctgtgcgaga ga 22
<210> 139
   <211> 22
   <212> DNA
   <213> Artificial Sequence
<220>
   <223> Description of Artificial Sequence: Synthetic oligonucleotide
<400> 139
   ctgtgtatta ctgtgcgaga ga 22
<210> 140
   <211> 22
   <212> DNA
   <213> Artificial Sequence
<220>
   <223> Description of Artificial Sequence: Synthetic oligonucleotide
<400> 140
   ccgtgtatta ctgtgcgaga gg 22
<210> 141
   <211> 22
   <212> DNA
   <213> Artificial Sequence
<220>
   <223> Description of Artificial Sequence: Synthetic oligonucleotide
<400> 141
   ccgtgtatta ctgtgcaaca ga 22
<210> 142
   <211> 22
   <212> DNA
   <213> Artificial Sequence
<220>
   <223> Description of Artificial Sequence: Synthetic oligonucleotide
<400> 142
   ccatgtatta ctgtgcaaga ta 22
<210> 143
   <211> 22
   <212> DNA
   <213> Artificial Sequence
<220>
   <223> Description of Artificial Sequence: Synthetic oligonucleotide
<400> 143
   ccgtgtatta ctgtgcggca ga 22
<210> 144
   <211> 22
   <212> DNA
   <213> Artificial Sequence
<220>
   <223> Description of Artificial Sequence: Synthetic oligonucleotide
<400> 144
   ccacatatta ctgtgcacac ag 22
<210> 145
   <211> 22
   <212> DNA
   <213> Artificial Sequence
<220>
   <223> Description of Artificial Sequence: Synthetic oligonucleotide
<400> 145
   ccacatatta ctgtgcacgg at 22
<210> 146
   <211> 22
   <212> DNA
   <213> Artificial Sequence
<220>
   <223> Description of Artificial Sequence: Synthetic oligonucleotide
<400> 146
   ccacgtatta ctgtgcacgg at 22
<210> 147
   <211> 22
   <212> DNA
   <213> Artificial Sequence
<220>
   <223> Description of Artificial Sequence: Synthetic oligonucleotide
<400> 147
   ccttgtatta ctgtgcaaaa ga 22
<210> 148
   <211> 22
   <212> DNA
   <213> Artificial Sequence
<220>
   <223> Description of Artificial Sequence: Synthetic oligonucleotide
<400> 148
   ctgtgtatta ctgtgcaaga ga 22
<210> 149
   <211> 22
   <212> DNA
   <213> Artificial Sequence
<220>
   <223> Description of Artificial Sequence: Synthetic oligonucleotide
<400> 149
   ccgtgtatta ctgtaccaca ga 22
<210> 150
   <211> 22
   <212> DNA
   <213> Artificial Sequence
<220>
   <223> Description of Artificial Sequence: Synthetic oligonucleotide
<400> 150
   ccttgtatca ctgtgcgaga ga 22
<210> 151
   <211> 22
   <212> DNA
   <213> Artificial Sequence
<220>
   <223> Description of Artificial Sequence: Synthetic oligonucleotide
<400> 151
   ccgtatatta ctgtgcgaaa ga 22
<210> 152
   <211> 22
   <212> DNA
   <213> Artificial Sequence
<220>
   <223> Description of Artificial Sequence: Synthetic oligonucleotide
<400> 152
   ctgtgtatta ctgtgcgaaa ga 22
<210> 153
   <211> 22
   <212> DNA
   <213> Artificial Sequence
<220>
   <223> Description of Artificial Sequence: Synthetic oligonucleotide
<400> 153
   ccgtgtatta ctgtactaga ga 22
<210> 154
   <211> 22
   <212> DNA
   <213> Artificial Sequence
<220>
   <223> Description of Artificial Sequence: Synthetic oligonucleotide
<400> 154
   ccgtgtatta ctgtgctaga ga 22
<210> 155
   <211> 22
   <212> DNA
   <213> Artificial Sequence
<220>
   <223> Description of Artificial Sequence: Synthetic oligonucleotide
<400> 155
   ccgtgtatta ctgtactaga ca 22
<210> 156
   <211> 22
   <212> DNA
   <213> Artificial Sequence
<220>
   <223> Description of Artificial Sequence: Synthetic oligonucleotide
<400> 156
   ctgtgtatta ctgtaagaaa ga 22
<210> 157
   <211> 22
   <212> DNA
   <213> Artificial Sequence
<220>
   <223> Description of Artificial Sequence: Synthetic oligonucleotide
<400> 157
   ccgtgtatta ctgtgcgaga aa 22
<210> 158
   <211> 22
   <212> DNA
   <213> Artificial Sequence
<220>
   <223> Description of Artificial Sequence: Synthetic oligonucleotide
<400> 158
   ccgtgtatta ctgtgccaga ga 22
<210> 159
   <211> 22
   <212> DNA
   <213> Artificial Sequence
<220>
   <223> Description of Artificial Sequence: Synthetic oligonucleotide
<400> 159
   ctgtgtatta ctgtgcgaga ca 22
<210> 160
   <211> 22
   <212> DNA
   <213> Artificial Sequence
<220>
   <223> Description of Artificial Sequence: Synthetic oligonucleotide
<400> 160
   ccatgtatta ctgtgcgaga ca 22
<210> 161
   <211> 20
   <212> DNA
   <213> Artificial Sequence
<220>
   <223> Description of Artificial Sequence: Synthetic oligonucleotide
<400> 161
   ccatgtatta ctgtgcgaga 20
<210> 162
   <211> 21
   <212> DNA
   <213> Artificial Sequence
<220>
   <223> Description of Artificial Sequence: Synthetic oligonucleotide
<400> 162
   ccgtgtatta ctgtgcgaga g 21

<210> 163
   <211> 21
   <212> DNA
   <213> Artificial Sequence
<220>
   <223> Description of Artificial Sequence: Synthetic oligonucleotide
<400> 163
   ctgtgtatta ctgtgcgaga g 21
<210> 164
   <211> 21
   <212> DNA
   <213> Artificial Sequence
<220>
   <223> Description of Artificial Sequence: Synthetic oligonucleotide
<400> 164
   ccgtgtatta ctgtgcgaga g 21
<210> 165
   <211> 21
   <212> DNA
   <213> Artificial Sequence
<220>
   <223> Description of Artificial Sequence: Synthetic oligonucleotide
<400> 165
   ccgtatatta ctgtgcgaaa g 21
<210> 166
   <211> 21
   <212> DNA
   <213> Artificial Sequence
<220>
   <223> Description of Artificial Sequence: Synthetic oligonucleotide
<400> 166
   ctgtgtatta ctgtgcgaaa g 21
<210> 167
   <211> 21
   <212> DNA
   <213> Artificial Sequence
<220>
   <223> Description of Artificial Sequence: Synthetic oligonucleotide
<400> 167
   ctgtgtatta ctgtgcgaga c 21
<210> 168
   <211> 21
   <212> DNA
   <213> Artificial Sequence
<220>
   <223> Description of Artificial Sequence: Synthetic oligonucleotide
<400> 168
   ccatgtatta ctgtgcgaga c 21
<210> 169
   <211> 20
   <212> DNA
   <213> Artificial Sequence
<220>
   <223> Description of Artificial Sequence: Synthetic oligonucleotide
<400> 169
   ccatgtatta ctgtgcgaga 20
<210> 170
   <211> 94
   <212> DNA
   <213> Artificial Sequence
<220>
   <223> Description of Artificial Sequence: Synthetic oligonucleotide
<400> 170
   ggtgtagtga tctagtgaca actctaagaa tactctctac ttgcagatga acagctttag 60
   ggctgaggac actgcagtct actattgtgc gaga                             94
<210> 171
   <211> 94
   <212> DNA
   <213> Artificial Sequence
<220>
   <223> Description of Artificial Sequence: Synthetic oligonucleotide
<400> 171
   ggtgtagtga tctagtgaca actctaagaa tactctctac ttgcagatga acagctttag 60
   ggctgaggac actgcagtct actattgtgc gaaa                             94
<210> 172
   <211> 85
   <212> DNA
   <213> Artificial Sequence
<220>
   <223> Description of Artificial Sequence: Synthetic oligonucleotide
<400> 172
   atagtagact gcagtgtcct cagcccttaa gctgttcatc tgcaagtaga gagtattctt 60
   agagttgtct ctagatcact acacc                                       85
<210> 173
   <211> 22
   <212> DNA
   <213> Artificial Sequence
<220>
   <223> Description of Artificial Sequence: Synthetic oligonucleotide
<400> 173
   ggtgtagtga tctagagaca ac 22
<210> 174
   <211> 55
   <212> DNA
   <213> Artificial Sequence
<220>
   <223> Description of Artificial Sequence: Synthetic oligonucleotide
<400> 174
   ggtgtagtga aacagcttta gggctgagga cactgcagtc tactattgtg cgaga 55
<210> 175
   <211> 55
   <212> DNA
   <213> Artificial Sequence
<220>
   <223> Description of Artificial Sequence: Synthetic oligonucleotide
<400> 175
   ggtgtagtga aacagcttta gggctgagga cactgcagtc tactattgtg cgaaa 55
<210> 176
   <211> 46
   <212> DNA
   <213> Artificial Sequence
<220>
   <223> Description of Artificial Sequence: Synthetic oligonucleotide
<400> 176
   atagtagact gcagtgtcct cagcccttaa gctgtttcac tacacc 46
<210> 177
   <211> 26
   <212> DNA
   <213> Artificial Sequence
<220>
   <223> Description of Artificial Sequence: Synthetic oligonucleotide
<400> 177
   ggtgtagtga aacagcttaa gggctg 26
<210> 178
   <211> 25
   <212> DNA
   <213> Artificial Sequence
<220>
   <223> Description of Artificial Sequence: Synthetic oligonucleotide
<400> 178
   cacatccgtg ttgttcacgg atgtg 25
<210> 179
   <211> 88
   <212> DNA
   <213> Artificial Sequence
<220>
   <223> Description of Artificial Sequence: Synthetic oligonucleotide
<400> 179
   aatagtagac tgcagtgtcc tcagccctta agctgttcat ctgcaagtag agagtattct 60
   tagagttgtc tctagactta gtgaagcg 88
<210> 180
   <211> 95
   <212> DNA
   <213> Artificial Sequence
<220>
   <223> Description of Artificial Sequence: Synthetic oligonucleotide
<400> 180
   cgcttcacta agtctagaga caactctaag aatactctct acttgcagat gaacagctta 60
   agggctgagg acactgcagt ctactattgt gcgag 95
<210> 181
   <211> 95
   <212> DNA
   <213> Artificial Sequence
<220>
   <223> Description of Artificial Sequence: Synthetic oligonucleotide
<400> 181
   cgcttcacta agtctagaga caactctaag aatactctct acttgcagat gaacagctta 60
   agggctgagg acactgcagt ctactattgt acgag 95
<210> 182
   <211> 24
   <212> DNA
   <213> Artificial Sequence
<220>
   <223> Description of Artificial Sequence: Synthetic oligonucleotide
<400> 182
   cgcttcacta agtctagaga caac 24
<210> 183
   <211> 419
   <212> DNA
   <213> Unknown Organism
<220>
   <223> Description of Unknown Organism: V3-23
<400> 183
   ctgtctgaac ggcccagccg gccatggccg aagttcaatt gttagagtct ggtggcggtc 60
   ttgttcagcc tggtggttct ttacgtcttt cttgcgctgc ttccggattc actttctctt 120
   cgtacgctat gtcttgggtt cgccaagctc ctggtaaagg tttggagtgg gtttctgcta 180
   tctctggttc tggtggcagt acttactatg ctgactccgt taaaggtcgc ttcactatct 240
   ctagagacaa ctctaagaat actctctact tgcagatgaa cagcttaagg gctgaggaca 300
   ctgcagtcta ctattgcgct aaagactatg aaggtactgg ttatgctttc gacatatggg 360
   gtcaaggtac tatggtcacc gtctctagtg cctccaccaa gggcccatcg gtcttcccc  419
<210> 184
   <211> 136
   <212> PRT
   <213> Unknown Organism
<220>
   <223> Description of Unknown Organism: V3-23
<400> 184
   Ala Gln Pro Ala Met Ala Glu Val Gln Leu Leu Glu Ser Gly Gly Gly
   1               5                   10                  15
   Leu Val Gln Pro Gly Gly Ser Leu Arg Leu Ser Cys Ala Ala Ser Gly
               20                  25                  30
   Phe Thr Phe Ser Ser Tyr Ala Met Ser Trp Val Arg Gln Ala Pro Gly
           35                  40                  45
   Lys Gly Leu Glu Trp Val Ser Ala Ile Ser Gly Ser Gly Gly Ser Thr
       50                  55                  60
   Tyr Tyr Ala Asp Ser Val Lys Gly Arg Phe Thr Ile Ser Arg Asp Asn
   65                  70                  75                  80
   Ser Lys Asn Thr Leu Tyr Leu Gln Met Asn Ser Leu Arg Ala Glu Asp
                   85                  90                  95
   Thr Ala Val Tyr Tyr Cys Ala Lys Asp Tyr Glu Gly Thr Gly Tyr Ala
               100                 105                 110
   Phe Asp Ile Trp Gly Gln Gly Thr Met Val Thr Val Ser Ser Ala Ser
           115                 120                 125
   Thr Lys Gly Pro Ser Val Phe Pro
       130                 135
<210> 185
   <211> 20
   <212> DNA
   <213> Artificial Sequence
<220>
   <223> Description of Artificial Sequence: Synthetic oligonucleotide
<400> 185
   ctgtctgaac ggcccagccg 20
<210> 186
   <211> 83
   <212> DNA
   <213> Artificial Sequence
<220>
   <223> Description of Artificial Sequence: Synthetic oligonucleotide
<400> 186
   ctgtctgaac ggcccagccg gccatggccg aagttcaatt gttagagtct ggtggcggtc 60
   ttgttcagcc tggtggttct tta 83
<210> 187
   <211> 54
   <212> DNA
   <213> Artificial Sequence
<220>
   <223> Description of Artificial Sequence: Synthetic oligonucleotide
<400> 187
   gaaagtgaat ccggaagcag cgcaagaaag acgtaaagaa ccaccaggct gaac 54
<210> 188
   <211> 42
   <212> DNA
   <213> Artificial Sequence
<220>
   <223> Description of Artificial Sequence: Synthetic oligonucleotide
<400> 188
   agaaacccac tccaaacctt taccaggagc ttggcgaacc ca 42
<210> 189
   <211> 94
   <212> DNA
   <213> Artificial Sequence
<220>
   <223> Description of Artificial Sequence: Synthetic oligonucleotide
<400> 189
   agtgtcctca gcccttaagc tgttcatctg caagtagaga gtattcttag agttgtctct 60
   agagatagtg aagcgacctt taacggagtc agca 94
<210> 190
   <211> 81
   <212> DNA
   <213> Artificial Sequence
<220>
   <223> Description of Artificial Sequence: Synthetic oligonucleotide
<400> 190
   gcttaagggc tgaggacact gcagtctact attgcgctaa agactatgaa ggtactggtt 60
   atgctttcga catatggggt c                                           81
<210> 191
   <211> 75
   <212> DNA
   <213> Artificial Sequence
<220>
   <223> Description of Artificial Sequence: Synthetic oligonucleotide
<400> 191
   ggggaagacc gatgggccct tggtggaggc actagagacg gtgaccatag taccttgacc 60
   ccatatgtcg aaagc                                                  75
<210> 192
   <211> 47
   <212> DNA
   <213> Artificial Sequence
<220>
   <223> Description of Artificial Sequence: Synthetic oligonucleotide
<400> 192
   gcttccggat tcactttctc ttacatgtgg gttcgccaag ctcctgg 47
<210> 193
   <211> 53
   <212> DNA
   <213> Artificial Sequence
<220>
   <223> Description of Artificial Sequence: Synthetic oligonucleotide
<400> 193
   ggtttggagt gggtttctat ctctggtggc acttatgctg actccgttaa agg 53
<210> 194
   <211> 9472
   <212> DNA
   <213> Unknown Organism
<220>
   <223> Description of Unknown Organism: MALIA3
<400> 194
   aatgctacta ctattagtag aattgatgcc accttttcag ctcgcgcccc aaatgaaaat 60
   atagctaaac aggttattga ccatttgcga aatgtatcta atggtcaaac taaatctact 120
   cgttcgcaga attgggaatc aactgttaca tggaatgaaa cttccagaca ccgtacttta 180
   gttgcatatt taaaacatgt tgagctacag caccagattc agcaattaag ctctaagcca 240
   tccgcaaaaa tgacctctta tcaaaaggag caattaaagg tactctctaa tcctgacctg 300
   tctttcgggc ttcctcttaa tctttttgat gcaatccgct ttgcttctga ctataatagt 360
   cagggtaaag acctgatttt tgatttatgg tcattctcgt tttctgaact gtttaaagca 420
   tttgaggggg attcaatgaa tatttatgac gattccgcag tattggacgc tatccagtct 480
   aaacatttta ctattacccc ctctggcaaa acttcttttg caaaagcctc tcgctatttt 540
   ggtttttatc gtcgtctggt aaacgagggt tatgatagtg ttgctcttac tatgcctcgt 600
   aattcctttt ggcgttatgt atctgcatta gttgaatgtg gtattcctaa atctcaactg 660
   atgaatcttt ctacctgtaa taatgttgtt ccgttagttc gttttattaa cgtagatttt 720
   tcttcccaac gtcctgactg gtataatgag ccagttctta aaatcgcata aggtaattca 780
   caatgattaa agttgaaatt aaaccatctc aagcccaatt tactactcgt tctggtgttt 840
   ctcgtcaggg caagccttat tcactgaatg agcagctttg ttacgttgat ttgggtaatg 900
   aatatccggt tcttgtcaag attactcttg atgaaggtca gccagcctat gcgcctggtc 960
   tgtacaccgt tcatctgtcc tctttcaaag ttggtcagtt cggttccctt atgattgacc 1020
   gtctgcgcct cgttccggct aagtaacatg gagcaggtcg cggatttcga cacaatttat 1080
   caggcgatga tacaaatctc cgttgtactt tgtttcgcgc ttggtataat cgctgggggt 1140
   caaagatgag tgttttagtg tattctttcg cctctttcgt tttaggttgg tgccttcgta 1200
   gtggcattac gtattttacc cgtttaatgg aaacttcctc atgaaaaagt ctttagtcct 1260
   caaagcctct gtagccgttg ctaccctcgt tccgatgctg tctttcgctg ctgagggtga 1320
   cgatcccgca aaagcggcct ttaactccct gcaagcctca gcgaccgaat atatcggtta 1380
   tgcgtgggcg atggttgttg tcattgtcgg cgcaactatc ggtatcaagc tgtttaagaa 1440
   attcacctcg aaagcaagct gataaaccga tacaattaaa ggctcctttt ggagcctttt 1500
   tttttggaga ttttcaacgt gaaaaaatta ttattcgcaa ttcctttagt tgttcctttc 1560
   tattctcaca gtgcacagtc tgtcgtgacg cagccgccct cagtgtctgg ggccccaggg 1620
   cagagggtca ccatctcctg cactgggagc agctccaaca tcggggcagg ttatgatgta 1680
   cactggtacc agcagcttcc aggaacagcc cccaaactcc tcatctatgg taacagcaat 1740
   cggccctcag gggtccctga ccgattctct ggctccaagt ctggcacctc agcctccctg 1800
   gccatcactg ggctccaggc tgaggatgag gctgattatt actgccagtc ctatgacagc 1860
   agcctgagtg gcctttatgt cttcggaact gggaccaagg tcaccgtcct aggtcagccc 1920
   aaggccaacc ccactgtcac tctgttcccg ccctcctctg aggagctcca agccaacaag 1980
   gccacactag tgtgtctgat cagtgacttc tacccgggag ctgtgacagt ggcctggaag 2040
   gcagatagca gccccgtcaa ggcgggagtg gagaccacca caccctccaa acaaagcaac 2100
   aacaagtacg cggccagcag ctatctgagc ctgacgcctg agcagtggaa gtcccacaga 2160
   agctacagct gccaggtcac gcatgaaggg agcaccgtgg agaagacagt ggcccctaca 2220
   gaatgttcat aataaaccgc ctccaccggg cgcgccaatt ctatttcaag gagacagtca 2280
   taatgaaata cctattgcct acggcagccg ctggattgtt attactcgcg gcccagccgg 2340
   ccatggccga agttcaattg ttagagtctg gtggcggtct tgttcagcct ggtggttctt 2400
   tacgtctttc ttgcgctgct tccggattca ctttctcttc gtacgctatg tcttgggttc 2460
   gccaagctcc tggtaaaggt ttggagtggg tttctgctat ctctggttct ggtggcagta 2520
   cttactatgc tgactccgtt aaaggtcgct tcactatctc tagagacaac tctaagaata 2580
   ctctctactt gcagatgaac agcttaaggg ctgaggacac tgcagtctac tattgcgcta 2640
   aagactatga aggtactggt tatgctttcg acatatgggg tcaaggtact atggtcaccg 2700
   tctctagtgc ctccaccaag ggcccatcgg tcttccccct ggcaccctcc tccaagagca 2760
   cctctggggg cacagcggcc ctgggctgcc tggtcaagga ctacttcccc gaaccggtga 2820
   cggtgtcgtg gaactcaggc gccctgacca gcggcgtcca caccttcccg gctgtcctac 2880
   agtctagcgg actctactcc ctcagcagcg tagtgaccgt gccctcttct agcttgggca 2940
   cccagaccta catctgcaac gtgaatcaca agcccagcaa caccaaggtg gacaagaaag 3000
   ttgagcccaa atcttgtgcg gccgctcatc accaccatca tcactctgct gaacaaaaac 3060
   tcatctcaga agaggatctg aatggtgccg cagatatcaa cgatgatcgt atggctggcg 3120
   ccgctgaaac tgttgaaagt tgtttagcaa aaccccatac agaaaattca tttactaacg 3180
   tctggaaaga cgacaaaact ttagatcgtt acgctaacta tgagggttgt ctgtggaatg 3240
   ctacaggcgt tgtagtttgt actggtgacg aaactcagtg ttacggtaca tgggttccta 3300
   ttgggcttgc tatccctgaa aatgagggtg gtggctctga gggtggcggt tctgagggtg 3360
   gcggttctga gggtggcggt actaaacctc ctgagtacgg tgatacacct attccgggct 3420
   atacttatat caaccctctc gacggcactt atccgcctgg tactgagcaa aaccccgcta 3480
   atcctaatcc ttctcttgag gagtctcagc ctcttaatac tttcatgttt cagaataata 3540
   ggttccgaaa taggcagggg gcattaactg tttatacggg cactgttact caaggcactg 3600
   accccgttaa aacttattac cagtacactc ctgtatcatc aaaagccatg tatgacgctt 3660
   actggaacgg taaattcaga gactgcgctt tccattctgg ctttaatgaa gatccattcg 3720
   tttgtgaata tcaaggccaa tcgtctgacc tgcctcaacc tcctgtcaat gctggcggcg 3780
   gctctggtgg tggttctggt ggcggctctg agggtggtgg ctctgagggt ggcggttctg 3840
   agggtggcgg ctctgaggga ggcggttccg gtggtggctc tggttccggt gattttgatt 3900
   atgaaaagat ggcaaacgct aataaggggg ctatgaccga aaatgccgat gaaaacgcgc 3960
   tacagtctga cgctaaaggc aaacttgatt ctgtcgctac tgattacggt gctgctatcg 4020
   atggtttcat tggtgacgtt tccggccttg ctaatggtaa tggtgctact ggtgattttg 4080
   ctggctctaa ttcccaaatg gctcaagtcg gtgacggtga taattcacct ttaatgaata 4140
   atttccgtca atatttacct tccctccctc aatcggttga atgtcgccct tttgtcttta 4200
   gcgctggtaa accatatgaa ttttctattg attgtgacaa aataaactta ttccgtggtg 4260
   tctttgcgtt tcttttatat gttgccacct ttatgtatgt attttctacg tttgctaaca 4320
   tactgcgtaa taaggagtct taatcatgcc agttcttttg ggtattccgt tattattgcg 4380
   tttcctcggt ttccttctgg taactttgtt cggctatctg cttacttttc ttaaaaaggg 4440
   cttcggtaag atagctattg ctatttcatt gtttcttgct cttattattg ggcttaactc 4500
   aattcttgtg ggttatctct ctgatattag cgctcaatta ccctctgact ttgttcaggg 4560
   tgttcagtta attctcccgt ctaatgcgct tccctgtttt tatgttattc tctctgtaaa 4620
   ggctgctatt ttcatttttg acgttaaaca aaaaatcgtt tcttatttgg attgggataa 4680
   ataatatggc tgtttatttt gtaactggca aattaggctc tggaaagacg ctcgttagcg 4740
   ttggtaagat tcaggataaa attgtagctg ggtgcaaaat agcaactaat cttgatttaa 4800
   ggcttcaaaa cctcccgcaa gtcgggaggt tcgctaaaac gcctcgcgtt cttagaatac 4860
   cggataagcc ttctatatct gatttgcttg ctattgggcg cggtaatgat tcctacgatg 4920
   aaaataaaaa cggcttgctt gttctcgatg agtgcggtac ttggtttaat acccgttctt 4980
   ggaatgataa ggaaagacag ccgattattg attggtttct acatgctcgt aaattaggat 5040
   gggatattat ttttcttgtt caggacttat ctattgttga taaacaggcg cgttctgcat 5100
   tagctgaaca tgttgtttat tgtcgtcgtc tggacagaat tactttacct tttgtcggta 5160
   ctttatattc tcttattact ggctcgaaaa tgcctctgcc taaattacat gttggcgttg 5220
   ttaaatatgg cgattctcaa ttaagcccta ctgttgagcg ttggctttat actggtaaga 5280
   atttgtataa cgcatatgat actaaacagg ctttttctag taattatgat tccggtgttt 5340
   attcttattt aacgccttat ttatcacacg gtcggtattt caaaccatta aatttaggtc 5400
   agaagatgaa attaactaaa atatatttga aaaagttttc tcgcgttctt tgtcttgcga 5460
   ttggatttgc atcagcattt acatatagtt atataaccca acctaagccg gaggttaaaa 5520
   aggtagtctc tcagacctat gattttgata aattcactat tgactcttct cagcgtctta 5580
   atctaagcta tcgctatgtt ttcaaggatt ctaagggaaa attaattaat agcgacgatt 5640
   tacagaagca aggttattca ctcacatata ttgatttatg tactgtttcc attaaaaaag 5700
   gtaattcaaa tgaaattgtt aaatgtaatt aattttgttt tcttgatgtt tgtttcatca 5760
   tcttcttttg ctcaggtaat tgaaatgaat aattcgcctc tgcgcgattt tgtaacttgg 5820
   tattcaaagc aatcaggcga atccgttatt gtttctcccg atgtaaaagg tactgttact 5880
   gtatattcat ctgacgttaa acctgaaaat ctacgcaatt tctttatttc tgttttacgt 5940
   gctaataatt ttgatatggt tggttcaatt ccttccataa ttcagaagta taatccaaac 6000
   aatcaggatt atattgatga attgccatca tctgataatc aggaatatga tgataattcc 6060
   gctccttctg gtggtttctt tgttccgcaa aatgataatg ttactcaaac ttttaaaatt 6120
   aataacgttc gggcaaagga tttaatacga gttgtcgaat tgtttgtaaa gtctaatact 6180
   tctaaatcct caaatgtatt atctattgac ggctctaatc tattagttgt ttctgcacct 6240
   aaagatattt tagataacct tcctcaattc ctttctactg ttgatttgcc aactgaccag 6300
   atattgattg agggtttgat atttgaggtt cagcaaggtg atgctttaga tttttcattt 6360
   gctgctggct ctcagcgtgg cactgttgca ggcggtgtta atactgaccg cctcacctct 6420
   gttttatctt ctgctggtgg ttcgttcggt atttttaatg gcgatgtttt agggctatca 6480
   gttcgcgcat taaagactaa tagccattca aaaatattgt ctgtgccacg tattcttacg 6540
   ctttcaggtc agaagggttc tatctctgtt ggccagaatg tcccttttat tactggtcgt 6600
   gtgactggtg aatctgccaa tgtaaataat ccatttcaga cgattgagcg tcaaaatgta 6660
   ggtatttcca tgagcgtttt tcctgttgca atggctggcg gtaatattgt tctggatatt 6720
   accagcaagg ccgatagttt gagttcttct actcaggcaa gtgatgttat tactaatcaa 6780
   agaagtattg ctacaacggt taatttgcgt gatggacaga ctcttttact cggtggcctc 6840
   actgattata aaaacacttc tcaagattct ggcgtaccgt tcctgtctaa aatcccttta 6900
   atcggcctcc tgtttagctc ccgctctgat tccaacgagg aaagcacgtt atacgtgctc 6960
   gtcaaagcaa ccatagtacg cgccctgtag cggcgcatta agcgcggcgg gtgtggtggt 7020
   tacgcgcagc gtgaccgcta cacttgccag cgccctagcg cccgctcctt tcgctttctt 7080
   cccttccttt ctcgccacgt tcgccggctt tccccgtcaa gctctaaatc gggggctccc 7140
   tttagggttc cgatttagtg ctttacggca cctcgacccc aaaaaacttg atttgggtga 7200
   tggttcacgt agtgggccat cgccctgata gacggttttt cgccctttga cgttggagtc 7260
   cacgttcttt aatagtggac tcttgttcca aactggaaca acactcaacc ctatctcggg 7320
   ctattctttt gatttataag ggattttgcc gatttcggaa ccaccatcaa acaggatttt 7380
   cgcctgctgg ggcaaaccag cgtggaccgc ttgctgcaac tctctcaggg ccaggcggtg 7440
   aagggcaatc agctgttgcc cgtctcactg gtgaaaagaa aaaccaccct ggatccaagc 7500
   ttgcaggtgg cacttttcgg ggaaatgtgc gcggaacccc tatttgttta tttttctaaa 7560
   tacattcaaa tatgtatccg ctcatgagac aataaccctg ataaatgctt caataatatt 7620
   gaaaaaggaa gagtatgagt attcaacatt tccgtgtcgc ccttattccc ttttttgcgg 7680
   cattttgcct tcctgttttt gctcacccag aaacgctggt gaaagtaaaa gatgctgaag 7740
   atcagttggg cgcacgagtg ggttacatcg aactggatct caacagcggt aagatccttg 7800
   agagttttcg ccccgaagaa cgttttccaa tgatgagcac ttttaaagtt ctgctatgtc 7860
   atacactatt atcccgtatt gacgccgggc aagagcaact cggtcgccgg gcgcggtatt 7920
   ctcagaatga cttggttgag tactcaccag tcacagaaaa gcatcttacg gatggcatga 7980
   cagtaagaga attatgcagt gctgccataa ccatgagtga taacactgcg gccaacttac 8040
   ttctgacaac gatcggagga ccgaaggagc taaccgcttt tttgcacaac atgggggatc 8100
   atgtaactcg ccttgatcgt tgggaaccgg agctgaatga agccatacca aacgacgagc 8160
   gtgacaccac gatgcctgta gcaatgccaa caacgttgcg caaactatta actggcgaac 8220
   tacttactct agcttcccgg caacaattaa tagactggat ggaggcggat aaagttgcag 8280
   gaccacttct gcgctcggcc cttccggctg gctggtttat tgctgataaa tctggagccg 8340
   gtgagcgtgg gtctcgcggt atcattgcag cactggggcc agatggtaag ccctcccgta 8400
   tcgtagttat ctacacgacg gggagtcagg caactatgga tgaacgaaat agacagatcg 8460
   ctgagatagg tgcctcactg attaagcatt ggtaactgtc agaccaagtt tactcatata 8520
   tactttagat tgatttaaaa cttcattttt aatttaaaag gatctaggtg aagatccttt 8580
   ttgataatct catgaccaaa atcccttaac gtgagttttc gttccactgt acgtaagacc 8640
   cccaagcttg tcgactgaat ggcgaatggc gctttgcctg gtttccggca ccagaagcgg 8700
   tgccggaaag ctggctggag tgcgatcttc ctgaggccga tactgtcgtc gtcccctcaa 8760
   actggcagat gcacggttac gatgcgccca tctacaccaa cgtaacctat cccattacgg 8820
   tcaatccgcc gtttgttccc acggagaatc cgacgggttg ttactcgctc acatttaatg 8880
   ttgatgaaag ctggctacag gaaggccaga cgcgaattat ttttgatggc gttcctattg 8940
   gttaaaaaat gagctgattt aacaaaaatt taacgcgaat tttaacaaaa tattaacgtt 9000
   tacaatttaa atatttgctt atacaatctt cctgtttttg gggcttttct gattatcaac 9060
   cggggtacat atgattgaca tgctagtttt acgattaccg ttcatcgatt ctcttgtttg 9120
   ctccagactc tcaggcaatg acctgatagc ctttgtagat ctctcaaaaa tagctaccct 9180
   ctccggcatg aatttatcag ctagaacggt tgaatatcat attgatggtg atttgactgt 9240
   ctccggcctt tctcaccctt ttgaatcttt acctacacat tactcaggca ttgcatttaa 9300
   aatatatgag ggttctaaaa atttttatcc ttgcgttgaa ataaaggctt ctcccgcaaa 9360
   agtattacag ggtcataatg tttttggtac aaccgattta gctttatgct ctgaggcttt 9420
   attgcttaat tttgctaatt ctttgccttg cctgtatgat ttattggatg tt         9472

<210> 195
   <211> 20
   <212> PRT
   <213> Unknown Organism
<220>
   <223> Description of Unknown Organism: MALIA3
<400> 195
   Met Lys Lys Leu Leu Phe Ala Ile Pro Leu Val Val Pro Phe Tyr Ser
     1               5                  10                  15
   His Ser Ala Gln
                20
<210> 196
   <211> 368
   <212> PRT
   <213> Unknown Organism
<220>
   <223> Description of Unknown Organism: MALIA3
<400> 196
   Met Lys Tyr Leu Leu Pro Thr Ala Ala Ala Gly Leu Leu Leu Leu Ala
   1               5                   10                  15
   Ala Gln Pro Ala Met Ala Glu Val Gln Leu Leu Glu Ser Gly Gly Gly
               20                  25                  30
   Leu Val Gln Pro Gly Gly Ser Leu Arg Leu Ser Cys Ala Ala Ser Gly
           35                  40                  45
   Phe Thr Phe Ser Ser Tyr Ala Met Ser Trp Val Arg Gln Ala Pro Gly
       50                  55                  60
   Lys Gly Leu Glu Trp Val Ser Ala Ile Ser Gly Ser Gly Gly Ser Thr
   65                  70                  75                  80
   Tyr Tyr Ala Asp Ser Val Lys Gly Arg Phe Thr Ile Ser Arg Asp Asn
                   85                  90                  95
   Ser Lys Asn Thr Leu Tyr Leu Gln Met Asn Ser Leu Arg Ala Glu Asp
               100                 105                 110
   Thr Ala Val Tyr Tyr Cys Ala Lys Asp Tyr Glu Gly Thr Gly Tyr Ala
           115                 120                 125
   Phe Asp Ile Trp Gly Gln Gly Thr Met Val Thr Val Ser Ser Ala Ser
       130                 135                 140
   Thr Lys Gly Pro Ser Val Phe Pro Leu Ala Pro Ser Ser Lys Ser Thr
   145                 150                 155                 160
   Ser Gly Gly Thr Ala Ala Leu Gly Cys Leu Val Lys Asp Tyr Phe Pro
                   165                 170                 175
   Glu Pro Val Thr Val Ser Trp Asn Ser Gly Ala Leu Thr Ser Gly Val
               180                 185                 190
   His Thr Phe Pro Ala Val Leu Gln Ser Ser Gly Leu Tyr Ser Leu Ser
   195 200 205
   Ser Val Val Thr Val Pro Ser Ser Ser Leu Gly Thr Gln Thr Tyr Ile
   210 215 220
   Cys Asn Val Asn His Lys Pro Ser Asn Thr Lys Val Asp Lys Lys Val
   225 230 235 240
   Glu Pro Lys Ser Cys Ala Ala Ala His His His His His His Ser Ala
   245 250 255
   Glu Gln Lys Leu Ile Ser Glu Glu Asp Leu Asn Gly Ala Ala Asp Ile
   260 265 270
   Asn Asp Asp Arg Met Ala Ser Gly Ala Ala Glu Thr Val Glu Ser Cys
   275 280 285
   Leu Ala Lys Pro His Thr Glu Ile Ser Phe Thr Asn Val Trp Lys Asp
   290 295 300
   Asp Lys Thr Leu Asp Arg Tyr Ala Asn Tyr Glu Gly Cys Leu Trp Asn
   305 310 315 320
   Ala Thr Gly Val Val Val Cys Thr Gly Asp Glu Thr Gln Cys Tyr Gly
   325 330 335
   Thr Trp Val Pro Ile Gly Leu Ala Ile Pro Glu Asn Glu Gly Gly Gly
   340 345 350
   Ser Glu Gly Gly Gly Ser Glu Gly Gly Gly Ser Glu Gly Gly Gly Thr
   355 360 365
<210> 197
   <211> 152
   <212> PRT
   <213> Unknown Organism
<220>
   <223> Description of Unknown Organism: MALIA3
<400> 197
   Ser Gly Asp Phe Asp Tyr Glu Lys Met Ala Asn Ala Asn Lys Gly Ala
   1 5 10 15
   Met Thr Glu Asn Ala Asp Glu Asn Ala Leu Gln Ser Asp Ala Lys Gly
   20 25 30
   Lys Leu Asp Ser Val Ala Thr Asp Tyr Gly Ala Ala Ile Asp Gly Phe
   35 40 45
   Ile Gly Asp Val Ser Gly Leu Ala Asn Gly Asn Gly Ala Thr Gly Asp
   50 55 60
   Phe Ala Gly Ser Asn Ser Gln Met Ala Gln Val Gly Asp Gly Asp Asn
   65 70 75 80
   Ser Pro Leu Met Asn Asn Phe Arg Gln Tyr Leu Pro Ser Leu Pro Gln
   85 90 95
   Ser Val Glu Cys Arg Pro Phe Val Phe Ser Ala Gly Lys Pro Tyr Glu
   100 105 110
   Phe Ser Ile Asp Cys Asp Lys Ile Asn Leu Phe Arg Gly Val Phe Ala
   115 120 125
   Phe Leu Leu Tyr Val Ala Thr Phe Met Tyr Val Phe Ser Thr Phe Ala
   130 135 140
   Asn Ile Leu Arg Asn Lys Glu Ser
   145 150
<210> 198
   <211> 15
   <212> PRT
   <213> Unknown Organism
<220>
   <223> Description of Unknown Organism: MALIA3
<400> 198
   Met Pro Val Leu Leu Gly Ile Pro Leu Leu Leu Arg Phe Leu Gly
   1 5 10 15
<210> 199
   <211> 335
   <212> PRT
   <213> Unknown Organism
<220>
   <223> Description of Unknown Organism: MALIA3
<400> 199
   Lys Thr Leu Val Ser Val Gly Lys Ile Gln Asp Lys Ile Val Ala Gly
   1               5                   10                  15
   Cys Lys Ile Ala Thr Asn Leu Asp Leu Arg Leu Gln Asn Leu Pro Gln
               20                  25                  30
   Val Gly Arg Phe Ala Lys Thr Pro Arg Val Leu Arg Ile Pro Asp Lys
           35                  40                  45
   Pro Ser Ile Ser Asp Leu Leu Ala Ile Gly Arg Gly Asn Asp Ser Tyr
       50                  55                  60
   Asp Glu Asn Lys Asn Gly Leu Leu Val Leu Asp Glu Cys Gly Thr Trp
   65                  70                  75                  80
   Phe Asn Thr Arg Ser Trp Asn Asp Lys Glu Arg Gln Pro Ile Ile Asp
                   85                  90                  95
   Trp Phe Leu His Ala Arg Lys Leu Gly Trp Asp Ile Ile Phe Leu Val
               100                 105                 110
   Gln Asp Leu Ser Ile Val Asp Lys Gln Ala Arg Ser Ala Leu Ala Glu
           115                 120                 125
   His Val Val Tyr Cys Arg Arg Leu Asp Arg Ile Thr Leu Pro Phe Val
   130 135 140
   Gly Thr Leu Tyr Ser Leu Ile Thr Gly Ser Lys Met Pro Leu Pro Lys
   145 150 155 160
   Leu His Val Gly Val Val Lys Tyr Gly Asp Ser Gln Leu Ser Pro Thr
   165 170 175
   Val Glu Arg Trp Leu Tyr Thr Gly Lys Asn Leu Tyr Asn Ala Tyr Asp
   180 185 190
   Thr Lys Gln Ala Phe Ser Ser Asn Tyr Asp Ser Gly Val Tyr Ser Tyr
   195 200 205
   Leu Thr Pro Tyr Leu Ser His Gly Arg Tyr Phe Lys Pro Leu Asn Leu
   210 215 220
   Gly Gln Lys Met Lys Leu Thr Lys Ile Tyr Leu Lys Lys Phe Ser Arg
   225 230 235 240
   Val Leu Cys Leu Ala Ile Gly Phe Ala Ser Ala Phe Thr Tyr Ser Tyr
   245 250 255
   Ile Thr Gln Pro Lys Pro Glu Val Lys Lys Val Val Ser Gln Thr Tyr
   260 265 270
   Asp Phe Asp Lys Phe Thr Ile Asp Ser Ser Gln Arg Leu Asn Leu Ser
   275 280 285
   Tyr Arg Tyr Val Phe Lys Asp Ser Lys Gly Lys Leu Ile Asn Ser Asp
   290 295 300
   Asp Leu Gln Lys Gln Gly Tyr Ser Leu Thr Tyr Ile Asp Leu Cys Thr
   305 310 315 320
   Val Ser Ile Lys Lys Gly Asn Ser Asn Glu Ile Val Lys Cys Asn
   325 330 335
<210> 200
   <211> 10
   <212> PRT
   <213> Unknown Organism
<220>
   <223> Description of Unknown Organism: MALIA3
<400> 200
   Met Lys Leu Leu Asn Val Ile Asn Phe Val
   1 5 10
<210> 201
   <211> 90
   <212> DNA
   <213> Homo sapiens
<400> 201
   actatctcta gagacaactc taagaatact ctctacttgc agatgaacag cttaagggct 60
   gaggacactg cagtctacta ttgcgctaaa                                  90
<210> 202
   <211> 30
   <212> PRT
   <213> Homo sapiens
<400> 202
   Thr Ile Ser Arg Asp Asn Ser Lys Asn Thr Leu Tyr Leu Gln Met Asn
    1               5                  10                  15
   Ser Leu Arg Ala Glu Asp Thr Ala Val Tyr Tyr Cys Ala Lys
               20                  25                  30
<210> 203
   <211> 90
   <212> DNA
   <213> Consensus sequence
<220>
   <221> modified_base
   <222> (3)
   <223> a, t, c, g, other or unknown
<220>
   <221> modified_base
   <222> (9)
   <223> a, t, c, g, other or unknown
<220>
   <221> modified_base
   <222> (12)
   <223> a, t, c, g, other or unknown
<220>
   <221> modified_base
   <222> (21)
   <223> a, t, c, g, other or unknown
<220>
   <221> modified_base
   <222> (30)
   <223> a, t, c, g, other or unknown
<220>
   <221> modified_base
   <222> (51)
   <223> a, t, c, g, other or unknown
<220>
   <221> modified_base
   <222> (57)
   <223> a, t, c, g, other or unknown
<220>
   <221> modified_base
   <222> (60)
   <223> a, t, c, g, other or unknown
<220>
   <221> modified_base
   <222> (69)
   <223> a, t, c, g, other or unknown
<220>
   <221> modified_base
   <222> (72)
   <223> a, t, c, g, other or unknown
<220>
   <221> modified_base
   <222> (75)
   <223> a, t, c, g, other or unknown
<220>
   <221> modified_base
   <222> (87)
   <223> a, t, c, g, other or unknown
<400> 203
   acnathtcnc gngayaaytc naaraayacn ttrtayttrc aratgaaytc nttrcgngcn 60
   gargayacng cngtntayta ytgygcnaar                                  90
<210> 204
   <211> 90
   <212> DNA
   <213> Homo sapiens
<400> 204
   caggtgcagc tggtgcagtc tggggctgag gtgaagaagc ctggggcctc agtgaaggtc 60
   tcctgcaagg cttctggata caccttcacc                                  90
<210> 205
   <211> 90
   <212> DNA
   <213> Homo sapiens
<400> 205
   caggtccagc ttgtgcagtc tggggctgag gtgaagaagc ctggggcctc agtgaaggtt 60
   tcctgcaagg cttctggata caccttcact                                  90
<210> 206
   <211> 90
   <212> DNA
   <213> Homo sapiens
<400> 206
   caggtgcagc tggtgcagtc tggggctgag gtgaagaagc ctggggcctc agtgaaggtc 60
   tcctgcaagg cttctggata caccttcacc                                  90
<210> 207
   <211> 90
   <212> DNA
   <213> Homo sapiens
<400> 207
   caggttcagc tggtgcagtc tggagctgag gtgaagaagc ctggggcctc agtgaaggtc 60
   tcctgcaagg cttctggtta cacctttacc                                  90
<210> 208
   <211> 90
   <212> DNA
   <213> Homo sapiens
<400> 208
   caggtccagc tggtacagtc tggggctgag gtgaagaagc ctggggcctc agtgaaggtc 60
   tcctgcaagg tttccggata caccctcact                                  90
<210> 209
   <211> 90
   <212> DNA
   <213> Homo sapiens
<400> 209
   cagatgcagc tggtgcagtc tggggctgag gtgaagaaga ctgggtcctc agtgaaggtt 60
   tcctgcaagg cttccggata caccttcacc                                  90
<210> 210
   <211> 90
   <212> DNA
   <213> Homo sapiens
<400> 210
   caggtgcagc tggtgcagtc tggggctgag gtgaagaagc ctggggcctc agtgaaggtt 60
   tcctgcaagg catctggata caccttcacc                                  90
<210> 211
   <211> 90
   <212> DNA
   <213> Homo sapiens
<400> 211
   caaatgcagc tggtgcagtc tgggcctgag gtgaagaagc ctgggacctc agtgaaggtc 60
   tcctgcaagg cttctggatt cacctttact                                  90
<210> 212
   <211> 90
   <212> DNA
   <213> Homo sapiens
<400> 212
   caggtgcagc tggtgcagtc tggggctgag gtgaagaagc ctgggtcctc ggtgaaggtc 60
   tcctgcaagg cttctggagg caccttcagc                                  90
<210> 213
   <211> 90
   <212> DNA
   <213> Homo sapiens
<400> 213
   caggtgcagc tggtgcagtc tggggctgag gtgaagaagc ctgggtcctc ggtgaaggtc 60
   tcctgcaagg cttctggagg caccttcagc                                  90
<210> 214
   <211> 90
   <212> DNA
   <213> Homo sapiens
<400> 214
   gaggtccagc tggtacagtc tggggctgag gtgaagaagc ctggggctac agtgaaaatc 60
   tcctgcaagg tttctggata caccttcacc                                  90
<210> 215
   <211> 90
   <212> DNA
   <213> Homo sapiens
<400> 215
   cagatcacct tgaaggagtc tggtcctacg ctggtgaaac ccacacagac cctcacgctg 60
   acctgcacct tctctgggtt ctcactcagc                                  90
<210> 216
   <211> 90
   <212> DNA
   <213> Homo sapiens
<400> 216
   caggtcacct tgaaggagtc tggtcctgtg ctggtgaaac ccacagagac cctcacgctg 60
   acctgcaccg tctctgggtt ctcactcagc                                  90
<210> 217
   <211> 90
   <212> DNA
   <213> Homo sapiens
<400> 217
   caggtcacct tgaaggagtc tggtcctgcg ctggtgaaac ccacacagac cctcacactg 60
   acctgcacct tctctgggtt ctcactcagc 90
<210> 218
   <211> 90
   <212> DNA
   <213> Homo sapiens
<400> 218
   gaggtgcagc tggtggagtc tgggggaggc ttggtccagc ctggggggtc cctgagactc 60
   tcctgtgcag cctctggatt cacctttagt 90
<210> 219
   <211> 90
   <212> DNA
   <213> Homo sapiens
<400> 219
   gaagtgcagc tggtggagtc tgggggaggc ttggtacagc ctggcaggtc cctgagactc 60
   tcctgtgcag cctctggatt cacctttgat 90
<210> 220
   <211> 90
   <212> DNA
   <213> Homo sapiens
<400> 220
   caggtgcagc tggtggagtc tgggggaggc ttggtcaagc ctggagggtc cctgagactc 60
   tcctgtgcag cctctggatt caccttcagt 90
<210> 221
   <211> 90
   <212> DNA
   <213> Homo sapiens
<400> 221
   gaggtgcagc tggtggagtc tgggggaggc ttggtacagc ctggggggtc cctgagactc 60
   tcctgtgcag cctctggatt caccttcagt 90
<210> 222
   <211> 90
   <212> DNA
   <213> Homo sapiens
<400> 222
   gaggtgcagc tggtggagtc tgggggaggc ttggtaaagc ctggggggtc ccttagactc 60
   tcctgtgcag cctctggatt cactttcagt                                  90
<210> 223
   <211> 90
   <212> DNA
   <213> Homo sapiens
<400> 223
   gaggtgcagc tggtggagtc tgggggaggt gtggtacggc ctggggggtc cctgagactc 60
   tcctgtgcag cctctggatt cacctttgat                                  90
<210> 224
   <211> 90
   <212> DNA
   <213> Homo sapiens
<400> 224
   gaggtgcagc tggtggagtc tgggggaggc ctggtcaagc ctggggggtc cctgagactc 60
   tcctgtgcag cctctggatt caccttcagt                                  90
<210> 225
   <211> 90
   <212> DNA
   <213> Homo sapiens
<400> 225
   gaggtgcagc tgttggagtc tgggggaggc ttggtacagc ctggggggtc cctgagactc 60
   tcctgtgcag cctctggatt cacctttagc                                  90
<210> 226
   <211> 90
   <212> DNA
   <213> Homo sapiens
<400> 226
   caggtgcagc tggtggagtc tgggggaggc gtggtccagc ctgggaggtc cctgagactc 60
   tcctgtgcag cctctggatt caccttcagt                                  90
<210> 227
   <211> 90
   <212> DNA
   <213> Homo sapiens
<400> 227
   caggtgcagc tggtggagtc tgggggaggc gtggtccagc ctgggaggtc cctgagactc 60
   tcctgtgcag cctctggatt caccttcagt                                  90
<210> 228
   <211> 90
   <212> DNA
   <213> Homo sapiens
<400> 228
   caggtgcagc tggtggagtc tgggggaggc gtggtccagc ctgggaggtc cctgagactc 60
   tcctgtgcag cctctggatt caccttcagt 90
<210> 229
   <211> 90
   <212> DNA
   <213> Homo sapiens
<400> 229
   caggtgcagc tggtggagtc tgggggaggc gtggtccagc ctgggaggtc cctgagactc 60
   tcctgtgcag cgtctggatt caccttcagt 90
<210> 230
   <211> 90
   <212> DNA
   <213> Homo sapiens
<400> 230
   gaagtgcagc tggtggagtc tgggggagtc gtggtacagc ctggggggtc cctgagactc 60
   tcctgtgcag cctctggatt cacctttgat 90
<210> 231
   <211> 90
   <212> DNA
   <213> Homo sapiens
<400> 231
   gaggtgcagc tggtggagtc tgggggaggc ttggtacagc ctggggggtc cctgagactc 60
   tcctgtgcag cctctggatt caccttcagt 90
<210> 232
   <211> 90
   <212> DNA
   <213> Homo sapiens

<400> 232
   gaggtgcagc tggtggagtc tgggggaggc ttggtacagc cagggcggtc cctgagactc 60
   tcctgtacag cttctggatt cacctttggt 90
<210> 233
   <211> 90
   <212> DNA
   <213> Homo sapiens
<400> 233
   gaggtgcagc tggtggagac tggaggaggc ttgatccagc ctggggggtc cctgagactc 60
   tcctgtgcag cctctgggtt caccgtcagt                                  90
<210> 234
   <211> 90
   <212> DNA
   <213> Homo sapiens
<400> 234
   gaggtgcagc tggtggagtc tgggggaggc ttggtccagc ctggggggtc cctgagactc 60
   tcctgtgcag cctctggatt caccttcagt                                  90
<210> 235
   <211> 90
   <212> DNA
   <213> Homo sapiens
<400> 235
   gaggtgcagc tggtggagtc tgggggaggc ttggtccagc ctggggggtc cctgagactc 60
   tcctgtgcag cctctggatt caccgtcagt                                  90
<210> 236
   <211> 90
   <212> DNA
   <213> Homo sapiens
<400> 236
   gaggtgcagc tggtggagtc tgggggaggc ttggtccagc ctggagggtc cctgagactc 60
   tcctgtgcag cctctggatt caccttcagt                                  90
<210> 237
   <211> 90
   <212> DNA
   <213> Homo sapiens
<400> 237
   gaggtgcagc tggtggagtc tgggggaggc ttggtccagc ctggggggtc cctgaaactc 60
   tcctgtgcag cctctgggtt caccttcagt                                  90
<210> 238
   <211> 90
   <212> DNA
   <213> Homo sapiens
<400> 238
   gaggtgcagc tggtggagtc cgggggaggc ttagttcagc ctggggggtc cctgagactc 60
   tcctgtgcag cctctggatt caccttcagt 90
<210> 239
   <211> 90
   <212> DNA
   <213> Homo sapiens
<400> 239
   gaggtgcagc tggtggagtc tcggggagtc ttggtacagc ctggggggtc cctgagactc 60
   tcctgtgcag cctctggatt caccgtcagt 90
<210> 240
   <211> 90
   <212> DNA
   <213> Homo sapiens
<400> 240
   caggtgcagc tgcaggagtc gggcccagga ctggtgaagc cttcggggac cctgtccctc 60
   acctgcgctg tctctggtgg ctccatcagc 90
<210> 241
   <211> 90
   <212> DNA
   <213> Homo sapiens
<400> 241
   caggtgcagc tgcaggagtc gggcccagga ctggtgaagc cttcggacac cctgtccctc 60
   acctgcgctg tctctggtta ctccatcagc 90
<210> 242
   <211> 90
   <212> DNA
   <213> Homo sapiens
<400> 242
   caggtgcagc tgcaggagtc gggcccagga ctggtgaagc cttcacagac cctgtccctc 60
   acctgcactg tctctggtgg ctccatcagc 90
<210> 243
   <211> 90
   <212> DNA
   <213> Homo sapiens
<400> 243
   cagctgcagc tgcaggagtc cggctcagga ctggtgaagc cttcacagac cctgtccctc 60
   acctgcgctg tctctggtgg ctccatcagc 90
<210> 244
   <211> 90
   <212> DNA
   <213> Homo sapiens
<400> 244
   caggtgcagc tgcaggagtc gggcccagga ctggtgaagc cttcacagac cctgtccctc 60
   acctgcactg tctctggtgg ctccatcagc                                  90
<210> 245
   <211> 90
   <212> DNA
   <213> Homo sapiens
<400> 245
   caggtgcagc tgcaggagtc gggcccagga ctggtgaagc cttcacagac cctgtccctc 60
   acctgcactg tctctggtgg ctccatcagc                                  90
<210> 246
   <211> 90
   <212> DNA
   <213> Homo sapiens
<400> 246
   caggtgcagc tacagcagtg gggcgcagga ctgttgaagc cttcggagac cctgtccctc 60
   acctgcgctg tctatggtgg gtccttcagt                                  90
<210> 247
   <211> 90
   <212> DNA
   <213> Homo sapiens
<400> 247
   cagctgcagc tgcaggagtc gggcccagga ctggtgaagc cttcggagac cctgtccctc 60
   acctgcactg tctctggtgg ctccatcagc                                  90
<210> 248
   <211> 90
   <212> DNA
   <213> Homo sapiens
<400> 248
   caggtgcagc tgcaggagtc gggcccagga ctggtgaagc cttcggagac cctgtccctc 60
   acctgcactg tctctggtgg ctccatcagt                                  90
<210> 249
   <211> 90
   <212> DNA
   <213> Homo sapiens
<400> 249
   caggtgcagc tgcaggagtc gggcccagga ctggtgaagc cttcggagac cctgtccctc 60
   acctgcactg tctctggtgg ctccgtcagc 90
<210> 250
   <211> 90
   <212> DNA
   <213> Homo sapiens
<400> 250
   caggtgcagc tgcaggagtc gggcccagga ctggtgaagc cttcggagac cctgtccctc 60
   acctgcgctg tctctggtta ctccatcagc 90
<210> 251
   <211> 90
   <212> DNA
   <213> Homo sapiens
<400> 251
   gaggtgcagc tggtgcagtc tggagcagag gtgaaaaagc ccggggagtc tctgaagatc 60
   tcctgtaagg gttctggata cagctttacc 90
<210> 252
   <211> 90
   <212> DNA
   <213> Homo sapiens
<400> 252
   gaagtgcagc tggtgcagtc tggagcagag gtgaaaaagc ccggggagtc tctgaggatc 60
   tcctgtaagg gttctggata cagctttacc 90
<210> 253
   <211> 90
   <212> DNA
   <213> Homo sapiens
<400> 253
   caggtacagc tgcagcagtc aggtccagga ctggtgaagc cctcgcagac cctctcactc 60
   acctgtgcca tctccgggga cagtgtctct 90
<210> 254
   <211> 90
   <212> DNA
   <213> Homo sapiens
<400> 254
   caggtgcagc tggtgcaatc tgggtctgag ttgaagaagc ctggggcctc agtgaaggtt 60
   tcctgcaagg cttctggata caccttcact 90
<210> 255
   <211> 22
   <212> DNA
   <213> Artificial Sequence
<220>
   <223> Description of Artificial Sequence: Probe
<400> 255
   agttctccct gcagctgaac tc 22
<210> 256
   <211> 22
   <212> DNA
   <213> Artificial Sequence
<220>
   <223> Description of Artificial Sequence: Probe
<400> 256
   cactgtatct gcaaatgaac ag 22
<210> 257
   <211> 22
   <212> DNA
   <213> Artificial Sequence
<220>
   <223> Description of Artificial Sequence: Probe
<400> 257
   ccctgtatct gcaaatgaac ag 22
<210> 258
   <211> 22
   <212> DNA
   <213> Artificial Sequence
<220>
   <223> Description of Artificial Sequence: Probe
<400> 258
   ccgcctacct gcagtggagc ag 22
<210> 259
   <211> 22
   <212> DNA
   <213> Artificial Sequence
<220>
   <223> Description of Artificial Sequence: Probe
<400> 259
   cgctgtatct gcaaatgaac ag 22
<210> 260
   <211> 22
   <212> DNA
   <213> Artificial Sequence
<220>
   <223> Description of Artificial Sequence: Probe
<400> 260
   cggcatatct gcagatctgc ag 22
<210> 261
   <211> 22
   <212> DNA
   <213> Artificial Sequence
<220>
   <223> Description of Artificial Sequence: Probe
<400> 261
   cggcgtatct gcaaatgaac ag 22
<210> 262
   <211> 22
   <212> DNA
   <213> Artificial Sequence
<220>
   <223> Description of Artificial Sequence: Probe
<400> 262
   ctgcctacct gcagtggagc ag 22
<210> 263
   <211> 22
   <212> DNA
   <213> Artificial Sequence
<220>
   <223> Description of Artificial Sequence: Probe
<400> 263
   tcgcctatct gcaaatgaac ag 22
<210> 264
   <211> 22
   <212> DNA
   <213> Artificial Sequence
<220>
   <223> Description of Artificial Sequence: Synthetic oligonucleotide
<400> 264
   acatggagct gagcagcctg ag 22
<210> 265
   <211> 22
   <212> DNA
   <213> Artificial Sequence
<220>
   <223> Description of Artificial Sequence: Synthetic oligonucleotide
<400> 265
   acatggagct gagcaggctg ag 22
<210> 266
   <211> 22
   <212> DNA
   <213> Artificial Sequence
<220>
   <223> Description of Artificial Sequence: Synthetic oligonucleotide
<400> 266
   acatggagct gaggagcctg ag 22
<210> 267
   <211> 22
   <212> DNA
   <213> Artificial Sequence
<220>
   <223> Description of Artificial Sequence: Synthetic oligonucleotide
<400> 267
   acctgcagtg gagcagcctg aa 22
<210> 268
   <211> 22
   <212> DNA
   <213> Artificial Sequence
<220>
   <223> Description of Artificial Sequence: Synthetic oligonucleotide
<400> 268
   atctgcaaat gaacagcctg aa 22
<210> 269
   <211> 22
   <212> DNA
   <213> Artificial Sequence
<220>
   <223> Description of Artificial Sequence: Synthetic oligonucleotide
<400> 269
   atctgcaaat gaacagcctg ag 22
<210> 270
   <211> 22
   <212> DNA
   <213> Artificial Sequence
<220>
   <223> Description of Artificial Sequence: Synthetic oligonucleotide
<400> 270
   atctgcaaat gaacagtctg ag 22
<210> 271
   <211> 22
   <212> DNA
   <213> Artificial Sequence
<220>
   <223> Description of Artificial Sequence: Synthetic oligonucleotide
<400> 271
   atctgcagat ctgcagccta aa 22
<210> 272
   <211> 22
   <212> DNA
   <213> Artificial Sequence
<220>
   <223> Description of Artificial Sequence: Synthetic oligonucleotide
<400> 272
   atcttcaaat gaacagcctg ag 22
<210> 273
   <211> 22
   <212> DNA
   <213> Artificial Sequence
<220>
   <223> Description of Artificial Sequence: Synthetic oligonucleotide
<400> 273
   atcttcaaat gggcagcctg ag 22
<210> 274
   <211> 22
   <212> DNA
   <213> Artificial Sequence
<220>
   <223> Description of Artificial Sequence: Synthetic oligonucleotide
<400> 274
   ccctgaagct gagctctgtg ac 22
<210> 275
   <211> 22
   <212> DNA
   <213> Artificial Sequence
<220>
   <223> Description of Artificial Sequence: Synthetic oligonucleotide
<400> 275
   ccctgcagct gaactctgtg ac 22
<210> 276
   <211> 22
   <212> DNA
   <213> Artificial Sequence
<220>
   <223> Description of Artificial Sequence: Synthetic oligonucleotide
<400> 276
   tccttacaat gaccaacatg ga 22
<210> 277
   <211> 22
   <212> DNA
   <213> Artificial Sequence
<220>
   <223> Description of Artificial Sequence: Synthetic oligonucleotide
<400> 277
   tccttaccat gaccaacatg ga 22
<210> 278
   <211> 22
   <212> DNA
   <213> Artificial Sequence
<220>
   <223> Description of Artificial Sequence: Synthetic oligonucleotide
<400> 278
   ccgtgtatta ctgtgcgaga ga 22
<210> 279
   <211> 22
   <212> DNA
   <213> Artificial Sequence
<220>
   <223> Description of Artificial Sequence: Synthetic oligonucleotide
<400> 279
   ctgtgtatta ctgtgcgaga ga 22
<210> 280
   <211> 22
   <212> DNA
   <213> Artificial Sequence
<220>
   <223> Description of Artificial Sequence: Synthetic oligonucleotide
<400> 280
   ccgtgtatta ctgtgcgaga gg 22
<210> 281
   <211> 22
   <212> DNA
   <213> Artificial Sequence
<220>
   <223> Description of Artificial Sequence: Synthetic oligonucleotide
<400> 281
   ccgtgtatta ctgtgcaaca ga 22
<210> 282
   <211> 22
   <212> DNA
   <213> Artificial Sequence
<220>
   <223> Description of Artificial Sequence: Synthetic oligonucleotide
<400> 282
   ccatgtatta ctgtgcaaga ta 22
<210> 283
   <211> 22
   <212> DNA
   <213> Artificial Sequence
<220>
   <223> Description of Artificial Sequence: Synthetic oligonucleotide
<400> 283
   ccgtgtatta ctgtgcggca ga 22
<210> 284
   <211> 22
   <212> DNA
   <213> Artificial Sequence
<220>
   <223> Description of Artificial Sequence: Synthetic oligonucleotide
<400> 284
   ccacatatta ctgtgcacac ag 22
<210> 285
   <211> 22
   <212> DNA
   <213> Artificial Sequence

<220>
   <223> Description of Artificial Sequence: Synthetic oligonucleotide
<400> 285
   ccacatatta ctgtgcacgg at 22
<210> 286
   <211> 22
   <212> DNA
   <213> Artificial Sequence
<220>
   <223> Description of Artificial Sequence: Synthetic oligonucleotide
<400> 286
   ccacgtatta ctgtgcacgg at 22
<210> 287
   <211> 22
   <212> DNA
   <213> Artificial Sequence
<220>
   <223> Description of Artificial Sequence: Synthetic oligonucleotide
<400> 287
   ccttgtatta ctgtgcaaaa ga 22
<210> 288
   <211> 22
   <212> DNA
   <213> Artificial Sequence
<220>
   <223> Description of Artificial Sequence: Synthetic oligonucleotide
<400> 288
   ctgtgtatta ctgtgcaaga ga 22
<210> 289
   <211> 22
   <212> DNA
   <213> Artificial Sequence
<220>
   <223> Description of Artificial Sequence: Synthetic oligonucleotide
<400> 289
   ccgtgtatta ctgtaccaca ga 22
<210> 290
   <211> 22
   <212> DNA
   <213> Artificial Sequence
<220>
   <223> Description of Artificial Sequence: Synthetic oligonucleotide
<400> 290
   ccttgtatca ctgtgcgaga ga 22
<210> 291
   <211> 22
   <212> DNA
   <213> Artificial Sequence
<220>
   <223> Description of Artificial Sequence: Synthetic oligonucleotide
<400> 291
   ccgtatatta ctgtgcgaaa ga 22
<210> 292
   <211> 22
   <212> DNA
   <213> Artificial Sequence
<220>
   <223> Description of Artificial Sequence: Synthetic oligonucleotide
<400> 292
   ctgtgtatta ctgtgcgaaa ga 22
<210> 293
   <211> 22
   <212> DNA
   <213> Artificial Sequence
<220>
   <223> Description of Artificial Sequence: Synthetic oligonucleotide
<400> 293
   ccgtgtatta ctgtactaga ga 22
<210> 294
   <211> 22
   <212> DNA
   <213> Artificial Sequence
<220>
   <223> Description of Artificial Sequence: Synthetic oligonucleotide
<400> 294
   ccgtgtatta ctgtgctaga ga 22
<210> 295
   <211> 22
   <212> DNA
   <213> Artificial Sequence
<220>
   <223> Description of Artificial Sequence: Synthetic oligonucleotide
<400> 295
   ccgtgtatta ctgtactaga ca 22
<210> 296
   <211> 22
   <212> DNA
   <213> Artificial Sequence
<220>
   <223> Description of Artificial Sequence: Synthetic oligonucleotide
<400> 296
   ctgtgtatta ctgtaagaaa ga 22
<210> 297
   <211> 22
   <212> DNA
   <213> Artificial Sequence
<220>
   <223> Description of Artificial Sequence: Synthetic oligonucleotide
<400> 297
   ccgtgtatta ctgtgcgaga aa 22
<210> 298
   <211> 22
   <212> DNA
   <213> Artificial Sequence
<220>
   <223> Description of Artificial Sequence: Synthetic oligonucleotide
<400> 298
   ccgtgtatta ctgtgccaga ga 22
<210> 299
   <211> 22
   <212> DNA
   <213> Artificial Sequence
<220>
   <223> Description of Artificial Sequence: Synthetic oligonucleotide
<400> 299
   ctgtgtatta ctgtgcgaga ca 22
<210> 300
   <211> 22
   <212> DNA
   <213> Artificial Sequence
<220>
   <223> Description of Artificial Sequence: Synthetic oligonucleotide
<400> 300
   ccatgtatta ctgtgcgaga ca 22
<210> 301
   <211> 22
   <212> DNA
   <213> Artificial Sequence
<220>
   <223> Description of Artificial Sequence: Synthetic oligonucleotide
<400> 301
   ccatgtatta ctgtgcgaga aa 22
<210> 302
   <211> 69
   <212> DNA
   <213> Unknown Organism
<220>
   <223> Description of Unknown Organism: Kappa FR1 GLGs
<400> 302
   gacatccaga tgacccagtc tccatcctcc ctgtctgcat ctgtaggaga cagagtcacc 60
   atcacttgc                                                         69
<210> 303
   <211> 66
   <212> DNA
   <213> Unknown Organism
<220>
   <223> Description of Unknown Organism: Lambda FR1 GLG sequence
<400> 303
   cagtctgtgc tgactcagcc accctcggtg tctgaagccc ccaggcagag ggtcaccatc 60
   tcctgt                                                            66
<210> 304
   <211> 668
   <212> DNA
   <213> Unknown Organism
<220>
   <223> Description of Unknown Organism: h3401-h2
<400> 304
   agtgcacaag acatccagat gacccagtct ccagccaccc tgtctgtgtc tccaggggaa 60
   agggccaccc tctcctgcag ggccagtcag agtgttagta acaacttagc ctggtaccag 120
   cagaaacctg gccaggttcc caggctcctc atctatggtg catccaccag ggccactgat 180
   atcccagcca ggttcagtgg cagtgggtct gggacagact tcactctcac catcagcaga 240
   ctggagcctg aagattttgc agtgtattac tgtcagcggt atggtagctc accggggtgg 300
   acgttcggcc aagggaccaa ggtggaaatc aaacgaactg tggctgcacc atctgtcttc 360
   atcttcccgc catctgatga gcagttgaaa tctggaactg cctctgttgt gtgcctgctg 420
   aataacttct atcccagaga ggccaaagta cagtggaagg tggataacgc cctccaatcg 480
   ggtaactccc aggagagtgt cacagagcag gacagcaagg acagcaccta cagcctcagc 540
   agcaccctga cgctgagcaa agcagactac gagaaacaca aagtctacgc ctgcgaagtc 600
   acccatcagg gcctgagctc gcctgtcaca aagagcttca acaaaggaga gtgtaagggc 660
   gaattcgc                                                          668
<210> 305
   <211> 223
   <212> PRT
   <213> Unknown Organism
<220>
   <223> Description of Unknown Organism: h3401-h2
<400> 305
   Ser Ala Gln Asp Ile Gln Met Thr Gln Ser Pro Ala Thr Leu Ser Val
    1               5                  10                  15
   Ser Pro Gly Glu Arg Ala Thr Leu Ser Cys Arg Ala Ser Gln Ser Val
               20                  25                  30
   Ser Asn Asn Leu Ala Trp Tyr Gln Gln Lys Pro Gly Gln Val Pro Arg
           35                  40                  45
   Leu Leu Ile Tyr Gly Ala Ser Thr Arg Ala Thr Asp Ile Pro Ala Arg
       50                  55                  60
   Phe Ser Gly Ser Gly Ser Gly Thr Asp Phe Thr Leu Thr Ile Ser Arg
   65                  70                  75                  80
   Leu Glu Pro Glu Asp Phe Ala Val Tyr Tyr Cys Gln Arg Tyr Gly Ser
                   85                  90                  95
   Ser Pro Gly Trp Thr Phe Gly Gln Gly Thr Lys Val Glu Ile Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Lys Gly Glu Cys Lys Gly Glu Phe Ala
       210                 215                 220
<210> 306
   <211> 700
   <212> DNA
   <213> Unknown Organism
<220>
   <223> Description of Unknown Organism: h3401-d8 KAPPA
<400> 306
   agtgcacaag acatccagat gacccagtct cctgccaccc tgtctgtgtc tccaggtgaa 60
   agagccaccc tctcctgcag ggccagtcag gtgtctccag gggaaagagc caccctctcc 120
   tgcaatcttc tcagcaactt agcctggtac cagcagaaac ctggccaggc tcccaggctc 180
   ctcatctatg gtgcttccac cggggccatt ggtatcccag ccaggttcag tggcagtggg 240
   tctgggacag agttcactct caccatcagc agcctgcagt ctgaagattt tgcagtgtat 300
   ttctgtcagc agtatggtac ctcaccgccc actttcggcg gagggaccaa ggtggagatc 360
   aaacgaactg tggctgcacc atctgtcttc atcttcccgc catctgatga gcagttgaaa 420
   tctggaactg cctctgttgt gtgcccgctg aataacttct atcccagaga ggccaaagta 480
   cagtggaagg tggataacgc cctccaatcg ggtaactccc aggagagtgt cacagagcag 540
   gacaacaagg acagcaccta cagcctcagc agcaccctga cgctgagcaa agtagactac 600
   gagaaacacg aagtctacgc ctgcgaagtc acccatcagg gccttagctc gcccgtcacg 660
   aagagcttca acaggggaga gtgtaagaaa gaattcgttt                      700
<210> 307
   <211> 222
   <212> PRT
   <213> Unknown Organism
<220>
   <223> Description of Unknown Organism: h3401-d8 KAPPA
<400> 307
   Ser Ala Gln Asp Ile Gln Met Thr Gln Ser Pro Ala Thr Leu Ser Val
    1               5                  10                  15
   Ser Pro Gly Glu Arg Ala Thr Leu Ser Cys Arg Ala Ser Gln Asn Leu
               20                  25                  30
   Leu Ser Asn Leu Ala Trp Tyr Gln Gln Lys Pro Gly Gln Ala Pro Arg
           35                  40                  45
   Leu Leu Ile Tyr Gly Ala Ser Thr Gly Ala Ile Gly Ile Pro Ala Arg
       50                  55                  60
   Phe Ser Gly Ser Gly Ser Gly Thr Glu Phe Thr Leu Thr Ile Ser Ser
   65                  70                  75                  80
   Leu Gln Ser Glu Asp Phe Ala Val Tyr Phe Cys Gln Gln Tyr Gly Thr
                   85                  90                  95
   Ser Pro Pro Thr Phe Gly Gly Gly Thr Lys Val Glu Ile Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Pro Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Asn Lys Asp Ser Thr Tyr
                   165                 170                 175
   Ser Leu Ser Ser Thr Leu Thr Leu Ser Lys Val Asp Tyr Glu Lys His
               180                 185                 190
   Glu Val Tyr Ala Cys Glu Val Thr His Gln Gly Leu Ser Ser Pro Val
           195                 200                 205
   Thr Lys Ser Phe Asn Arg Gly Glu Cys Lys Lys Glu Phe Val
       210                 215                 220
<210> 308
   <211> 45
   <212> DNA
   <213> Artificial Sequence
<220>
   <223> Description of Artificial Sequence: Synthetic oligonucleotide
<400> 308
   gctgtgtatt actgtgcgag cacatccgtg ttgttcacgg atgtg 45
<210> 309
   <211> 45
   <212> DNA
   <213> Artificial Sequence
<220>
   <223> Description of Artificial Sequence: Synthetic oligonucleotide
<400> 309
   gccgtgtatt actgtgcgag cacatccgtg ttgttcacgg atgtg 45
<210> 310
   <211> 45
   <212> DNA
   <213> Artificial Sequence
<220>
   <223> Description of Artificial Sequence: Synthetic oligonucleotide
<400> 310
   gccgtatatt actgtgcgag cacatccgtg ttgttcacgg atgtg 45
<210> 311
   <211> 45
   <212> DNA
   <213> Artificial Sequence
<220>
   <223> Description of Artificial Sequence: Synthetic oligonucleotide
<400> 311
   gccgtgtatt actgtacgag cacatccgtg ttgttcacgg atgtg 45
<210> 312
   <211> 45
   <212> DNA
   <213> Artificial Sequence
<220>
   <223> Description of Artificial Sequence: Synthetic oligonucleotide
<400> 312
   gccatgtatt actgtgcgag cacatccgtg ttgttcacgg atgtg 45
<210> 313
   <211> 88
   <212> DNA
   <213> Artificial Sequence
<220>
   <223> Description of Artificial Sequence: Synthetic oligonucleotide
<400> 313
   aatagtagac tgcagtgtcc tcagccctta agctgttcat ctgcaagtag agagtattct 60
   tagagttgtc tctagactta gtgaagcg 88
<210> 314
   <211> 95
   <212> DNA
   <213> Artificial Sequence
<220>
   <223> Description of Artificial Sequence: Synthetic oligonucleotide
<400> 314
   cgcttcacta agtctagaga caactctaag aatactctct acttgcagat gaacagctta 60
   agggctgagg acactgcagt ctactattgt gcgag 95
<210> 315
   <211> 95
   <212> DNA
   <213> Artificial Sequence
<220>
   <223> Description of Artificial Sequence: Synthetic oligonucleotide
<400> 315
   cgcttcacta agtctagaga caactctaag aatactctct acttgcagat gaacagctta 60
   agggctgagg acactgcagt ctactattgt acgag 95
<210> 316
   <211> 24
   <212> DNA
   <213> Artificial Sequence
<220>
   <223> Description of Artificial Sequence: Synthetic oligonucleotide
<400> 316
   cgcttcacta agtctagaga caac 24
<210> 317
   <211> 44
   <212> DNA
   <213> Artificial Sequence
<220>
   <223> Description of Artificial Sequence: Synthetic oligonucleotide
<400> 317
   cacatccgtg ttgttcacgg atgtgggagg atggagactg ggtc 44
<210> 318
   <211> 44
   <212> DNA
   <213> Artificial Sequence
<220>
   <223> Description of Artificial Sequence: Synthetic oligonucleotide
<400> 318
   cacatccgtg ttgttcacgg atgtgggaga gtggagactg agtc 44
<210> 319
   <211> 44
   <212> DNA
   <213> Artificial Sequence
<220>
   <223> Description of Artificial Sequence: Synthetic oligonucleotide
<400> 319
   cacatccgtg ttgttcacgg atgtgggtgc ctggagactg cgtc 44
<210> 320
   <211> 44
   <212> DNA
   <213> Artificial Sequence
<220>
   <223> Description of Artificial Sequence: Synthetic oligonucleotide
<400> 320
   cacatccgtg ttgttcacgg atgtgggtgg ctggagactg cgtc 44
<210> 321
   <211> 34
   <212> DNA
   <213> Artificial Sequence
<220>
   <223> Description of Artificial Sequence: Synthetic oligonucleotide
<400> 321
   cctctactct tgtcacagtg cacaagacat ccag 34
<210> 322
   <211> 20
   <212> DNA
   <213> Artificial Sequence
<220>
   <223> Description of Artificial Sequence: Synthetic oligonucleotide
<400> 322
   cctctactct tgtcacagtg 20
<210> 323
   <211> 44
   <212> DNA
   <213> Artificial Sequence
<220>
   <223> Description of Artificial Sequence: Synthetic oligonucleotide
<400> 323
   ggaggatgga ctggatgtct tgtgcactgt gacaagagta gagg 44
<210> 324
   <211> 44
   <212> DNA
   <213> Artificial Sequence

<220>
   <223> Description of Artificial Sequence: Synthetic oligonucleotide
<400> 324
   ggagagtgga ctggatgtct tgtgcactgt gacaagagta gagg 44
<210> 325
   <211> 44
   <212> DNA
   <213> Artificial Sequence
<220>
   <223> Description of Artificial Sequence: Synthetic oligonucleotide
<400> 325
   ggtgcctgga ctggatgtct tgtgcactgt gacaagagta gagg 44
<210> 326
   <211> 44
   <212> DNA
   <213> Artificial Sequence
<220>
   <223> Description of Artificial Sequence: Synthetic oligonucleotide
<400> 326
   ggtggctgga ctggatgtct tgtgcactgt gacaagagta gagg 44
<210> 327
   <211> 44
   <212> DNA
   <213> Artificial Sequence
<220>
   <223> Description of Artificial Sequence: Synthetic oligonucleotide
<400> 327
   cacatccgtg ttgttcacgg atgtggatcg actgtccagg agac 44
<210> 328
   <211> 44
   <212> DNA
   <213> Artificial Sequence
<220>
   <223> Description of Artificial Sequence: Synthetic oligonucleotide
<400> 328
   cacatccgtg ttgttcacgg atgtggactg tctgtcccaa ggcc 44
<210> 329
   <211> 44
   <212> DNA
   <213> Artificial Sequence
<220>
   <223> Description of Artificial Sequence: Synthetic oligonucleotide
<400> 329
   cacatccgtg ttgttcacgg atgtggactg actgtccagg agac 44
<210> 330
   <211> 44
   <212> DNA
   <213> Artificial Sequence
<220>
   <223> Description of Artificial Sequence: Synthetic oligonucleotide
<400> 330
   cacatccgtg ttgttcacgg atgtggaccc tctgccctgg ggcc 44
<210> 331
   <211> 59
   <212> DNA
   <213> Artificial Sequence
<220>
   <223> Description of Artificial Sequence: Synthetic oligonucleotide
<400> 331
   cctctgactg agtgcacaga gtgctttaac ccaaccggct agtgttagcg gttccccgg 59
<210> 332
   <211> 69
   <212> DNA
   <213> Artificial Sequence
<220>
   <223> Description of Artificial Sequence: Synthetic oligonucleotide
<400> 332
   cctctgactg agtgcacaga gtgctttaac ccaaccggct agtgttagcg gttccccggg 60
   acagtcgat 69
<210> 333
   <211> 69
   <212> DNA
   <213> Artificial Sequence
<220>
   <223> Description of Artificial Sequence: Synthetic oligonucleotide
<400> 333
   cctctgactg agtgcacaga gtgctttaac ccaaccggct agtgttagcg gttccccggg 60
   acagacagt 69
<210> 334
   <211> 69
   <212> DNA
   <213> Artificial Sequence
<220>
   <223> Description of Artificial Sequence: Synthetic oligonucleotide
<400> 334
   cctctgactg agtgcacaga gtgctttaac ccaaccggct agtgttagcg gttccccggg 60
   acagtcagt 69
<210> 335
   <211> 70
   <212> DNA
   <213> Artificial Sequence
<220>
   <223> Description of Artificial Sequence: Synthetic oligonucleotide
<400> 335
   cctctgactg agtgcacaga gtgctttaac ccaaccggct agtgttagcg gtstccccgg 60
   ggcagagggt 70
<210> 336
   <211> 24
   <212> DNA
   <213> Artificial Sequence
<220>
   <223> Description of Artificial Sequence: Synthetic oligonucleotide
<400> 336
   cctctgactg agtgcacaga gtgc 24
<210> 337
   <211> 20
   <212> DNA
   <213> Artificial Sequence
<220>
   <223> Description of Artificial Sequence: Synthetic oligonucleotide
<400> 337
   gggaggatgg agactgggtc 20
<210> 338
   <211> 20
   <212> DNA
   <213> Artificial Sequence
<220>
   <223> Description of Artificial Sequence: Synthetic oligonucleotide
<400> 338
   gggaagatgg agactgggtc 20
<210> 339
   <211> 20
   <212> DNA
   <213> Artificial Sequence
<220>
   <223> Description of Artificial Sequence: Synthetic oligonucleotide
<400> 339
   gggagagtgg agactgagtc 20
<210> 340
   <211> 20
   <212> DNA
   <213> Artificial Sequence
<220>
   <223> Description of Artificial Sequence: Synthetic oligonucleotide
<400> 340
   gggtgcctgg agactgcgtc 20
<210> 341
   <211> 20
   <212> DNA
   <213> Artificial Sequence
<220>
   <223> Description of Artificial Sequence: Synthetic oligonucleotide
<400> 341
   gggtggctgg agactgcgtc 20
<210> 342
   <211> 20
   <212> DNA
   <213> Artificial Sequence
<220>
   <223> Description of Artificial Sequence: Synthetic oligonucleotide
<400> 342
   gggagtctgg agactgggtc 20
<210> 343
   <211> 50
   <212> DNA
   <213> Artificial Sequence
<220>
   <223> Description of Artificial Sequence: Synthetic oligonucleotide
<400> 343
   gggaggatgg agactgggtc atctggatgt cttgtgcact gtgacagagg 50
<210> 344
   <211> 50
   <212> DNA
   <213> Artificial Sequence
<220>
   <223> Description of Artificial Sequence: Synthetic oligonucleotide
<400> 344
   gggaagatgg agactgggtc atctggatgt cttgtgcact gtgacagagg 50
<210> 345
   <211> 50
   <212> DNA
   <213> Artificial Sequence
<220>
   <223> Description of Artificial Sequence: Synthetic oligonucleotide
<400> 345
   gggagagtgg agactgggtc atctggatgt cttgtgcact gtgacagagg 50
<210> 346
   <211> 50
   <212> DNA
   <213> Artificial Sequence
<220>
   <223> Description of Artificial Sequence: Synthetic oligonucleotide
<400> 346
   gggtgcctgg agactgggtc atctggatgt cttgtgcact gtgacagagg 50
<210> 347
   <211> 50
   <212> DNA
   <213> Artificial Sequence
<220>
   <223> Description of Artificial Sequence: Synthetic oligonucleotide
<400> 347
   gggtggctgg agactgggtc atctggatgt cttgtgcact gtgacagagg 50
<210> 348
   <211> 50
   <212> DNA
   <213> Artificial Sequence
<220>
   <223> Description of Artificial Sequence: Synthetic oligonucleotide
<400> 348
   gggagtctgg agactgggtc atctggatgt cttgtgcact gtgacagagg 50
<210> 349
   <211> 42
   <212> DNA
   <213> Artificial Sequence
<220>
   <223> Description of Artificial Sequence: Synthetic oligonucleotide
<400> 349
   cctctgtcac agtgcacaag acatccagat gacccagtct cc 42
<210> 350
   <211> 22
   <212> DNA
   <213> Artificial Sequence
<220>
   <223> Description of Artificial Sequence: Synthetic oligonucleotide
<400> 350
   cctctgtcac agtgcacaag ac 22
<210> 351
   <211> 24
   <212> DNA
   <213> Artificial Sequence
<220>
   <223> Description of Artificial Sequence: Synthetic oligonucleotide
<400> 351
   acactctccc ctgttgaagc tctt 24
<210> 352
   <211> 912
   <212> DNA
   <213> Artificial Sequence
<220>
   <223> Description of Artificial Sequence: Synthetic oligonucleotide
<400> 352
   tccggagctt cagatctgtt tgcctttttg tggggtggtg cagatcgcgt tacggagatc 60
   gaccgactgc ttgagcaaaa gccacgctta actgctgatc aggcatggga tgttattcgc 120
   caaaccagtc gtcaggatct taacctgagg ctttttttac ctactctgca agcagcgaca 180
   tctggtttga cacagagcga tccgcgtcgt cagttggtag aaacattaac acgttgggat 240
   ggcatcaatt tgcttaatga tgatggtaaa acctggcagc agccaggctc tgccatcctg 300
   aacgtttggc tgaccagtat gttgaagcgt accgtagtgg ctgccgtacc tatgccattt 360
   gataagtggt acagcgccag tggctacgaa acaacccagg acggcccaac tggttcgctg 420
   aatataagtg ttggagcaaa aattttgtat gaggcggtgc agggagacaa atcaccaatc 480
   ccacaggcgg ttgatctgtt tgctgggaaa ccacagcagg aggttgtgtt ggctgcgctg 540
   gaagatacct gggagactct ttccaaacgc tatggcaata atgtgagtaa ctggaaaaca 600
   cctgcaatgg ccttaacgtt ccgggcaaat aatttctttg gtgtaccgca ggccgcagcg 660
   gaagaaacgc gtcatcaggc ggagtatcaa aaccgtggaa cagaaaacga tatgattgtt 720
   ttctcaccaa cgacaagcga tcgtcctgtg cttgcctggg atgtggtcgc acccggtcag 780
   agtgggttta ttgctcccga tggaacagtt gataagcact atgaagatca gctgaaaatg 840
   tacgaaaatt ttggccgtaa gtcgctctgg ttaacgaagc aggatgtgga ggcgcataag 900
   gagtcgtcta ga                                                      912
<210> 353
   <211> 6680
   <212> DNA
   <213> Unknown Organism
<220>
   <223> Description of Unknown Organism: pCES5
<400> 353
   gacgaaaggg cctcgtgata cgcctatttt tataggttaa tgtcatgata ataatggttt 60
   cttagacgtc aggtggcact tttcggggaa atgtgcgcgg aacccctatt tgtttatttt 120
   tctaaataca ttcaaatatg tatccgctca tgagacaata accctgataa atgcttcaat 180
   aatattgaaa aaggaagagt atgagtattc aacatttccg tgtcgccctt attccctttt 240
   ttgcggcatt ttgccttcct gtttttgctc acccagaaac gctggtgaaa gtaaaagatg 300
   ctgaagatca gttgggtgcc cgagtgggtt acatcgaact ggatctcaac agcggtaaga 360
   tccttgagag ttttcgcccc gaagaacgtt ttccaatgat gagcactttt aaagttctgc 420
   tatgtggcgc ggtattatcc cgtattgacg ccgggcaaga gcaactcggt cgccgcatac 480
   actattctca gaatgacttg gttgagtact caccagtcac agaaaagcat cttacggatg 540
   gcatgacagt aagagaatta tgcagtgctg ccataaccat gagtgataac actgcggcca 600
   acttacttct gacaacgatc ggaggaccga aggagctaac cgcttttttg cacaacatgg 660
   gggatcatgt aactcgcctt gatcgttggg aaccggagct gaatgaagcc ataccaaacg 720
   acgagcgtga caccacgatg cctgtagcaa tggcaacaac gttgcgcaaa ctattaactg 780
   gcgaactact tactctagct tcccggcaac aattaataga ctggatggag gcggataaag 840
   ttgcaggacc acttctgcgc tcggcccttc cggctggctg gtttattgct gataaatctg 900
   gagccggtga gcgtgggtct cgcggtatca ttgcagcact ggggccagat ggtaagccct 960
   cccgtatcgt agttatctac acgacgggga gtcaggcaac tatggatgaa cgaaatagac 1020
   agatcgctga gataggtgcc tcactgatta agcattggta actgtcagac caagtttact 1080
   catatatact ttagattgat ttaaaacttc atttttaatt taaaaggatc taggtgaaga 1140
   tcctttttga taatctcatg accaaaatcc cttaacgtga gttttcgttc cactgagcgt 1200
   cagaccccgt agaaaagatc aaaggatctt cttgagatcc tttttttctg cgcgtaatct 1260
   gctgcttgca aacaaaaaaa ccaccgctac cagcggtggt ttgtttgccg gatcaagagc 1320
   taccaactct ttttccgaag gtaactggct tcagcagagc gcagatacca aatactgtcc 1380
   ttctagtgta gccgtagtta ggccaccact tcaagaactc tgtagcaccg cctacatacc 1440
   tcgctctgct aatcctgtta ccagtggctg ctgccagtgg cgataagtcg tgtcttaccg 1500
   ggttggactc aagacgatag ttaccggata aggcgcagcg gtcgggctga acggggggtt 1560
   cgtgcataca gcccagcttg gagcgaacga cctacaccga actgagatac ctacagcgtg 1620
   agcattgaga aagcgccacg cttcccgaag ggagaaaggc ggacaggtat ccggtaagcg 1680
   gcagggtcgg aacaggagag cgcacgaggg agcttccagg gggaaacgcc tggtatcttt 1740
   atagtcctgt cgggtttcgc cacctctgac ttgagcgtcg atttttgtga tgctcgtcag 1800
   gggggcggag cctatggaaa aacgccagca acgcggcctt tttacggttc ctggcctttt 1860
   gctggccttt tgctcacatg ttctttcctg cgttatcccc tgattctgtg gataaccgta 1920
   ttaccgcctt tgagtgagct gataccgctc gccgcagccg aacgaccgag cgcagcgagt 1980
   cagtgagcga ggaagcggaa gagcgcccaa tacgcaaacc gcctctcccc gcgcgttggc 2040
   cgattcatta atgcagctgg cacgacaggt ttcccgactg gaaagcgggc agtgagcgca 2100
   acgcaattaa tgtgagttag ctcactcatt aggcacccca ggctttacac tttatgcttc 2160
   cggctcgtat gttgtgtgga attgtgagcg gataacaatt tcacacagga aacagctatg 2220
   accatgatta cgccaagctt tggagccttt tttttggaga ttttcaacgt gaaaaaatta 2280
   ttattcgcaa ttcctttagt tgttcctttc tattctcaca gtgcacaggt ccaactgcag 2340
   gtcgacctcg agatcaaacg tggaactgtg gctgcaccat ctgtcttcat cttcccgcca 2400
   tctgatgagc agttgaaatc tggaactgcc tctgttgtgt gcctgctgaa taacttctat 2460
   cccagagagg ccaaagtaca gtggaaggtg gataacgccc tccaatcggg taactcccag 2520
   gagagtgtca cagagcagga cagcaaggac agcacctaca gcctcagcag caccctgacg 2580
   ctgagcaaag cagactacga gaaacacaaa gtctacgcct gcgaagtcac ccatcagggc 2640
   ctgagttcac cggtgacaaa gagcttcaac aggggagagt gttaataagg cgcgccaatt 2700
   ctatttcaag gagacagtca taatgaaata cctattgcct acggcagccg ctggattgtt 2760
   attactcgcg gcccagccgg ccatggccga agttcaattg ttagagtctg gtggcggtct 2820
   tgttcagcct ggtggttctt tacgtctttc ttgcgctgct tccggagctt cagatctgtt 2880
   tgcctttttg tggggtggtg cagatcgcgt tacggagatc gaccgactgc ttgagcaaaa 2940
   gccacgctta actgctgatc aggcatggga tgttattcgc caaaccagtc gtcaggatct 3000
   taacctgagg ctttttttac ctactctgca agcagcgaca tctggtttga cacagagcga 3060
   tccgcgtcgt cagttggtag aaacattaac acgttgggat ggcatcaatt tgcttaatga 3120
   tgatggtaaa acctggcagc agccaggctc tgccatcctg aacgtttggc tgaccagtat 3180
   gttgaagcgt accgtagtgg ctgccgtacc tatgccattt gataagtggt acagcgccag 3240
   tggctacgaa acaacccagg acggcccaac tggttcgctg aatataagtg ttggagcaaa 3300
   aattttgtat gaggcggtgc agggagacaa atcaccaatc ccacaggcgg ttgatctgtt 3360
   tgctgggaaa ccacagcagg aggttgtgtt ggctgcgctg gaagatacct gggagactct 3420
   ttccaaacgc tatggcaata atgtgagtaa ctggaaaaca cctgcaatgg ccttaacgtt 3480
   ccgggcaaat aatttctttg gtgtaccgca ggccgcagcg gaagaaacgc gtcatcaggc 3540
   ggagtatcaa aaccgtggaa cagaaaacga tatgattgtt ttctcaccaa cgacaagcga 3600
   tcgtcctgtg cttgcctggg atgtggtcgc acccggtcag agtgggttta ttgctcccga 3660
   tggaacagtt gataagcact atgaagatca gctgaaaatg tacgaaaatt ttggccgtaa 3720
   gtcgctctgg ttaacgaagc aggatgtgga ggcgcataag gagtcgtcta gagacaactc 3780
   taagaatact ctctacttgc agatgaacag cttaagtctg agcattcggt ccgggcaaca 3840
   ttctccaaac tgaccagacg acacaaacgg cttacgctaa atcccgcgca tgggatggta 3900
   aagaggtggc gtctttgctg gcctggactc atcagatgaa ggccaaaaat tggcaggagt 3960
   ggacacagca ggcagcgaaa caagcactga ccatcaactg gtactatgct gatgtaaacg 4020
   gcaatattgg ttatgttcat actggtgctt atccagatcg tcaatcaggc catgatccgc 4080
   gattacccgt tcctggtacg ggaaaatggg actggaaagg gctattgcct tttgaaatga 4140
   accctaaggt gtataacccc cagaagctag cctgcggctt cggtcaccgt ctcaagcgcc 4200
   tccaccaagg gcccatcggt cttccccctg gcaccctcct ccaagagcac ctctgggggc 4260
   acagcggccc tgggctgcct ggtcaaggac tacttccccg aaccggtgac ggtgtcgtgg 4320
   aactcaggcg ccctgaccag cggcgtccac accttcccgg ctgtcctaca gtcctcagga 4380
   ctctactccc tcagcagcgt agtgaccgtg ccctccagca gcttgggcac ccagacctac 4440
   atctgcaacg tgaatcacaa gcccagcaac accaaggtgg acaagaaagt tgagcccaaa 4500
   tcttgtgcgg ccgcacatca tcatcaccat cacggggccg cagaacaaaa actcatctca 4560
   gaagaggatc tgaatggggc cgcatagact gttgaaagtt gtttagcaaa acctcataca 4620
   gaaaattcat ttactaacgt ctggaaagac gacaaaactt tagatcgtta cgctaactat 4680
   gagggctgtc tgtggaatgc tacaggcgtt gtggtttgta ctggtgacga aactcagtgt 4740
   tacggtacat gggttcctat tgggcttgct atccctgaaa atgagggtgg tggctctgag 4800
   ggtggcggtt ctgagggtgg cggttctgag ggtggcggta ctaaacctcc tgagtacggt 4860
   gatacaccta ttccgggcta tacttatatc aaccctctcg acggcactta tccgcctggt 4920
   actgagcaaa accccgctaa tcctaatcct tctcttgagg agtctcagcc tcttaatact 4980
   ttcatgtttc agaataatag gttccgaaat aggcagggtg cattaactgt ttatacgggc 5040
   actgttactc aaggcactga ccccgttaaa acttattacc agtacactcc tgtatcatca 5100
   aaagccatgt atgacgctta ctggaacggt aaattcagag actgcgcttt ccattctggc 5160
   tttaatgagg atccattcgt ttgtgaatat caaggccaat cgtctgacct gcctcaacct 5220
   cctgtcaatg ctggcggcgg ctctggtggt ggttctggtg gcggctctga gggtggcggc 5280
   tctgagggtg gcggttctga gggtggcggc tctgagggtg gcggttccgg tggcggctcc 5340
   ggttccggtg attttgatta tgaaaaaatg gcaaacgcta ataagggggc tatgaccgaa 5400
   aatgccgatg aaaacgcgct acagtctgac gctaaaggca aacttgattc tgtcgctact 5460
   gattacggtg ctgctatcga tggtttcatt ggtgacgttt ccggccttgc taatggtaat 5520
   ggtgctactg gtgattttgc tggctctaat tcccaaatgg ctcaagtcgg tgacggtgat 5580
   aattcacctt taatgaataa tttccgtcaa tatttacctt ctttgcctca gtcggttgaa 5640
   tgtcgccctt atgtctttgg cgctggtaaa ccatatgaat tttctattga ttgtgacaaa 5700
   ataaacttat tccgtggtgt ctttgcgttt cttttatatg ttgccacctt tatgtatgta 5760
   ttttcgacgt ttgctaacat actgcgtaat aaggagtctt aataagaatt cactggccgt 5820
   cgttttacaa cgtcgtgact gggaaaaccc tggcgttacc caacttaatc gccttgcagc 5880
   acatccccct ttcgccagct ggcgtaatag cgaagaggcc cgcaccgatc gcccttccca 5940
   acagttgcgc agcctgaatg gcgaatggcg cctgatgcgg tattttctcc ttacgcatct 6000
   gtgcggtatt tcacaccgca tataaattgt aaacgttaat attttgttaa aattcgcgtt 6060
   aaatttttgt taaatcagct cattttttaa ccaataggcc gaaatcggca aaatccctta 6120
   taaatcaaaa gaatagcccg agatagggtt gagtgttgtt ccagtttgga acaagagtcc 6180
   actattaaag aacgtggact ccaacgtcaa agggcgaaaa accgtctatc agggcgatgg 6240
   cccactacgt gaaccatcac ccaaatcaag ttttttgggg tcgaggtgcc gtaaagcact 6300
   aaatcggaac cctaaaggga gcccccgatt tagagcttga cggggaaagc cggcgaacgt 6360
   ggcgagaaag gaagggaaga aagcgaaagg agcgggcgct agggcgctgg caagtgtagc 6420
   ggtcacgctg cgcgtaacca ccacacccgc cgcgcttaat gcgccgctac agggcgcgta 6480
   ctatggttgc tttgacgggt gcagtctcag tacaatctgc tctgatgccg catagttaag 6540
   ccagccccga cacccgccaa cacccgctga cgcgccctga cgggcttgtc tgctcccggc 6600
   atccgcttac agacaagctg tgaccgtctc cgggagctgc atgtgtcaga ggttttcacc 6660
   gtcatcaccg aaacgcgcga                                             6680
<210> 354
   <211> 286
   <212> PRT
   <213> Unknown Organism
<220>
   <223> Description of Unknown Organism: pCES5
<400> 354
   Met Ser Ile Gln His Phe Arg Val Ala Leu Ile Pro Phe Phe Ala Ala
   1               5                   10                  15
   Phe Cys Leu Pro Val Phe Ala His Pro Glu Thr Leu Val Lys Val Lys
               20                  25                  30
   Asp Ala Glu Asp Gln Leu Gly Ala Arg Val Gly Tyr Ile Glu Leu Asp
           35                  40                  45
   Leu Asn Ser Gly Lys Ile Leu Glu Ser Phe Arg Pro Glu Glu Arg Phe
       50                  55                  60
   Pro Met Met Ser Thr Phe Lys Val Leu Leu Cys Gly Ala Val Leu Ser
   65                  70                  75                  80
   Arg Ile Asp Ala Gly Gln Glu Gln Leu Gly Arg Arg Ile His Tyr Ser
                   85                  90                  95
   Gln Asn Asp Leu Val Glu Tyr Ser Pro Val Thr Glu Lys His Leu Thr
               100                 105                 110
   Asp Gly Met Thr Val Arg Glu Leu Cys Ser Ala Ala Ile Thr Met Ser
           115                 120                 125
   Asp Asn Thr Ala Ala Asn Leu Leu Leu Thr Thr Ile Gly Gly Pro Lys
       130                 135                 140
   Glu Leu Thr Ala Phe Leu His Asn Met Gly Asp His Val Thr Arg Leu
   145                 150                 155                 160
   Asp Arg Trp Glu Pro Glu Leu Asn Glu Ala Ile Pro Asn Asp Glu Arg
                   165                 170                 175
   Asp Thr Thr Met Pro Val Ala Met Ala Thr Thr Leu Arg Lys Leu Leu
               180                 185                 190
   Thr Gly Glu Leu Leu Thr Leu Ala Ser Arg Gln Gln Leu Ile Asp Trp
           195                 200                 205
   Met Glu Ala Asp Lys Val Ala Gly Pro Leu Leu Arg Ser Ala Leu Pro
       210                 215                 220
   Ala Gly Trp Phe Ile Ala Asp Lys Ser Gly Ala Gly Glu Arg Gly Ser
   225                 230                 235                 240
   Arg Gly Ile Ile Ala Ala Leu Gly Pro Asp Gly Lys Pro Ser Arg Ile
                   245                 250                 255
   Val Val Ile Tyr Thr Thr Gly Ser Gln Ala Thr Met Asp Glu Arg Asn
               260                 265                 270
   Arg Gln Ile Ala Glu Ile Gly Ala Ser Leu Ile Lys His Trp
           275                 280                 285
<210> 355
   <211> 138
   <212> PRT
   <213> Unknown Organism
<220>
   <223> Description of Unknown Organism: pCES5
<400> 355

<210> 356
   <211> 48
   <212> PRT
   <213> Unknown Organism
<220>
   <223> Description of Unknown Organism: pCES5
<400> 356
   Met Lys Tyr Leu Leu Pro Thr Ala Ala Ala Gly Leu Leu Leu Leu Ala
   1               5                   10                  15
   Ala Gln Pro Ala Met Ala Glu Val Gln Leu Leu Glu Ser Gly Gly Gly
               20                  25                  30
   Leu Val Gln Pro Gly Gly Ser Leu Arg Leu Ser Cys Ala Ala Ser Gly
           35                  40                  45
<210> 357
   <211> 28
   <212> PRT
   <213> Unknown Organism
<220>
   <223> Description of Unknown Organism: pCES5
<400> 357
   Ser Arg Asp Asn Ser Lys Asn Thr Leu Tyr Leu Gln Met Asn Ser Leu
   1               5                   10                  15
   Ser Leu Ser Ile Arg Ser Gly Gln His Ser Pro Thr
               20                  25
<210> 358
   <211> 129
   <212> PRT
   <213> Unknown Organism
<220>
   <223> Description of Unknown Organism: pCES5
<400> 358
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Ala Ala Ala His His His His His His
               100                 105                 110
   Gly Ala Ala Glu Gln Lys Leu Ile Ser Glu Glu Asp Leu Asn Gly Ala
           115                 120                 125
   Ala
<210> 359
   <211> 404
   <212> PRT
   <213> Unknown Organism
<220>
   <223> Description of Unknown Organism: pCES5
<400> 359
   Thr Val Glu Ser Cys Leu Ala Lys Pro His Thr Glu Asn Ser Phe Thr
    1               5                  10                  15
   Asn Val Trp Lys Asp Asp Lys Thr Leu Asp Arg Tyr Ala Asn Tyr Glu
               20                  25                  30
   Gly Cys Leu Trp Asn Ala Thr Gly Val Val Val Cys Thr Gly Asp Glu
           35                  40                  45
   Thr Gln Cys Tyr Gly Thr Trp Val Pro Ile Gly Leu Ala Ile Pro Glu
       50                  55                  60
   Asn Glu Gly Gly Gly Ser Glu Gly Gly Gly Ser Glu Gly Gly Gly Ser
   65                  70                  75                  80
   Glu Gly Gly Gly Thr Lys Pro Pro Glu Tyr Gly Asp Thr Pro Ile Pro
                   85                  90                  95
   Gly Tyr Thr Tyr Ile Asn Pro Leu Asp Gly Thr Tyr Pro Pro Gly Thr
               100                 105                 110
   Glu Gln Asn Pro Ala Asn Pro Asn Pro Ser Leu Glu Glu Ser Gln Pro
           115                 120                 125
   Leu Asn Thr Phe Met Phe Gln Asn Asn Arg Phe Arg Asn Arg Gln Gly
       130                 135                 140
   Ala Leu Thr Val Tyr Thr Gly Thr Val Thr Gln Gly Thr Asp Pro Val
   145                 150                 155                 160
   Lys Thr Tyr Tyr Gln Tyr Thr Pro Val Ser Ser Lys Ala Met Tyr Asp
                   165                 170                 175
   Ala Tyr Trp Asn Gly Lys Phe Arg Asp Cys Ala Phe His Ser Gly Phe
   180 185 190
   Asn Glu Asp Pro Phe Val Cys Glu Tyr Gln Gly Gln Ser Ser Asp Leu
   195 200 205
   Pro Gln Pro Pro Val Asn Ala Gly Gly Gly Ser Gly Gly Gly Ser Gly
   210 215 220
   Gly Gly Ser Glu Gly Gly Gly Ser Glu Gly Gly Gly Ser Glu Gly Gly
   225 230 235 240
   Gly Ser Glu Gly Gly Gly Ser Gly Gly Gly Ser Gly Ser Gly Asp Phe
   245 250 255
   Asp Tyr Glu Lys Met Ala Asn Ala Asn Lys Gly Ala Met Thr Glu Asn
   260 265 270
   Ala Asp Glu Asn Ala Leu Gln Ser Asp Ala Lys Gly Lys Leu Asp Ser
   275 280 285
   Val Ala Thr Asp Tyr Gly Ala Ala Ile Asp Gly Phe Ile Gly Asp Val
   290 295 300
   Ser Gly Leu Ala Asn Gly Asn Gly Ala Thr Gly Asp Phe Ala Gly Ser
   305 310 315 320
   Asn Ser Gln Met Ala Gln Val Gly Asp Gly Asp Asn Ser Pro Leu Met
   325 330 335
   Asn Asn Phe Arg Gln Tyr Leu Pro Ser Leu Pro Gln Ser Val Glu Cys
   340 345 350
   Arg Pro Tyr Val Phe Gly Ala Gly Lys Pro Tyr Glu Phe Ser Ile Asp
   355 360 365
   Cys Asp Lys Ile Asn Leu Phe Arg Gly Val Phe Ala Phe Leu Leu Tyr
   370 375 380
   Val Ala Thr Phe Met Tyr Val Phe Ser Thr Phe Ala Asn Ile Leu Arg
   385 390 395 400
   Asn Lys Glu Ser
<210> 360
   <211> 69
   <212> DNA
   <213> Unknown Organism
<220>
   <223> Description of Unknown Organism: Kappa FR1 GLGs
<400> 360
   gacatccaga tgacccagtc tccatcctcc ctgtctgcat ctgtaggaga cagagtcacc 60
   atcacttgc 69
<210> 361
   <211> 69
   <212> DNA
   <213> Unknown Organism
<220>
   <223> Description of Unknown Organism: Kappa FR1 GLGs
<400> 361
   gacatccaga tgacccagtc tccatcctcc ctgtctgcat ctgtaggaga cagagtcacc 60
   atcacttgc                                                         69
<210> 362
   <211> 69
   <212> DNA
   <213> Unknown Organism
<220>
   <223> Description of Unknown Organism: Kappa FR1 GLGs
<400> 362
   gacatccaga tgacccagtc tccatcctcc ctgtctgcat ctgtaggaga cagagtcacc 60
   atcacttgc                                                         69
<210> 363
   <211> 69
   <212> DNA
   <213> Unknown Organism
<220>
   <223> Description of Unknown Organism: Kappa FR1 GLGs
<400> 363
   gacatccaga tgacccagtc tccatcctcc ctgtctgcat ctgtaggaga cagagtcacc 60
   atcacttgc                                                         69
<210> 364
   <211> 69
   <212> DNA
   <213> Unknown Organism
<220>
   <223> Description of Unknown Organism: Kappa FR1 GLGs
<400> 364
   gacatccaga tgacccagtc tccatcctcc ctgtctgcat ctgtaggaga cagagtcacc 60
   atcacttgc                                                         69
<210> 365
   <211> 69
   <212> DNA
   <213> Unknown Organism
<220>
   <223> Description of Unknown Organism: Kappa FR1 GLGs
<400> 365
   aacatccaga tgacccagtc tccatctgcc atgtctgcat ctgtaggaga cagagtcacc 60
   atcacttgt                                                         69
<210> 366
   <211> 69
   <212> DNA
   <213> Unknown Organism
<220>
   <223> Description of Unknown Organism: Kappa FR1 GLGs
<400> 366
   gacatccaga tgacccagtc tccatcctca ctgtctgcat ctgtaggaga cagagtcacc 60
   atcacttgt                                                         69
<210> 367
   <211> 69
   <212> DNA
   <213> Unknown Organism
<220>
   <223> Description of Unknown Organism: Kappa FR1 GLGs
<400> 367
   gacatccaga tgacccagtc tccatcctca ctgtctgcat ctgtaggaga cagagtcacc 60
   atcacttgt                                                         69
<210> 368
   <211> 69
   <212> DNA
   <213> Unknown Organism
<220>
   <223> Description of Unknown Organism: Kappa FR1 GLGs
<400> 368
   gccatccagt tgacccagtc tccatcctcc ctgtctgcat ctgtaggaga cagagtcacc 60
   atcacttgc                                                         69
<210> 369
   <211> 69
   <212> DNA
   <213> Unknown Organism
<220>
   <223> Description of Unknown Organism: Kappa FR1 GLGs
<400> 369
   gccatccagt tgacccagtc tccatcctcc ctgtctgcat ctgtaggaga cagagtcacc 60
   atcacttgc 69
<210> 370
   <211> 69
   <212> DNA
   <213> Unknown Organism
<220>
   <223> Description of Unknown Organism: Kappa FR1 GLGs
<400> 370
   gacatccaga tgacccagtc tccatcttcc gtgtctgcat ctgtaggaga cagagtcacc 60
   atcacttgt 69
<210> 371
   <211> 69
   <212> DNA
   <213> Unknown Organism
<220>
   <223> Description of Unknown Organism: Kappa FR1 GLGs
<400> 371
   gacatccaga tgacccagtc tccatcttct gtgtctgcat ctgtaggaga cagagtcacc 60
   atcacttgt 69
<210> 372
   <211> 69
   <212> DNA
   <213> Unknown Organism
<220>
   <223> Description of Unknown Organism: Kappa FR1 GLGs
<400> 372
   gacatccagt tgacccagtc tccatccttc ctgtctgcat ctgtaggaga cagagtcacc 60
   atcacttgc 69
<210> 373
   <211> 69
   <212> DNA
   <213> Unknown Organism
<220>
   <223> Description of Unknown Organism: Kappa FR1 GLGs
<400> 373
   gccatccgga tgacccagtc tccattctcc ctgtctgcat ctgtaggaga cagagtcacc 60
   atcacttgc 69
<210> 374
   <211> 69
   <212> DNA
   <213> Unknown Organism
<220>
   <223> Description of Unknown Organism: Kappa FR1 GLGs
<400> 374
   gccatccgga tgacccagtc tccatcctca ttctctgcat ctacaggaga cagagtcacc 60
   atcacttgt 69
<210> 375
   <211> 69
   <212> DNA
   <213> Unknown Organism
<220>
   <223> Description of Unknown Organism: Kappa FR1 GLGs
<400> 375
   gtcatctgga tgacccagtc tccatcctta ctctctgcat ctacaggaga cagagtcacc 60
   atcagttgt 69
<210> 376
   <211> 69
   <212> DNA
   <213> Unknown Organism
<220>
   <223> Description of Unknown Organism: Kappa FR1 GLGs
<400> 376
   gccatccaga tgacccagtc tccatcctcc ctgtctgcat ctgtaggaga cagagtcacc 60
   atcacttgc 69
<210> 377
   <211> 69
   <212> DNA
   <213> Unknown Organism
<220>
   <223> Description of Unknown Organism: Kappa FR1 GLGs
<400> 377
   gacatccaga tgacccagtc tccttccacc ctgtctgcat ctgtaggaga cagagtcacc 60
   atcacttgc 69
<210> 378
   <211> 69
   <212> DNA
   <213> Unknown Organism
<220>
   <223> Description of Unknown Organism: Kappa FR1 GLGs
<400> 378
   gatattgtga tgacccagac tccactctcc ctgcccgtca cccctggaga gccggcctcc 60
   atctcctgc 69
<210> 379
   <211> 69
   <212> DNA
   <213> Unknown Organism
<220>
   <223> Description of Unknown Organism: Kappa FR1 GLGs
<400> 379
   gatattgtga tgacccagac tccactctcc ctgcccgtca cccctggaga gccggcctcc 60
   atctcctgc 69
<210> 380
   <211> 69
   <212> DNA
   <213> Unknown Organism
<220>
   <223> Description of Unknown Organism: Kappa FR1 GLGs
<400> 380
   gatgttgtga tgactcagtc tccactctcc ctgcccgtca cccttggaca gccggcctcc 60
   atctcctgc 69
<210> 381
   <211> 69
   <212> DNA
   <213> Unknown Organism
<220>
   <223> Description of Unknown Organism: Kappa FR1 GLGs
<400> 381
   gatgttgtga tgactcagtc tccactctcc ctgcccgtca cccttggaca gccggcctcc 60
   atctcctgc 69
<210> 382
   <211> 69
   <212> DNA
   <213> Unknown Organism
<220>
   <223> Description of Unknown Organism: Kappa FR1 GLGs
<400> 382
   gatattgtga tgacccagac tccactctct ctgtccgtca cccctggaca gccggcctcc 60
   atctcctgc 69
<210> 383
   <211> 69
   <212> DNA
   <213> Unknown Organism
<220>
   <223> Description of Unknown Organism: Kappa FR1 GLGs
<400> 383
   gatattgtga tgacccagac tccactctct ctgtccgtca cccctggaca gccggcctcc 60
   atctcctgc 69
<210> 384
   <211> 69
   <212> DNA
   <213> Unknown Organism
<220>
   <223> Description of Unknown Organism: Kappa FR1 GLGs
<400> 384
   gatattgtga tgactcagtc tccactctcc ctgcccgtca cccctggaga gccggcctcc 60
   atctcctgc 69
<210> 385
   <211> 69
   <212> DNA
   <213> Unknown Organism
<220>
   <223> Description of Unknown Organism: Kappa FR1 GLGs
<400> 385
   gatattgtga tgactcagtc tccactctcc ctgcccgtca cccctggaga gccggcctcc 60
   atctcctgc 69
<210> 386
   <211> 69
   <212> DNA
   <213> Unknown Organism
<220>
   <223> Description of Unknown Organism: Kappa FR1 GLGs
<400> 386
   gatattgtga tgacccagac tccactctcc tcacctgtca cccttggaca gccggcctcc 60
   atctcctgc 69
<210> 387
   <211> 69
   <212> DNA
   <213> Unknown Organism
<220>
   <223> Description of Unknown Organism: Kappa FR1 GLGs
<400> 387
   gaaattgtgt tgacgcagtc tccaggcacc ctgtctttgt ctccagggga aagagccacc 60
   ctctcctgc 69
<210> 388
   <211> 69
   <212> DNA
   <213> Unknown Organism
<220>
   <223> Description of Unknown Organism: Kappa FR1 GLGs
<400> 388
   gaaattgtgt tgacgcagtc tccagccacc ctgtctttgt ctccagggga aagagccacc 60
   ctctcctgc 69
<210> 389
   <211> 69
   <212> DNA
   <213> Unknown Organism
<220>
   <223> Description of Unknown Organism: Kappa FR1 GLGs
<400> 389
   gaaatagtga tgacgcagtc tccagccacc ctgtctgtgt ctccagggga aagagccacc 60
   ctctcctgc                                                         69
<210> 390
   <211> 69
   <212> DNA
   <213> Unknown Organism
<220>
   <223> Description of Unknown Organism: Kappa FR1 GLGs
<400> 390
   gaaatagtga tgacgcagtc tccagccacc ctgtctgtgt ctccagggga aagagccacc 60
   ctctcctgc                                                         69
<210> 391
   <211> 69
   <212> DNA
   <213> Unknown Organism
<220>
   <223> Description of Unknown Organism: Kappa FR1 GLGs
<400> 391
   gaaattgtgt tgacacagtc tccagccacc ctgtctttgt ctccagggga aagagccacc 60
   ctctcctgc                                                         69
<210> 392
   <211> 69
   <212> DNA
   <213> Unknown Organism
<220>
   <223> Description of Unknown Organism: Kappa FR1 GLGs
<400> 392
   gaaattgtgt tgacacagtc tccagccacc ctgtctttgt ctccagggga aagagccacc 60
   ctctcctgc                                                         69
<210> 393
   <211> 69
   <212> DNA
   <213> Unknown Organism
<220>
   <223> Description of Unknown Organism: Kappa FR1 GLGs
<400> 393
   gaaattgtaa tgacacagtc tccagccacc ctgtctttgt ctccagggga aagagccacc 60
   ctctcctgc 69
<210> 394
   <211> 69
   <212> DNA
   <213> Unknown Organism

<220>
   <223> Description of Unknown Organism: Kappa FR1 GLGs
<400> 394
   gacatcgtga tgacccagtc tccagactcc ctggctgtgt ctctgggcga gagggccacc 60
   atcaactgc 69
<210> 395
   <211> 69
   <212> DNA
   <213> Unknown Organism
<220>
   <223> Description of Unknown Organism: Kappa FR1 GLGs
<400> 395
   gaaacgacac tcacgcagtc tccagcattc atgtcagcga ctccaggaga caaagtcaac 60
   atctcctgc 69
<210> 396
   <211> 69
   <212> DNA
   <213> Unknown Organism
<220>
   <223> Description of Unknown Organism: Kappa FR1 GLGs
<400> 396
   gaaattgtgc tgactcagtc tccagacttt cagtctgtga ctccaaagga gaaagtcacc 60
   atcacctgc 69
<210> 397
   <211> 69
   <212> DNA
   <213> Unknown Organism
<220>
   <223> Description of Unknown Organism: Kappa FR1 GLGs
<400> 397
   gaaattgtgc tgactcagtc tccagacttt cagtctgtga ctccaaagga gaaagtcacc 60
   atcacctgc                                                         69
<210> 398
   <211> 69
   <212> DNA
   <213> Unknown Organism
<220>
   <223> Description of Unknown Organism: Kappa FR1 GLGs
<400> 398
   gatgttgtga tgacacagtc tccagctttc ctctctgtga ctccagggga gaaagtcacc 60
   atcacctgc                                                         69
<210> 399
   <211> 66
   <212> DNA
   <213> Unknown Organism
<220>
   <223> Description of Unknown Organism: Lambda FR1 GLG sequence
<400> 399
   cagtctgtgc tgacgcagcc gccctcagtg tctggggccc cagggcagag ggtcaccatc 60
   tcctgc                                                            66
<210> 400
   <211> 66
   <212> DNA
   <213> Unknown Organism
<220>
   <223> Description of Unknown Organism: Lambda FR1 GLG sequence
<400> 400
   cagtctgtgc tgactcagcc accctcagcg tctgggaccc ccgggcagag ggtcaccatc 60
   tcttgt                                                            66
<210> 401
   <211> 66
   <212> DNA
   <213> Unknown Organism
<220>
   <223> Description of Unknown Organism: Lambda FR1 GLG sequence
<400> 401
   cagtctgtgc tgactcagcc accctcagcg tctgggaccc ccgggcagag ggtcaccatc 60
   tcttgt                                                            66
<210> 402
   <211> 66
   <212> DNA
   <213> Unknown Organism
<220>
   <223> Description of Unknown Organism: Lambda FR1 GLG sequence
<400> 402
   cagtctgtgt tgacgcagcc gccctcagtg tctgcggccc caggacagaa ggtcaccatc 60
   tcctgc                                                            66
<210> 403
   <211> 66
   <212> DNA
   <213> Unknown Organism
<220>
   <223> Description of Unknown Organism: Lambda FR1 GLG sequence
<400> 403
   cagtctgccc tgactcagcc tccctccgcg tccgggtctc ctggacagtc agtcaccatc 60
   tcctgc                                                            66
<210> 404
   <211> 66
   <212> DNA
   <213> Unknown Organism
<220>
   <223> Description of Unknown Organism: Lambda FR1 GLG sequence
<400> 404
   cagtctgccc tgactcagcc tcgctcagtg tccgggtctc ctggacagtc agtcaccatc 60
   tcctgc                                                            66
<210> 405
   <211> 66
   <212> DNA
   <213> Unknown Organism
<220>
   <223> Description of Unknown Organism: Lambda FR1 GLG sequence
<400> 405
   cagtctgccc tgactcagcc tgcctccgtg tctgggtctc ctggacagtc gatcaccatc 60
   tcctgc 66
<210> 406
   <211> 66
   <212> DNA
   <213> Unknown Organism
<220>
   <223> Description of Unknown Organism: Lambda FR1 GLG sequence
<400> 406
   cagtctgccc tgactcagcc tccctccgtg tccgggtctc ctggacagtc agtcaccatc 60
   tcctgc 66
<210> 407
   <211> 66
   <212> DNA
   <213> Unknown Organism
<220>
   <223> Description of Unknown Organism: Lambda FR1 GLG sequence
<400> 407
   cagtctgccc tgactcagcc tgcctccgtg tctgggtctc ctggacagtc gatcaccatc 60
   tcctgc 66
<210> 408
   <211> 66
   <212> DNA
   <213> Unknown Organism
<220>
   <223> Description of Unknown Organism: Lambda FR1 GLG sequence
<400> 408
   tcctatgagc tgactcagcc accctcagtg tccgtgtccc caggacagac agccagcatc 60
   acctgc 66
<210> 409
   <211> 66
   <212> DNA
   <213> Unknown Organism
<220>
   <223> Description of Unknown Organism: Lambda FR1 GLG sequence
<400> 409
   tcctatgagc tgactcagcc actctcagtg tcagtggccc tgggacagac ggccaggatt 60
   acctgt 66
<210> 410
   <211> 66
   <212> DNA
   <213> Unknown Organism
<220>
   <223> Description of Unknown Organism: Lambda FR1 GLG sequence
<400> 410
   tcctatgagc tgacacagcc accctcggtg tcagtgtccc caggacaaac ggccaggatc 60
   acctgc 66
<210> 411
   <211> 66
   <212> DNA
   <213> Unknown Organism
<220>
   <223> Description of Unknown Organism: Lambda FR1 GLG sequence
<400> 411
   tcctatgagc tgacacagcc accctcggtg tcagtgtccc taggacagat ggccaggatc 60
   acctgc 66
<210> 412
   <211> 66
   <212> DNA
   <213> Unknown Organism
<220>
   <223> Description of Unknown Organism: Lambda FR1 GLG sequence
<400> 412
   tcttctgagc tgactcagga ccctgctgtg tctgtggcct tgggacagac agtcaggatc 60
   acatgc 66
<210> 413
   <211> 66
   <212> DNA
   <213> Unknown Organism
<220>
   <223> Description of Unknown Organism: Lambda FR1 GLG sequence
<400> 413
   tcctatgtgc tgactcagcc accctcagtg tcagtggccc caggaaagac ggccaggatt 60
   acctgt                                                            66
<210> 414
   <211> 66
   <212> DNA
   <213> Unknown Organism
<220>
   <223> Description of Unknown Organism: Lambda FR1 GLG sequence
<400> 414
   tcctatgagc tgacacagct accctcggtg tcagtgtccc caggacagac agccaggatc 60
   acctgc                                                            66
<210> 415
   <211> 66
   <212> DNA
   <213> Unknown Organism
<220>
   <223> Description of Unknown Organism: Lambda FR1 GLG sequence
<400> 415
   tcctatgagc tgatgcagcc accctcggtg tcagtgtccc caggacagac ggccaggatc 60
   acctgc                                                            66
<210> 416
   <211> 66
   <212> DNA
   <213> Unknown Organism
<220>
   <223> Description of Unknown Organism: Lambda FR1 GLG sequence
<400> 416
   tcctatgagc tgacacagcc atcctcagtg tcagtgtctc cgggacagac agccaggatc 60
   acctgc                                                            66
<210> 417
   <211> 66
   <212> DNA
   <213> Unknown Organism
<220>
   <223> Description of Unknown Organism: Lambda FR1 GLG sequence
<400> 417
   ctgcctgtgc tgactcagcc cccgtctgca tctgccttgc tgggagcctc gatcaagctc 60
   acctgc                                                            66
<210> 418
   <211> 66
   <212> DNA
   <213> Unknown Organism
<220>
   <223> Description of Unknown Organism: Lambda FR1 GLG sequence
<400> 418
   cagcctgtgc tgactcaatc atcctctgcc tctgcttccc tgggatcctc ggtcaagctc 60
   acctgc                                                            66
<210> 419
   <211> 66
   <212> DNA
   <213> Unknown Organism
<220>
   <223> Description of Unknown Organism: Lambda FR1 GLG sequence
<400> 419
   cagcttgtgc tgactcaatc gccctctgcc tctgcctccc tgggagcctc ggtcaagctc 60
   acctgc                                                            66
<210> 420
   <211> 66
   <212> DNA
   <213> Unknown Organism
<220>
   <223> Description of Unknown Organism: Lambda FR1 GLG sequence
<400> 420
   cagcctgtgc tgactcagcc accttcctcc tccgcatctc ctggagaatc cgccagactc 60
   acctgc                                                            66
<210> 421
   <211> 66
   <212> DNA
   <213> Unknown Organism
<220>
   <223> Description of Unknown Organism: Lambda FR1 GLG sequence
<400> 421
   caggctgtgc tgactcagcc ggcttccctc tctgcatctc ctggagcatc agccagtctc 60
   acctgc                                                            66
<210> 422
   <211> 66
   <212> DNA
   <213> Unknown Organism
<220>
   <223> Description of Unknown Organism: Lambda FR1 GLG sequence
<400> 422
   cagcctgtgc tgactcagcc atcttcccat tctgcatctt ctggagcatc agtcagactc 60
   acctgc                                                            66
<210> 423
   <211> 66
   <212> DNA
   <213> Unknown Organism
<220>
   <223> Description of Unknown Organism: Lambda FR1 GLG sequence
<400> 423
   aattttatgc tgactcagcc ccactctgtg tcggagtctc cggggaagac ggtaaccatc 60
   tcctgc                                                            66
<210> 424
   <211> 66
   <212> DNA
   <213> Unknown Organism
<220>
   <223> Description of Unknown Organism: Lambda FR1 GLG sequence
<400> 424
   cagactgtgg tgactcagga gccctcactg actgtgtccc caggagggac agtcactctc 60
   acctgt                                                            66
<210> 425
   <211> 66
   <212> DNA
   <213> Unknown Organism
<220>
   <223> Description of Unknown Organism: Lambda FR1 GLG sequence
<400> 425
   caggctgtgg tgactcagga gccctcactg actgtgtccc caggagggac agtcactctc 60
   acctgt                                                            66
<210> 426
   <211> 66
   <212> DNA
   <213> Unknown Organism
<220>
   <223> Description of Unknown Organism: Lambda FR1 GLG sequence
<400> 426
   cagactgtgg tgacccagga gccatcgttc tcagtgtccc ctggagggac agtcacactc 60
   acttgt                                                            66
<210> 427
   <211> 66
   <212> DNA
   <213> Unknown Organism
<220>
   <223> Description of Unknown Organism: Lambda FR1 GLG sequence
<400> 427
   cagcctgtgc tgactcagcc accttctgca tcagcctccc tgggagcctc ggtcacactc 60
   acctgc                                                            66
<210> 428
   <211> 66
   <212> DNA
   <213> Unknown Organism
<220>
   <223> Description of Unknown Organism: Lambda FR1 GLG sequence
<400> 428
   caggcagggc tgactcagcc accctcggtg tccaagggct tgagacagac cgccacactc 60
   acctgc                                                            66

## Description

The present disclosure relates to constructing libraries of genetic packages that display a member of a diverse family of peptides, polypeptides or proteins and collectively display at least a portion of the diversity of the family. In a preferred embodiment, the displayed polypeptides are human Fabs.

More specifically, the disclosure is directed to the methods of cleaving single-stranded nucleic acids at chosen locations, the cleaved nucleic acids encoding, at least in part, the peptides, polypeptides or proteins displayed on the genetic packages of the libraries of the disclosure. In a preferred embodiment, the genetic packages are filamentous phage or phagemids.

The present disclosure further relates to methods of screening the libraries of genetic packages that display useful peptides, polypeptides and proteins and to the peptides, polypeptides and proteins identified by such screening.

### BACKGROUND OF THE INVENTION

It is now common practice in the art to prepare libraries of genetic packages that display a member of a diverse family of peptides, polypeptides or proteins and collectively display at least a portion of the diversity of the family. In many common libraries, the displayed peptides, polypeptides or proteins are related to antibodies. Often, they are Fabs or single chain antibodies.

In general, the DNAs that encode members of the families to be displayed must be amplified before they are cloned and used to display the desired member on the surface of a genetic package. Such amplification typically makes use of forward and backward primers.

Such primers can be complementary to sequences native to the DNA to be amplified or complementary to oligonucleotides attached at the 5' or 3' ends of that DNA. Primers that are complementary to sequences native to the DNA to be amplified are disadvantaged in that they bias the members of the families to be displayed. Only those members that contain a sequence in the native DNA that is substantially complementary to the primer will be amplified. Those that do not will be absent from the family. For those members that are amplified, any diversity within the primer region will be suppressed.

For example, in European patent 368,684 B1, the primer that is used is at the 5' end of the V_{H} region of an antibody gene. It anneals to a sequence region in the native DNA that is said to be "sufficiently well conserved" within a single species. Such primer will bias the members amplified to those having this "conserved" region. Any diversity within this region is extinguished.

It is generally accepted that human antibody genes arise through a process that involves a combinatorial selection of V and J or V, D, and J followed by somatic mutations. Although most diversity occurs in the Complementary Determining Regions (CDRs), diversity also occurs in the more conserved Framework Regions (FRs) and at least some of this diversity confers or enhances specific binding to antigens (Ag). As a consequence, libraries should contain as much of the CDR and FR diversity as possible.

To clone the amplified DNAs for display on a genetic package of the peptides, polypeptides or proteins that they encode, the DNAs must be cleaved to produce appropriate ends for ligation to a vector. Such cleavage is generally effected using restriction endonuclease recognition sites carried on the primers. When the primers are at the 5' end of DNA produced from reverse transcription of RNA, such restriction leaves deleterious 5' untranslated regions in the amplified DNA. These regions interfere with expression of the cloned genes and thus the display of the peptides, polypeptides and proteins coded for by them.

### SUNMARY OF THE INVENTION

The invention relates to the embodiments as defined in the claims.

It is an object of this disclosure to provide novel methods for constructing libraries of genetic packages that display a member of a diverse family of peptides, polypeptides or proteins and collectively display at least a portion of the diversity of the family. These methods are not biased toward DNAs that contain native sequences that are complementary to the primers used for amplification. They also enable any sequences that may be deleterious to expression to be removed from the amplified DNA before cloning and displaying.

It is another object of this disclosure to provide a method for cleaving single-stranded nucleic acid sequences at a desired location, the method comprising the steps of:
(i) contacting the nucleic acid with a single-stranded oligonucleotide, the oligonucleotide being functionally complementary to the nucleic acid in the region in which cleavage is desired and including a sequence that with its complement in the nucleic acid forms a restriction endonuclease recognition site that on restriction results in cleavage of the nucleic acid at the desired location; and
(ii) cleaving the nucleic acid solely at the recognition site formed by the complementation of the nucleic acid and the oligonucleotide;
the contacting and the cleaving steps being performed at a temperature sufficient to maintain the nucleic acid in substantially single-stranded form, the oligonucleotide being functionally complementary to the nucleic acid over a large enough region to allow the two strands to associate such that cleavage may occur at the chosen temperature and at the desired location, and the cleavage being carried out using a restriction endonuclease that is active at the chosen temperature.

It is a further object of this disclosure to provide an alternative method for cleaving single-stranded nucleic acid sequences at a desired location, the method comprising the steps of:
(i) contacting the nucleic acid with a partially double-stranded oligonucleotide, the single-stranded region of the oligonucleotide being functionally complementary to the nucleic acid in the region in which cleavage is desired, and the double-stranded region of the oligonucleotide having a Type II-S restriction endonuclease recognition site, whose cleavage site is located at a known distance from the recognition site; and
(ii) cleaving the nucleic acid solely at the cleavage site formed by the complementation of the nucleic acid and the single-stranded region of the oligonucleotide;
the contacting and the cleaving steps being performed at a temperature sufficient to maintain the nucleic acid in substantially single-stranded form, the oligonucleotide being functionally complementary to the nucleic acid over a large enough region to allow the two strands to associate such that cleavage may occur at the chosen temperature and at the desired location, and the cleavage being carried out using a restriction endonuclease that is active at the chosen temperature.

It is another objective of the present disclosure to provide a method of capturing DNA molecules that comprise a member of a diverse family of DNAs and collectively comprise at least a portion of the diversity of the family. These DNA molecules in single-stranded form have been cleaved by one of the methods of this disclosure . This method involves ligating the individual single-stranded DNA members of the family to a partially duplex DNA complex. The method comprises the steps of:
(i) contacting a single-stranded nucleic acid sequence that has been cleaved with a restriction endonuclease with a partially double-stranded oligonucleotide, the single-stranded region of the oligonucleotide being functionally complementary to the nucleic acid in the region that remains after cleavage, the double-stranded region of the oligonucleotide including any sequences necessary to return the sequences that remain after cleavage into proper reading frame for expression and containing a restriction endonuclease recognition site 5' of those sequences; and
(ii) cleaving the partially double-stranded oligonucleotide sequence solely at the restriction endonuclease recognition site contained within the double-stranded region of the partially double-stranded oligonucleotide.

It is another object of this disclosure to prepare libraries, that display a diverse family of peptides, polypeptides or proteins and collectively display at least part of the diversity of the family, using the methods and DNAs described above.

It is an object of this disclosure to screen those libraries to identify useful peptides, polypeptides and proteins and to use those substances in human therapy.

### BRIEF DESCRIPTION OF THE DRAWINGS

FIG. 1 is a schematic of various methods that may be employed to amplify VH genes without using primers specific for VH sequences.
FIG. 2 is a schematic of various methods that may be employed to amplify VL genes without using VL sequences.
FIG. 3 depicts gel analysis of cleaved kappa DNA from Example 2.
FIG. 4 depicts gel analysis of cleaved kappa DNA from Example 2.
FIG. 5 depicts gel analysis of amplified kappa DNA from Example 2.
FIG. 6 depicts gel purified amplified kappa DNA from Example 2.

### TERMS

In this application, the following terms and abbreviations are used:
- Sense strand: The upper strand of ds DNA as usually written. In the sense strand, 5'-ATG-3' codes for Met.
- Antisense strand: The lower strand of ds DNA as usually written. In the antisense strand, 3'-TAC-5' would correspond to a Met codon in the sense strand.
- Forward primer:: A "forward" primer is complementary to a part of the sense strand and primes for synthesis of a new antisense-strand molecule. "Forward primer" and "lower-strand primer" are equivalent.
- Backward primer:: A "backward" primer is complementary to a part of the antisense strand and primes for synthesis of a new sense-strand molecule. "Backward primer" and "top-strand primer" are equivalent.
- Bases:: Bases are specified either by their position in a vector or gene as their position within a gene by codon and base. For example, "89.1" is the first base of codon 89, 89.2 is the second base of codon 89.
- Sv: Streptavidin
- Ap: Ampicillin
- *ap^{R}*: A gene conferring ampicillin resistance.
- RE: Restriction endonuclease
- URE: Universal restriction endonuclease
- Functionally complementary: Two sequences are sufficiently complementary so as to anneal under the chosen conditions.
- RERS: Restriction endonuclease recognition site
- AA: Amino acid
- PCR: Polymerization chain reaction
- GLGs: Germline genes
- Ab: Antibody: an immunoglobin. The term also covers any protein having a binding domain which is homologous to an immunoglobin binding domain. A few examples of antibodies within this definition are, *inter alia,* immunoglobin isotypes and the Fab, F(ab¹)₂, scfv, Fv, dAb and Fd fragments.
- Fab: Two chain molecule comprising an Ab light chain and part of a heavy-chain.
- scFv: A single-chain Ab comprising either VH::linker::VL or VL::linker::VH
- w.t.: Wild type
- HC: Heavy chain
- LC: Light chain
- VK: A variable domain of a Kappa light chain.
- VH: A variable domain of a heavy chain.
- VL: A variable domain of a lambda light chain.

### DETAILED DESCRIPTION OF THE PREFERRED EMBODIMENTS

The nucleic acid sequences that are useful in the methods of this disclosure, i.e., those that encode at least in part the individual peptides, polypeptides and proteins displayed on the genetic packages of this disclosure:, may be naturally occurring, synthetic or a combination thereof. They may be mRNA, DNA or cDNA. In the preferred embodiment, the nucleic acids encode antibodies. Most preferably, they encode Fabs.

The nucleic acids useful in this disclosure may be naturally diverse, synthetic diversity may be introduced into those naturally diverse members, or the diversity may be entirely synthetic. For example, synthetic diversity can be introduced into one or more CDRs of antibody genes.

Synthetic diversity may be created, for example, through the use of TRIM technology (U.S. 5,869,644). TRIM technology allows control over exactly which amino-acid types are allowed at variegated positions and in what proportions. In TRIM technology, codons to be diversified are synthesized using mixtures of trinucleotides. This allows any set of amino acid types to be included in any proportion.

Another alternative that may be used to generate diversified DNA is mixed oligonucleotide synthesis. With TRIM technology, one could allow Ala and Trp. With mixed oligonucleotide synthesis, a mixture that included Ala and Trp would also necessarily include Ser and Gly. The amino-acid types allowed at the variegated positions are picked with reference to the structure of antibodies, or other peptides, polypeptides or proteins of the family, the observed diversity in germline genes, the observed somatic mutations frequently observed, and the desired areas and types of variegation.

In a preferred embodiment of this disclosure, the nucleic acid sequences for at least one CDR or other region of the peptides, polypeptides or proteins of the family are cDNAs produced by reverse transcription from mRNA. More preferably, the mRNAs are obtained from peripheral blood cells, bone marrow cells, spleen cells or lymph node cells (such as B-lymphocytes or plasma cells) that express members of naturally diverse sets of related genes. More preferable, the mRNAs encode a diverse family of antibodies. Most preferably, the mRNAs are obtained from patients suffering from at least one autoimmune disorder or cancer. Preferably, mRNAs containing a high diversity of autoimmune diseases, such as systemic lupus erythematosus, systemic sclerosis, rheumatoid arthritis, antiphospholipid syndrome and vasculitis are used.

In a preferred embodiment of this disclosure, the cDNAs are produced from the mRNAs using reverse transcription. In this preferred embodiment, the mRNAs are separated from the cell and degraded using standard methods, such that only the full length (i.e., capped) mRNAs remain. The cap is then removed and reverse transcription used to produce the cDNAs.

The reverse transcription of the first (antisense) strand can be done in any manner with any suitable primer. See, e.g., HJ de Haard et al., Journal of Biological Chemistry, 274(26):18218-30 (1999). In the preferred embodiment of this disclosure where the mRNAs encode antibodies, primers that are complementary to the constant regions of antibody genes may be used. Those primers are useful because they do not generate bias toward subclasses of antibodies. In another embodiment, poly-dT primers may be used (and may be preferred for the heavy-chain genes). Alternatively, sequences complementary to the primer may be attached to the termini of the antisense strand.

In one preferred embodiment of this disclosure, the reverse transcriptase primer may be biotinylated, thus allowing the cDNA product to be immobilized on streptavidin (Sv) beads. Immobilization can also be effected using a primer labeled at the 5' end with one of a) free amine group, b) thiol, c) carboxylic acid, or d) another group not found in DNA that can react to form a strong bond to a known partner on an insoluble medium. If, for example, a free amine (preferably primary amine) is provided at the 5' end of a DNA primer, this amine can be reacted with carboxylic acid groups on a polymer bead using standard amide-forming chemistry. If such preferred immobilization is used during reverse transcription, the top strand RNA is degraded using well-known enzymes, such as a combination of RNAseH and RNAseA, either before or after immobilization.

The nucleic acid sequences useful in the methods of this disclosure are generally amplified before being used to display the peptides, polypeptides or proteins that they encode. Prior to amplification, the single-stranded DNAs may be cleaved using either of the methods described before. Alternatively, the single-stranded DNAs may be amplified and then cleaved using one of those methods.

Any of the well known methods for amplifying nucleic acid sequences may be used for such amplification. Methods that maximize, and do not bias, diversity are preferred. In a preferred embodiment of this disclosure where the nucleic acid sequences are derived from antibody genes, the present disclosure preferably utilizes primers in the constant regions of the heavy and light chain genes and primers to a synthetic sequence that are attached at the 5' end of the sense strand. Priming at such synthetic sequence avoids the use of sequences within the variable regions of the antibody genes. Those variable region priming sites generate bias against V genes that are either of rare subclasses or that have been mutated at the priming sites. This bias is partly due to suppression of diversity within the primer region and partly due to lack of priming when many mutations are present in the region complementary to the primer. The methods disclosed have the advantage of not biasing the population of amplified antibody genes for particular V gene types.

The synthetic sequences may be attached to the 5' end of the DNA strand by various methods well known for ligating DNA sequences together. RT CapExtention is one preferred method.

In RT CapExtention (derived from Smart PCR^{(™)}), a short overlap (5'-...GGG-3' in the upper-strand primer (USP-GGG) complements 3'-CCC....5' in the lower strand) and reverse transcriptases are used so that the reverse complement of the upper-strand primer is attached to the lower strand.

In a preferred embodiment of this disclosure, the upper strand or lower strand primer may be also biotinylated or labeled at the 5' end with one of a) free amino group, b) thiol, c) carboxylic acid and d) another group not found in DNA that can react to form a strong bond to a known partner as an insoluble medium. These can then be used to immobilize the labeled strand after amplification. The immobilized DNA can be either single or double-stranded.

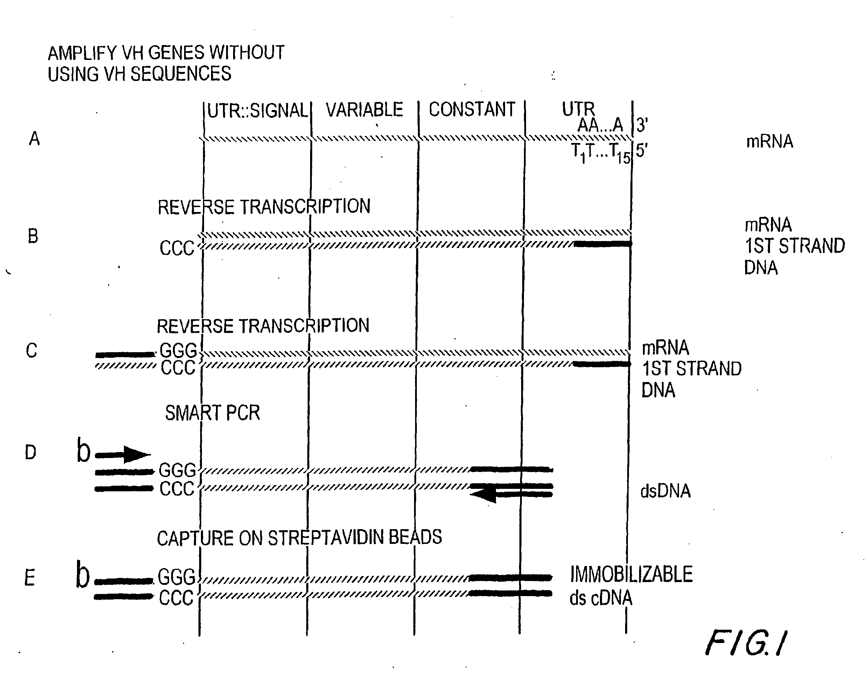

FIG. 1 shows a schematic of the amplification of VH genes. FIG. 1, Panel A shows a primer specific to the poly-dT region of the 3' UTR priming synthesis of the first, lower strand. Primers that bind in the constant region are also suitable. Panel B shows the lower strand extended at its 3' end by three Cs that are not complementary to the mRNA. Panel C shows the result of annealing a synthetic top-strand primer ending in three GGGs that hybridize to the 3' terminal CCCs and extending the reverse transcription extending the lower strand by the reverse complement of the synthetic primer sequence. Panel D shows the result of PCR amplification using a 5' biotinylated synthetic top-strand primer that replicates the 5' end of the synthetic primer of panel C and a bottom-strand primer complementary to part of the constant domain. Panel E shows immobilized double-stranded (ds) cDNA obtained by using a 5'-biotinylated top-strand primer.

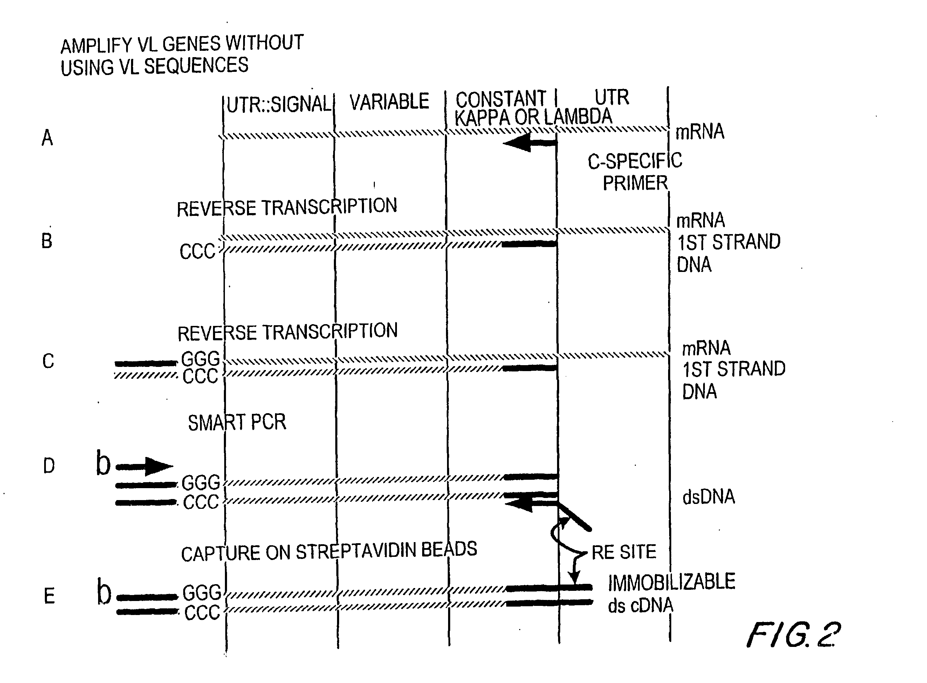

FIG. 2 shows a similar schematic for amplification of VL genes. FIG. 2, Panel A shows a primer specific to the constant region at or near the 3' end priming synthesis of the first, lower strand. Primers that bind in the poly-dT region are also suitable. Panel B shows the lower strand extended at its 3' end by three Cs that are not complementary to the mRNA. Panel C shows the result of annealing a synthetic top-strand primer ending in three GGGs that hybridize to the 3' terminal CCCs and extending the reverse transcription extending the lower strand by the reverse complement of the synthetic primer sequence. Panel D shows the result of PCR amplification using a 5' biotinylated synthetic top-strand primer that replicates the 5' end of the synthetic primer of panel C and a bottom-strand primer complementary to part of the constant domain. The bottom-strand primer also contains a useful restriction endonuclease site, such as AscI. Panel E shows immobilized ds cDNA obtained by using a 5'-biotinylated top-strand primer.

In FIGs. 1 and 2, each V gene consists of a 5' untranslated region (UTR) and a secretion signal, followed by the variable region, followed by a constant region, followed by a 3' untranslated region (which typically ends in poly-A). An initial primer for reverse transcription may be complementary to the constant region or to the poly A segment of the 3'-UTR. For human heavy-chain genes, a primer of 15 T is preferred. Reverse transcriptases attach several C residues to the 3' end of the newly synthesized DNA. RT CapExtention exploits this feature. The reverse transcription reaction is first run with only a lower-strand primer. After about 1 hour, a primer ending in GGG (USP-GGG) and more RTase are added. This causes the lower-strand cDNA to be extended by the reverse complement of the USP-GGG up to the final GGG. Using one primer identical to part of the attached synthetic sequence and a second primer complementary to a region of known sequence at the 3' end of the sense strand, all the V. genes are amplified irrespective of their V gene subclass.

After amplification, the DNAs of this disclosure are rendered single-stranded. For example, the strands can be separated by using a biotinylated primer, capturing the biotinylated product on streptavidin beads, denaturing the DNA, and washing away the complementary strand. Depending on which end of the captured DNA is wanted, one will choose to immobilize either the upper (sense) strand or the lower (antisense) strand.

To prepare the single-stranded amplified DNAs for cloning into genetic packages so as to effect display of the peptides, polypeptides or proteins encoded, at least in part, by those DNAs, they must be manipulated to provide ends suitable for cloning and expression. In particular, any 5' untranslated regions and mammalian signal sequences must be removed and replaced, in frame, by a suitable signal sequence that functions in the display host. Additionally, parts of the variable domains (in antibody genes) may be removed and replaced by synthetic segments containing synthetic diversity. The diversity of other gene families may likewise be expanded with synthetic diversity.

According to the methods of this disclosure, there are two ways to manipulate the single-stranded amplified DNAs for cloning. The first method comprises the steps of:
(i) contacting the nucleic acid with a single-stranded oligonucleotide, the oligonucleotide being functionally complementary to the nucleic acid in the region in which cleavage is desired and including a sequence that with its complement in the nucleic acid forms a restriction endonuclease recognition site that on restriction results in cleavage of the nucleic acid at the desired location; and
(ii) cleaving the nucleic acid solely at the recognition site formed by the complementation of the nucleic acid and the oligonucleotide;
the contacting and the cleaving steps being performed at a temperature sufficient to maintain the nucleic acid in substantially single-stranded form, the oligonucleotide being functionally complementary to the nucleic acid over a large enough region to allow the two strands to associate such that cleavage may occur at the chosen temperature and at the desired location, and the cleavage being carried out using a restriction endonuclease that is active at the chosen temperature.

In this first method, short oligonucleotides are annealed to the single-stranded DNA so that restriction endonuclease recognition sites formed within the now locally double-stranded regions of the DNA can be cleaved. In particular, a recognition site that occurs at the same position in a substantial fraction of the single-stranded DNAs is identical.

For antibody genes, this can be done using a catalog of germline sequences. See, e.g., "http://www.mrc-cpe.cam.ac.uk/imt-doc/restricted/ok.htm 1." Updates can be obtained from this site under the heading "Amino acid and nucleotide sequence alignments." For other families, similar comparisons exist and may be used to select appropriate regions for cleavage and to maintain diversity.

For example, Table 195 depicts the DNA sequences of the FR3 regions of the 51 known human VH germline genes. In this region, the genes contain restriction endonuclease recognition sites shown in Table 200. Restriction endonucleases that cleave a large fraction of germline genes at the same site are preferred over endonucleases that cut at a variety of sites. Furthermore, it is preferred that there be only one site for the restriction endonucleases within the region to which the short oligonucleotide binds on the single-stranded DNA, e.g., about 10 bases on either side of the restriction endonuclease recognition site.

An enzyme that cleaves downstream in FR3 is also more preferable because it captures fewer mutations in the framework. This may be advantageous is some cases. However, it is well known that framework mutations exist and confer and enhance antibody binding. The present disclosure, by choice of appropriate restriction site, allows all or part of FR3 diversity to be captured. Hence, the method also allows extensive diversity to be captured.

Finally, in the methods of this disclosure restriction endonucleases that are active between about 45° and about 75°C are used. Preferably enzymes that are active above 50°C, and more preferably active about 55°C, are used. Such temperatures maintain the nucleic acid sequence to be cleaved in substantially single-stranded form.

Enzymes shown in Table 200 that cut many of the heavy chain FR3 germline genes at a single position include: *Mae*III(24@4), *Tsp*45I(21@4), *Hph*I(44@5), *Bsa*JI (23@65), *Alu*I (23@47), *Blp*I (21@48), *Dde*I (29@58), *Bgl*II (10@61), *Msl*I (44@72), *Bsi*EI (23@74), *Eae*I (23@74), *Eag*I(23@74), *Hae*III(25@75), *Bst*4CI(51@86), *Hpy*CH4III(51@86), *Hin*fI(38@2), *Mly*I(18@2), *Ple*I (18@2), *Mnl*I(31@67), *Hpy*CH4V(21@44), *Bsm*AI(16@11), *Bpm*I(19@12), *Xmn*I(12@30), and *Sac*I(11@51). (The notation used means, for example that *Bsm*AI cuts 16 of the FR3 germline genes with a restriction endonuclease recognition site beginning at base 11 of FR3.)

For cleavage of human heavy chains in FR3, the preferred restriction endonucleases are: *Bst*4CI (or TaaI or *Hpy*CH4III), *Blp*I, *Hpy*CH4V, and *Msl*I. Because ACNGT (the restriction endonuclease recognition site for *Bs*t4CI, *Taa*I, and *Hpy*CH4III) is found at a consistent site in all the human FR3 germline genes, one of those enzymes is the most preferred for capture of heavy chain CDR3 diversity. *Blp*I and *Hpy*CH4V are complementary. *Blp*I cuts most members of the VH1 and VH4 families while *Hpy*CH4V cuts most members of the VH3, VH5, VH6, and VH7 families. Neither enzyme cuts VH2s, but this is a very small family, containing only three members. Thus, these enzymes may also be used in preferred embodiments of the methods of this disclosure..

The restriction endonucleases *Hpy*CH4III, *Bst*4CI, and *Taa*I all recognize 5'-ACnGT-3' and cut upper strand DNA after n and lower strand DNA before the base complementary to n. This is the most preferred restriction endonuclease recognition site for this method on human heavy chains because it is found in all germline genes. Furthermore, the restriction endonuclease recognition region (ACnGT) matches the second and third bases of a tyrosine codon (tay) and the following cysteine codon (tgy) as shown in Table 206. These codons are highly conserved, especially the cysteine in mature antibody genes.

Table 250 E shows the distinct oligonucleotides of length 22 (except the last one which is of length 20) bases. Table 255 C shows the analysis of 1617 actual heavy chain antibody genes. Of these, 1511 have the site and match one of the candidate oligonucleotides to within 4 mismatches. Eight oligonucleotides account for most of the matches and are given in Table 250 F.1. The 8 oligonucleotides are very similar so that it is likely that satisfactory cleavage will be achieved with only one oligonucleotide (such as H43.77.97.1-02#1) by adjusting temperature, pH, salinity, and the like. One or two oligonucleotides may likewise suffice whenever the germline gene sequences differ very little and especially if they differ very little close to the restriction endonuclease recognition region to be cleaved. Table 255 D shows a repeat analysis of 1617 actual heavy chain antibody genes using only the 8 chosen oligonucleotides. This shows that 1463 of the sequences match at least one of the oligonucleotides to within 4 mismatches and have the site as expected. Only 7 sequences have a second HpyCH4III restriction endonuclease recognition region in this region.

Another illustration of choosing an appropriate restriction endonuclease recognition site involves cleavage in FR1 of human heavy chains. Cleavage in FR1 allows capture of the entire CDR diversity of the heavy chain.

The germline genes for human heavy chain FR1 are shown in Table 217. Table 220 shows the restriction endonuclease recognition sites found in human germline genes FR1s. The preferred sites are *Bsg*I (GTGCAG; 39@4), *Bso*FI (GCngc;43@6,11@9,2@3,1@12), *Tse*I (Gcwgc;43@6,11@9,2@3,1@12), *Msp*A1I(CMGckg;46@7,2@1), *Pvu*II(CAGctg;46@7,2@1), *Alu*I(AGct;48@82@2), *Dde*I(Ctnag;22@52,9@48), *Hph*I(tcacc;22@80), *Bss*KI(Nccngg;35@39,2@40), *Bsa*JI(Ccnngg;32@40,2@41), *Bst*NI(CCwgg;33@40), *Scr*FI(CCngg;35@40,2@41), *Eco*O109I(RGgnccy;22@46, 11@43), *Sau*96I(Ggncc;23@47,11@44), AvaII(Ggwcc;23@47,4@44), *Ppu*MI(RGgwccy;22@46,4@43), *Bsm*FI(gtccc;20@48), *Hin*fI (Gantc;34@16,21@56,21@77), *Tfi*I(21@77), *Mly*I*(*GAGTC; 34@16), *Mly*I(gactc; 21@56), and *Alw*NI(CAGnnnctg;22@68). The more preferred sites are *MspAI* and *PvuII.* MspAI and PvuII have 46 sites at 7-12 and 2 at 1-6. To avoid cleavage at both sites, oligonucleotides are used that do not fully cover the site at 1-6. Thus, the DNA will not be cleaved at that site. We have shown that DNA that extends 3, 4, or 5 bases beyond a PvuII-site can be cleaved efficiently.

Another illustration of choosing an appropriate restriction endonuclease recognition site involves cleavage in FR1 of human kappa light chains. Table 300 shows the human kappa FR1 germline genes and Table 302 shows restriction endonuclease recognition sites that are found in a substantial number of human kappa FR1 germline genes at consistent locations. Of the restriction endonuclease recognition sites listed, *Bsm*AI and *Pfl*FI are the most preferred enzymes. BsmAI sites are found at base 18 in 35 of 40 germline genes. PflFI sites are found in 35 of 40 germline genes at base 12.

Another example of choosing an appropriate restriction endonuclease recognition site involves cleavage in FR1 of the human lambda light chain. Table 400 shows the 31 known human lambda FR1 germline gene sequences. Table 405 shows restriction endonuclease recognition sites found in human lambda FR1 germline genes. HinfI and DdeI are the most preferred restriction endonucleases for cutting human lambda chains in FR1.

After the appropriate site or sites for cleavage are chosen, one or more short oligonucleotides are prepared so as to functionally complement, alone or in combination, the chosen recognition site. The oligonucleotides also include sequences that flank the recognition site in the majority of the amplified genes. This flanking region allows the sequence to anneal to the single-stranded DNA sufficiently to allow cleavage by the restriction endonuclease specific for the site chosen.

The actual length and sequence of the oligonucleotide depends on the recognition site and the conditions to be used for contacting and cleavage. The length must be sufficient so that the oligonucleotide is functionally complementary to the single-stranded DNA over a large enough region to allow the two strands to associate such that cleavage may occur at the chosen temperature and solely at the desired location.

Typically, the oligonucleotides of this preferred method of the disclosure are about 17 to about 30 nucleotides in length. Below about 17 bases, annealing is too weak and above 30 bases there can be a loss of specificity. A preferred length is 18 to 24 bases.

Oligonucleotides of this length need not be identical complements of the germline genes. Rather, a few mismatches taken may be tolerated. Preferably, however, no more than 1-3 mismatches are allowed. Such mismatches do not adversely affect annealing of the oligonucleotide to the single-stranded DNA. Hence, the two DNAs are said to be functionally complementary.

The second method to manipulate the amplified single-stranded DNAs of this disclosure for cloning comprises the steps of:
(i) contacting the nucleic acid with a partially double-stranded oligonucleotide, the single-stranded region of the oligonucleotide being functionally complementary to the nucleic acid in the region in which cleavage is desired, and the double-stranded region of the oligonucleotide having a Type II-S restriction endonuclease recognition site, whose cleavage site is located at a known distance from the recognition site; and
(ii) cleaving the nucleic acid solely at the cleavage site formed by the complementation of the nucleic acid and the single-stranded region of the oligonucleotide;
the contacting and the cleaving steps being performed at a temperature sufficient to maintain the nucleic acid in substantially single-stranded form, the oligonucleotide being functionally complementary to the nucleic acid over a large enough region to allow the two strands to associate such that cleavage may occur at the chosen temperature and at the desired location, and the cleavage being carried out using a restriction endonuclease that is active at the chosen temperature.

This second method employs Universal Restriction Endonucleases ("URE"). UREs are partially double-stranded oligonucleotides. The single-stranded portion or overlap of the URE consists of a DNA adapter that is functionally complementary to the sequence to be cleaved in the single-stranded DNA. The double-stranded portion consists of a type II-S restriction endonuclease recognition site.

The URE method of this disclosure is specific and precise and can tolerate some (e.g., 1-3) mismatches in the complementary regions, i.e., it is functionally complementary to that region. Further, conditions under which the URE is used can be adjusted so that most of the genes that are amplified can be cut, reducing bias in the library produced from those genes.

The sequence of the single-stranded DNA adapter or overlap portion of the URE typically consists of about 14-22 bases. However, longer or shorter adapters may be used. The size depends on the ability of the adapter to associate with its functional complement in the single-stranded DNA and the temperature used for contacting the URE and the single-stranded DNA at the temperature used for cleaving the DNA with the type II-S enzyme. The adapter must be functionally complementary to the single-stranded DNA over a large enough region to allow the two strands to associate such that the cleavage may occur at the chosen temperature and at the desired location. We prefer singe-stranded or overlap portions of 14-17 bases in length, and more preferably 18-20 bases in length.

The site chosen for cleavage using the URE is preferably one that is substantially conserved in the family of amplified DNAs. As compared to the first cleavage method of this disclosure, these sites do not need to be endonuclease recognition sites. However, like the first method, the sites chosen can be synthetic rather than existing in the native DNA. Such sites may be chosen by references to the sequences of known antibodies or other families of genes. For example, the sequences of many germline genes are reported at http://www.mrc-cpe.cam.ac.uk/imt-doc/restricted/ok.html. For example, one preferred site occurs near the end of FR3 -- codon 89 through the second base of codon 93. CDR3 begins at codon 95.

The sequences of 79 human heavy-chain genes are also available at http://www.ncbi.nlm.nih.gov/entre2/nucleotide.html. This site can be used to identify appropriate sequences for URE cleavage according to the methods of this disclosure. See, *e.g*., Table 8B.

Most preferably, one or more sequences are identified using these sites or other available sequence information. These sequences together are present in a substantial fraction of the amplified DNAs. For example, multiple sequences could be used to allow for known diversity in germline genes or for frequent somatic mutations. Synthetic degenerate sequences could also be used. Preferably, a sequence(s) that occurs in at least 65% of genes examined with no more than 2-3 mismatches is chosen

URE single-stranded adapters or overlaps are then made to be complementary to the chosen regions. Conditions for using the UREs are determined empirically. These conditions should allow cleavage of DNA that contains the functionally complementary sequences with no more than 2 or 3 mismatches but that do not allow cleavage of DNA lacking such sequences.

As described above, the double-stranded portion of the URE includes a Type II-S endonuclease recognition site. Any Type II-S enzyme that is active at a temperature necessary to maintain the single-stranded DNA substantially in that form and to allow the single-stranded DNA adapter portion of the URE to anneal long enough to the single-stranded DNA to permit cleavage at the desired site may be used.

The preferred Type II-S enzymes for use in the URE methods of this disclosure provide asymmetrical cleavage of the single-stranded DNA. Among these are the enzymes listed in Table 800. The most preferred Type II-S enzyme is FokI.

When the preferred Fok I containing URE is used, several conditions are preferably used to effect cleavage:
1) Excess of the URE over target DNA should be present to activate the enzyme. URE present only in equimolar amounts to the target DNA would yield poor cleavage of ssDNA because the amount of active enzyme available would be limiting.
2) An activator may be used to activate part of the FokI enzyme to dimerize without causing cleavage. Examples of appropriate activators are shown in Table 510.
3) The cleavage reaction is performed at a temperature between 45°-75°C, preferably above 50°C and most preferably above 55°C.

The UREs used in the prior art contained a 14-base single-stranded segment, a 10-base stem (containing a FokI site), followed by the palindrome of the 10-base stem. While such UREs may be used in the methods of this disclosure, the preferred UREs of this disclosure also include a segment of three to eight bases (a loop) between the FokI restriction endonuclease recognition site containing segments. In the preferred embodiment, the stem (containing the FokI site) and its palindrome are also longer than 10 bases. Preferably, they are 10-14 bases in length. Examples of these "lollipop" URE adapters are shown in Table 5.

One example of using a URE to cleave an single-stranded DNA involves the FR3 region of human heavy chain. Table 508 shows an analysis of 840 full-length mature human heavy chains with the URE recognition sequences shown. The vast majority (718/84C=0.85) will be recognized with 2 or fewer mismatches using five UREs (VHS881-1.1, VHS881-1.2, VHS881-2.1, VHS881-4.1, and VRS881-9.1). Each has a 20-base adaptor sequence to complement the germline gene, a ten-base stem segment containing a FokI site, a five base loop, and the reverse complement of the first stem segment. Annealing those adapters, alone or in combination, to single-stranded antisense heavy chain DNA and treating with *Fok*I in the presence of, *e.g*., the activator FOKIact, will lead to cleavage of the antisense strand at the position indicated.

Another example of using a URE(s) to cleave a single-stranded DNA involves the FR1 region of the human Kappa light chains. Table 512 shows an analysis of 182 full-length human kappa chains for matching by the four 19-base probe sequences shown. Ninety-six percent of the sequences match one of the probes with 2 or fewer mismatches. The URE adapters shown in Table 512 are for cleavage of the sense strand of kappa chains. Thus, the adaptor sequences are the reverse complement of the germline gene sequences. The URE consists of a ten-base stem, a five base loop, the reverse complement of the stem and the complementation sequence. The loop shown here is TTGTT, but other sequences could be used. Its function is to interrupt the palindrome of the stems so that formation of a lollypop monomer is favored over dimerization. Table 512 also shows where the sense strand is cleaved.

Another example of using a URE to cleave a single-stranded DNA involves the human lambda light chain. Table 515 shows analysis of 128 human lambda light chains for matching the four 19-base probes shown. With three or fewer mismatches, 88 of 128 (69%) of the chains match one of the probes. Table 515 also shows URE adapters corresponding to these probes. Annealing these adapters to upper-strand ssDNA of lambda chains and treatment with FokI in the presence of FOKIact at a temperature at or above 45°C will lead to specific and precise cleavage of the chains.

The conditions under which the short oligonucleotide sequences of the first method and the UREs of the second method are contacted with the single-stranded DNAs may be empirically determined. The conditions must be such that the single-stranded DNA remains in substantially single-stranded form. More particularly, the conditions must be such that the single-stranded DNA does not form loops that may interfere with its association with the oligonucleotide sequence or the URE or that may themselves provide sites for cleavage by the chosen restriction endonuclease.

The effectiveness and specificity of short oligonucleotides (first method) and UREs (second method) can be adjusted by controlling the concentrations of the URE adapters/oligonucleotides and substrate DNA, the temperature, the pH, the concentration of metal ions, the ionic strength, the concentration of chaotropes (such as urea and formamide), the concentration of the restriction endonuclease(e.g., FokI), and the time of the digestion. These conditions can be optimized with synthetic oligonucleotides having: 1) target germline gene sequences, 2) mutated target gene sequences, or 3) somewhat related non-target sequences. The goal is to cleave most of the target sequences and minimal amounts of non-targets.

In the preferred embodiment of this disclosure, the single-stranded DNA is maintained in substantially that form using a temperature between 45°C to 75°C. More preferably, a temperature between 50°C and 60°C, most preferably between 55°C and 60°C, is used. These temperatures are employed both when contacting the DNA with the oligonucleotide or URE and when cleaving the DNA using the methods of this disclosure.

The two cleavage methods of this disclosure have several advantages. The first method allows the individual members of the family of single-stranded DNAs to be cleaved solely at one substantially conserved endonuclease recognition site. The method also does not require an endonuclease recognition site to be built in to the reverse transcription or amplification primers. Any native or synthetic site in the family can be used.

The second method has both of these advantages. In addition, the URE method allows the single-stranded DNAs to be cleaved at positions where no endonuclease recognition site naturally occurs or has been synthetically constructed.

Most importantly, both cleavage methods permit the use of 5' and 3' primers so as to maximize diversity and then cleavage to remove unwanted or deleterious sequences before cloning and display.

After cleavage of the amplified DNAs using one of the methods of this invention, the DNA is prepared for cloning. This is done by using a partially duplexed synthetic DNA adapter, whose terminal sequence is based on the specific cleavage site at which the amplified DNA has been cleaved.

The synthetic DNA is designed such that when it is ligated to the cleaved single-stranded DNA, it allows that DNA to be expressed in the correct reading frame so as to display the desired peptide, polypeptide or protein on the surface of the genetic package. Preferably, the double-stranded portion of the adapter comprises the sequence of several codons that encode the amino acid sequence characteristic of the family of peptides, polypeptides or proteins up to the cleavage site. For human heavy chains, the amino acids of the 3-23 framework are preferably used to provide the sequences required for expression of the cleaved DNA.

Preferably, the double-stranded portion of the adapter is about 12 to 100 bases in length. More preferably, about 20 to 100 bases are used. The double-standard region of the adapter also preferably contains at least one endonuclease recognition site useful for cloning the DNA into a suitable display vector (or a recipient vector used to archive the diversity). This endonuclease restriction site may be native to the germline gene sequences used to extend the DNA sequence. It may be also constructed using degenerate sequences to the native germline gene sequences. Or, it may be wholly synthetic.

The single-stranded portion of the adapter is complementary to the region of the cleavage in the single-stranded DNA. The overlap can be from about 2 bases up to about 15 bases. The longer the overlap, the more efficient the ligation is likely to be. A preferred length for the overlap is 7 to 10. This allows some mismatches in the region so that diversity in this region may be captured.

The single-stranded region or overlap of the partially duplexed adapter is advantageous because it allows DNA cleaved at the chosen site, but not other fragments to be captured. Such fragments would contaminate the library with genes encoding sequences that will not fold into proper antibodies and are likely to be non-specifically sticky.

One illustration of the use of a partially duplexed adaptor in the methods of this disclosure involves ligating such adaptor to a human FR3 region that has been cleaved, as described above, at 5'-ACnGT-3' using HpyCH4III, Bst4CI or TaaI.

Table 250 F.2 shows the bottom strand of the double-stranded portion of the adaptor for ligation to the cleaved bottom-strand DNA. Since the HpyCH4III-Site is so far to the right (as shown in Table 206), a sequence that includes the *Afl*II-site as well as the *Xba*I site can be added. This bottom strand portion of the partially-duplexed adaptor, H43.XAExt, incorporates both *Xba*I and *Afl*II-sites. The top strand of the double-stranded portion of the adaptor has neither site (due to planned mismatches in the segments opposite the *Xba*I and *Afl*II-Sites of H43.XAExt), but will anneal very tightly to H43.XAExt. H43AExt contains only the *Afl*II-site and is to be used with the top strands H43.ABr1 and H43.ABr2 (which have intentional alterations to destroy the *Afl*II-site).

After ligation, the desired, captured DNA can be PCR amplified again, if desired, using in the preferred embodiment a primer to the downstream constant region of the antibody gene and a primer to part of the double-standard region of the adapter. The primers may also carry restriction endonuclease sites for use in cloning the amplified DNA.

After ligation, and perhaps amplification, of the partially double-stranded adapter to the single-stranded amplified DNA, the composite DNA is cleaved at chosen 5' and 3' endonuclease recognition sites.

The cleavage sites useful for cloning depend on the phage or phagemid into which the cassette will be inserted and the available sites in the antibody genes. Table 1 provides restriction endonuclease data for 75 human light chains. Table 2 shows corresponding data for 79 human heavy chains. In each Table, the endonucleases are ordered by increasing frequency of cutting. In these Tables, Nch is the number of chains cut by the enzyme and Ns is the number of sites (some chains have more than one site).

From this analysis, *Sfl*I, *No*tI, *Afl*II, *Apa*LI, and AscI are very suitable. SfiI and NotI are preferably used in pCES1 to insert the heavy-chain display segment. *Apa*LI and *Asc*I are preferably used in pCES1 to insert the light-chain display segment.

*Bst*EII-sites occur in 97% of germ-line JH genes. In rearranged V genes, only 54/79 (68%) of heavy-chain genes contain a BstEII-Site and 7/61 of these contain two sites. Thus, 47/79 (59%) contain a single *Bst*EII-Site. An alternative to using BstEII is to cleave via UREs at the end of JH and ligate to a synthetic oligonucleotide that encodes part of CH1.

One example of preparing a family of DNA sequences using the methods of this disclosure involves capturing human CDR 3 diversity. As described above, mRHAs from various autoimmune patients is reverse transcribed into lower strand cDNA. After the top strand RNA is degraded, the lower strand is immobilized and a short oligonucleotide used to cleave the cDNA upstream of CDR3. A partially duplexed synthetic DNA adapter is then annealed to the DNA and the DNA is amplified using a primer to the adapter and a primer to the constant region (after FR4). The DNA is then cleaved using BstEII (in FR4) and a restriction endonuclease appropriate to the partially double-stranded adapter (e.g., Xba I and AflII (in FR3)). The DNA is then ligated into a synthetic VH skeleton such as 3-23.

One example of preparing a single-stranded DNA that was cleaved using the URE method involves the human Kappa chain. The cleavage site in the sense strand of this chain is depicted in Table 512. The oligonucleotide kapextURE is annealed to the oligonucleotides (kaBR01UR, kaBR02UR, kaBR03UR, and kaBR04UR) to form a partially duplex DNA. This DNA is then ligated to the cleaved soluble kappa chains. The ligation product is then amplified using primers kapextUREPCR and CKForeAsc (which inserts a AscI site after the end of C kappa). This product is then cleaved with ApaLI and AscI and ligated to similarly cut recipient vector.

Another example involves the cleavage illustrated in Table 515. After cleavage, an extender (ON_LamEx133) and four bridge oligonucleotides (ON_LamB1-133, ON_LamB2-133, ON_LamB3-133, and ON_LamB4-133) are annealed to form a partially duplex DNA. That DNA is ligated to the cleaved lambda-chain sense strands. After ligation, the DNA is amplified with ON_Lam133PCR and a forward primer specific to the lambda constant domain, such as CL2ForeAsc or CL7ForeAsc (Table 130).

In human heavy chains, one can cleave almost all genes in FR4 (downstream, i.e. toward the 3' end of the sense strand, of CDR3) at a BstEII-Site that occurs at a constant position in a very large fraction of human heavy-chain V genes. One then needs a site in FR3, if only CDR3 diversity is to be captured, in FR2, if CDR2 and CDR3 diversity is wanted, or in FR1, if all the CDR diversity is wanted. These sites are preferably inserted as part of the partially double-stranded adaptor.

The preferred process of this disclosure is to provide recipient vectors having sites that allow cloning of either light or heavy chains. Such vectors are well known and widely used in the art. A preferred phage display vector in accordance with this disclosure is phage MALIA3. This displays in gene III. The sequence of the phage MALIA3 is shown in Table 120A (annotated) and Table 120B (condensed).

The DNA encoding the selected regions of the light or heavy chains can be transferred to the vectors using endonucleases that cut either light or heavy chains only very rarely. For example, light chains may be captured with ApaLI and AscI. Heavy-chain genes are preferably cloned into a recipient vector having SfiI, NcoI, XbaI, AflII, BstEII, ApaI, and NotI sites. The light chains are preferably moved into the library as ApaLI-AscI fragments. The heavy chains are preferably moved into the library as *Sfi*I-*Not*I fragments.

Most preferably, the display is had on the surface of a derivative of M13 phage. The most preferred vector contains all the genes of M13, an antibiotic resistance gene, and the display cassette. The preferred vector is provided with restriction sites that allow introduction and excision of members of the diverse family of genes, as cassettes. The preferred vector is stable against rearrangement under the growth conditions used to amplify phage.

In another embodiment of this disclosure, the diversity captured by the methods of the present disclosure may be displayed in a phagemid vector (e.g., pCES1) that displays the peptide, polypeptide or protein on the III protein. Such vectors may also be used to store the diversity for subsequent display using other vectors or phage.

In another embodiment, the mode of display may be through a short linker to three possible anchor domains. One anchor domain being the final portion of M13 III ("IIIstump"), a second anchor being the full length III mature protein, and the third being the M13 VIII mature protein.

The IIIstump fragment contains enough of M13 III to assemble into phage but not the domains involved in mediating infectivity. Because the w.t. III and VIII proteins are present, the phage is unlikely to delete the antibody genes and phage that do delete these segments receive only a very small growth advantage. For each of the anchor domains, the DNA encodes the w.t. AA sequence, but differs from the w.t. DNA sequence to a very high extent. This will greatly reduce the potential for homologous recombination between the display anchor and the w.t. gene that is also present. -

Most preferably, the present disclosure uses a complete phage carrying an antibiotic-resisiance gene (such as an ampicillin-resistance gene) and the display cassette. Because the w.t. iii and viii genes are present, the w.t. proteins are also present. The display cassette is transcribed from a regulatable promoter (*e.g*., P_{Lacz}). Use of a regulatable promoter allows control of the ratio of the fusion display gene to the corresponding w.t. coat protein. This ratio determines the average number of copies of the display fusion per phage (or phagemid) particle.

Another aspect of the disclosure- is a method of displaying peptides, polypeptides or proteins (and particularly Fabs) on filamentous phage. In the most preferred embodiment this method displays FABs and comprises:
a) obtaining a cassette capturing a diversity of segments of DNA encoding the elements:
   P_{reg}::RBS1::SS1::VL::CT::stop::RBS2::SS2::VH::CH1:: linker::anchor::stop::,
   where P*_{reg}* is a regulatable promoter, RBS1 is a first ribosome binding site, SS1 is a signal sequence operable in the host strain, VL is a member of a diverse set of light-chain variable regions, CL is a light-chain constant region, stop is one or more stop codons, RBS2 is a second ribosome binding site, SS2 is a second signal sequence operable in the host strain, VH is a member of a diverse set of heavy-chain variable regions, CH1 is an antibody heavy-chain first constant domain, linker is a sequence of amino acids of one to about 50 residues, anchor is a protein that will assemble into the filamentous phage particle and stop is a second example of one or more stop codons; and
b) positioning that cassette within the phage genome to maximize the viability of the phage and to minimize the potential for deletion of the cassette or parts thereof.

The DNA encoding the anchor protein in the above preferred cassette should be designed to encode the same (or a closely related) amino acid sequence as is found in one of the coat proteins of the phage, but with a distinct DNA sequence. This is to prevent unwanted homologous recombination with the w.t. gene. In addition, the cassette should be placed in the intergenic region. The positioning and orientation of the display cassette can influence the behavior of the phage.

In one embodiment of the disclosure , a transcription terminator may be placed after the second stop of the display cassette above (*e.g*., Trp). This will reduce interaction between the display cassette and other genes in the phage antibody display vector (PADV).

In another embodiment of the methods of this disclosure, the phage or phagemid can display proteins other than Fab, by replacing the Fab portions indicated above, with other protein genes.

Various hosts can be used for growth of the display phage or phagemids of this disclosure. Such hosts are well known in the art. In the preferred embodiment, where Fabs are being displayed, the preferred host should grow at 30°C and be RecA⁻ (to reduce unwanted genetic recombination) and EndA⁻ (to make recovery of RF DNA easier). It is also preferred that the host strain be easily transformed by electroporation.

XL1-Blue MRF' satisfies most of these preferences, but does not grow well at 30°C. XL1-Blue MRF' does grow slowly at 38°C and thus is an acceptable host. TG-1 is also an acceptable host although it is *RecA*⁺ and *EndA*⁺. XL1-Blue MRF' is more preferred for the intermediate host used to accumulate diversity prior to final construction of the library.

After display, the libraries of this disclosure may be screened using well known and conventionally used techniques. The selected peptides, polypeptides or proteins may then be used to treat disease. Generally, the peptides, polypeptides or proteins for use in therapy or in pharmaceutical compositions are produced by isolating the DNA encoding the desired peptide, polypeptide or protein from the member of the library selected. That DNA is then used in conventional methods to produce the peptide, polypeptides or protein it encodes in appropriate host cells, preferably mammalian host cells, e.g., CHO cells. After isolation, the peptide, polypeptide or protein is used alone or with pharmaceutically, acceptable compositions in therapy to treat disease.

### EXAMPLES

### Example 1: Capturing kappa chains with BsmAI:

A repertoire of human-kappa chain mRNAs was prepared by treating total or poly(A+) RNA isolated from a collection of patients having various autoimmune diseases with calf intestinal phosphatase to remove the 5'-phosphate from all molecules that have them, such as ribosomal RNA, fragmented mRNA, tRNA and genomic DNA. Full length mRNA (containing a protective 7-methyl cap structure) is unaffected. The RNA is then treated with tobacco acid pyrophosphatase to remove the cap structure from full length mRNAs leaving a 5'-monophosphate group.

Full length mRNA's were modified with an adaptor at the 5' end and then reversed transcribed and amplified using the GeneRACE™ method and kit (Invitrogen). A 5' biotinylated primer complementary to the adaptor and a 3' primer complementary to a portion of the construct region were used.

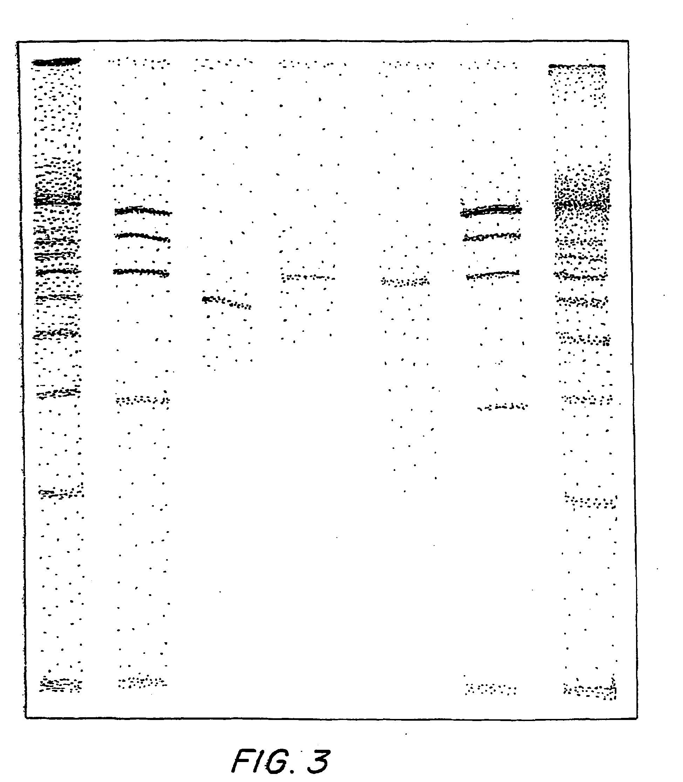

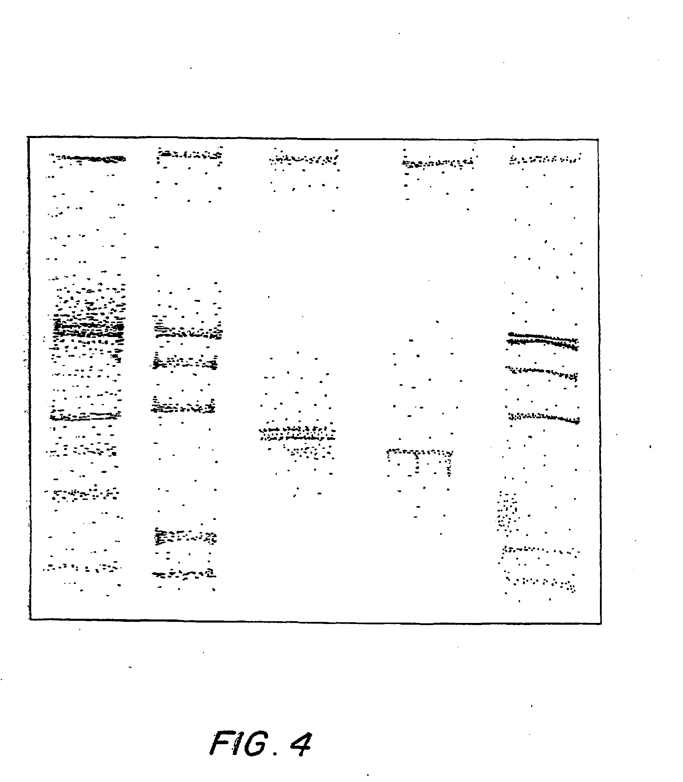

Approximately 2 micrograms (ug) of human kappa-chain (Igkappa) gene RACE material with biotin attached to 5'-end of upper strand was immobilized on 200 microliters (µL) of Seradyn magnetic beads. The lower strand was removed by washing the DNA with 2 aliquots 200 µL of 0.1 M NaOH (pH 13) for 3 minutes for the first aliquot followed by 30 seconds for the second aliquot. The beads were neutralized with 200 µL of 10 mM Tris (pH 7.5) 100 mM NaCl. The short oligonucleotides shown in Table 525 were added in 40 fold molar excess in 100 µL of NEB buffer 2 (50 mM NaCl, 10 mM Tris-HCl, 10 mM MgCl₂, 1 mM dithiothreitol pH 7.9) to the dry beads. The mixture was incubated at 95°C for 5 minutes then cooled down to 55°C over 30 minutes. Excess oligonucleotide was washed away with 2 washes of NEB buffer 3 (100 mM NaCl, 50 mM Tris-HCl, 10 mM MgCl₂, 1 mM dithiothreitol pH 7.9). Ten units of BsmAI (NEB) were added in NEB buffer 3 and incubated for 1 h at 55°C. The cleaved downstream DNA was collected and purified over a Qiagen PCR purification column (FIGs. 3 and 4).

A partially double-stranded adaptor was prepared using the oligonucleotide shown in Table 525. The adaptor was added to the single-stranded DNA in 100 fold molar excess along with 1000 units of T4 DNA ligase (NEB) and incubated overnight at 16°C. The excess oligonucleotide was removed with a Qiagen PCR purification column. The ligated material was amplified by PCR using the primers kapPCRt1 and kapfor shown in Table 525 for 10 cycles with the program shown in Table 530.

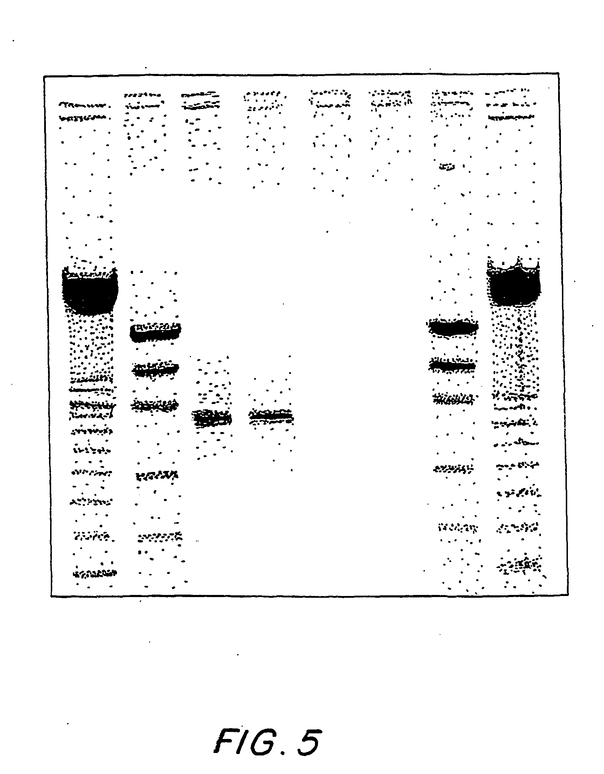

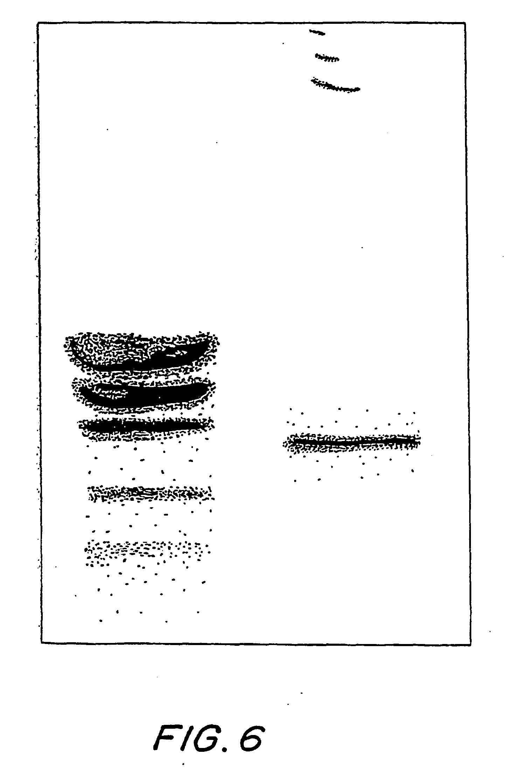

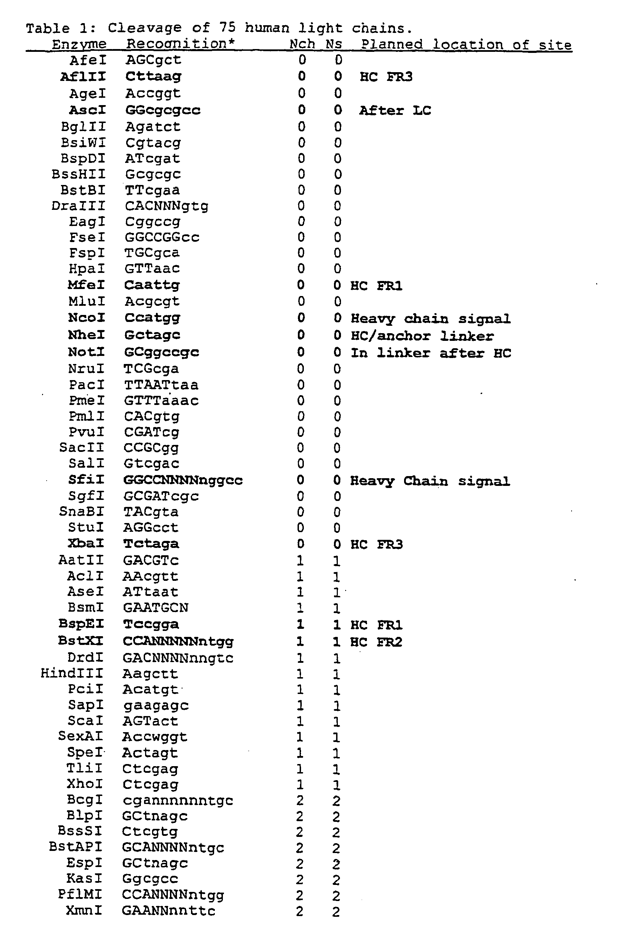

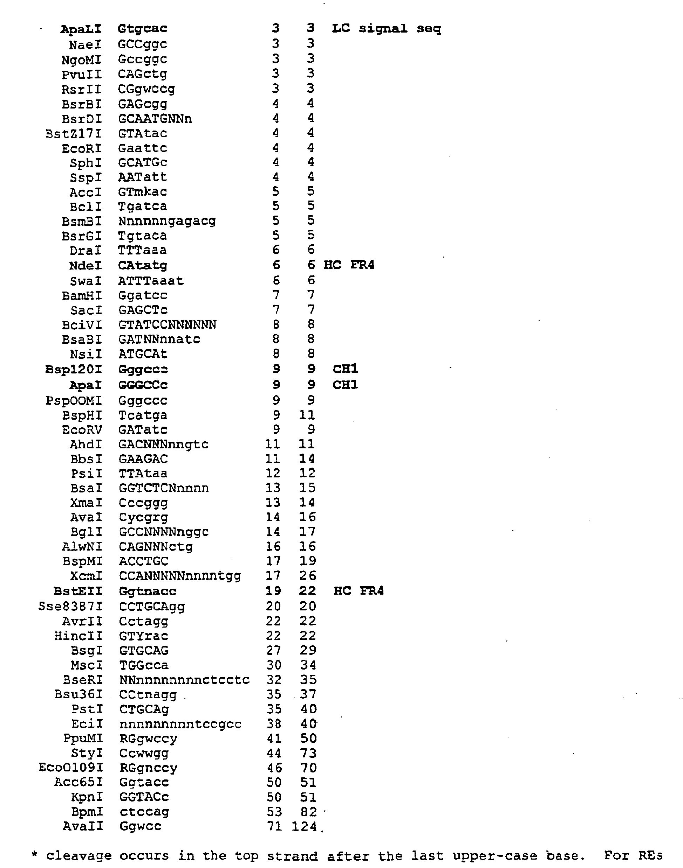

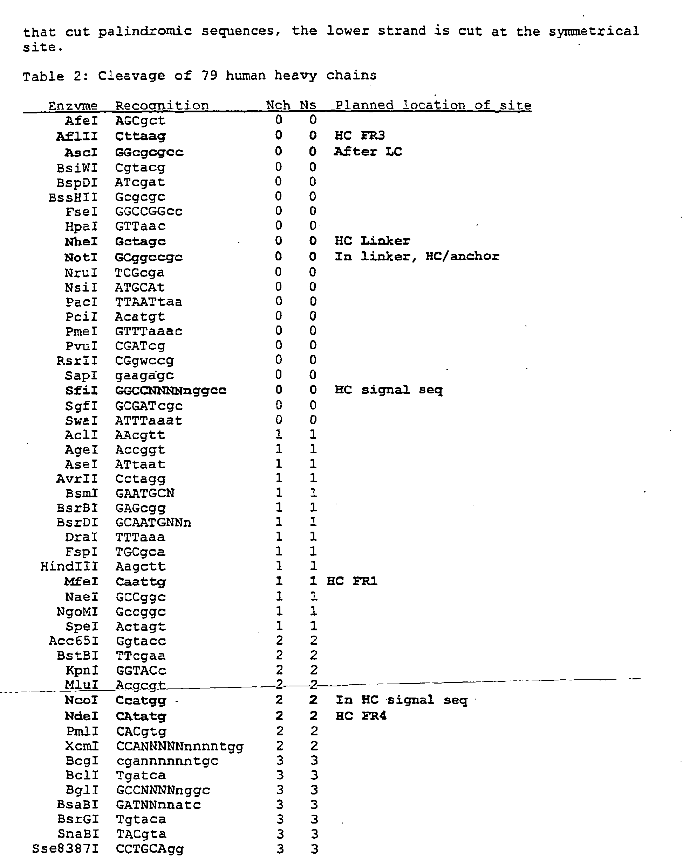

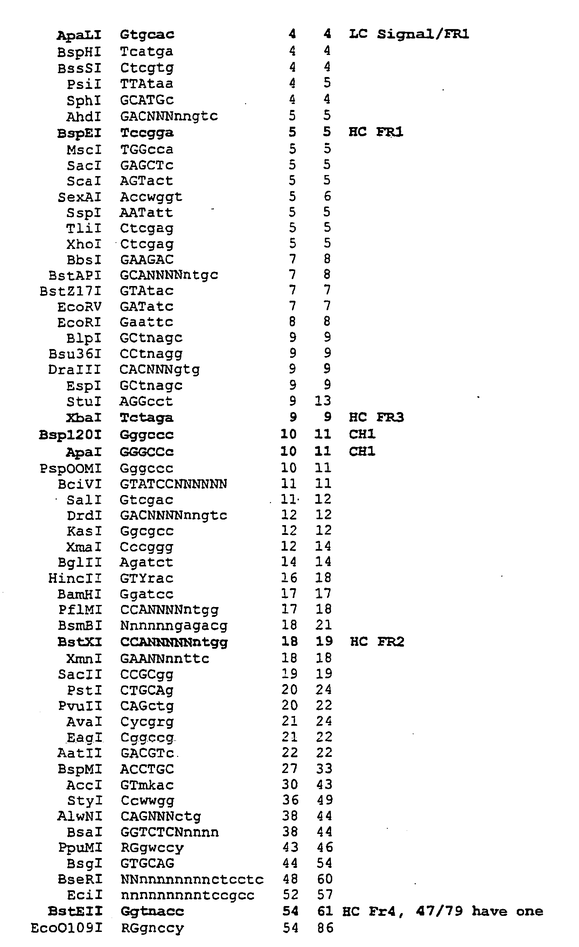

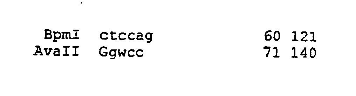

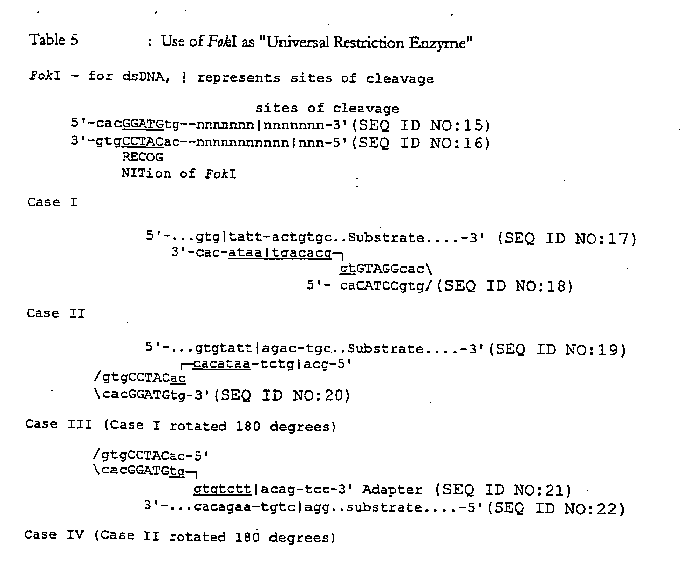

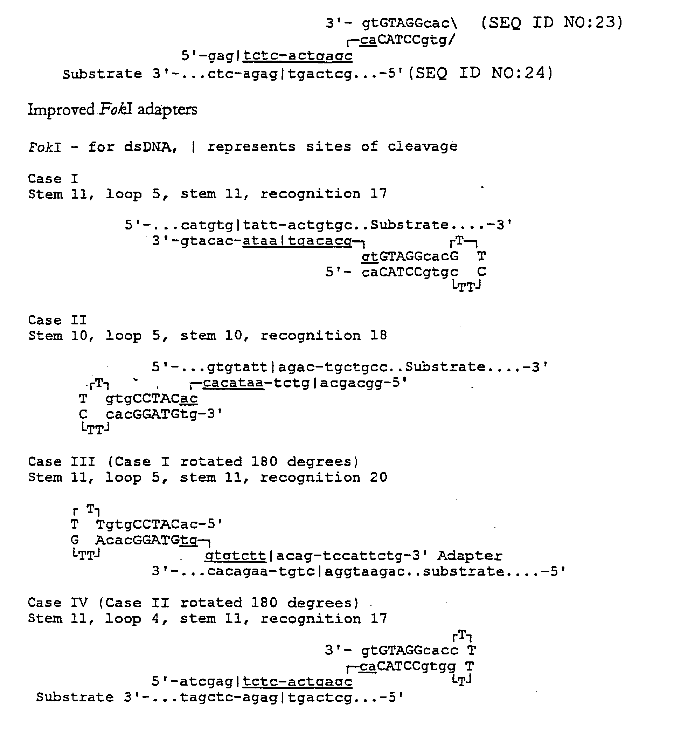

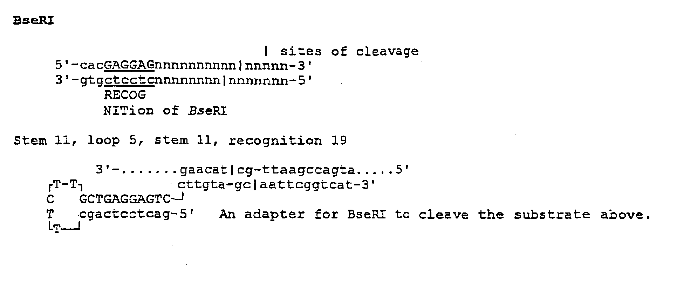

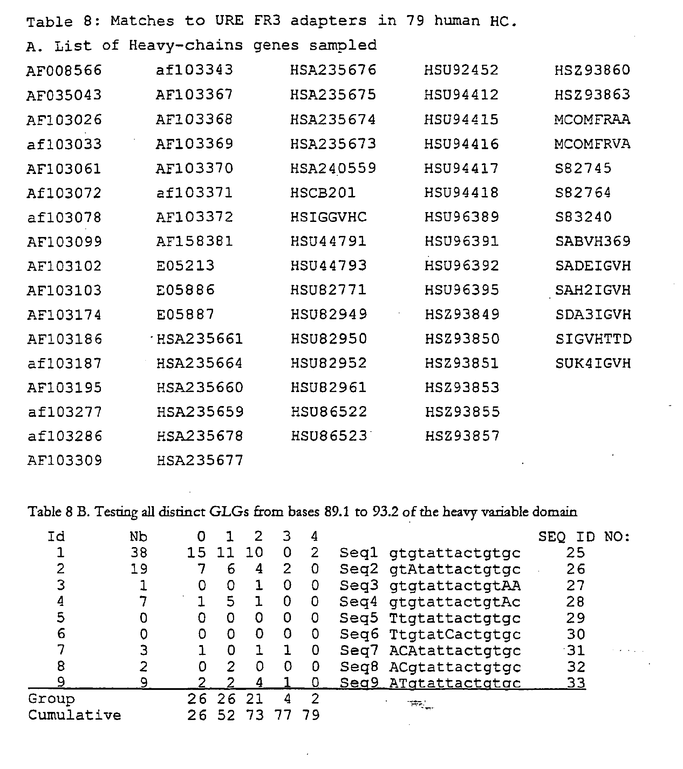

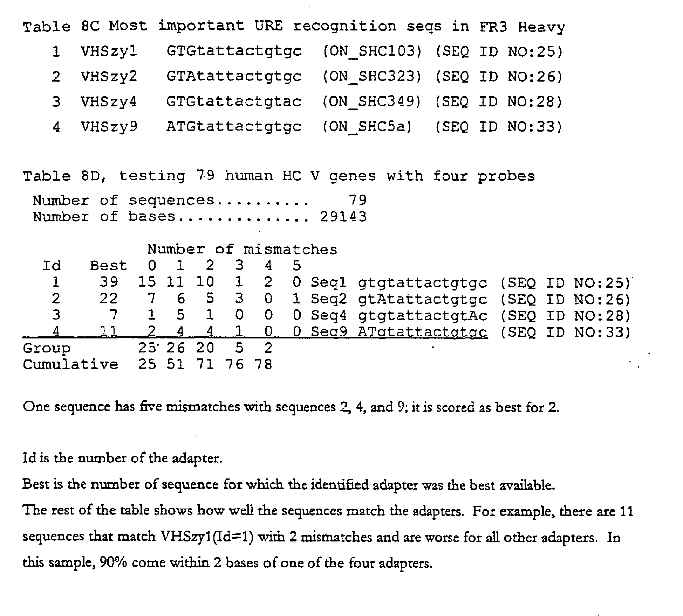

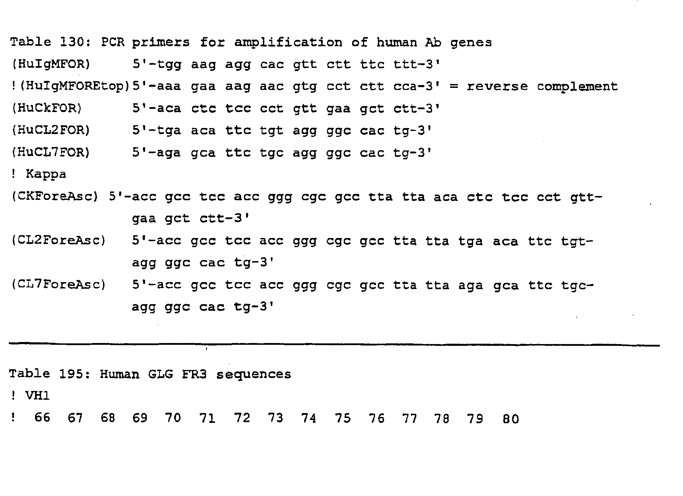

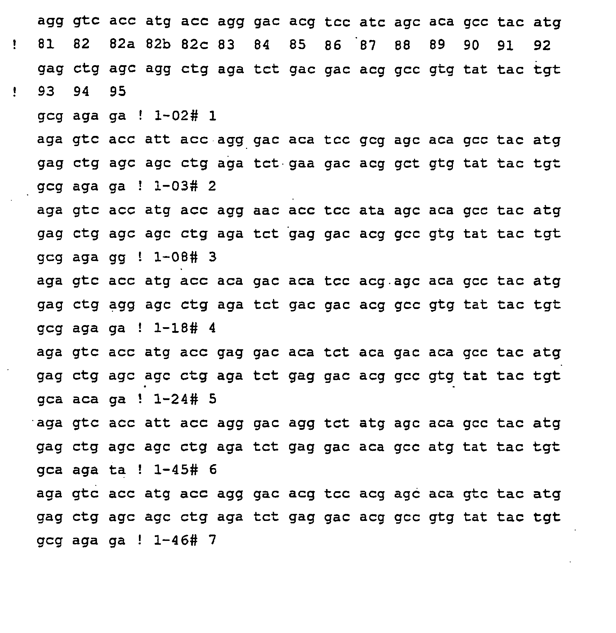

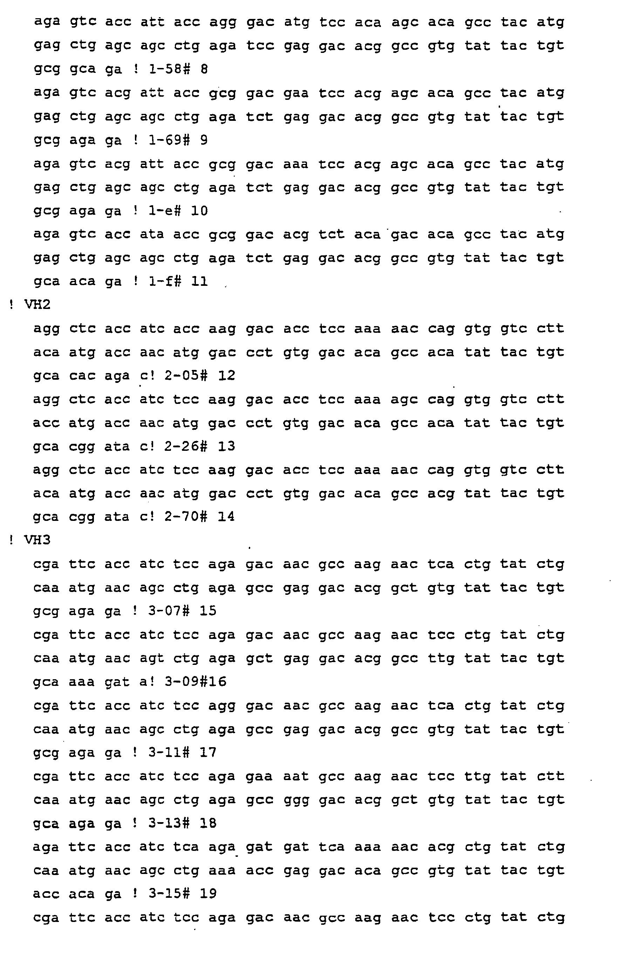

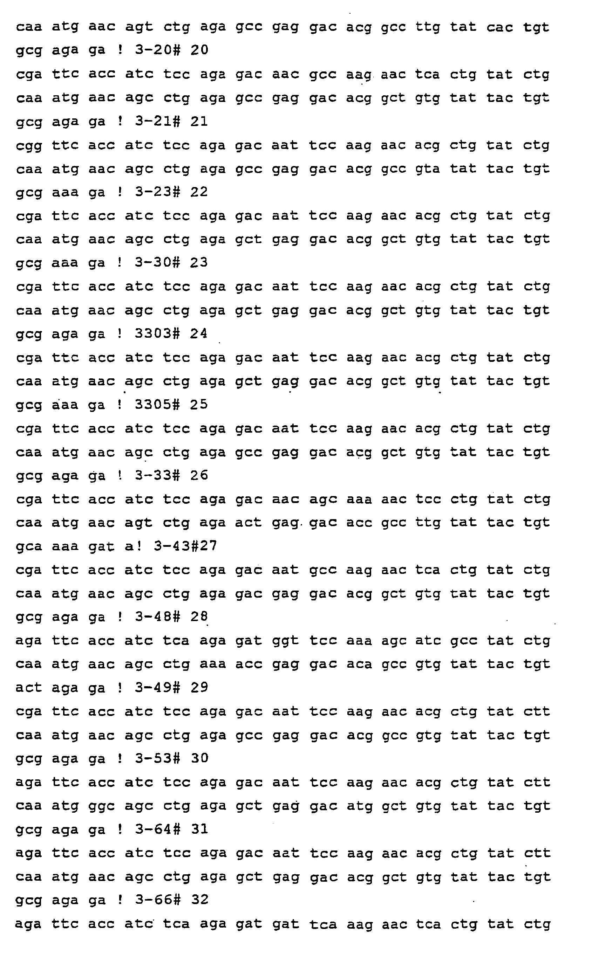

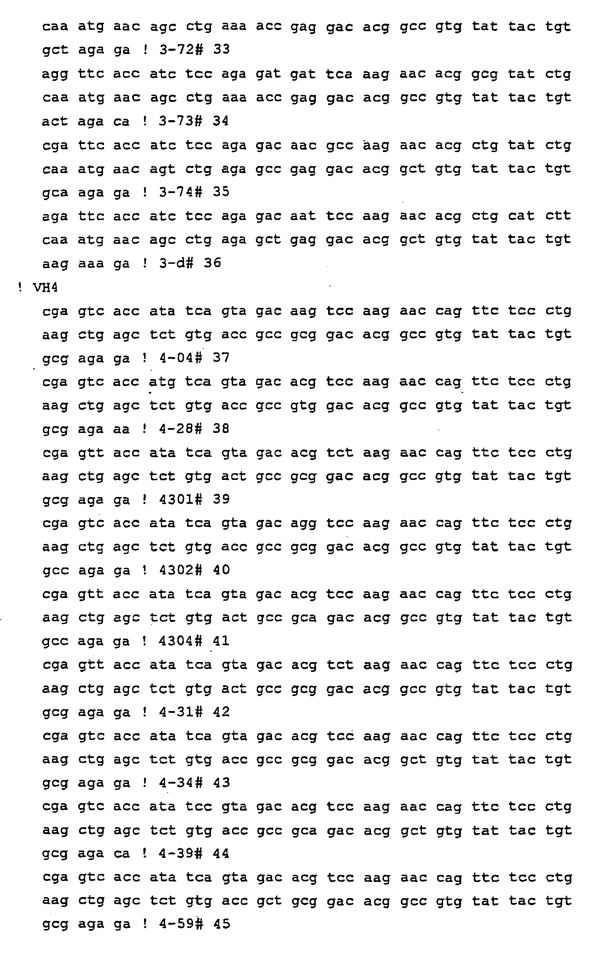

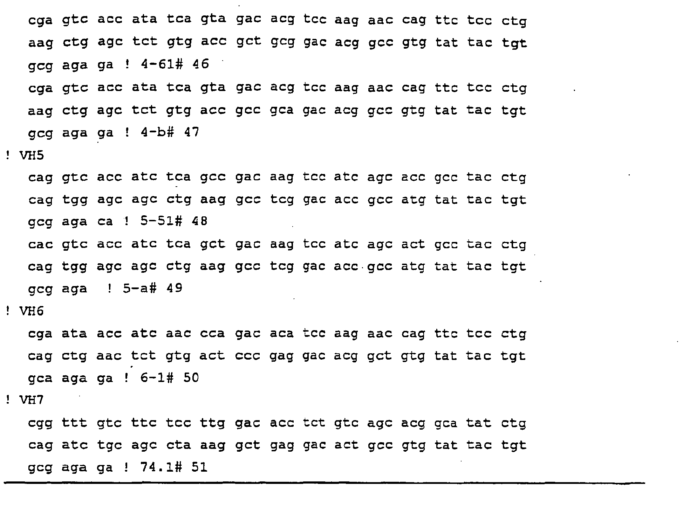

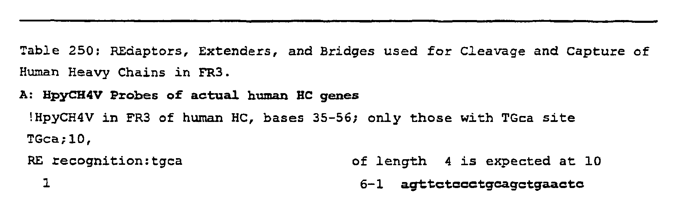

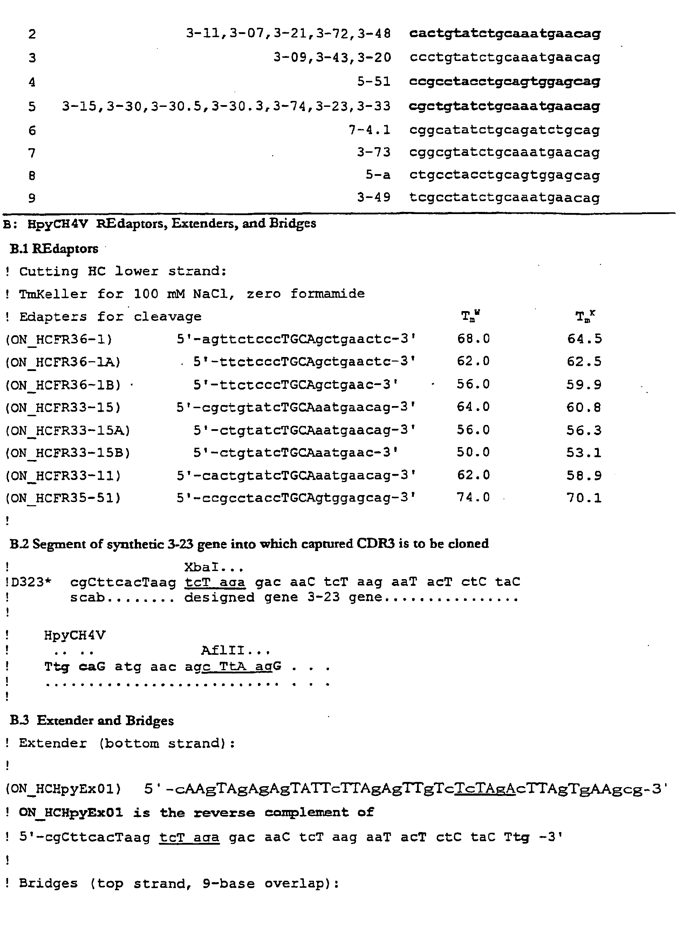

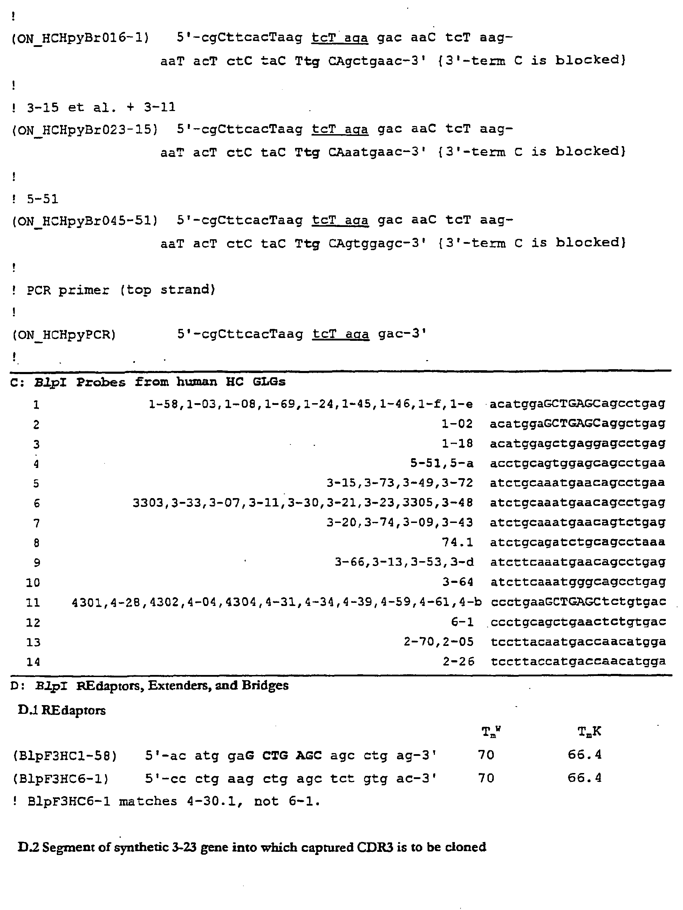

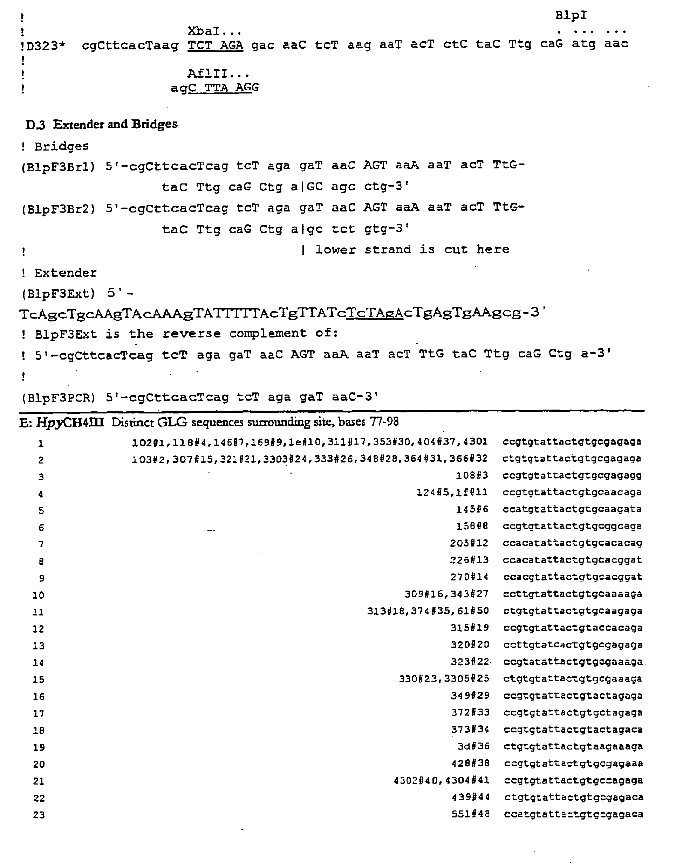

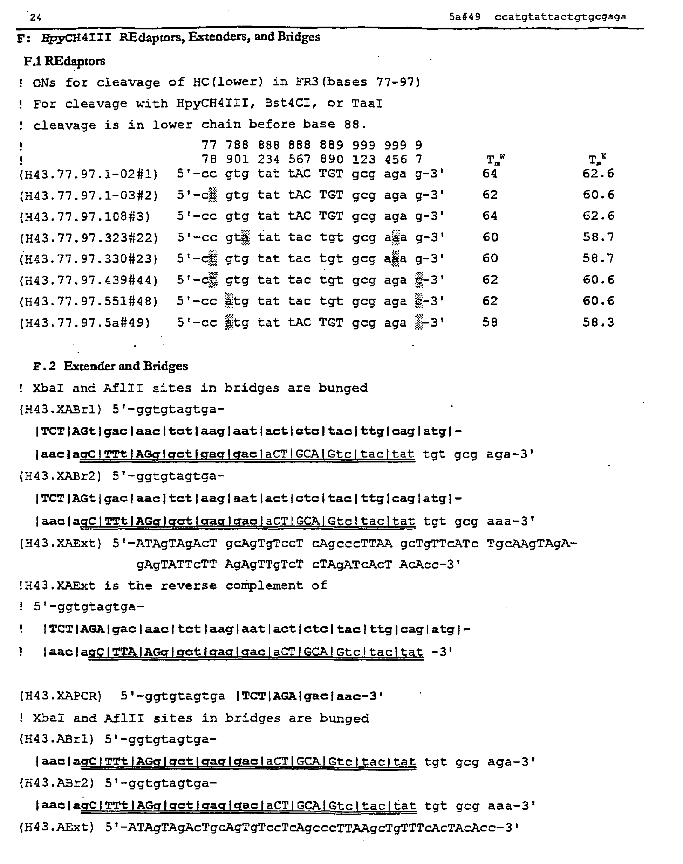

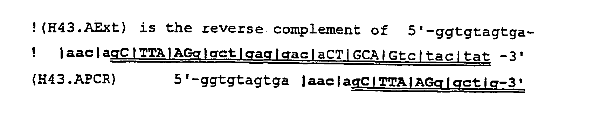

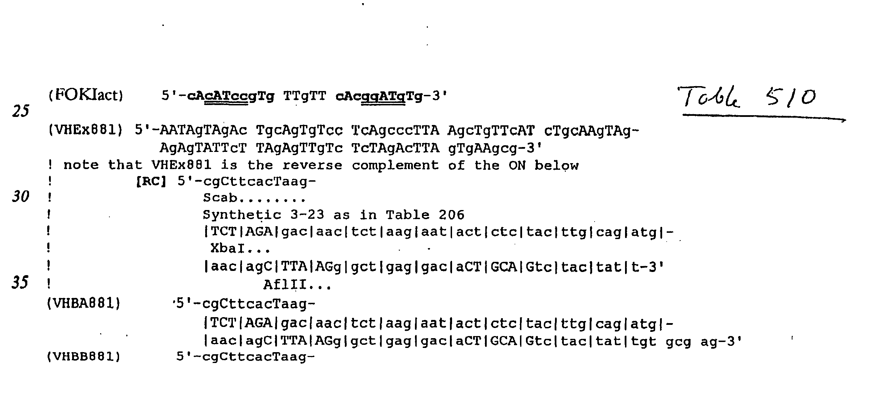

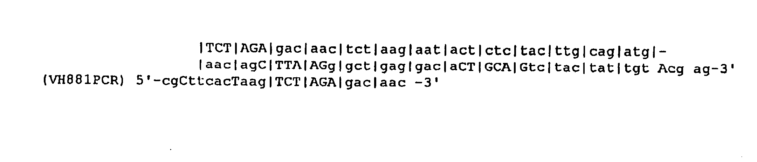

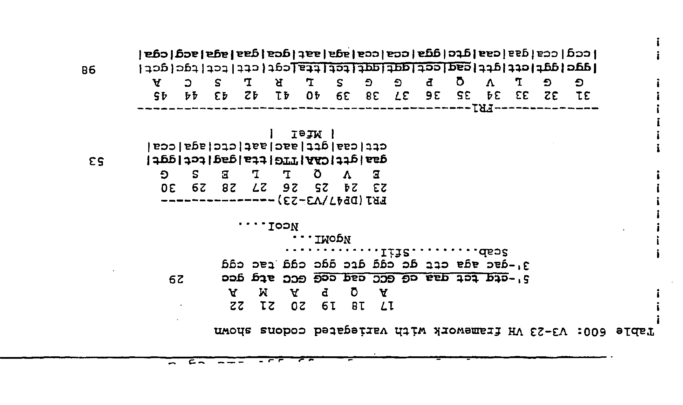

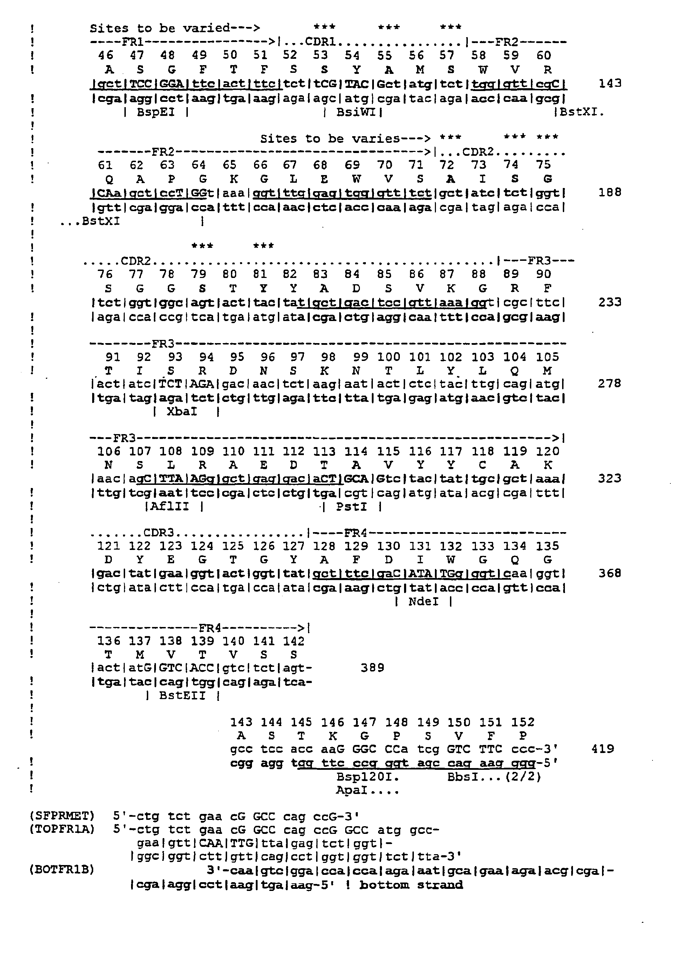

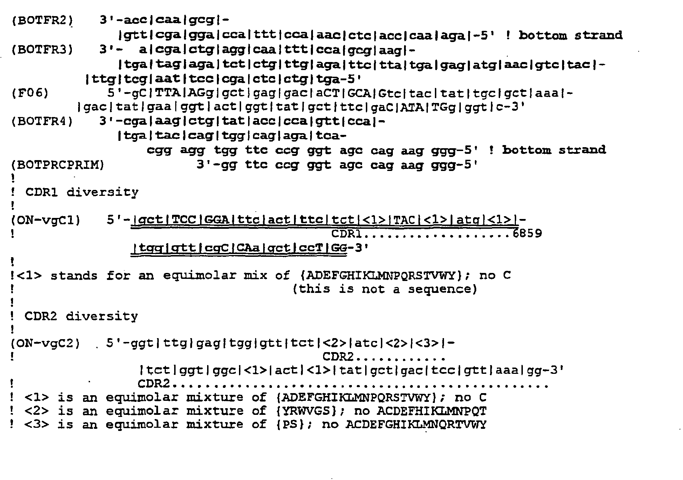

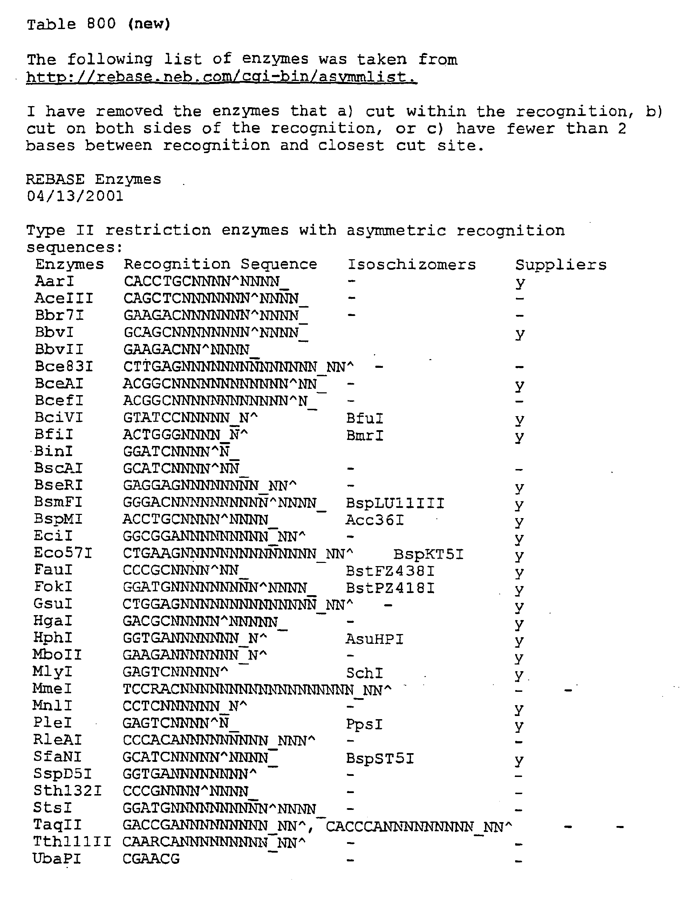

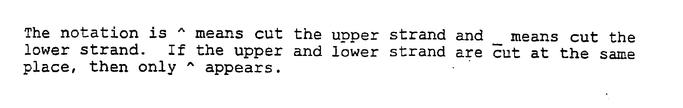

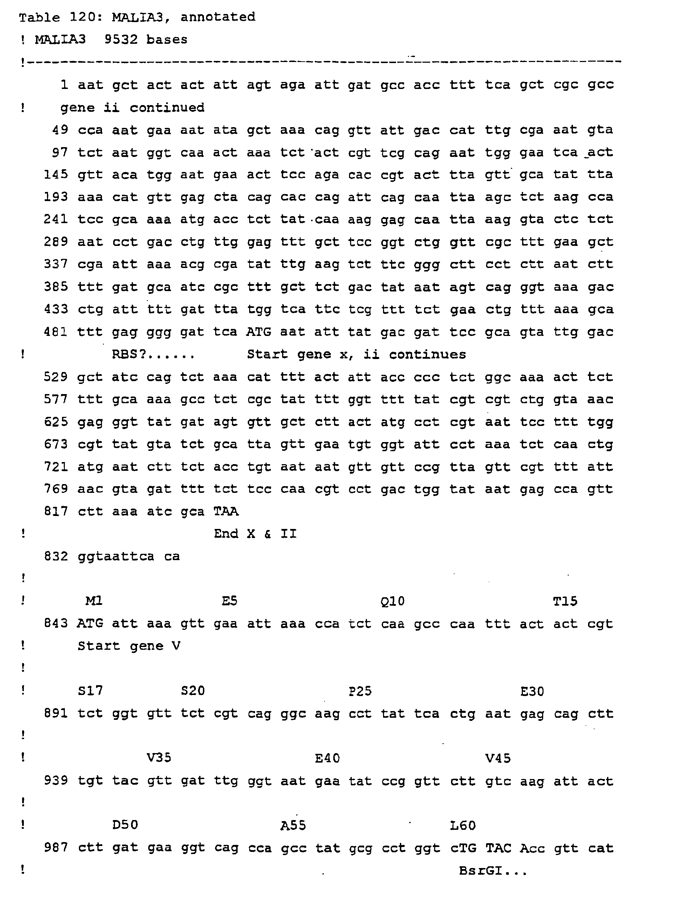

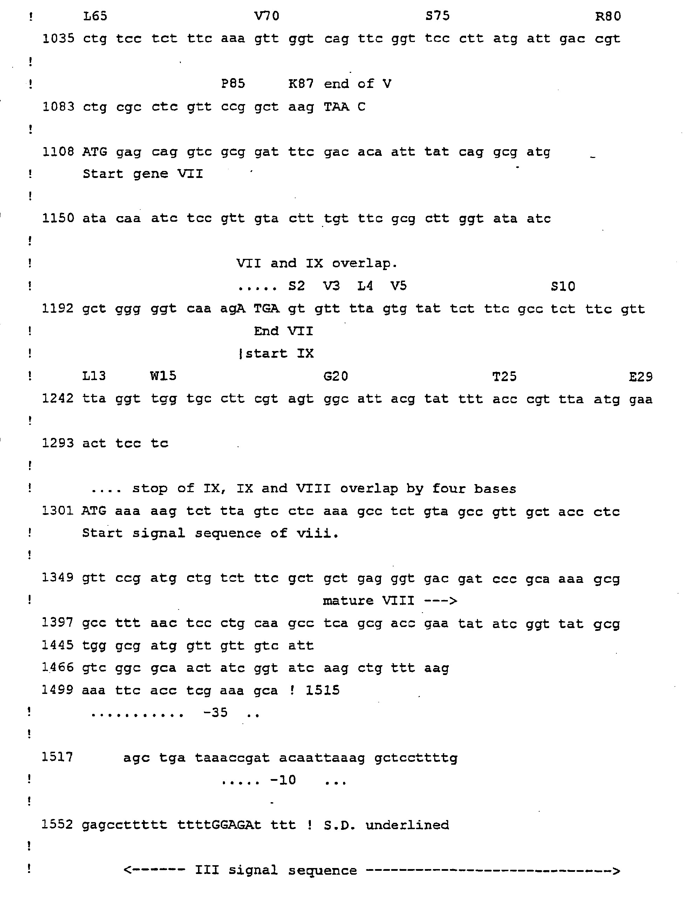

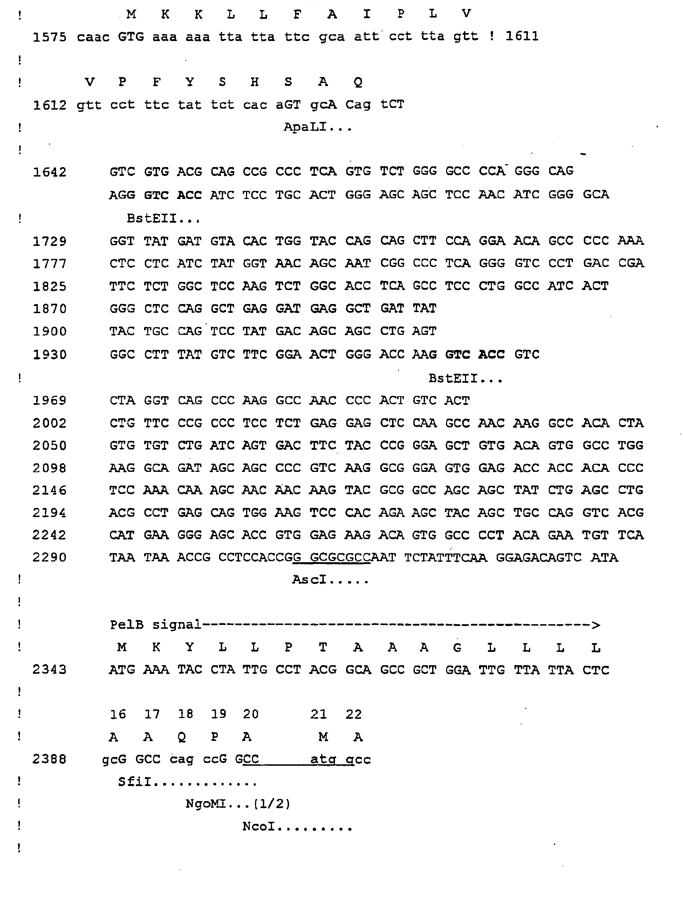

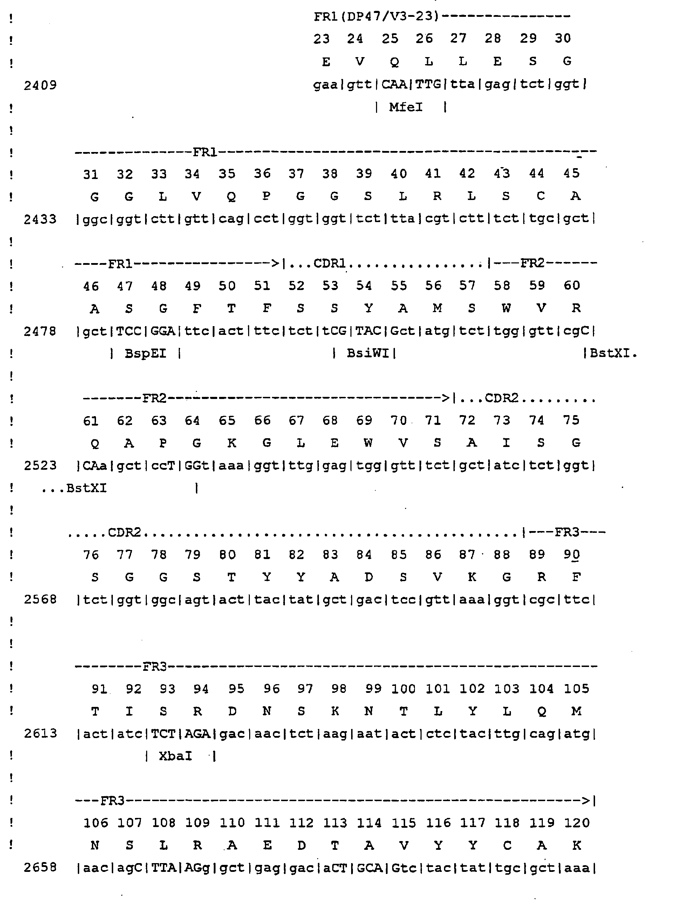

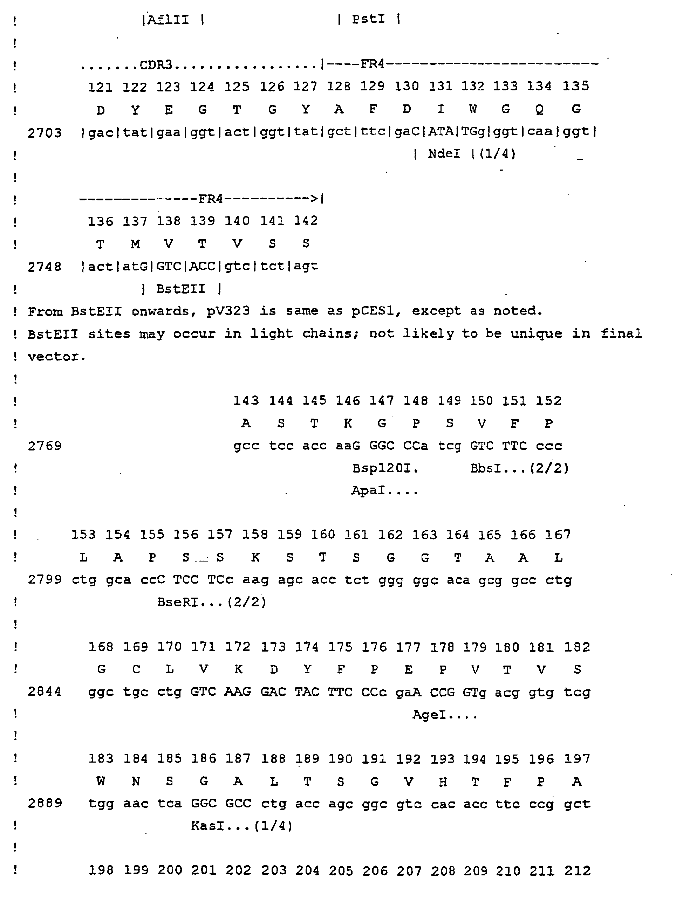

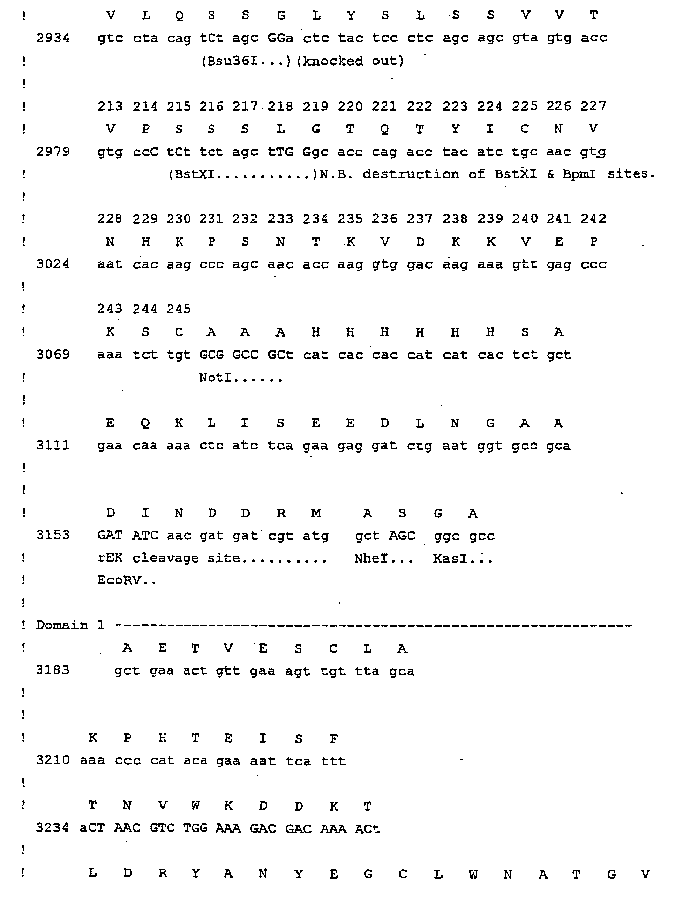

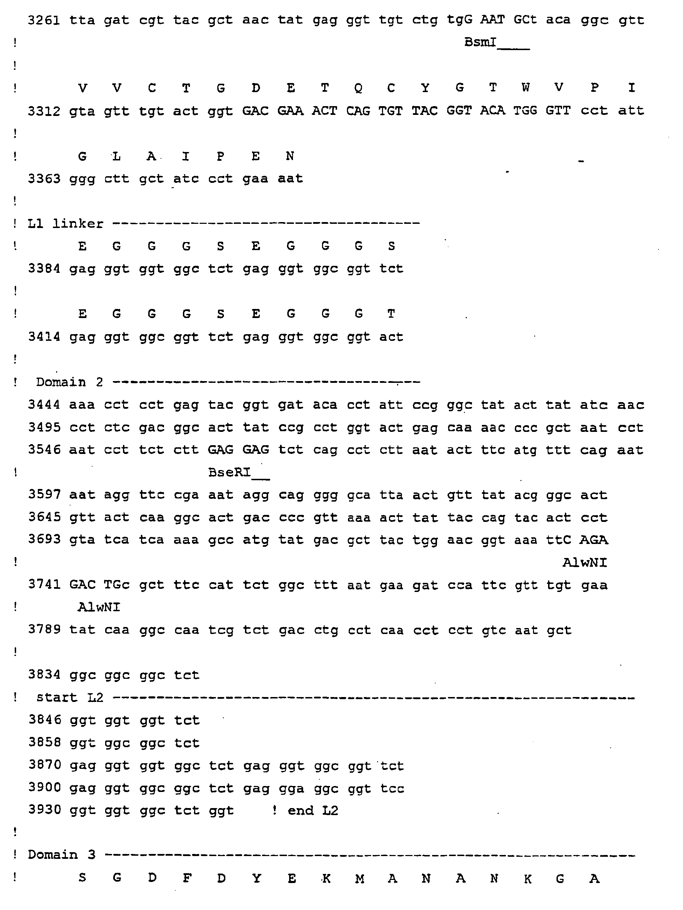

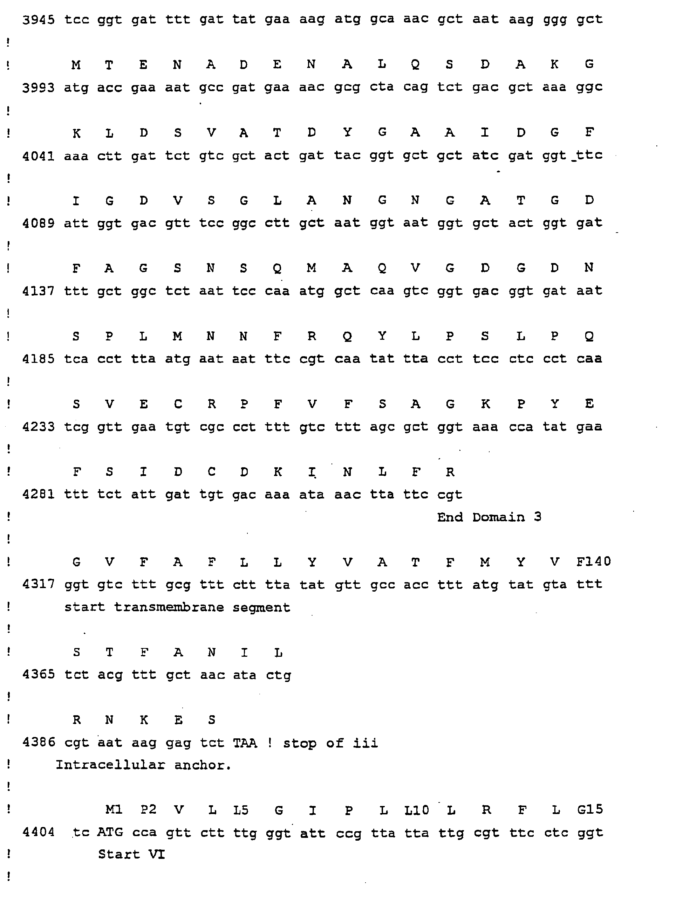

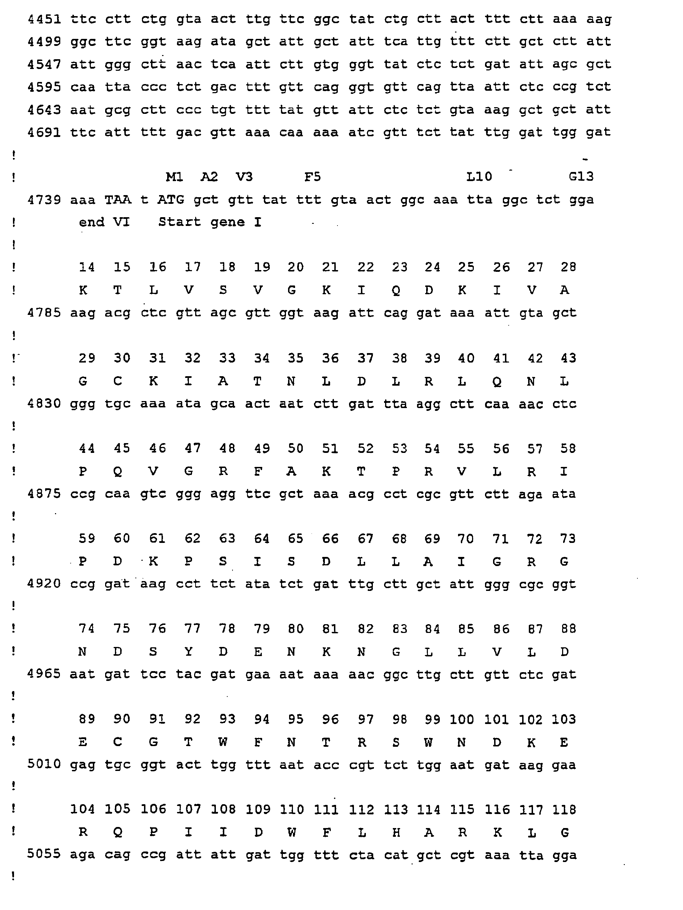

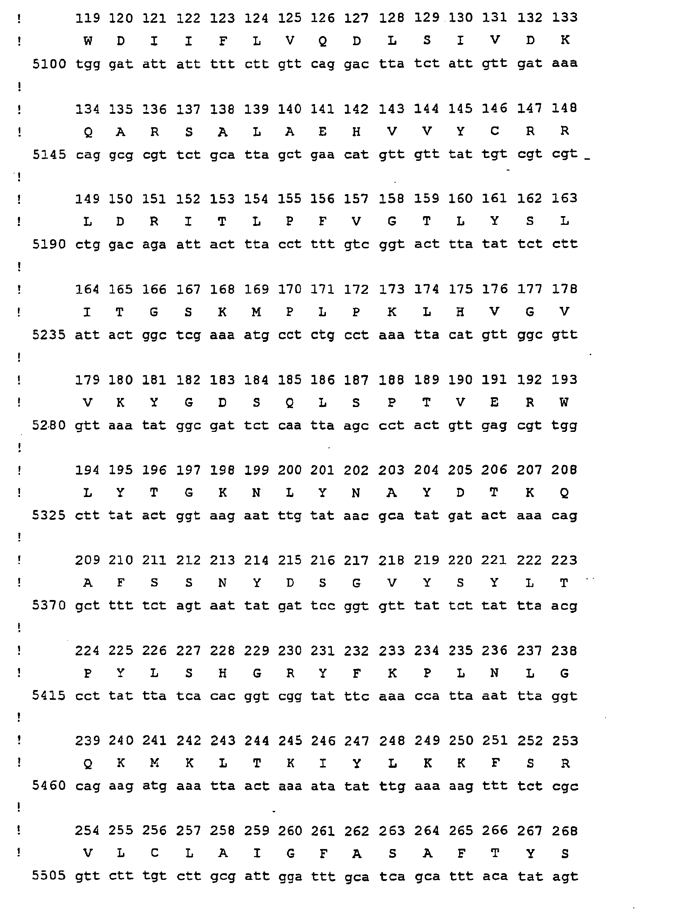

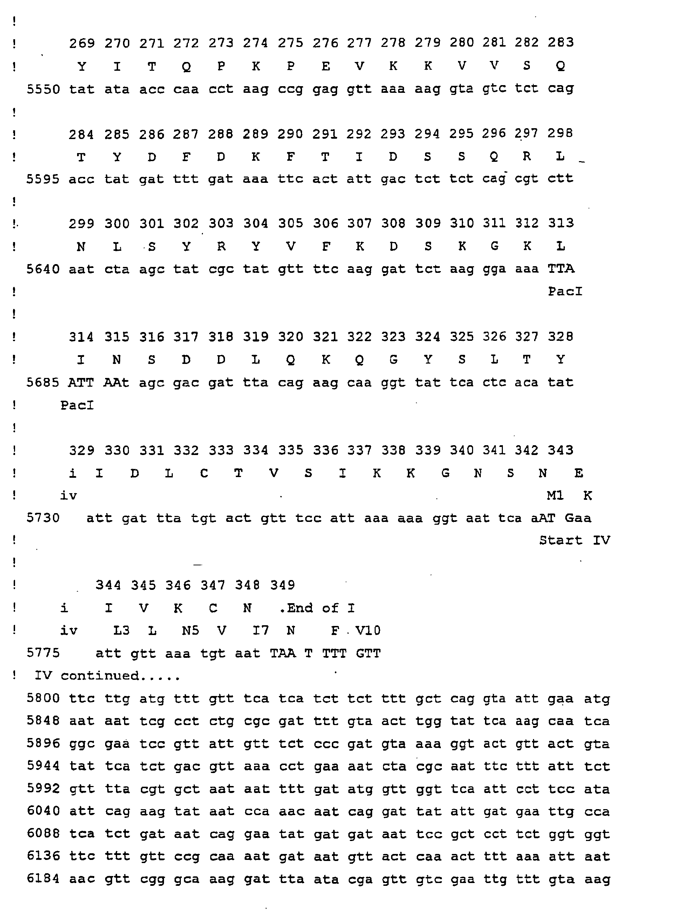

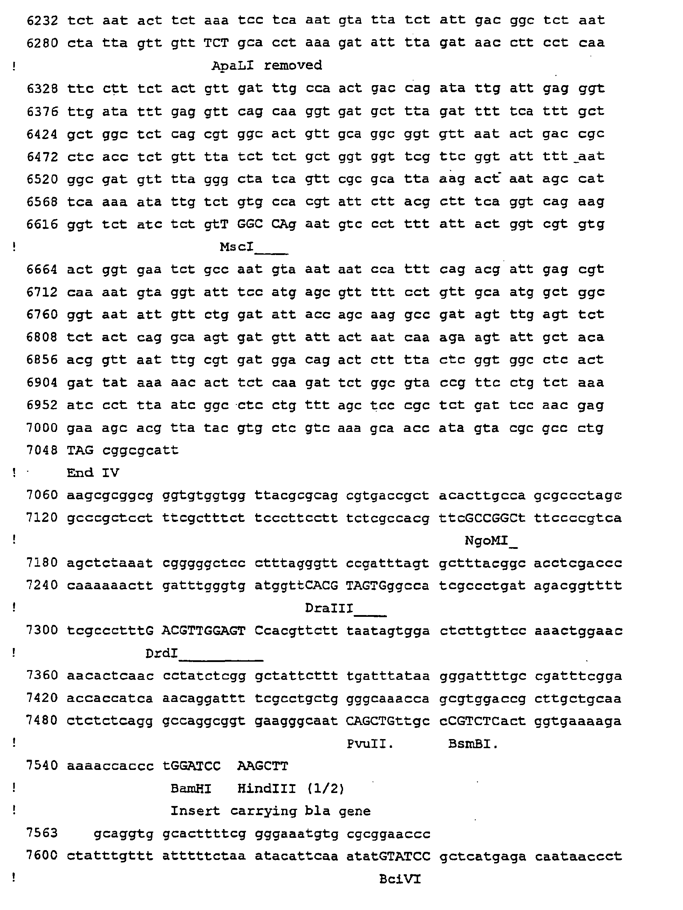

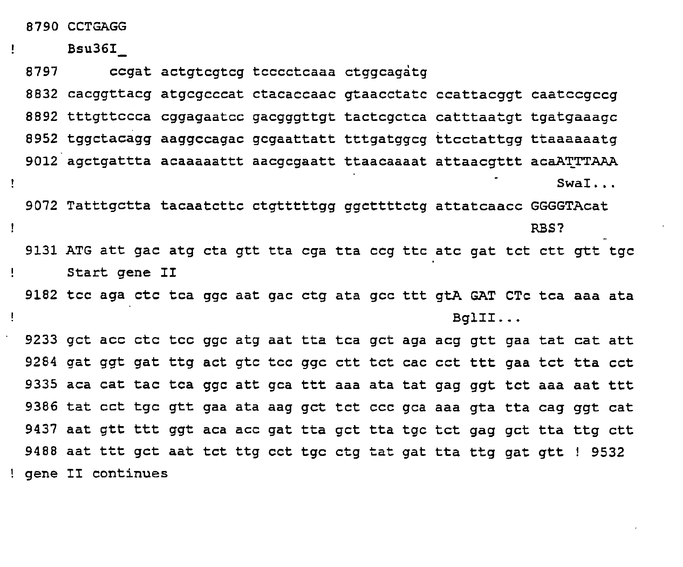

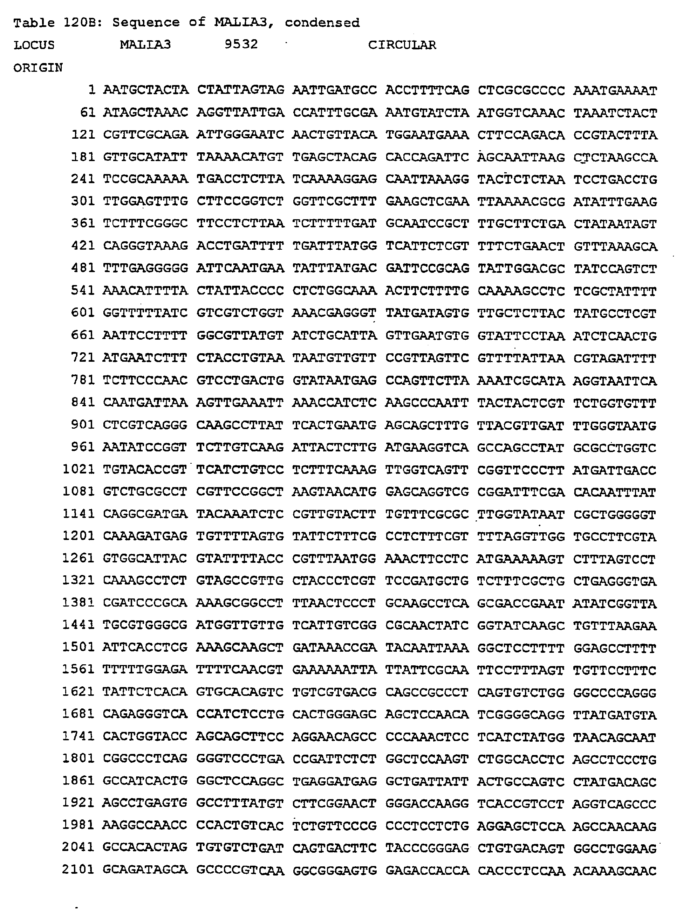

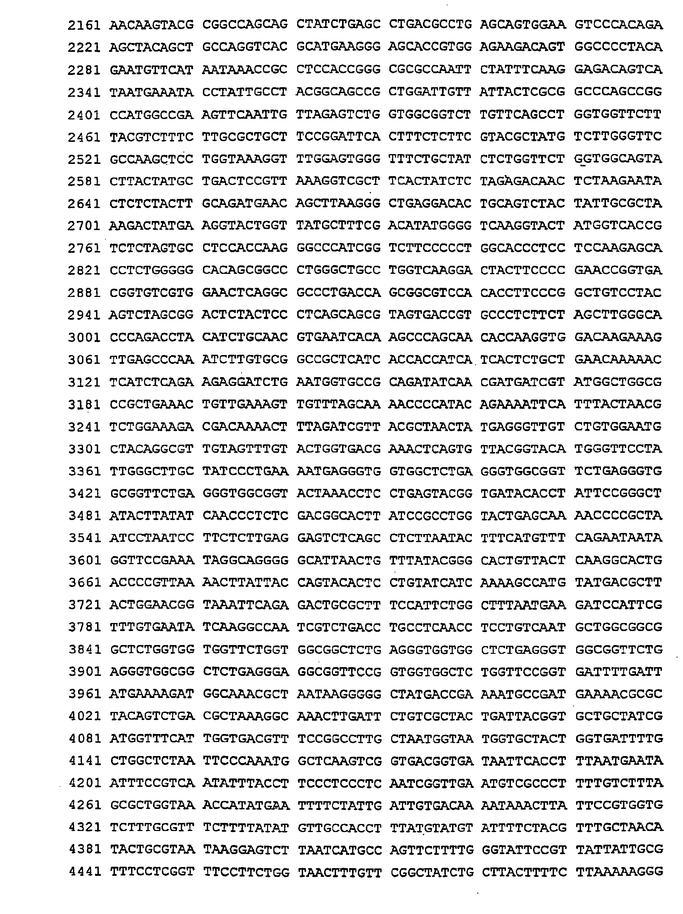

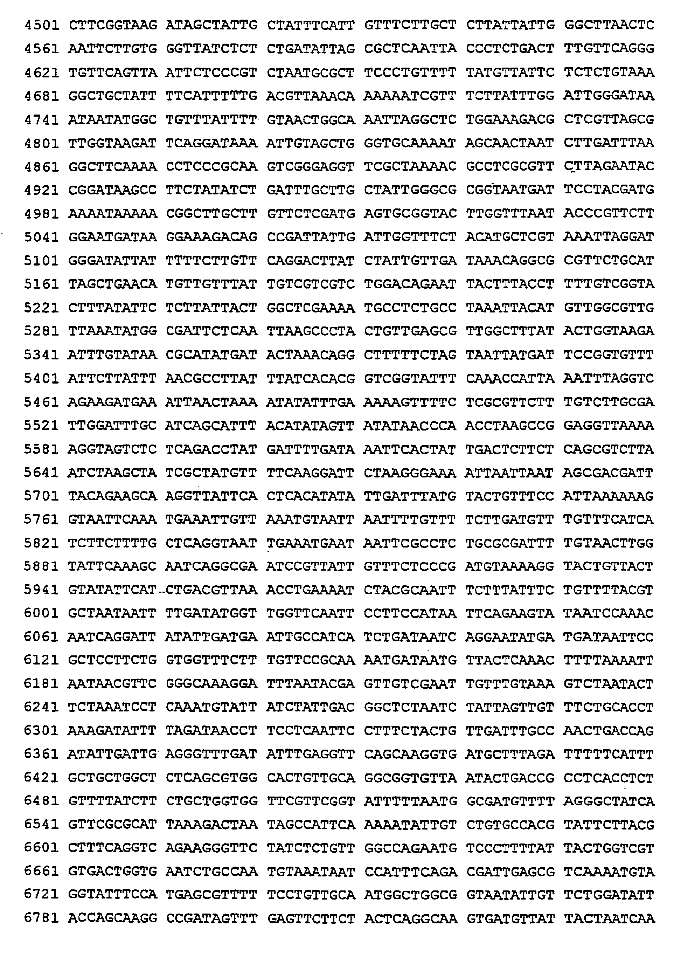

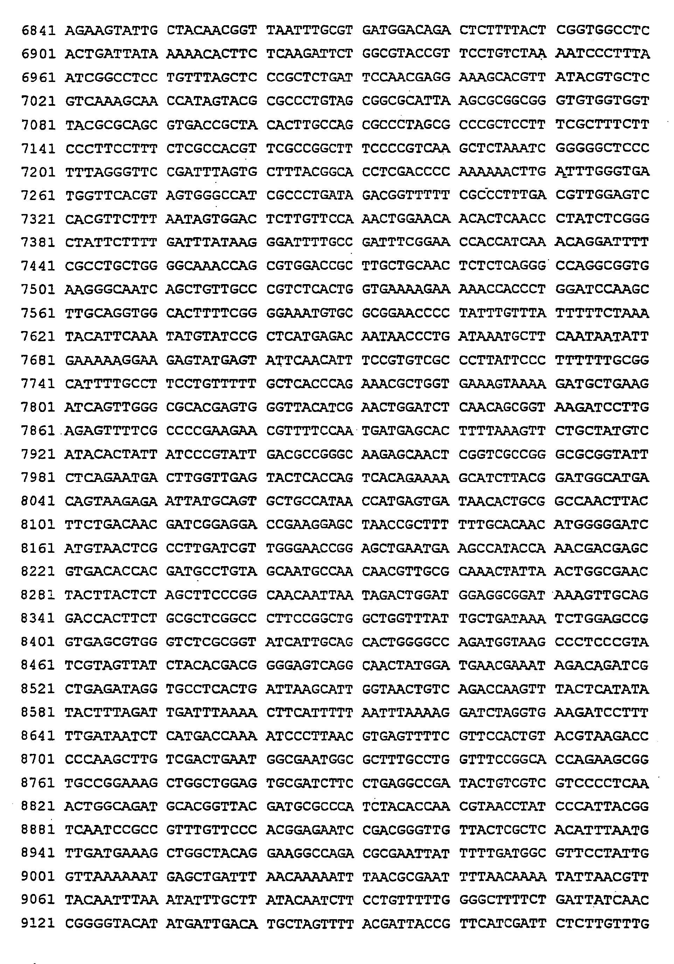

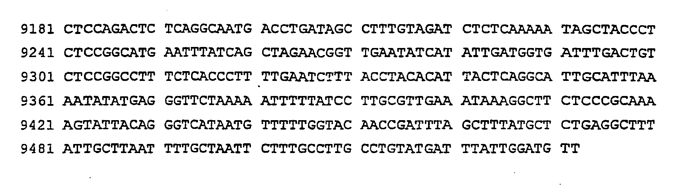

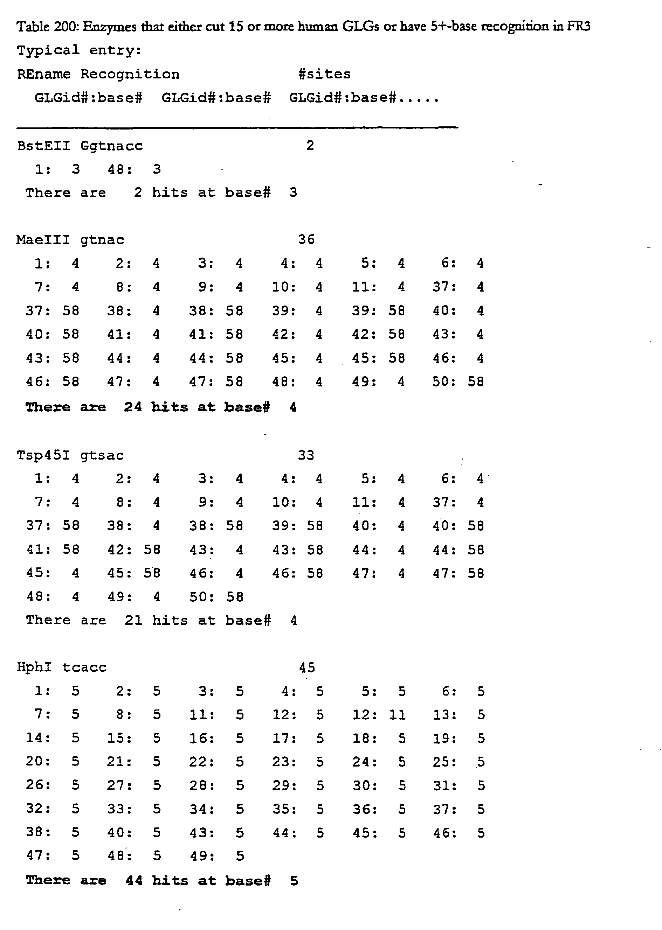

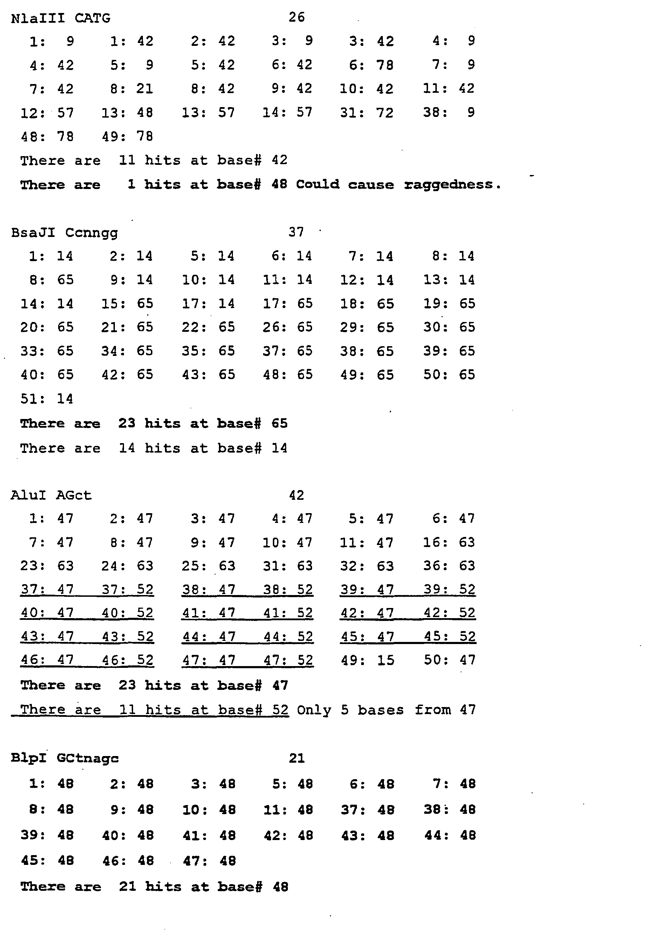

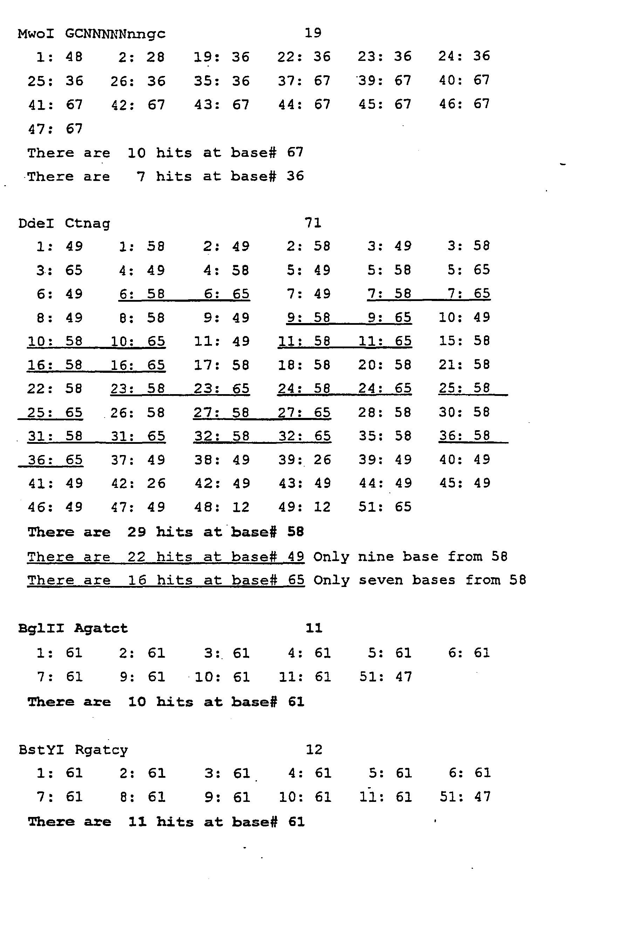

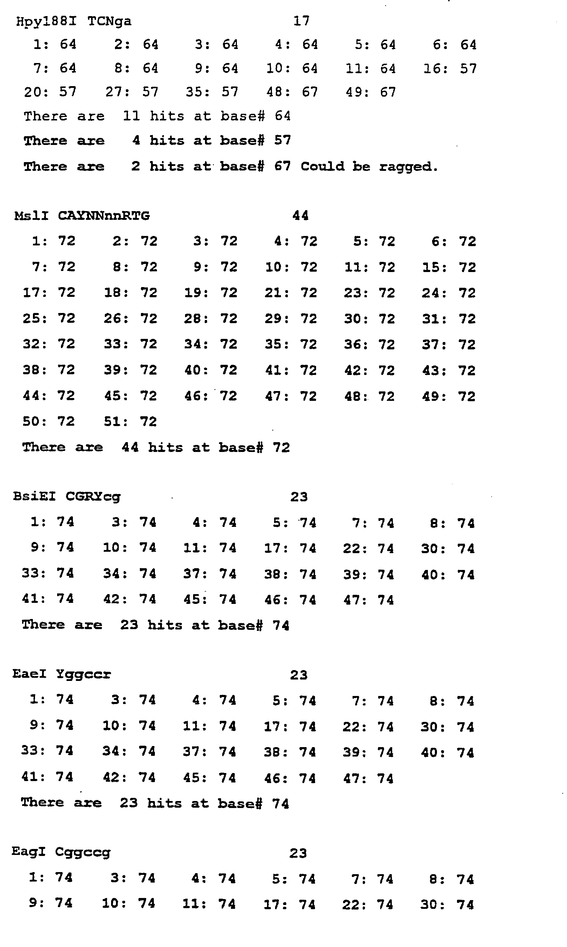

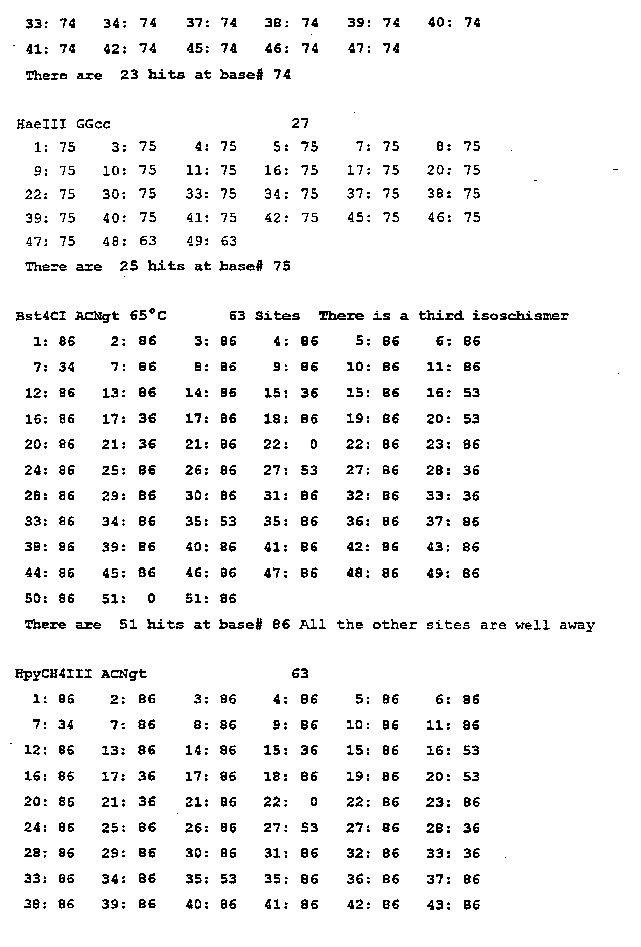

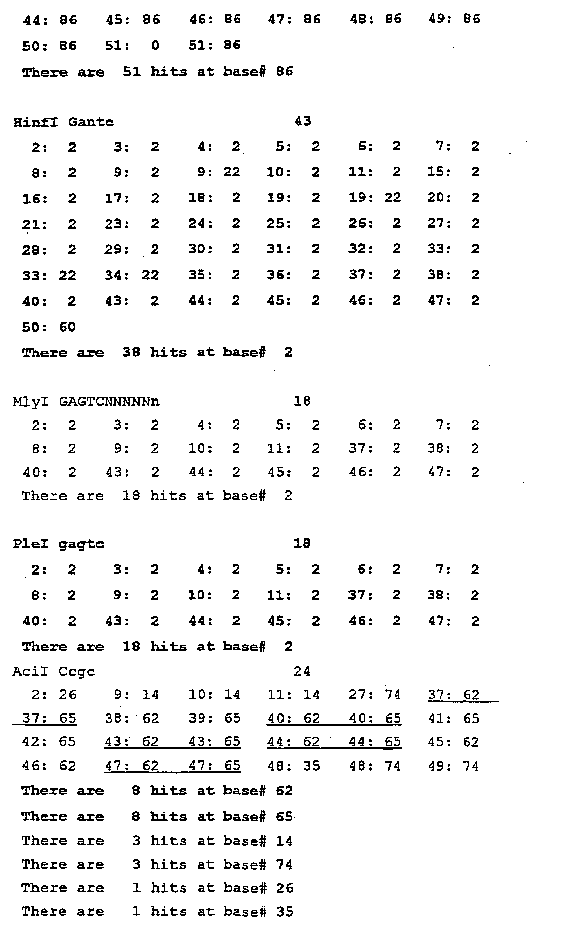

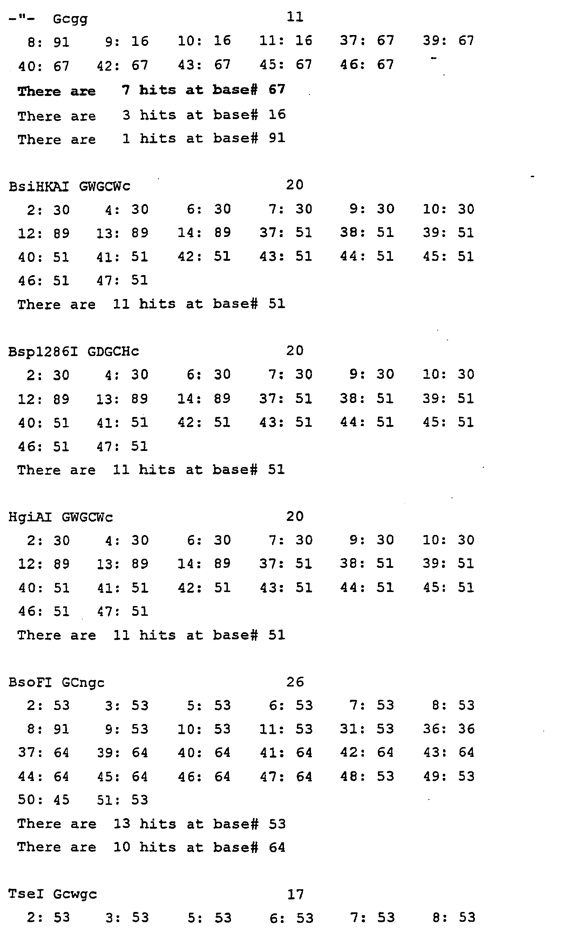

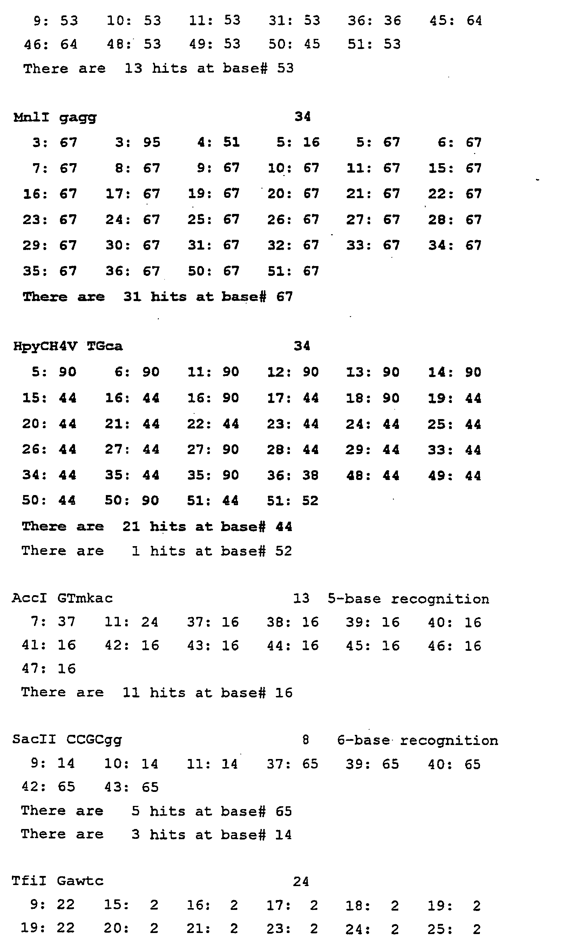

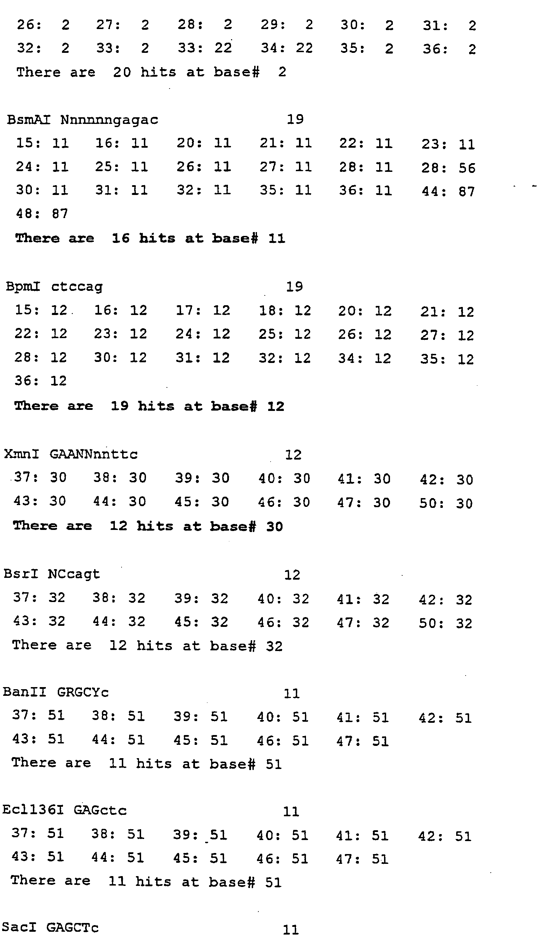

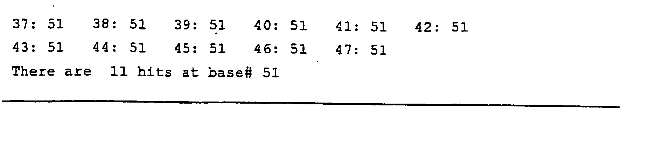

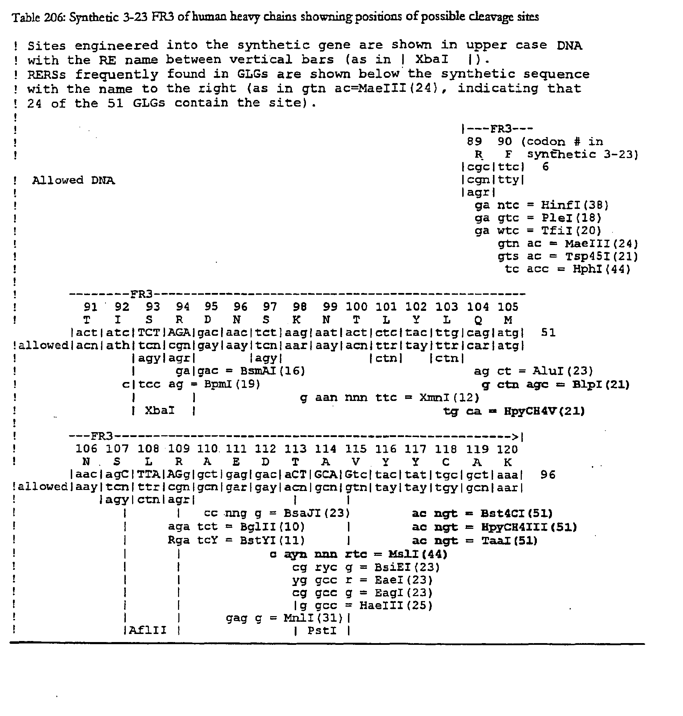

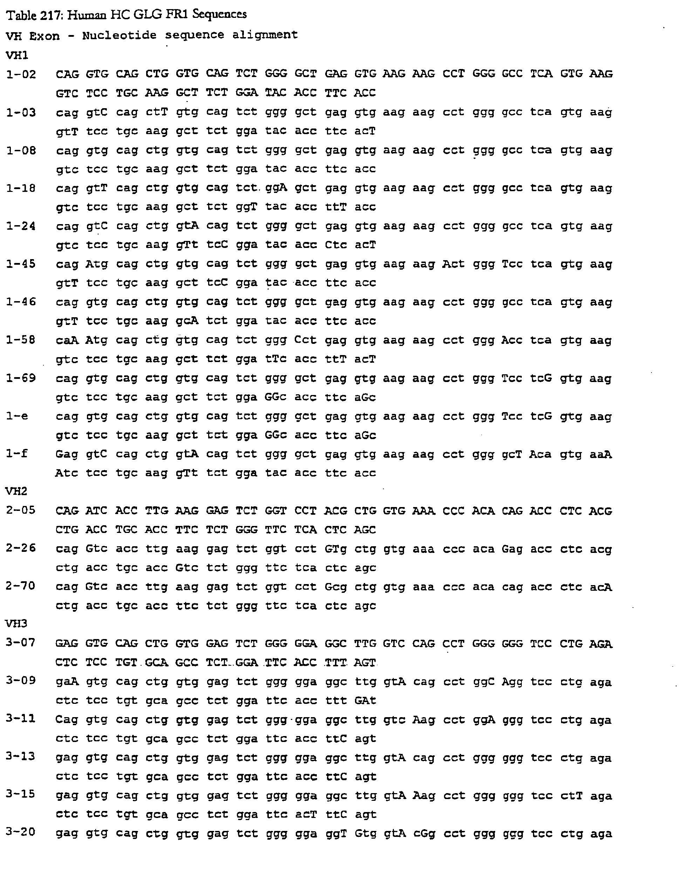

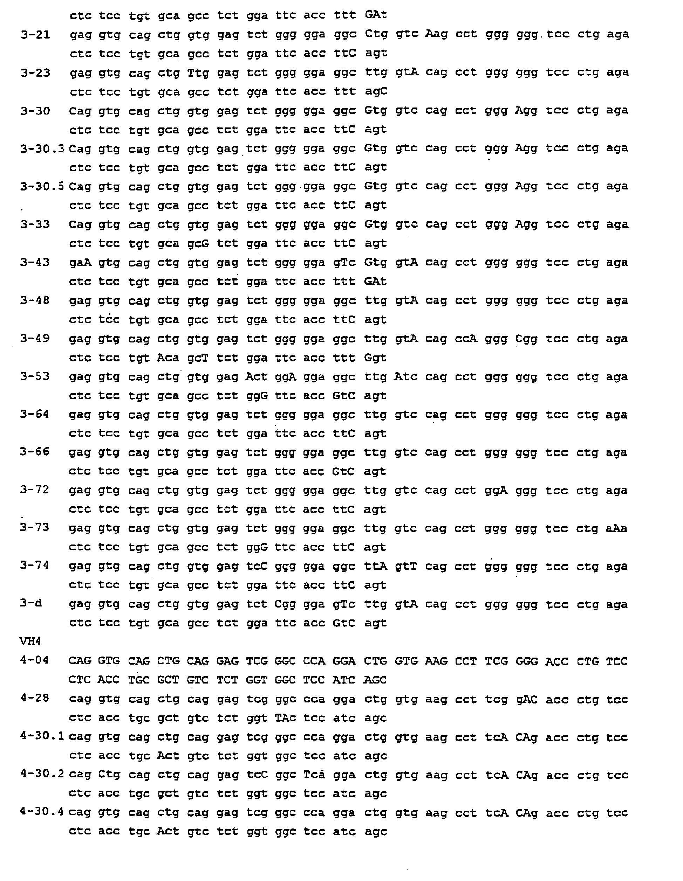

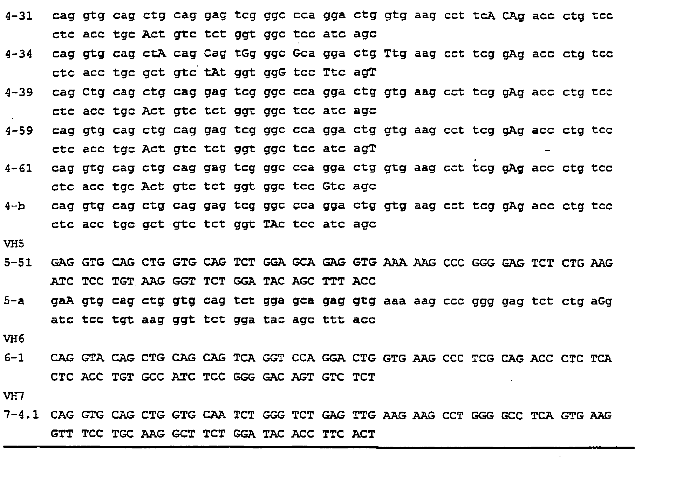

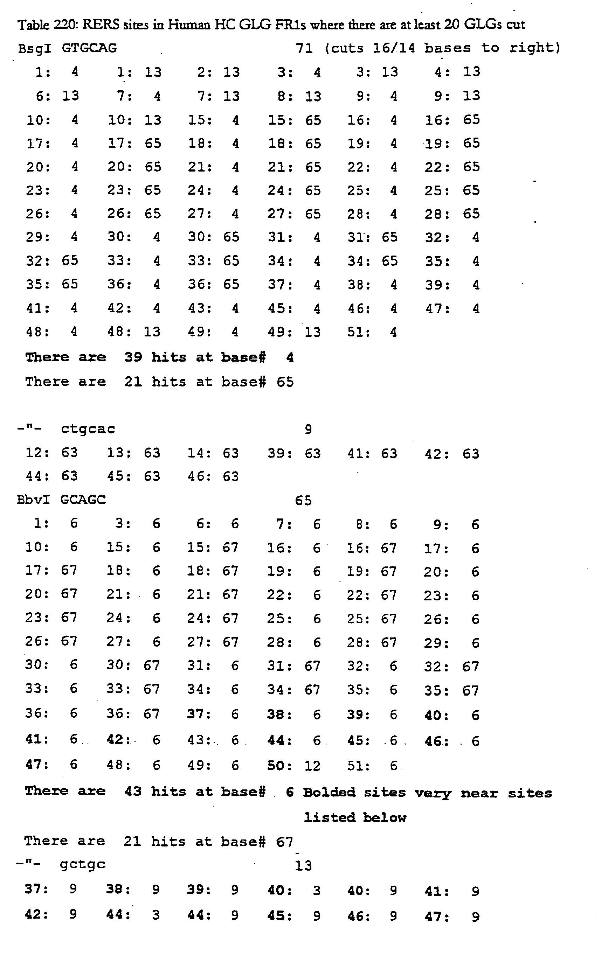

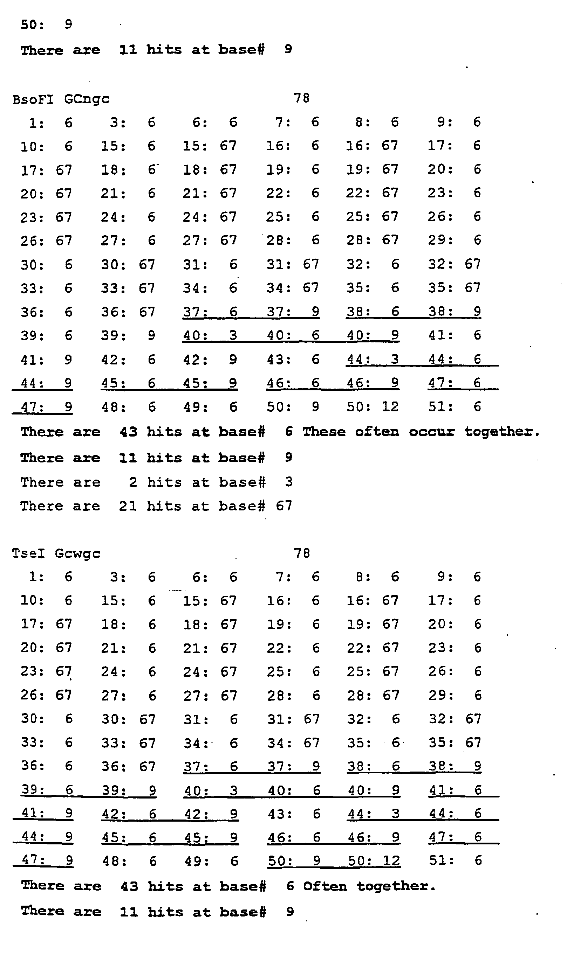

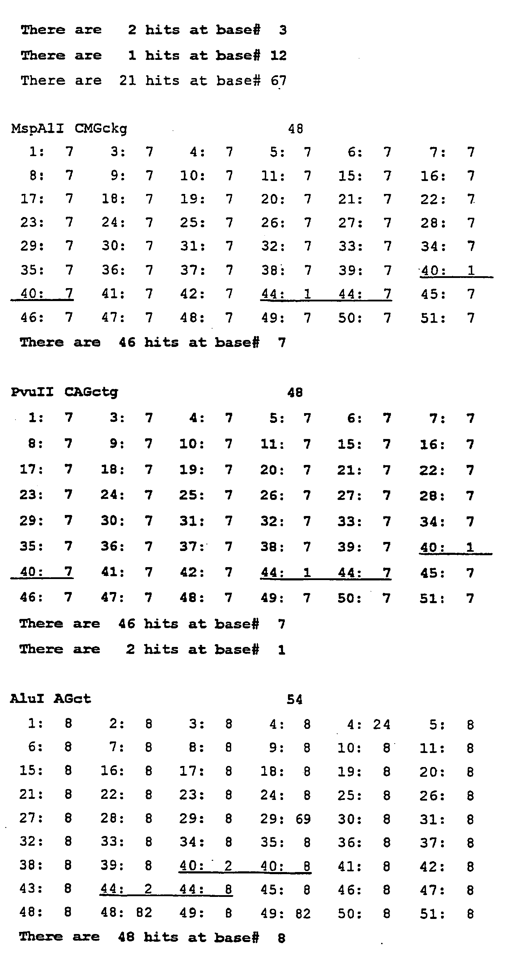

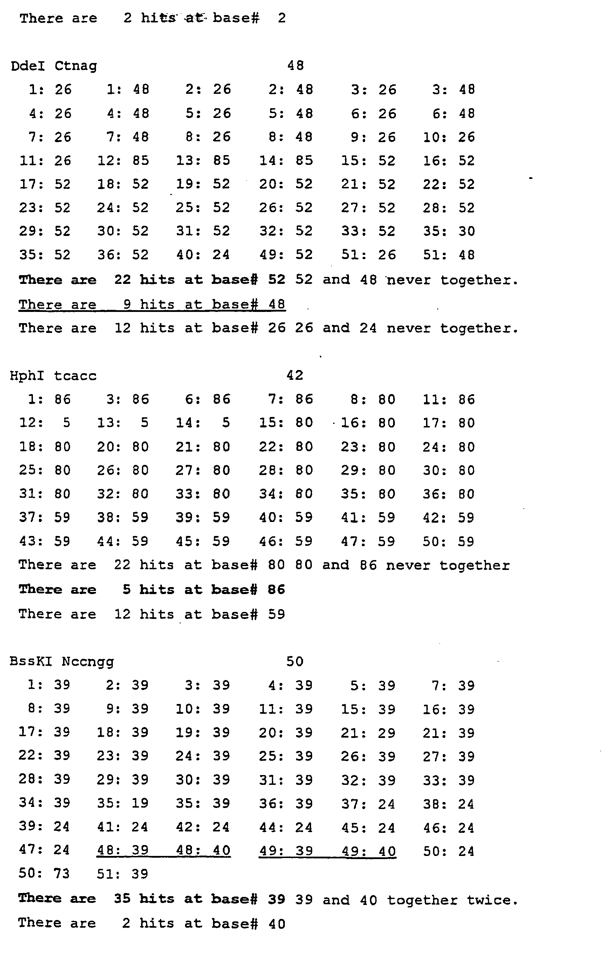

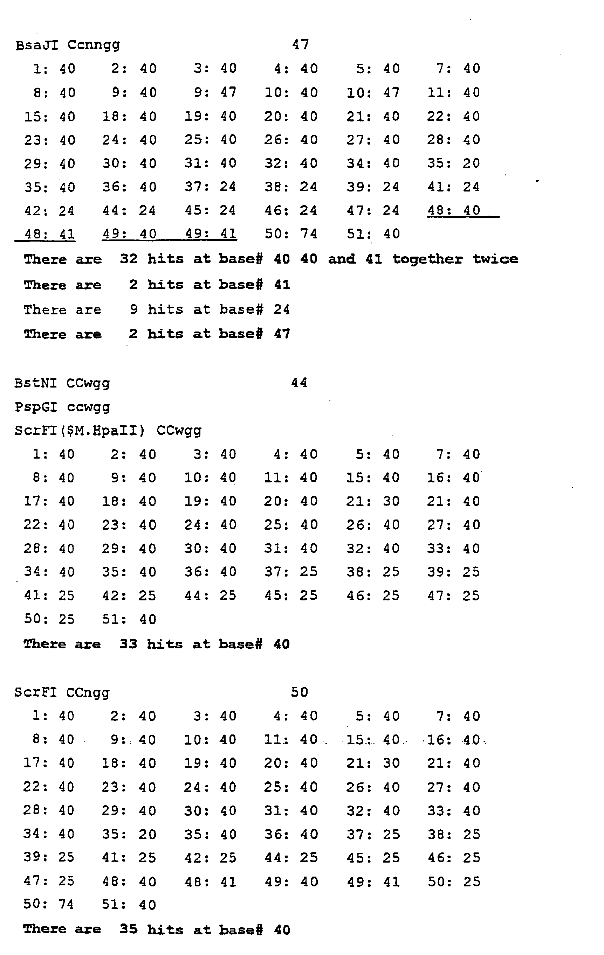

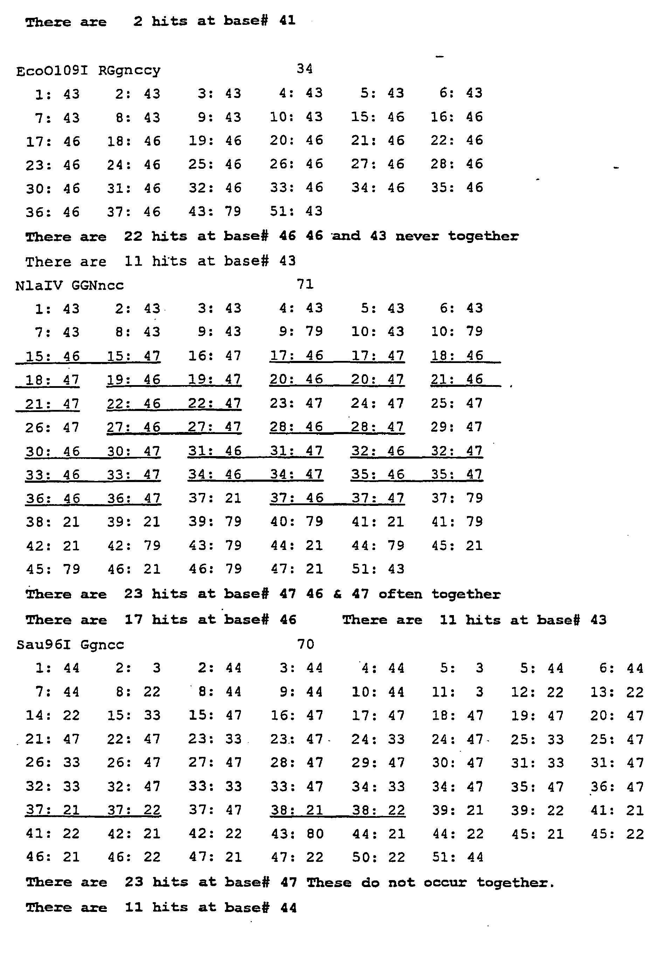

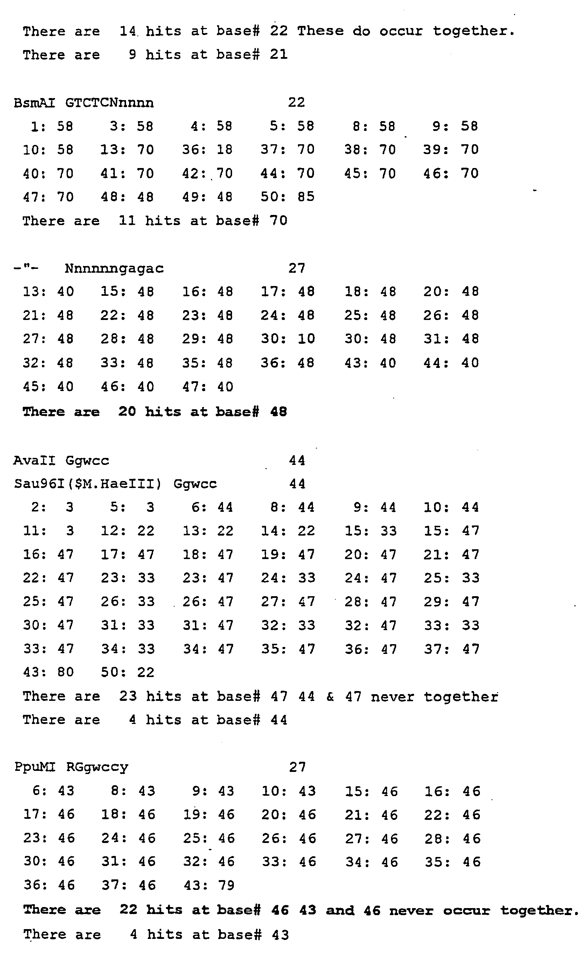

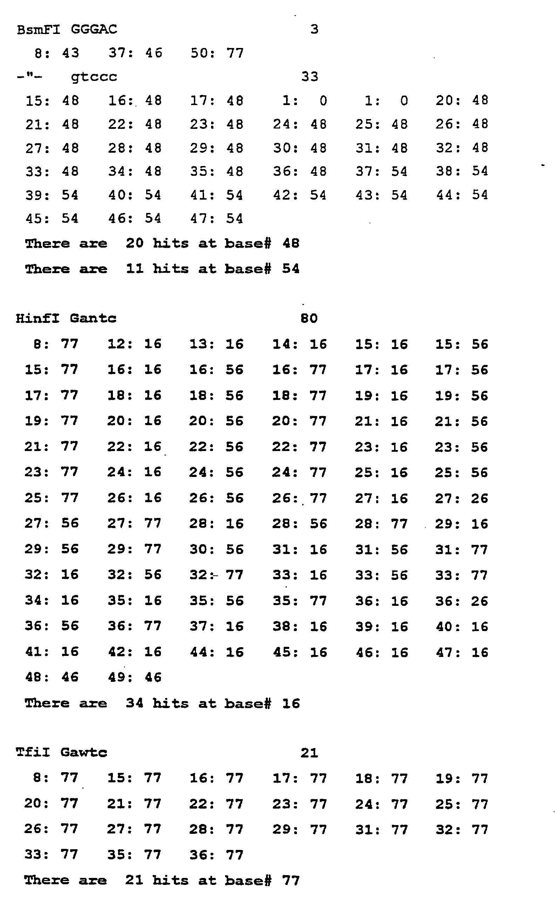

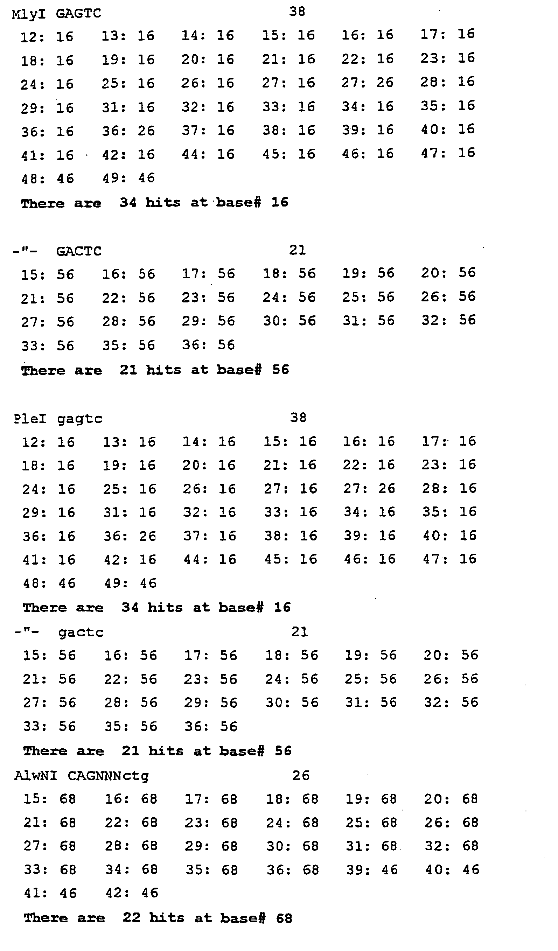

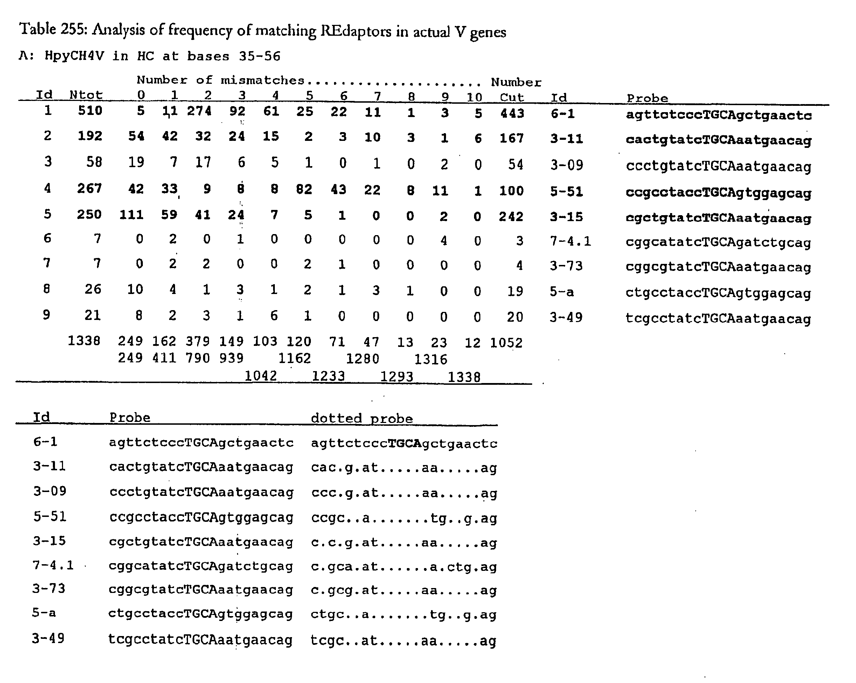

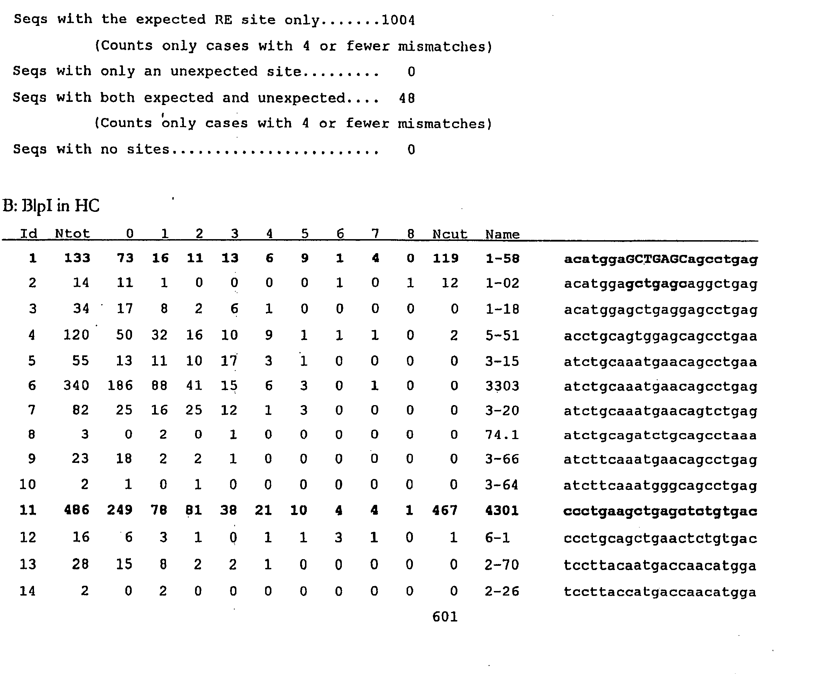

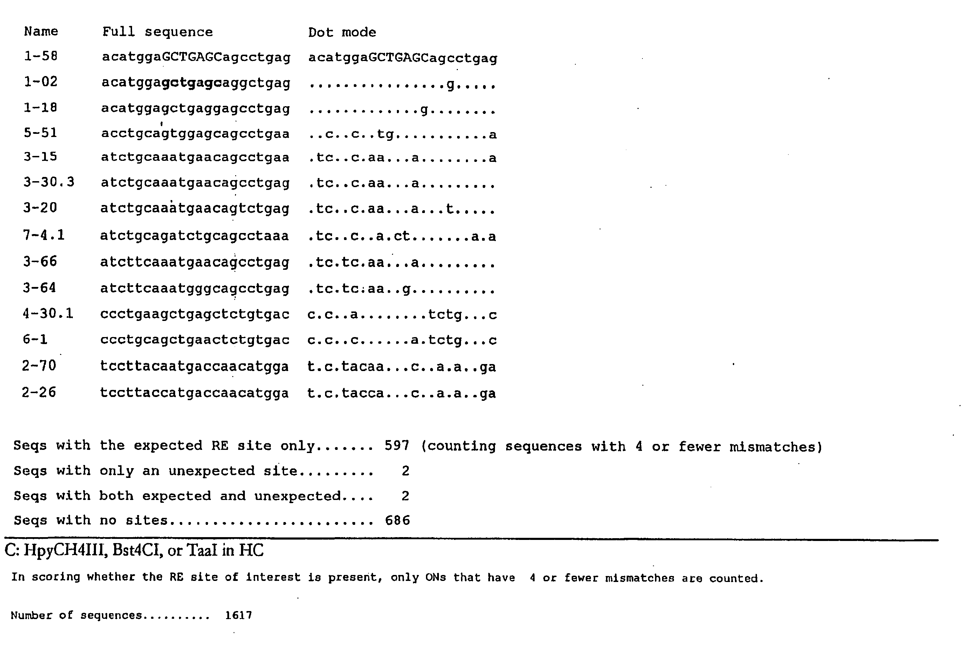

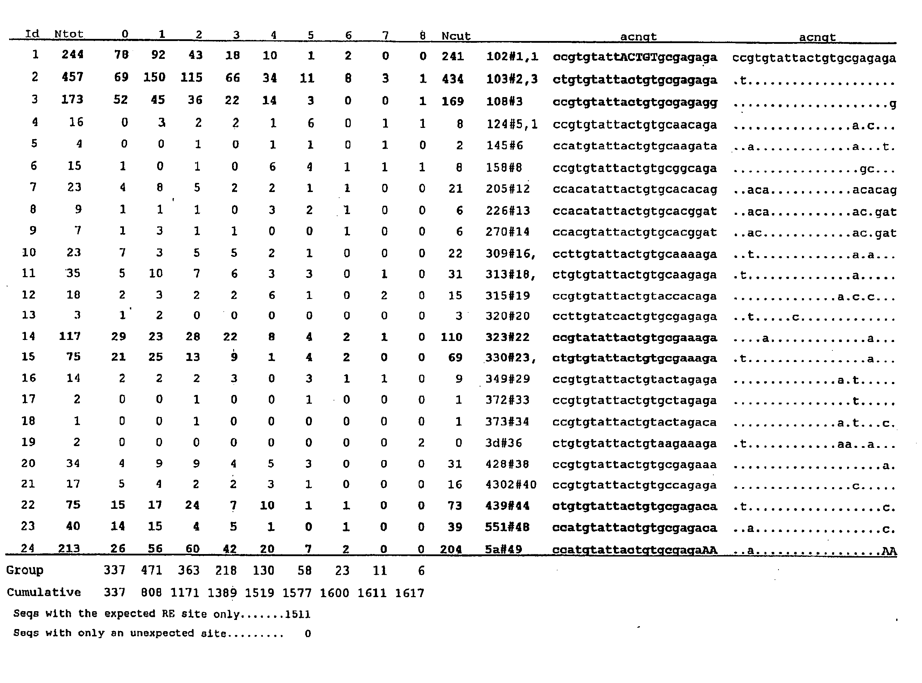

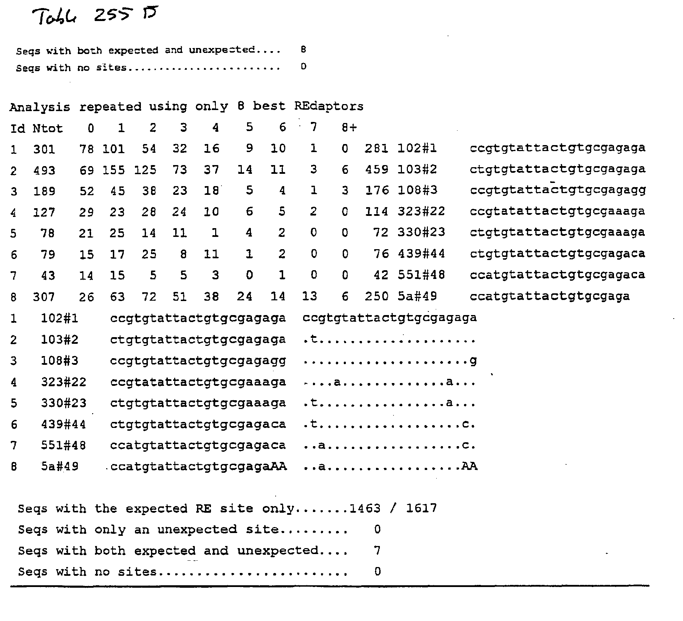

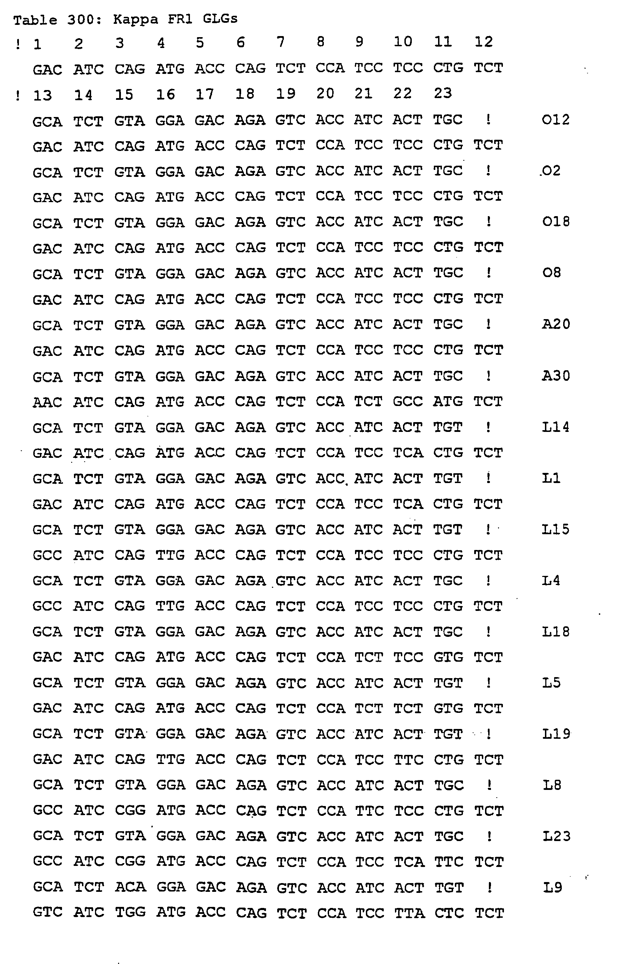

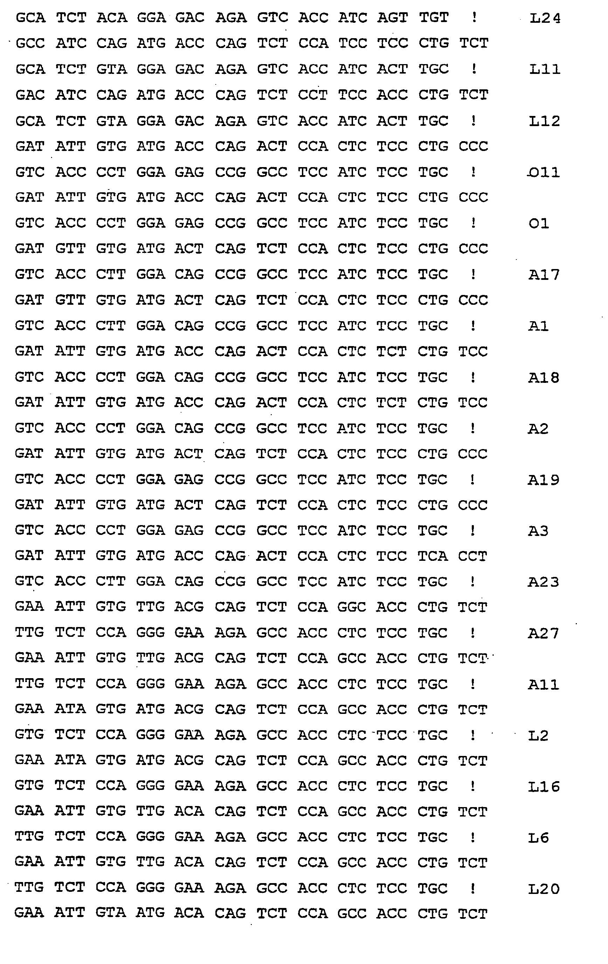

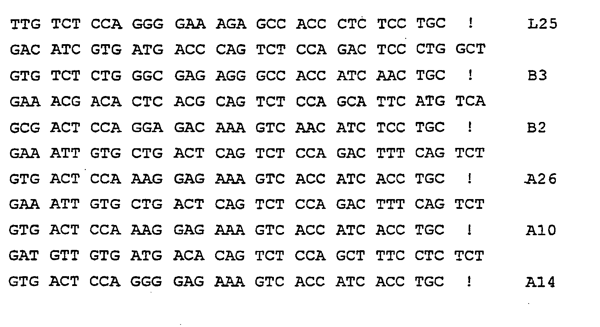

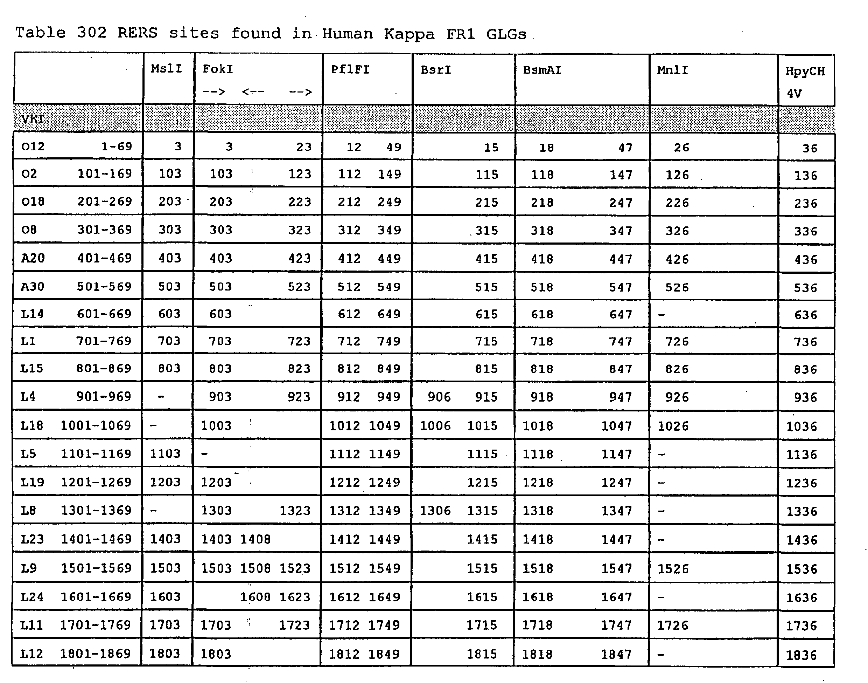

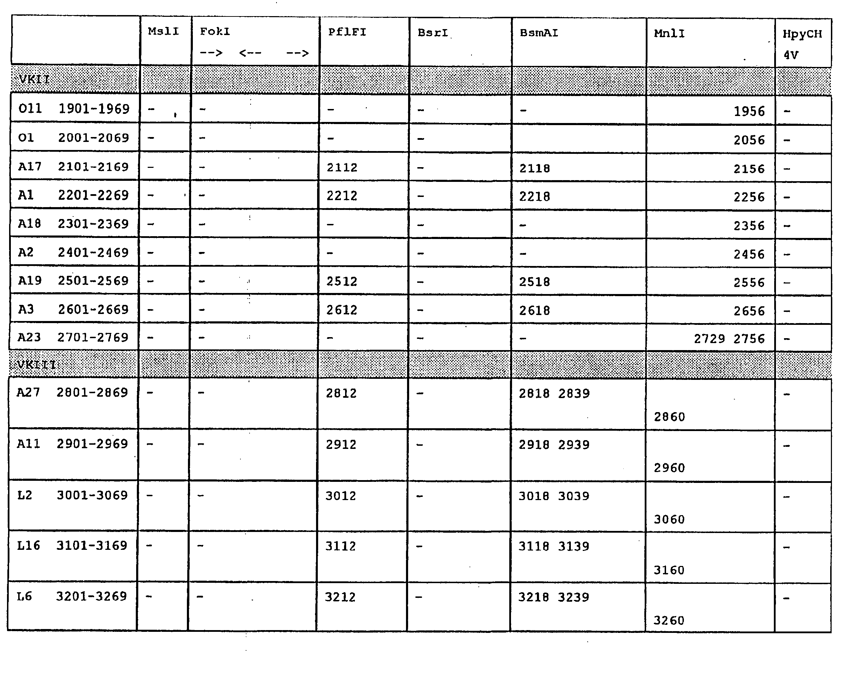

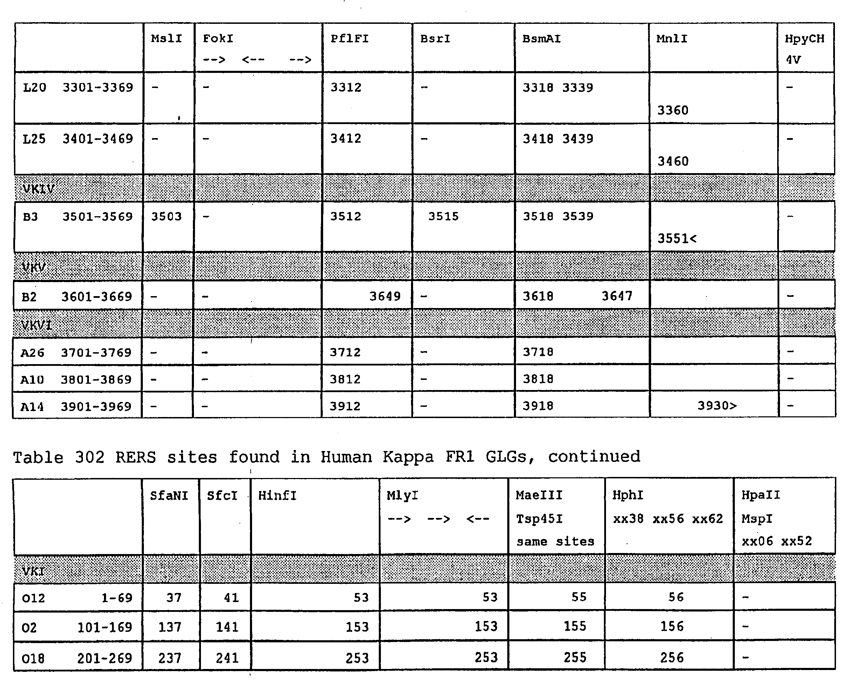

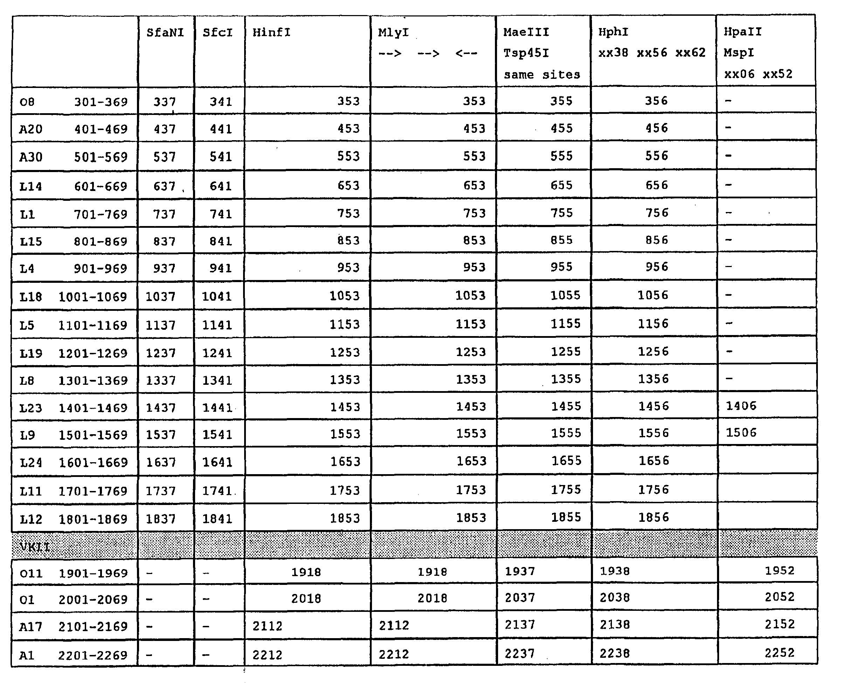

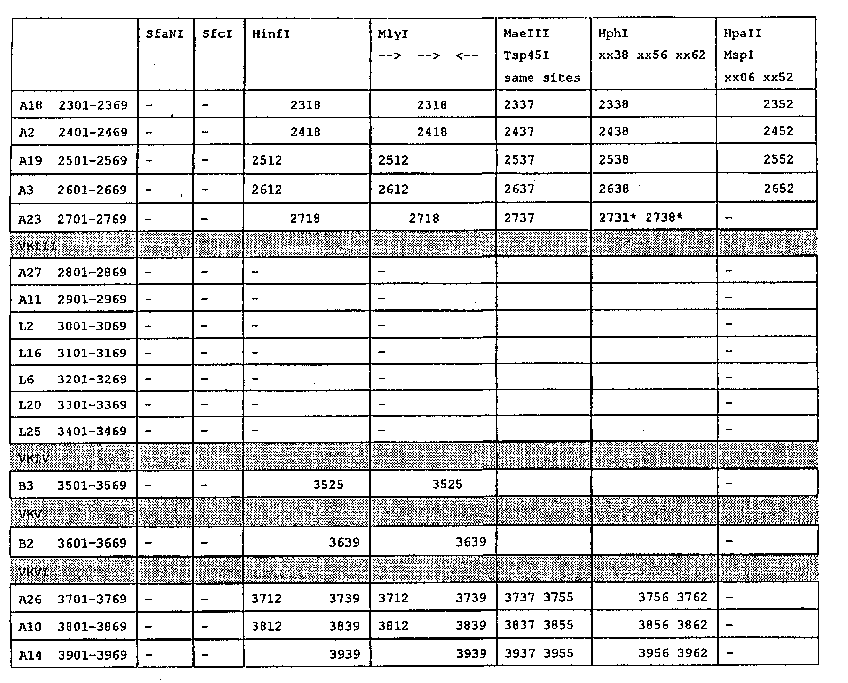

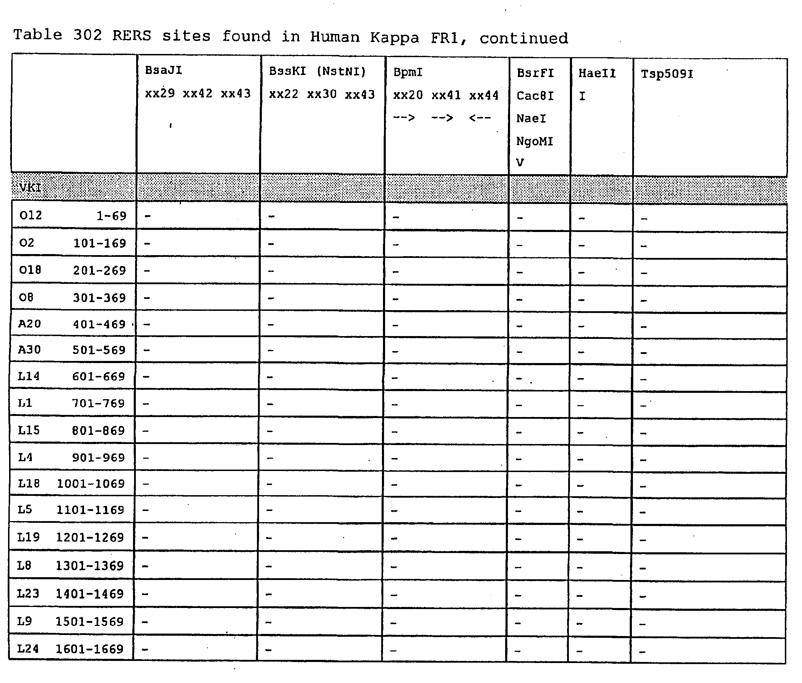

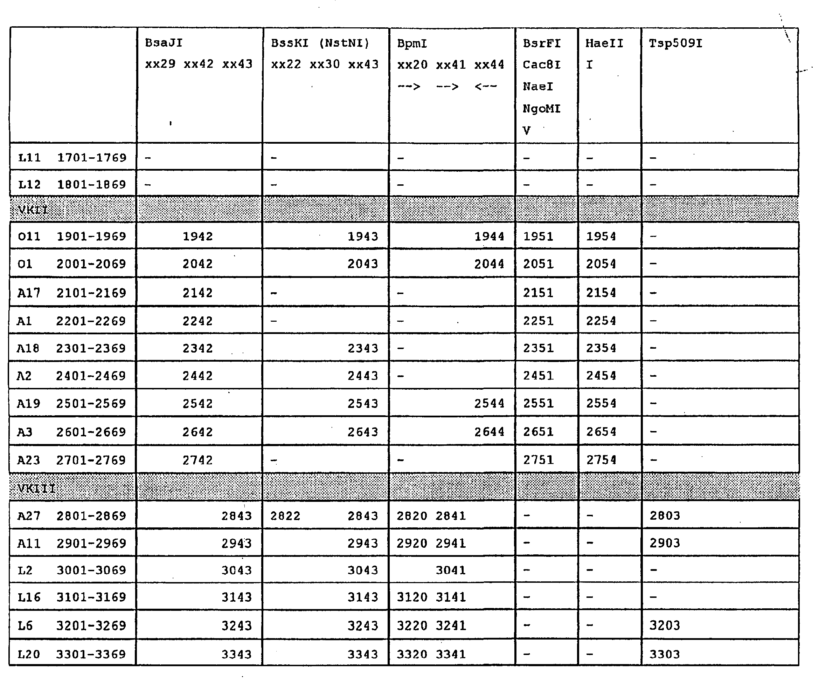

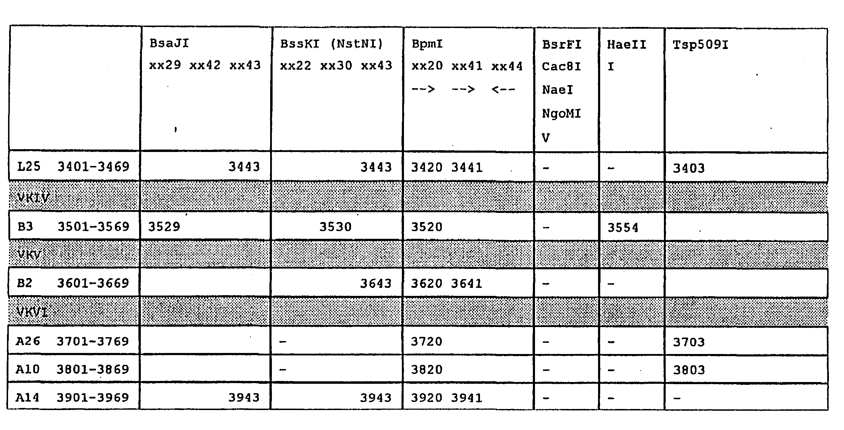

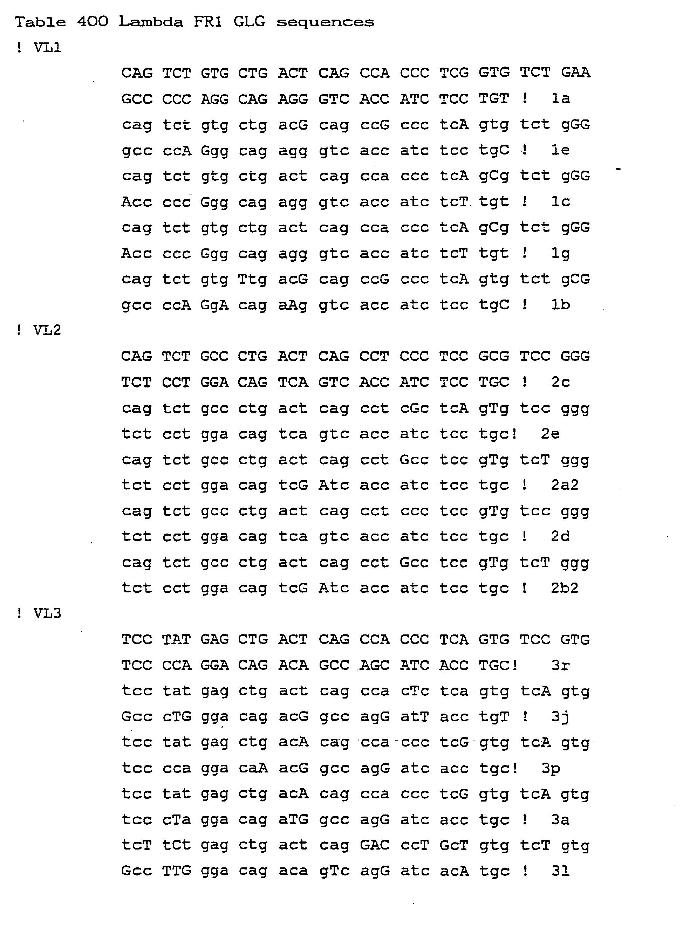

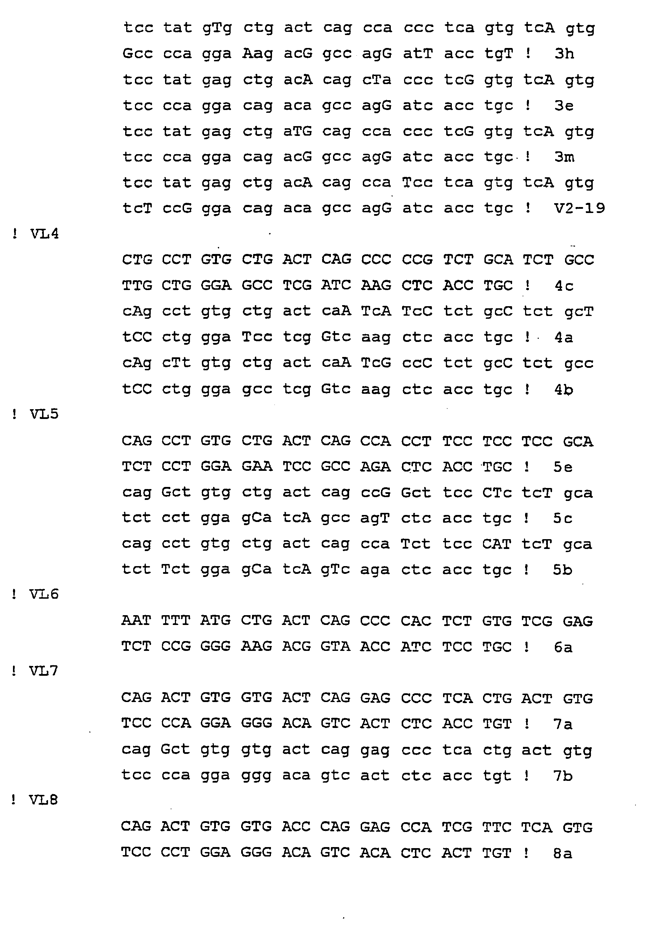

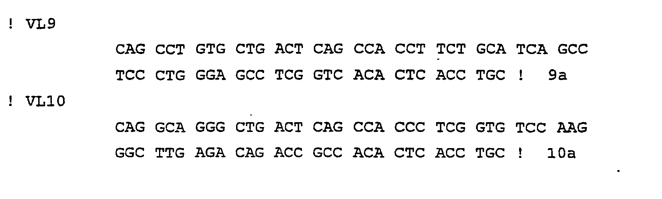

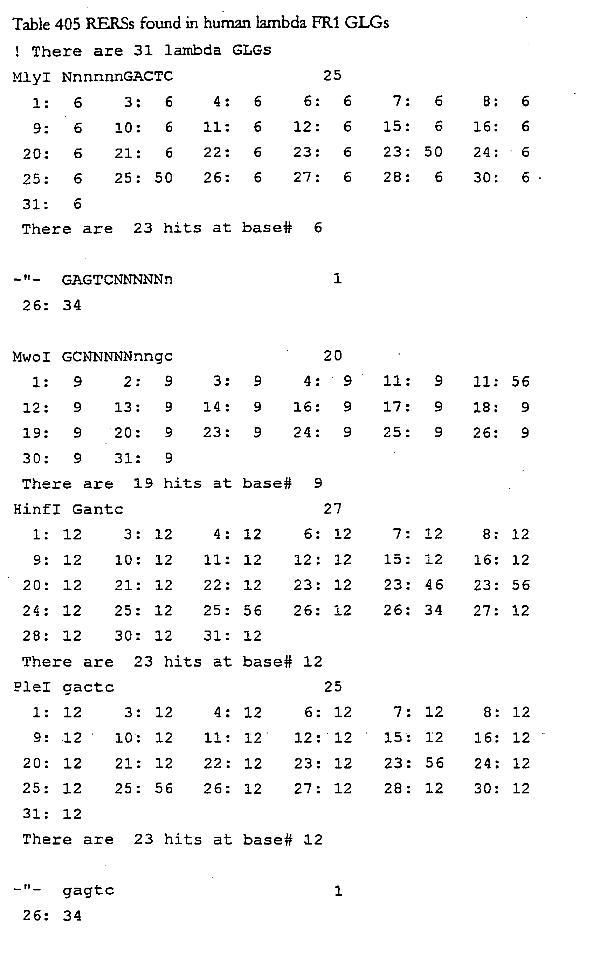

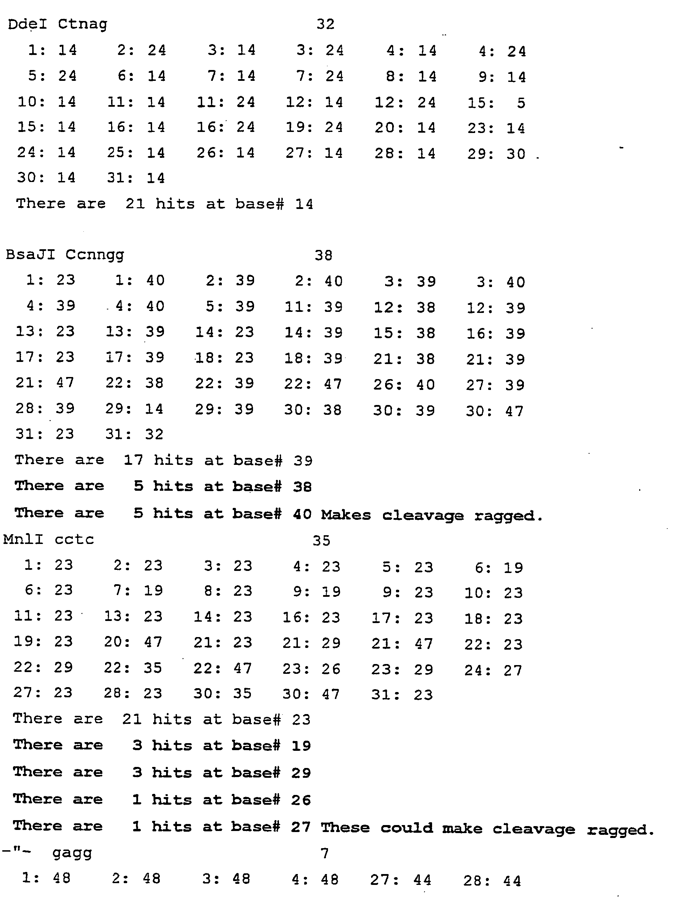

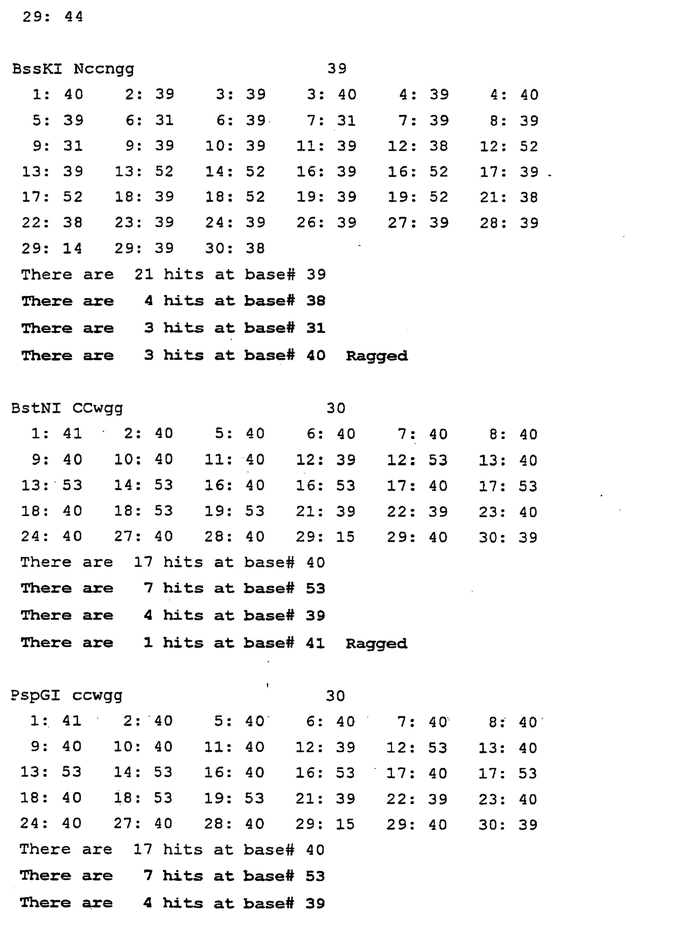

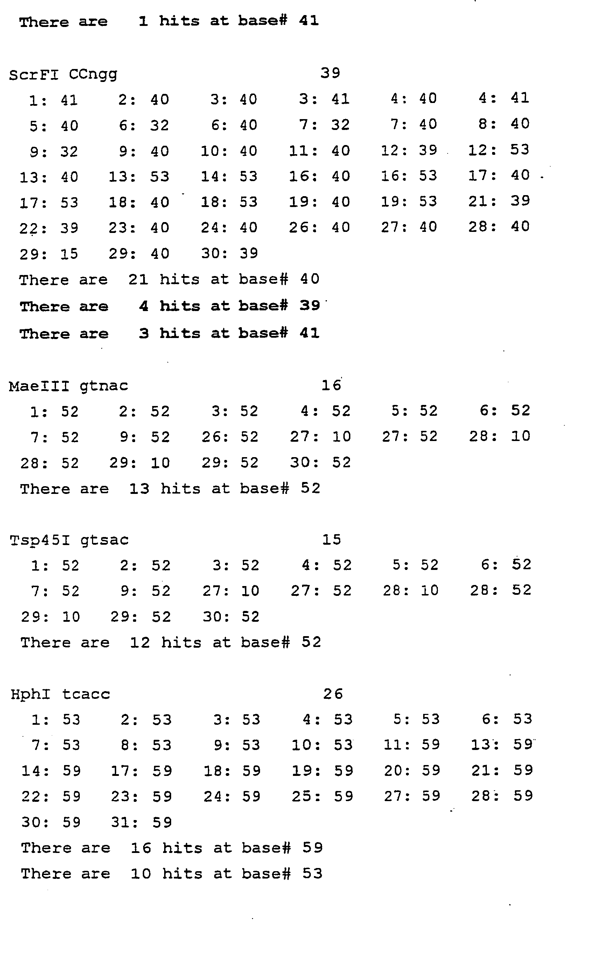

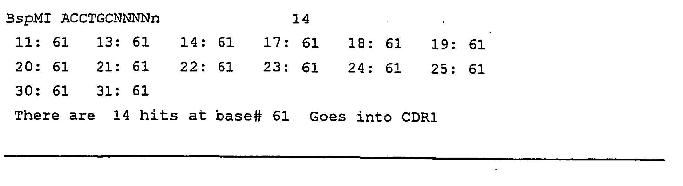

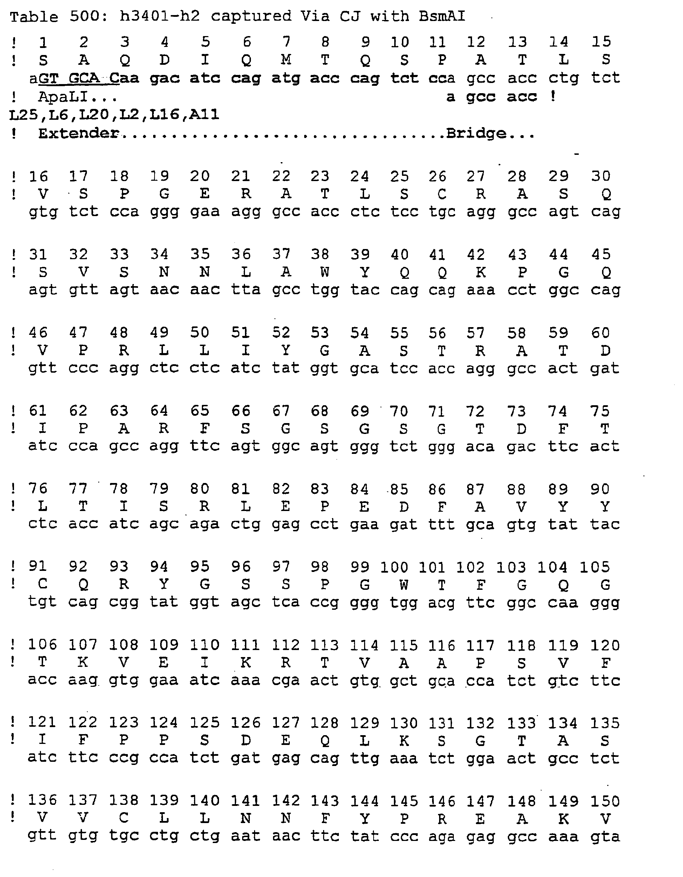

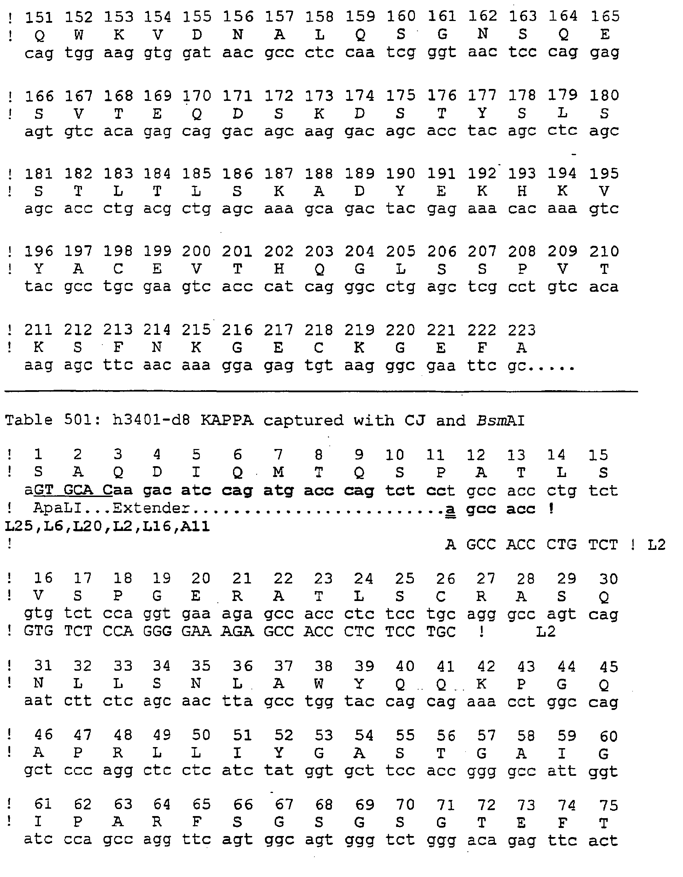

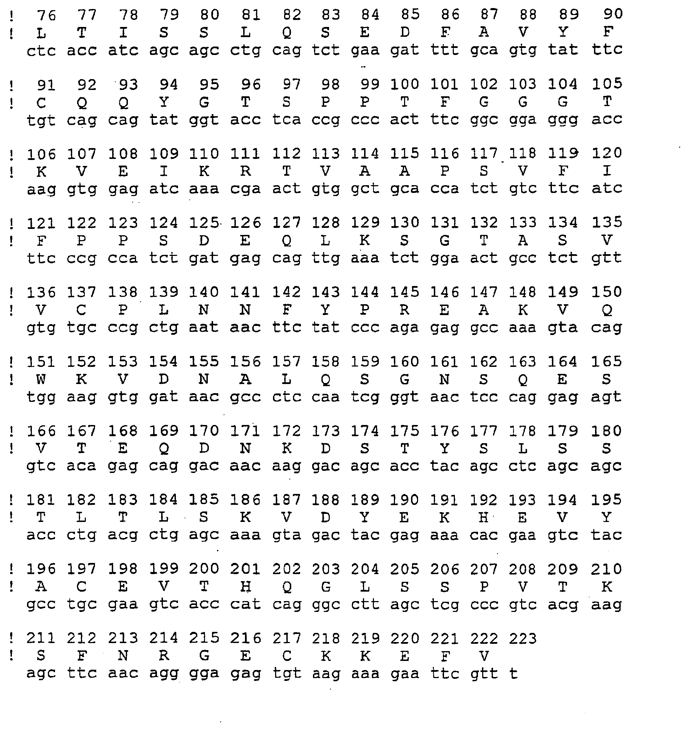

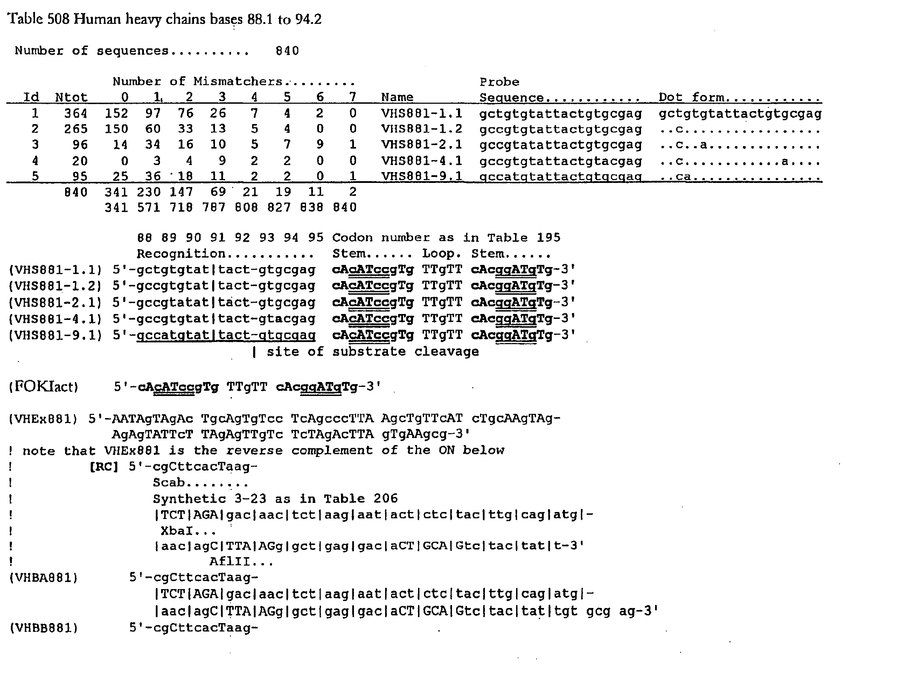

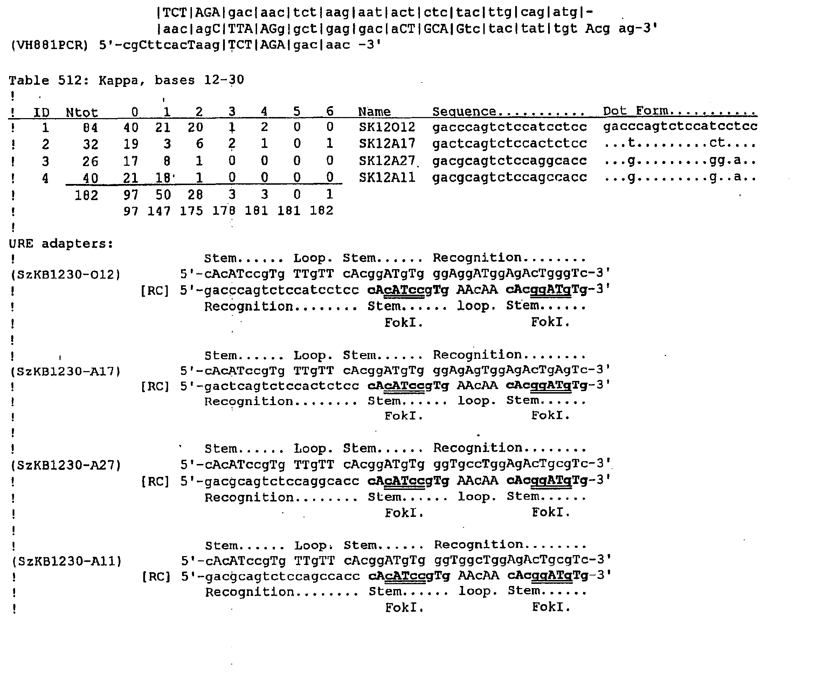

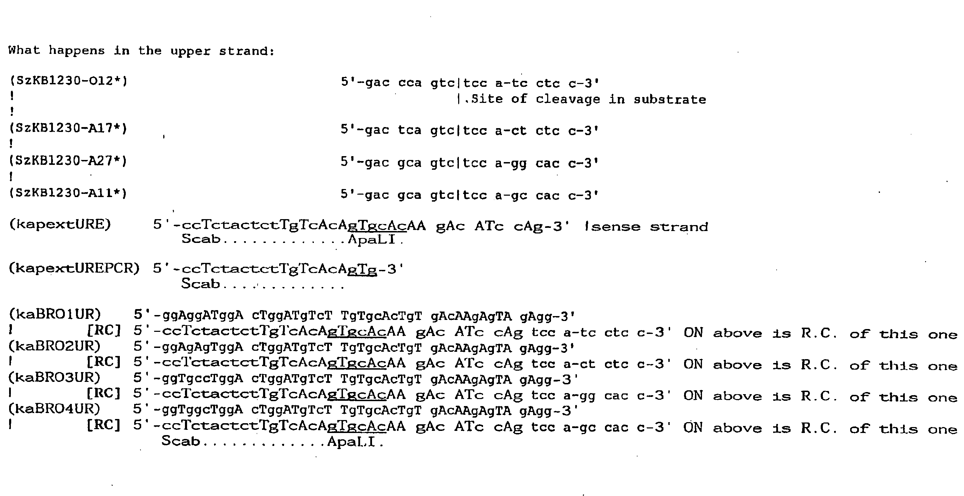

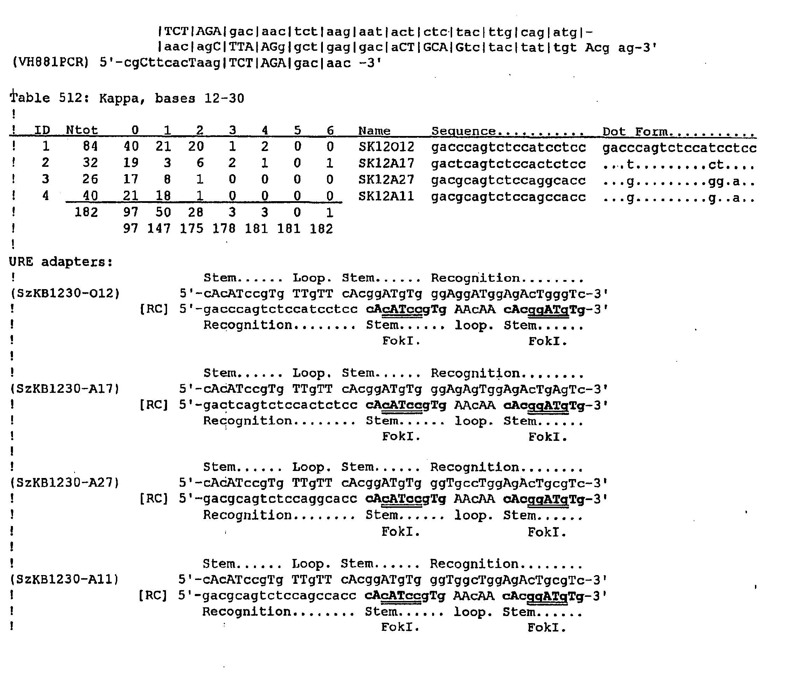

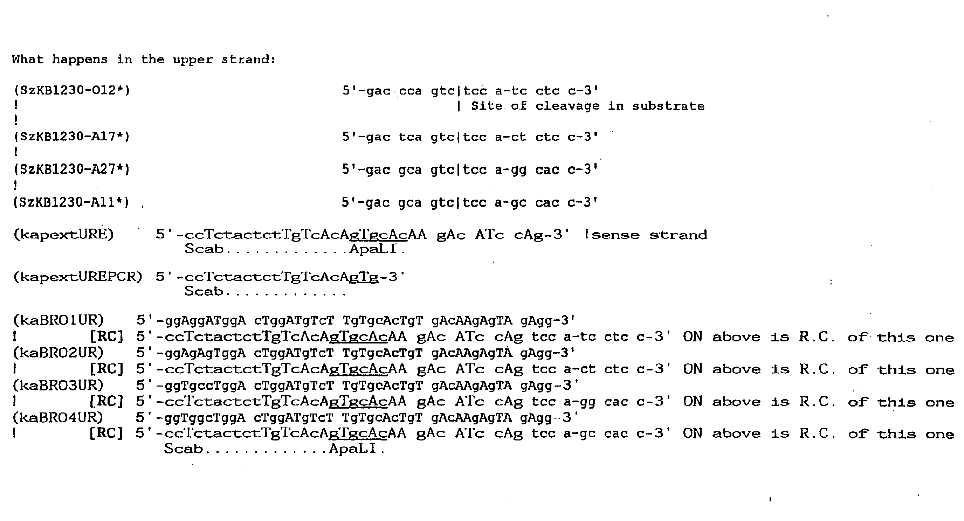

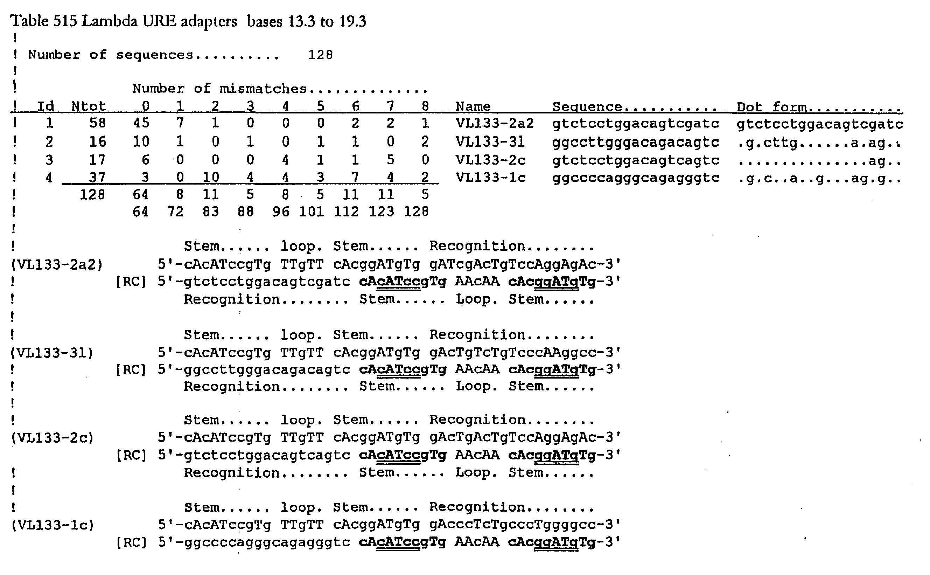

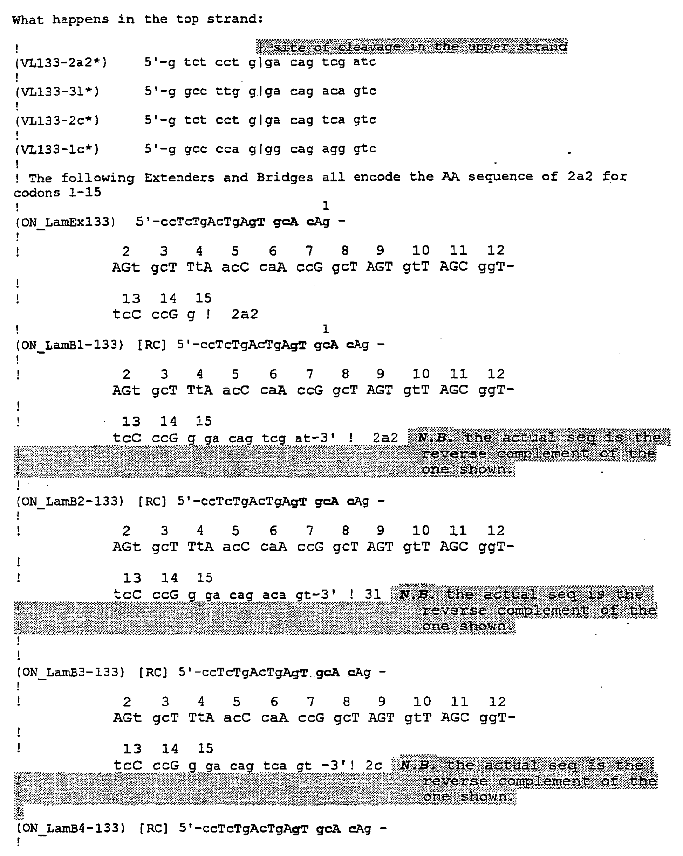

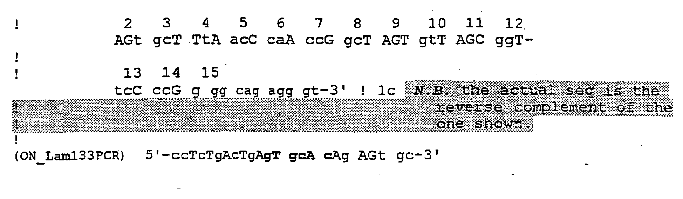

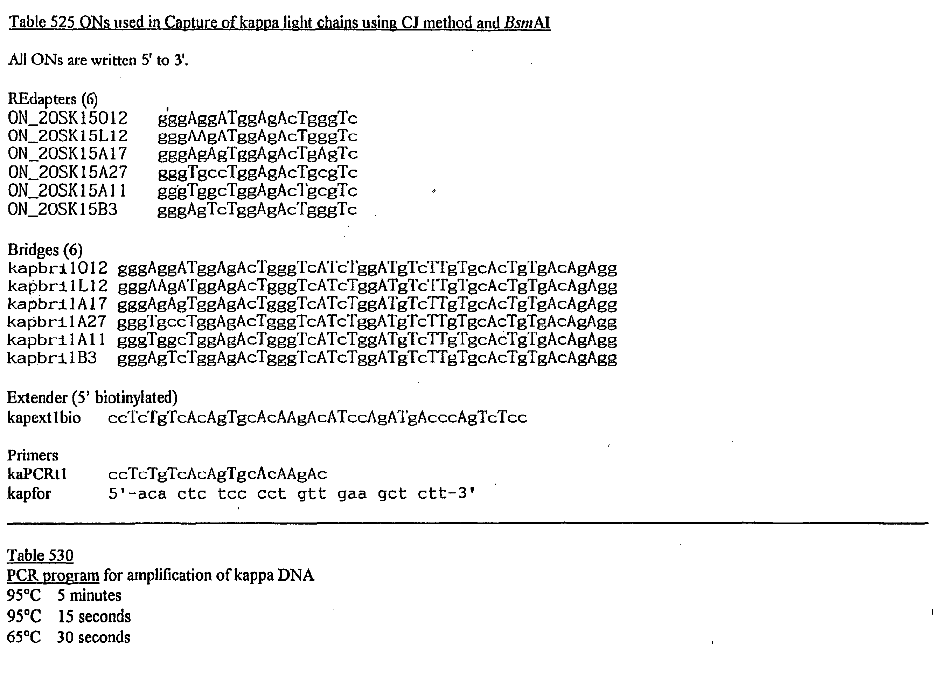

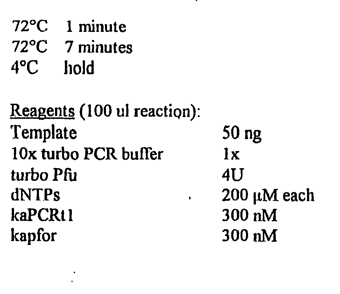

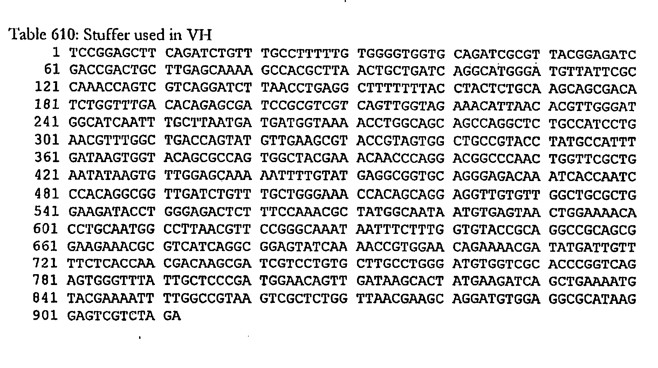

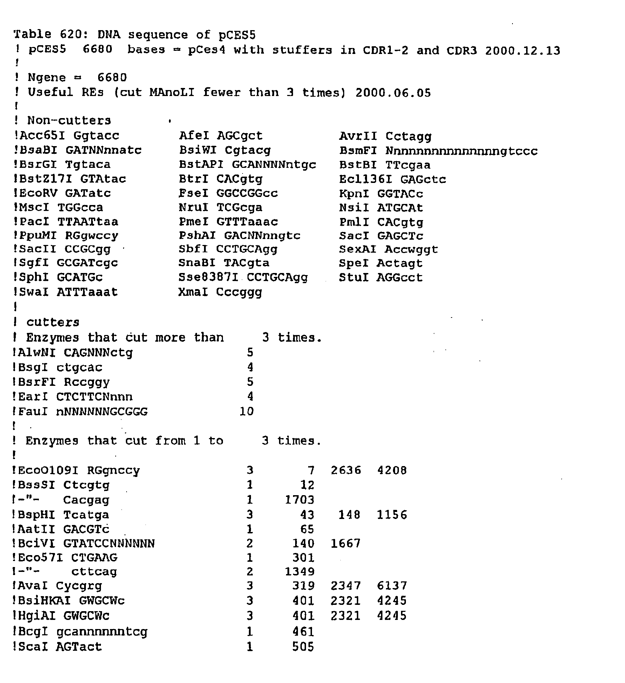

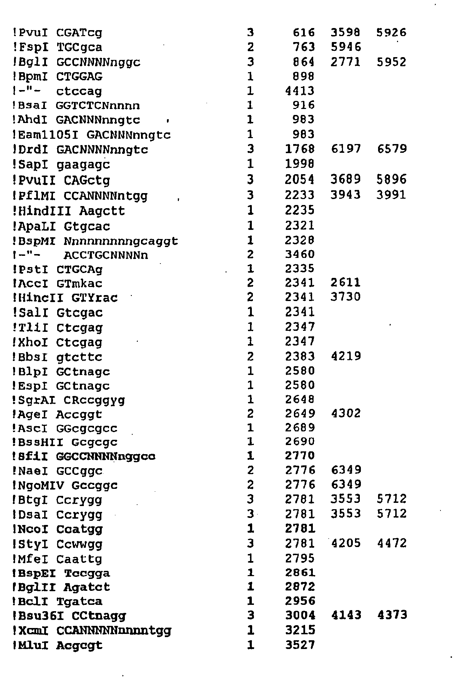

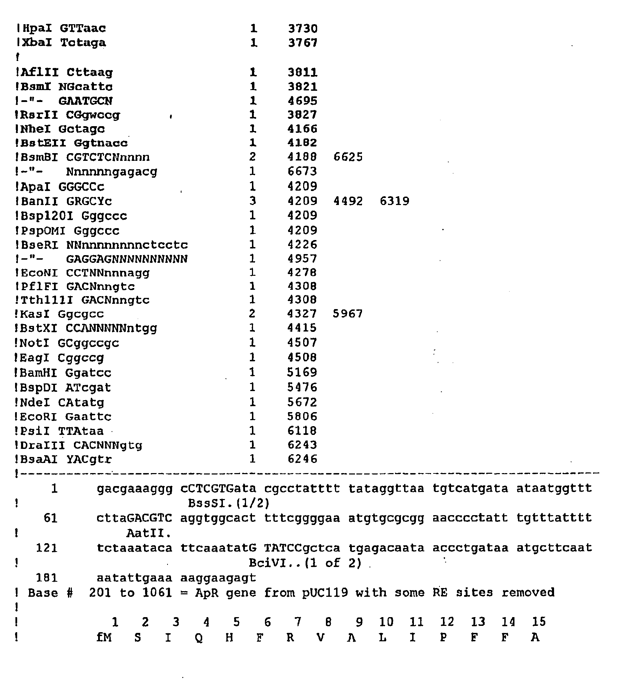

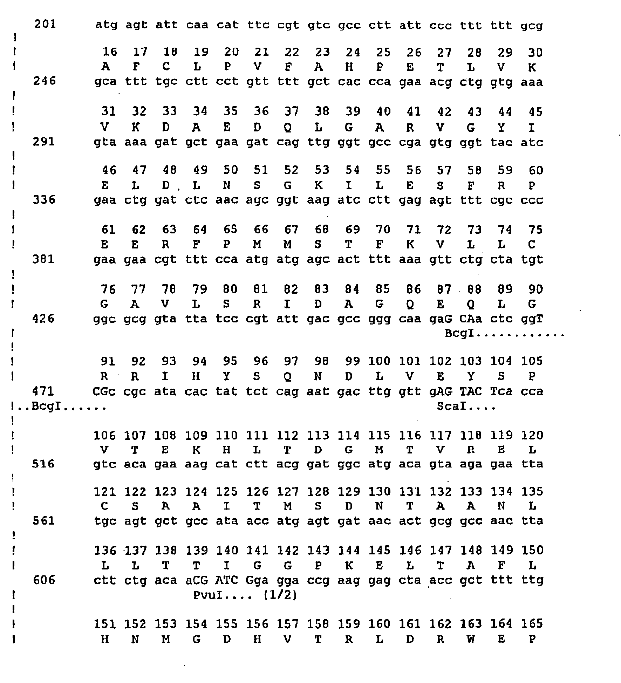

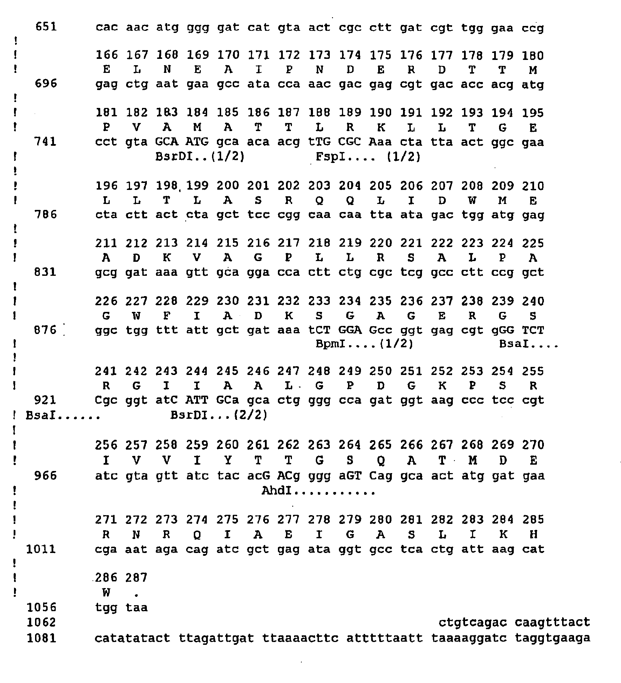

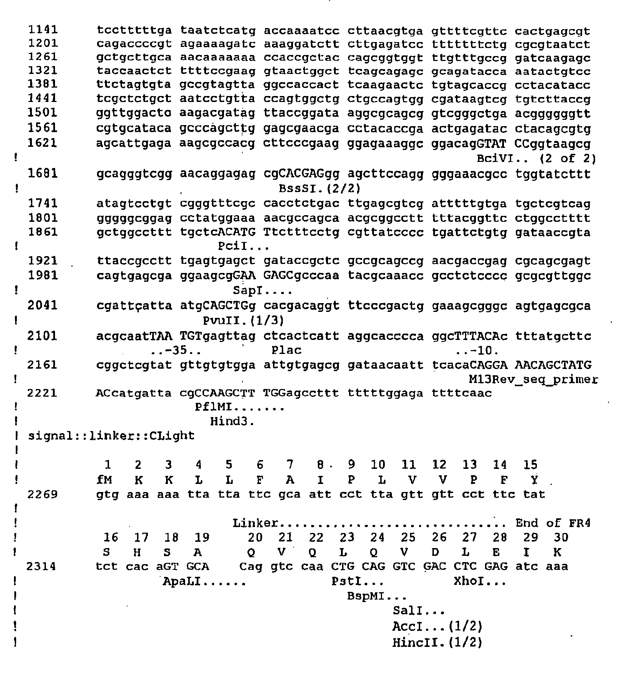

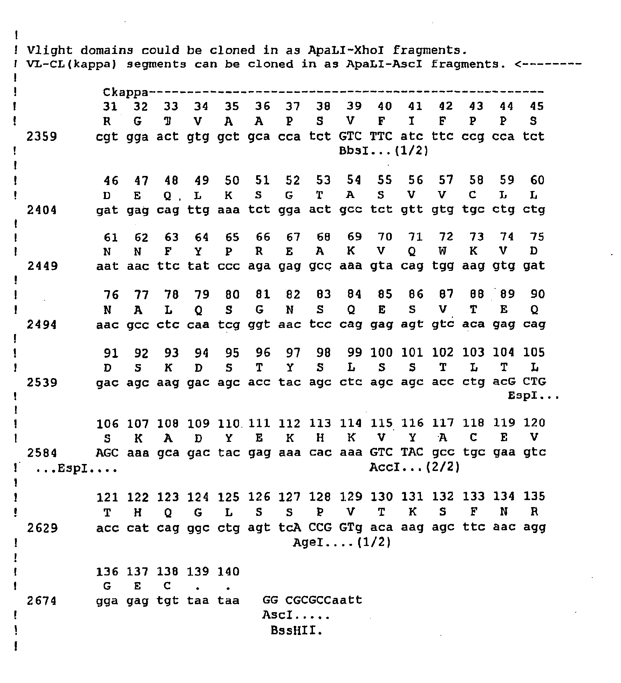

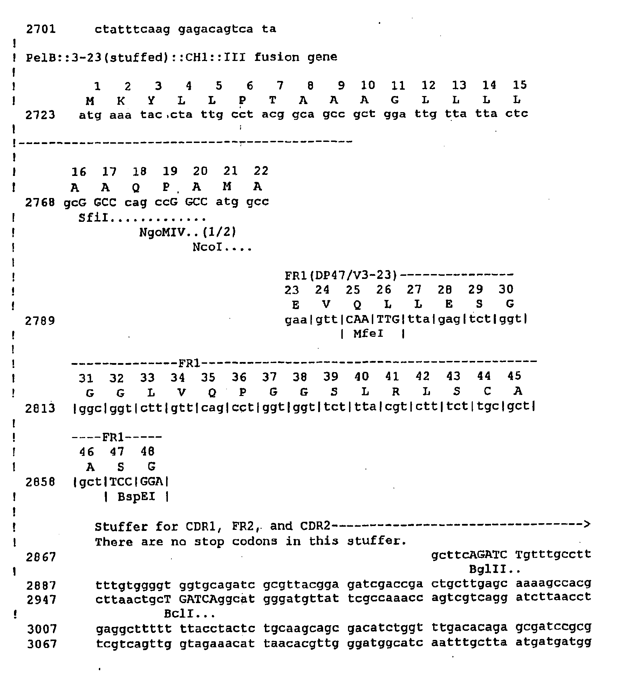

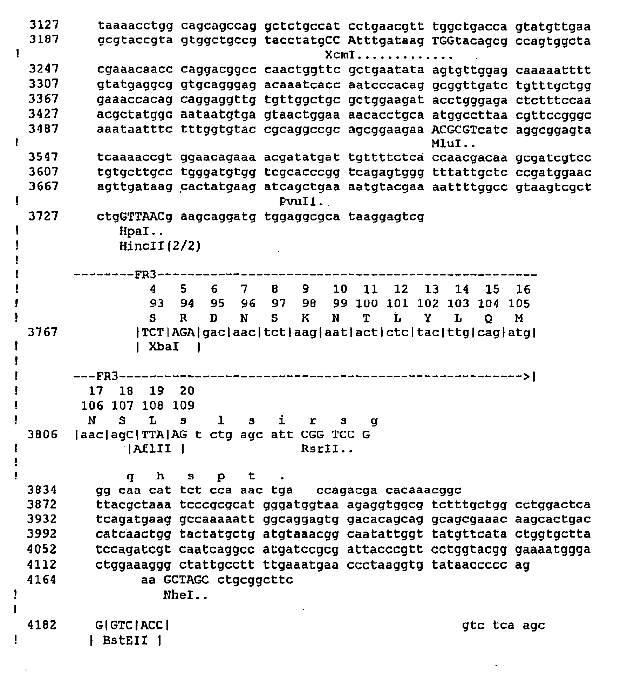

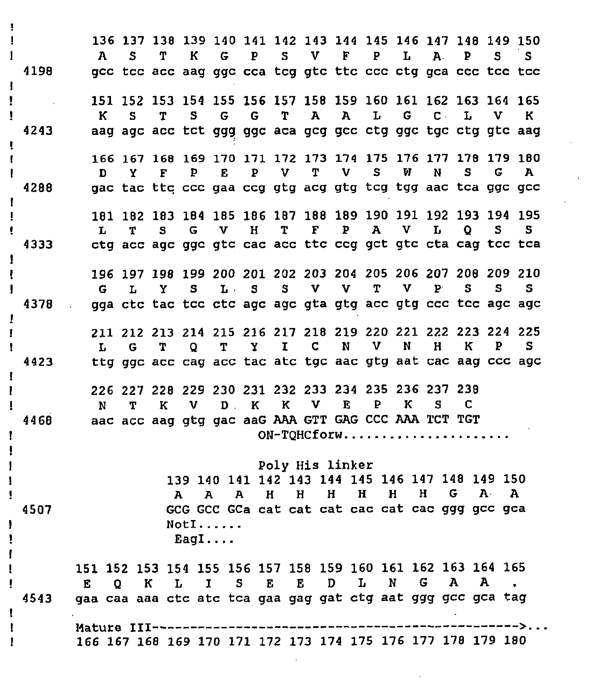

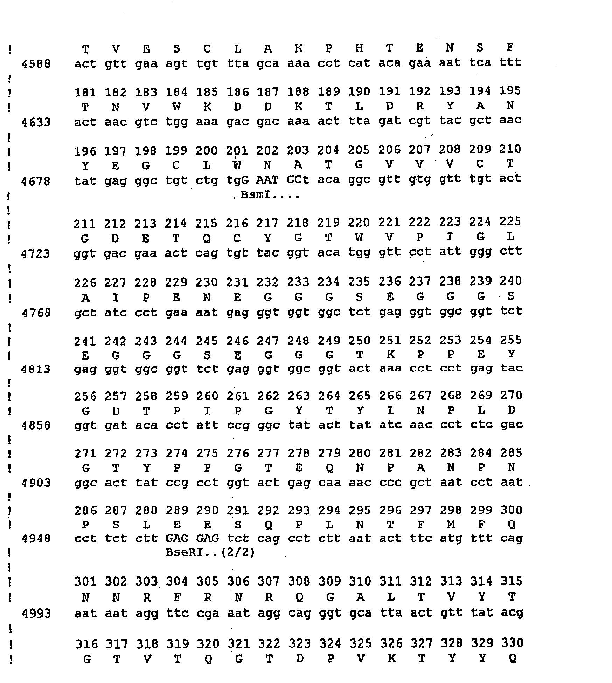

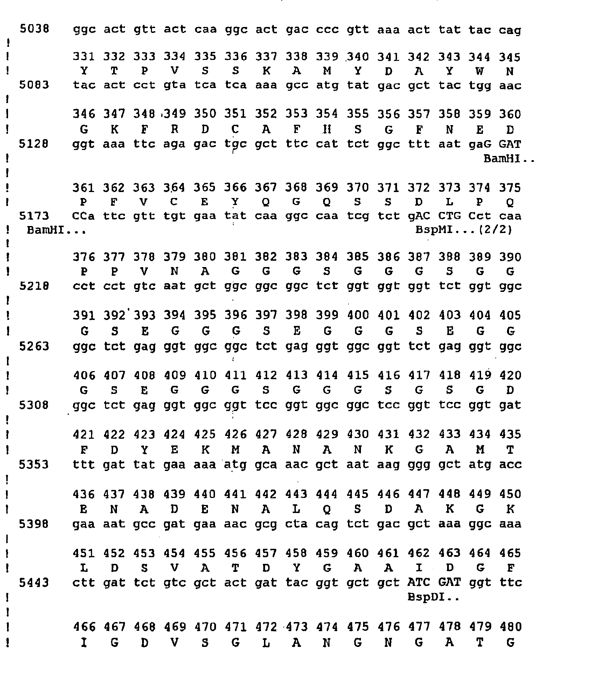

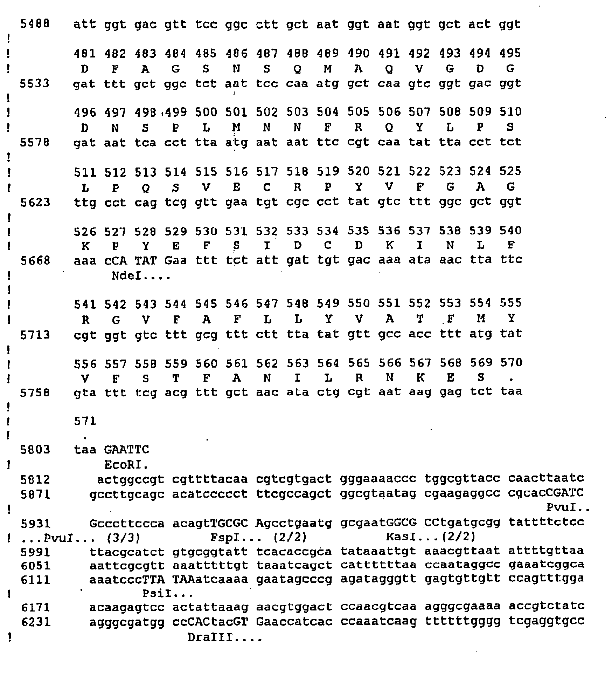

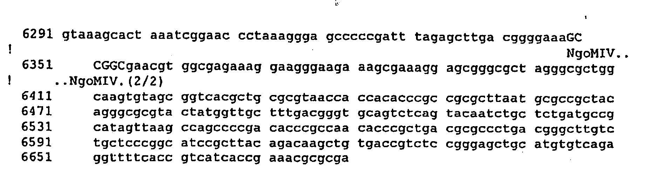

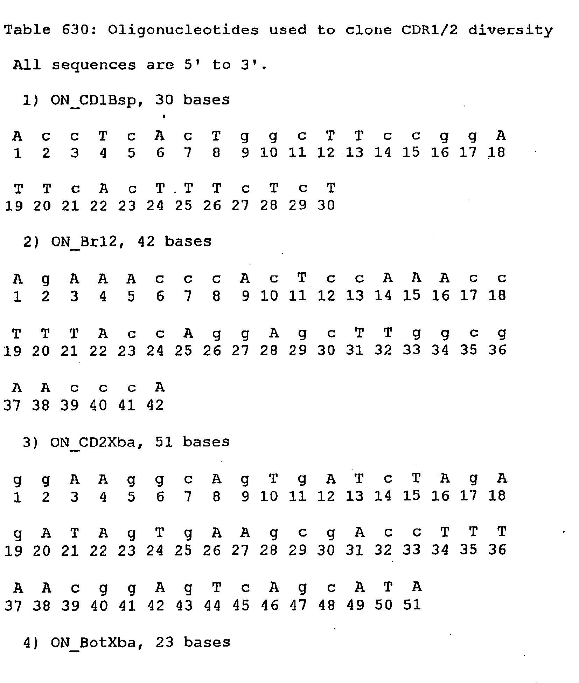

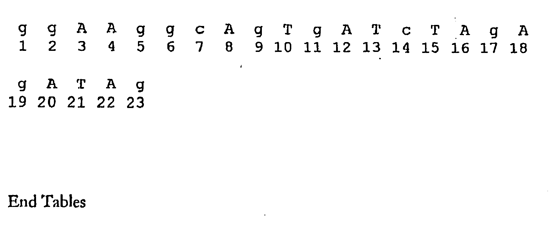

The soluble PCR product was run on a gel and showed a band of approximately 700 n, as expected (FIGs. 5 and 6). The DNA was cleaved with enzymes ApaLI and AscI, gel purified, and ligated to similarly cleaved vector pCES1. The presence of the correct size insert was checked by PCR in several clones as shown in FIG. 15.

Table 500 shows the DNA sequence of a kappa light chain captured by this procedure. Table 501 shows a second sequence captured by this procedure. The closest bridge sequence was complementary to the sequence 5'-agccacc-3', but the sequence captured reads 5'-Tgccacc-3', showing that some mismatch in the overlapped region is tolerated.

### Example 2: Construction of Synthetic CDR1 and CDR2 Diversity in V-3-23 VH Framework

A synthetic Complementary Determinant Region (CDR) 1 and 2 diversity was constructed in the 3-23 VH framework in a two step process: first, a vector containing the 3-23 VH framework was constructed, and then, a synthetic CDR 1 and 2 was assembled and cloned into this vector.

For construction of the V3-23 framework, 8 oligos and two PCR primers (long oligonucleotides: TOPFR1A, BOTFR1B, BOTFR2, BOTFR3, F06, BOTFR4, ON-vgC1, and ON-vgC2 and primers: SFPRMET and BOTPCRPRIM, shown in Table 600) that overlap were designed based on the Genebank sequence of V323 VH. The design incorporated at least one useful restriction site in each framework region, as shown in Table 600. In Table 600, the segments that were synthesized are shown as bold, the overlapping regions are underscored, and the PCR priming regions at each end are underscored. A mixture of these 8 oligos was combined at a final concentration of 2.5uM in a 20ul Polymerase Chain Reaction (PCR) reaction. The PCR mixture contained 200uM dNTPs, 2.5mM MgCl₂, 0.02U *Pfu Turbo*™ DNA Polymerase, 1U Qiagen HotStart Taq DNA Polymerase, and 1X Qiagen PCR buffer. The PCR program consisted of 10 cycles of 94°C for 30s, 55°C for 30s, and 72°C for 30s. The assembled V3-23 DNA sequence was then amplified, using 2.5ul of a 10-fold dilution from the initial PCR in 100ul PCR reaction. The PCR reaction contained 200uM dNTPs, 2.5mM MgCl₂, 0.02U *Pfu Turbo*™ DNA Polymerase, 1U Qiagen HotStart Taq DNA Polymerase, 1X Qiagen PCR Buffer and 2 outside primers (SFPRMET and BOTPCRPRIM) at a concentration of 1uM. The PCR program consisted of 23 cycles at 94°C for 30s, 55°C for 30s, and 72°C for 60s. The V3-23 VH DNA sequence was digested and cloned into pCES1 (phagemid vector) using the *Sfi*I and *Bst*EII restriction endonuclease sites (All restriction enzymes mentioned herein were supplied by New England BioLabs, Beverly, MA and used as per manufacturer's instructions).

Stuffer sequences (shown in Table 610 and Table 620) were introduced into pCES1 to replace CDR1/CDR2 sequences (900 bases between *Bsp*EI and *Xba*I RE sites) and CDR3 sequences (358 bases between *Afl*II and *Bst*EII), prior to cloning the CDR1/CDR2 diversity. The new vector is pCES5 and its sequence is given in Table 620. Having stuffers in place of the CDRs avoids the risk that a parental sequence would be over-represented in the library. The CDR1-2 stuffer contains restriction sites for *Bgl*II, *Bsu*36I, *Bcl*I, *Xcm*I, *Mlu*I, *Pvu*II, *Hpa*I, and *Hinc*II, the underscored sites being unique within the vector pCES5. The stuffer that replaces CDR3 contains the unique restriction endonuclease site *Rsr*II. The stuffer sequences are fragments from the penicillase gene of *E*. *coli*.

For the construction of the CDR1 and CDR2 diversity, 4 overlapping oligonucleotides (ON-vgC1, ON_Br12, ON_CD2Xba, and ON-vgC2, shown in Table 600 and Table 630) encoding CDR1/2, plus flanking regions, were designed. A mix of these 4 oligos was combined at a final concentration of 2.5uM in a 40ul PCR reaction. Two of the 4 oligos contained variegated sequences positioned at the CDR1 and the CDR2. The PCR mixture contained 200uM dNTPs, 2.5U Pwo DNA Polymerase (Roche), and 1X Pwo PCR buffer with 2mM MgSO₄. The PCR program consisted of 10 cycles at 94°C for 30s, 60°C for 30s, and 72°C for 60s. This assembled CDR1/2 DNA sequence was amplified, using 2.5ul of the mixture in 100ul PCR reaction. The PCR reaction contained 200uM dNTPs, 2.5U Pwo DNA Polymerase, 1X Pwo PCR Buffer with 2mM MgSO₄ and 2 outside primers at a concentration of 1uM. The PCR program consisted of 10 cycles at 94°C for 30s, 60°C for 30s, and 72°C for 60s. These variegated sequences were digested and cloned into the V3-23 framework in place of the CDR1/2 stuffer.

We obtained approximately 7 X 10⁷ independent transformants. Into this diversity, we can clone CDR3 diversity either from donor populations or from synthetic DNA.

It will be understood that the foregoing is only illustrative of the principles of this disclosure and that various modifications can be made by those skilled in the art without departing from the scope of and sprit of the disclosure.

Furthermore, the present disclosure relates to the following items:
1. A method for cleaving single-stranded nucleic acid sequences at a desired location, the method comprising the steps of:
   (i) contacting the nucleic acid with a single-stranded oligonucleotide, the oligonucleotide being functionally complementary to the nucleic acid in the region in which cleavage is desired and including a sequence that with its complement in the nucleic acid forms a restriction endonuclease recognition site that on restriction results in cleavage of the nucleic acid at the desired location; and
   (ii) cleaving the nucleic acid solely at the recognition site formed by the complementation of the nucleic acid and the oligonucleotide;
   the contacting and the cleaving steps being performed at a temperature sufficient to maintain the nucleic acid in substantially single-stranded form, the oligonucleotide being functionally complementary to the nucleic acid over a large enough region to allow the two strands to associate such that cleavage may occur at the chosen temperature and at the desired location, and the cleavage being carried out using a restriction endonuclease that is active at the chosen temperature.
2. A method for cleaving single-stranded nucleic acid sequences at a desired location, the method comprising the steps of:
   (i) contacting the nucleic acid with a partially double-stranded oligonucleotide, the single-stranded region of the oligonucleotide being functionally complementary to the nucleic acid in the region in which cleavage is desired, and the double-stranded region of the oligonucleotide having a Type II-S restriction endonuclease recognition site, whose cleavage site is located at a known distance from the recognition site; and
   (ii) cleaving the nucleic acid solely at the Type II-S cleavage site formed by the complementation of the nucleic acid and the single-stranded region of the oligonucleotide;
   the contacting and the cleaving steps being performed at a temperature sufficient to maintain the nucleic acid in substantially single-stranded form, the oligonucleotide being functionally complementary to the nucleic acid over a large enough region to allow the two strands to associate such that cleavage may occur at the chosen temperature and at the desired location, and the cleavage being carried out using a restriction endonuclease that is active at the chosen temperature.
3. In a method for displaying a member of a diverse family of peptides, polypeptides or proteins on the surface of a genetic package and collectively displaying at least a part of the diversity of the family, the improvement being characterized in that the displayed at least a part of peptide, polypeptide or protein is encoded at least in part by a nucleic acid that has been cleaved at a desired location by a method comprising the steps of:
   (i) contacting the nucleic acid with a single-stranded oligonucleotide, the oligonucleotide being functionally complementary to the nucleic acid in the region in which cleavage is desired and including a sequence that with its complement in the nucleic acid forms a restriction endonuclease recognition site that on restriction results in cleavage of the nucleic acid at the desired location; and
   (ii) cleaving the nucleic acid solely at the recognition site formed by the complementation of the nucleic acid and the oligonucleotide;
   the contacting and the cleaving steps being performed at a temperature sufficient to maintain the nucleic acid in substantially single-stranded form, the oligonucleotide being functionally complementary to the nucleic acid over a large enough region to allow the two strands to associate such that cleavage may occur at the chosen temperature and at the desired location, and the cleavage being carried out using a restriction endonuclease that is active at the chosen temperature.
4. In a method for displaying a member of a diverse family of peptides, polypeptides or proteins on the surface of a genetic package and collectively displaying at least a part of the diversity of the family, the improvement being characterized in that the displayed peptide, polypeptide or protein is encoded by a DNA sequence comprising a nucleic acid that has been cleaved at a desired location by
   (i) contacting the nucleic acid with a partially double-stranded oligonucleotide, the single-stranded region of the oligonucleotide being functionally complementary to the nucleic acid in the region in which cleavage is desired, and the double-stranded region of the oligonucleotide having a Type II-S restriction endonuclease recognition site, whose cleavage site is located at a known distance from the recognition site; and
   (ii) cleaving the nucleic acid solely at the Type II-S cleavage site formed by the complementation of the nucleic acid and the single-stranded region of the oligonucleotide,
   the contacting and the cleaving steps being performed at a temperature sufficient to maintain the nucleic acid in substantially single-stranded form, the oligonucleotide being functionally complementary to the nucleic acid over a large enough region to allow the two strands to associate such that cleavage may occur at the chosen temperature and at the desires location, and the cleavage being carried out using a restriction endonuclease that is active at the chosen temperature.
5. A method for displaying a member of a diverse family of peptides, polypeptides or proteins on the surface of a genetic package and collectively displaying at least a part of the diversity of the family, the method comprising the steps of:
   (i) preparing a collection of nucleic acids that code at least in part for members of the diverse family;
   (ii) rendering the nucleic acids single-stranded;
   (iii) cleaving the single-stranded nucleic acids at a desired location by a method comprising the steps of:
      (a) contacting the nucleic acid with a single-stranded oligonucleotide, the oligonucleotide being functionally complementary to the nucleic acid in the region in which cleavage is desired and including a sequence that with its complement in the nucleic acid forms a restriction endonuclease recognition site that on restriction results in cleavage of the nucleic acid at the desired location; and
      (b) cleaving the nucleic acid solely at the recognition site formed by the complementation of the nucleic acid and the oligonucleotide;
      the contacting and the cleaving steps being performed at a temperature sufficient to maintain the nucleic acid in substantially single-stranded form, the oligonucleotide being functionally complementary to the nucleic acid over a large enough region to allow the two strands to associate such that cleavage may occur at the chosen temperature and at the desired location, and the cleavage being carried out using a restriction endonuclease that is active at the chosen temperature; and
   (iv) displaying a member of the family of peptides, polypeptides or proteins coded, at least in part, by the cleaved nucleic acids on the surface of the genetic package and collectively displaying at least a portion of the diversity of the family.
6. A method for displaying a member of a diverse family of peptides, polypeptides or proteins on the surface of a genetic package and collectively displaying at least a portion of the diversity of the family, the method comprising the steps of:
   (i) preparing a collection of nucleic acids that code, at least in part, for members of the diverse family;
   (ii) rendering the nucleic acids single-stranded;
   (iii) cleaving the single-stranded nucleic acids at a desired location by a method comprising the steps of:
      (a) contacting the nucleic acid with a partially double-stranded oligonucleotide, the single-stranded region of the oligonucleotide being functionally complementary to the nucleic acid in the region in which cleavage is desired, and the double-stranded region of the oligonucleotide having a Type II-S restriction endonuclease recognition site, whose cleavage site is located at a known distance from the recognition site; and
      (b) cleaving the nucleic acid solely at the Type II-S cleavage site formed by the complementation of the nucleic acid and the single-stranded region of the oligonucleotide;
      the contacting and the cleaving steps being performed at a temperature sufficient to maintain the nucleic acid in substantially single-stranded form, the oligonucleotide being functionally complementary to the nucleic acid over a large enough region to allow the two strands to associate such that cleavage may occur at the chosen temperature and at the desired location, and the restriction being carried out using a cleavage endonuclease that is active at the chosen temperature; and
   (iv) displaying a member of the family of peptides, polypeptides or proteins coded, at least in part, by the cleaved nucleic acids on the surface of the genetic package and collectively displaying at least a portion of the diversity of the family.
7. A library comprising a collection of genetic packages that display a member of a diverse family of peptides, polypeptides or proteins and collectively display at least a portion of the diversity of the family, the library being produced using the methods of items 3, 4, 5 or 6.
8. A library comprising a collection of genetic packages that display a member of a diverse family of peptides, polypeptides or proteins and that collectively display at least a portion of the family, the displayed peptides, polypeptides or proteins being encoded by DNA sequences comprising at least in part sequences produced by cleaving single-stranded nucleic acid sequences at a desired location by a method comprising the steps of:
   (i) contacting the nucleic acid with a single-stranded oligonucleotide, the oligonucleotide being functionally complementary to the nucleic acid in the region in which cleavage is desired and including a sequence that with its complement in the nucleic acid forms a restriction endonuclease recognition site that on restriction results in cleavage of the nucleic acid at the desired location; and
   (ii) cleaving the nucleic acid solely at the recognition site formed by the complementation of the nucleic acid and the oligonucleotide;
   the contacting and the cleaving steps being performed at a temperature sufficient to maintain the nucleic acid in substantially single-stranded form, the oligonucleotide being functionally complementary to the nucleic acid over a large enough region to allow the two strands to associate such that cleavage may occur at the chosen temperature and at the desired location, and the cleavage being carried out using a restriction endonuclease that is active at the chosen temperature.
9. A library comprising a collection of genetic packages that display a member of a diverse family of peptides, polypeptides or proteins and that collectively display at least a portion of the diversity of the family of the displayed peptides, polypeptides or proteins being encoded by DNA sequences comprising at least in part sequences produced by cleaving single-stranded nucleic acid sequences at a desired location by a method comprising the steps of:
   (i) contacting the nucleic acid with a partially double-stranded oligonucleotide, the single-stranded region of the oligonucleotide being functionally complementary to the nucleic acid in the region in which cleavage is desired, and the double-stranded region of the oligonucleotide having a Type II S restriction endonuclease recognition site, whose cleavage site is located at a known distance from the recognition site where the cleavage of the nucleic acid is desired; and
   (ii) cleaving the nucleic acid solely at the Type II-S cleavage site formed by the complementation of the nucleic acid and the single-stranded region of the oligonucleotide;
   the contacting and the cleaving steps being performed at a temperature sufficient to maintain the nucleic acid in substantially single-stranded form, the oligonucleotide being functionally complementary to the nucleic acid over a large enough region to allow the two strands to associate such that cleavage may occur at the chosen temperature and at the desired location, and the cleavage being carried out using a restriction endonuclease that is active at the chosen temperature.
10. The methods according to any one of items 1 to 9, wherein the nucleic acids encode at least a portion of an immunoglobulin.
11. The methods according to item 10, wherein the immunoglobulin comprises a Fab or single chain Fv.
12. The methods according to item 10 or 11, wherein the immunoglobin comprises at least portion or a heavy chain.
13. The methods according to item 12, wherein at least a portion of the heavy chain is human.
14. The methods according to item 10 or 11, wherein the immunoglobulin comprises at least a portion of FR1.
15. The methods according to item 14, wherein at least a portion of the FR1 is human.
16. The methods according to item 10 or 11, wherein the immunoglobulin comprises at least a portion of a light chain.
17. The methods according to item 16, wherein at least a portion of the light chain is human.
18. The methods according to any one of items 1 to 9, wherein the nucleic acid sequences are at least in part derived from patients suffering from at least one autoimmune disease and/or cancer.
19. The methods according to item 18, wherein the autoimmune disease is selected from the group comprising lupus, erythematosus, systemic sclerosis, rheumatoid arthritis, antiphosolipid syndrome or vasculitis.
20. The methods according to item 18, wherein the nucleic acids are at least in part isolated from the group comprising peripheral blood cells, bone marrow cells spleen cells or lymph node cells.
21. The methods according to item 5 or 6 further comprising an nucleic acid amplification step between steps (i) and (ii), between steps (ii) and (iii) or between steps (iii) and (iv).
22. The methods according to item 21, wherein the amplification step uses geneRACE™.
23. The methods according to any one of items 1 to 9, wherein the temperature is between 45°C and 75°C.
24. The methods according to item 23, wherein the temperature is between 50°C and 60°C.
25. The methods according to item 24, wherein the temperature is between 55°C and 60°C.
26. The methods according to item 1, 3, 5 or 8, wherein the length of the single-stranded oligonucleotide is between 17 and 30 bases.
27. The methods according to item 26, wherein the length of the single-stranded oligonucleotide is between 18 and 24 bases.
28. The methods according to item 1, 3, 5 or 8, wherein the restriction endonuclease is selected from the group comprising *Mae*III, *Tsp*45I, *Hph*I, *Bsa*JI, *Alu*I, *Blp*I*, DdeI, Bgl*II, *Msl*I*, Bsi*EI, *Eae*I, *Eag*I, *Hae*III, *Bst*4CI, *Hpy*CH4III, *Hin*fI, *Mly*I, *Ple*I, *Mnl*I, *Hpy*CH4V, *Bsm*AI, *Bpm*I, *Xmn*I, or *Sac*I.
29. The methods according to item 28, wherein the restriction endonuclease is selected from the group comprising *Bst*4CI, *Taa*I, *HpyCH4*III, *Blp*I, *Hpy*CH4V or *Msl*I.
30. The methods according to item 2, 4, 6 or 9, wherein the length of the single-stranded region of the partially double-stranded oligonucleotide is between 14 and 22 bases.
31. The methods according to item 30, wherein the length of the single-stranded region of the partially double-stranded oligonucleotide is between 14 and 17 bases.
32. The methods according to item 31, wherein the length of the single-stranded region of the oligonucleotide is between 18 and 20 bases.
33. The methods according to item 2, 4, 6 or 9, wherein the length of the double-stranded region of the partially double-stranded oligonucleotide is between 10 and 14 base pairs formed by a stem and its palindrome.
34. The methods according to item 33 wherein, the partially double-stranded oligonucleotide comprises a loop of 3 to 8 bases between the stem and the palindrome.
35. The methods according to item 2, 4, 6 or 9, wherein the Type II-S restriction endonuclease is selected from the group comprising AarICAC, AceIII, Bbr7I, BbvI, BbvII, Bce83I, BceAI, BcefI, BciVI, BfiI, BinI, BscAI, BseRI, BsmFI, BspMI, EciI, Eco57I, FauI, FokI, GsuI, HgaI, HphI, MboII, MlyI, MmeI, MnlI, PleI, RleAI, SfaNI, SspD5I, Sth132I, StsI, TaqII, Tth111II, or UbaPI.
36. The methods according to item 35, wherein the Type II-S restriction endonuclease is *Fok*I.
37. A method for preparing single-stranded nucleic acids for cloning into an vector, the method comprising the steps of:
   (i) contacting a single-stranded nucleic acid sequence that has been cleaved with a restriction endonuclease with a partially double-stranded oligonucleotide, the single-stranded region of the oligonucleotide being functionally complementary to the nucleic acid in the region that remains after cleavage, the double-stranded region of the oligonucleotide including any sequences necessary to return the sequences that remain after cleavage into proper and original reading frame for expression and containing a restriction endonuclease recognition site 5' of those sequences; and
   (ii) cleaving the partially double-stranded oligonucleotide sequence solely at the restriction endonuclease recognition site contained within the double-stranded region of the partially double-stranded oligonucleotide.
38. The method according to item 37, wherein the length of the single-stranded portion of the partially double-stranded oligonucleotide is between 2 and 15 bases.
39. The method according to item 38, wherein the length of the single-stranded portion of the partially double-stranded oligonucleotide is between 7 and 10 bases.
40. The method according to item 37, wherein the length of the double-stranded portion of the partially double-stranded oligonucleotide is between 12 and 100 base pairs.
41. The method according to item 40, wherein the length of the double-stranded portion of the partially double-stranded oligonucleotide is between 20 and 100 base pairs.

## Claims

1. A library comprising a collection of genetic packages that display a member of a diverse family of peptides, polypeptides or proteins and that collectively display at least a portion of the family, the displayed peptides, polypeptides or proteins being encoded by DNA sequences comprising sequences encoding
(a) a heavy chain CDR1 having an amino acid sequence according to the formula S-<1>-Y-<1>-M-<1>, wherein <1> is_selected from the group consisting of A, D, E, F, G, H, I, K, L, M, N, P, Q, R, S, T, V, W, and Y;
(b) a heavy chain CDR2 having an amino acid sequence according to the formula <2>-I-<2>-<3>-S-G-G-<1>-T-<1>-Y-A-D-S-V-K-G, wherein <2> is selected from the group consisting of Y, R, W, V, G, and S, <3>is selected from the group consisting of P and S, <1> is_selected from the group consisting of A, D, E, F, G, H, I, K, L, M, N, P, Q, R, S, T, V, W, and Y, and
(c) sequences encoding heavy chain CDR3 diversity,
wherein said DNA sequences further comprise sequences encoding the framework regions of VH 3-23.

2. The library according to claim 1, wherein said genetic packages are M13 phage.

3. The library according to claim 2, wherein the M13 phage comprise an antibiotic resistance gene.

4. The library according to claim 2, wherein the M13 phage comprise restriction sites that allow introduction and excision of a member of the diverse family of peptides, polypeptides or proteins.

5. The library according to claim 1, wherein said DNA sequences are in a phage vector.

6. The library according to claim 5, wherein the phage vector comprises an antibiotic resistance gene.

7. The library according to claim 6, wherein the antibiotic resistance gene is an ampicillin resistance gene.

8. The library according to claim 5, wherein the phage vector comprises a regulatable promoter.

9. The library according to claim 1, wherein said DNA sequences are in a phagemid vector.

10. The library according to any one of claims 1 to 9, wherein said displayed peptides, polypeptides, or proteins are displayed through a short linker to the final portion of M13 gene III.

11. The library according to any one of claims 1 to 9, wherein said displayed peptides, polypeptides, or proteins are displayed through a short linker to the full length mature gene III protein.

12. The library according to any one of claims 1 to 9, wherein said displayed peptides, polypeptides, or proteins are displayed through a short linker to the full length mature gene VIII protein.

13. The library of claim 1, wherein the heavy chain CDR3 diversity comprises natural diversity.

14. The library of claim 13, wherein the natural diversity is captured from an autoimmune patient.

15. The library of claim 1, wherein the heavy chain CDR3 diversity comprises synthetic diversity.

## Patentansprüche

1. Bibliothek, die eine Sammlung genetischer Pakete umfasst, die ein Mitglied einer diversen Familie der Peptide, Polypeptide oder Proteine präsentieren und die kollektiv mindestens einen Abschnitt der Familie präsentieren, wobei die präsentierten Peptide, Polypeptide oder Proteine durch DNA-Sequenzen kodiert sind, die Sequenzen umfassen, die für
(a) eine schwere Kette CDR1 mit einer Aminosäuresequenz gemäß der Formel S-<1>-Y-<1>-M-<1>, wobei <1> ausgewählt ist aus der Gruppe bestehend aus A, D, E, F, G, H, I, K, L, M, N, P, Q, R, S, T, V, W und Y;
(b) eine schwere Kette CDR2 mit einer Aminosäuresequenz gemäß der Formel <2>-I-<2>-<3>-S-G-G-<1>-T-<1>-Y-A-D-S-V-K-G, wobei <2> ausgewählt ist aus der Gruppe bestehend aus Y, R, W, V, G und S, <3> ausgewählt ist aus der Gruppe bestehend aus P und S, <1> ausgewählt ist aus der Gruppe bestehend aus A, D, E, F, G, H, I, K, L, M, N, P, Q, R, S, T, V, W und Y, und
(c) Sequenzen kodieren, die für Diversität der schweren Kette CDR3 kodieren,
wobei die DNA-Sequenzen ferner Sequenzen umfassen, die für die Framework-Regionen von VH 3-23 kodieren.

2. Bibliothek nach Anspruch 1, wobei die genetischen Pakete M13-Phage sind.

3. Bibliothek nach Anspruch 2, wobei der M13-Phage ein Antibiotikaresistenzgen umfasst.

4. Bibliothek nach Anspruch 2, wobei der M13-Phage Restriktionsstellen umfasst, die die Einführung und Exzision eines Mitglieds der diversen Familie der Peptide, Polypeptide oder Proteine ermöglichen.

5. Bibliothek nach Anspruch 1, wobei die DNA-Sequenzen in einem Phagenvektor sind.

6. Bibliothek nach Anspruch 5, wobei der Phagenvektor ein Antibiotikaresistenzgen umfasst.

7. Bibliothek nach Anspruch 6, wobei das Antibiotikaresistenzgen ein Ampicillinresistenzgen ist.

8. Bibliothek nach Anspruch 5, wobei der Phagenvektor einen regulierbaren Promoter umfasst.

9. Bibliothek nach Anspruch 1, wobei die DNA-Sequenzen in einem Phagemidvektor sind.

10. Bibliothek nach einem der Ansprüche 1 bis 9, wobei die präsentierten Peptide, Polypeptide oder Proteine über einen kurzen Linker dem finalen Abschnitt von M13 Gen III präsentiert werden.

11. Bibliothek nach einem der Ansprüche 1 bis 9, wobei die präsentierten Peptide, Polypeptide oder Proteine über einen kurzen Linker dem in voller Länge vorliegenden, reifen Gen III-Protein präsentiert werden.

12. Bibliothek nach einem der Ansprüche 1 bis 9, wobei die präsentierten Peptide, Polypeptide oder Proteine über einen kurzen Linker dem in voller Länge vorliegenden, reifen Gen VIII Protein präsentiert werden.

13. Bibliothek nach Anspruch 1, wobei die Diversität der schweren Kette CDR3 natürliche Diversität umfasst.

14. Bibliothek nach Anspruch 13, wobei die natürliche Diversität von einem Autoimmunpatienten erfasst wurde.

15. Bibliothek nach Anspruch 1, wobei die Diversität der schweren Kette CDR3 synthetische Diversität umfasst.

## Revendications

1. Banque comprenant une collection d'ensembles génétiques affichant un membre d'une famille diverse de peptides, de polypeptiques ou de protéines et qui, collectivement, affichent au moins une partie de la famille, les peptides, polypeptides ou protéines affichées codées par les séquences ADN contenant des séquences codant pour
(a) une chaîne lourde CDR1 ayant une séquence acide aminé selon la formule S-< 1>-Y-<1>-M-<1>, <1> étant sélectionné dans le groupe composé de A, D, E,F,G,H,I,K,L,M,N,P,QR,S,T,V,W et Y;
(b) une chaîne lourde CDR2 ayant une séquence acide aminé selon la formule <2>-I-<2>-<3>-S-G-G-<1>-T-<1>-Y-A-D-S-V-K-G, <2> étant sélectionné dans le groupe composé de Y, R, W, V, G, and S, -<3> étant sélectionné dans le groupe composé de P et S, <1> étant sélectionné dans le groupe composé de A, D, E, F, G, H,I,K,L,M,N,P,QR,S,T,V,W et Y, et
(c) les séquences codant pour la diversité de la chaîne lourde CDR3, lesdites séquences d'ADN comprenant également les séquences codant pour les régions-cadres de VH 3-23.

2. Banque selon la revendication 1, les ensembles génétiques étant le phage M13.

3. Banque selon la revendication 2, le phage M13 comprenant un gène de résistance à un antibiotique.

4. Banque selon la revendication 2, le phage M13 comprenant des sites de restriction permettant l'introduction et l'excision d'un membre de la famille diverse de peptides, polypeptides ou protéines.

5. Banque selon la revendication 1, les séquences d'ADN étant dans un vecteur phagique.

6. Banque selon la revendication 5, le vecteur phagique comprenant un gène de résistance à un antibiotique.

7. Banque selon la revendication 6, le gène de résistance à l'antibiotique étant le gène de résistance à l'ampicilline.

8. Banque selon la revendication 5, le vecteur phagique comprenant un promoteur régulable.

9. Banque selon la revendication 1, les séquences d'ADN étant dans un vecteur phagemide.

10. Banque selon l'une quelconque des revendications 1 à 9, lesdits peptides, polypeptides ou protéines étant affichées à travers un court lieur à la partie finale du gène III de M13.

11. Banque selon l'une quelconque des revendications 1 à 9, lesdits peptides, polypeptides ou protéines étant affichées à travers un court lieur à la protéine de pleine longueur du gène III mature.

12. Banque selon l'une quelconque des revendications 1 à 9, lesdits peptides, polypeptides ou protéines étant affichées à travers un court lieur à la protéine de pleine longueur du gène VIII mature.

13. Banque de la revendication 1, la diversité de la chaîne lourde CDR3 comprenant une diversité naturelle.

14. Banque de la revendication 13, la diversité naturelle étant obtenue d'un patient auto-immun.

15. Banque de la revendication 1, la diversité de la chaîne lourde CDR3 comprenant une diversité synthétique.
